(19) Europäisches Patentamt
European Patent Office
Office européen des brevets

(11) EP 4 585 272 A2

(12) **EUROPEAN PATENT APPLICATION**

(43) Date of publication:
**16.07.2025 Bulletin 2025/29**

(21) Application number: **25164405.0**

(22) Date of filing: **30.03.2017**

(51) International Patent Classification (IPC):
***A61P 43/00*** *(2006.01)*

(52) Cooperative Patent Classification (CPC):
**C12N 15/111; A61P 1/16; A61P 43/00; C12N 9/22; C12N 15/88;** C12N 2310/20; C12N 2320/32

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.03.2016 US 201662315602 P**
**16.08.2016 US 201662375776 P**
**12.12.2016 US 201662433228 P**
**07.03.2017 US 201762468300 P**

(62) Document number(s) of the earlier application(s) in accordance with Art. 76 EPC:
**17719387.7 / 3 436 077**

(71) Applicant: **Intellia Therapeutics, Inc.**
**Cambridge, MA 02139 (US)**

(72) Inventors:
- **Morrissey, David V.**
  **Winchester, MA 01890 (US)**
- **Patel, Mihir Chandrakant**
  **Perkasie, PA 18944 (US)**
- **Finn, Jonathan D.**
  **Melrose, MA 02176 (US)**
- **Smith, Amy Madison Rhoden**
  **Watertown, MA 02472 (US)**
- **Shaw, Lucinda J.**
  **Lexington, MA 02421 (US)**
- **Dombrowski, Christian**
  **Boston, MA 02130 (US)**
- **Shah, Ruchi Rudraprasad**
  **Brookline, MA 02445 (US)**

(74) Representative: **Schlich**
**9 St Catherine's Road**
**Littlehampton**
**West Sussex BN17 5HS (GB)**

Remarks:
•The complete document including Reference Table(s) and the Sequence Listing(s) can be downloaded from the EPO website
•This application was filed on 18-03-2025 as a divisional application to the application mentioned under INID code 62.
•Claims filed after the date of filing of the application/ after the date of receipt of the divisional application (Rule 68(4) EPC).

# (54) LIPID NANOPARTICLE FORMULATIONS FOR CRISPR/CAS COMPONENTS

(57) The invention provides lipid nanoparticle-based compositions and methods useful for delivery of CRISPR/Cas gene editing components.

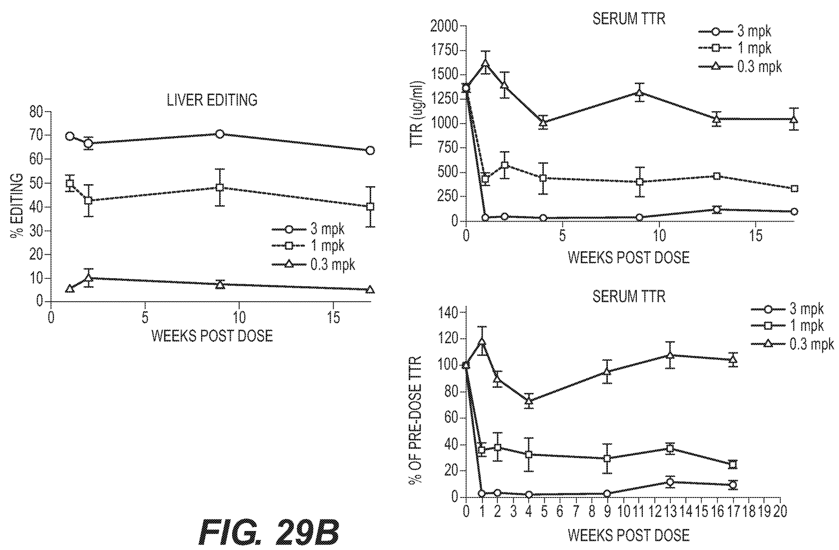


*FIG. 29B*

EP 4 585 272 A2

## Description

The present application claims the benefit of priority to U.S. Provisional Patent Application No. 62/315,602 filed March 30, 2016, U.S. Provisional Patent Application No. 62/375,776 filed August 16, 2016, U.S. Provisional Patent Application No. 62/433,228 filed December 12, 2016, and U.S. Provisional Patent Application No. 62/468,300 filed March 7, 2017; the entire contents of each are incorporated herein by reference.

The delivery of biologically active agents (including therapeutically relevant compounds) to subjects is often hindered by difficulties in the agents reaching the target cell or tissue. In particular, the trafficking of many biologically active agents into living cells can be restricted by the membrane systems of the cells.

One class of biologically active agents that is particularly difficult to deliver to cells are biologics including proteins, nucleic acid-based drugs, and derivatives thereof. Certain nucleic acids and proteins are stable for only a limited duration in cells or plasma, and sometimes are highly charged, which can complicate delivery across cell membranes. Compositions that can stabilize and deliver such agents into cells are therefore of particular interest. Lipid carriers, biodegradable polymers and various conjugate systems can be used to improve delivery of these biologically active agents to cells.

A number of components and compositions for editing genes in cells *in vivo* now exist, providing tremendous potential for treating genetic, viral, bacterial, autoimmune, cancer, aging-related, and inflammatory diseases. Several of these editing technologies take advantage of cellular mechanisms for repairing double-stranded breaks ("DSB") created by enzymes such as meganucleases, clustered regularly interspaced short palindromic repeats (CRISPR) associated ("Cas") nucleases, zinc finger nucleases ("ZFN"), and transcription activator-like effector nucleases ("TALEN"). When DSBs are made in a cell, the cell may repair the break by one of several processes. One such process involves non-homologous end joining ("NHEJ") of the cleaved ends of DNA. During NHEJ, nucleotides may be added or removed by the cell, resulting in a sequence altered from the cleaved sequence. In other circumstances, cells repair DSBs by homology-directed repair ("HDR") or homologous recombination ("HR") mechanisms, where an endogenous or exogenous template with homology to each end of a DSB, for example, is used to direct repair of the break. Several of these editing technologies take advantage of cellular mechanisms for repairing single-stranded breaks or double-stranded breaks ("DSB").

CRISPR/Cas gene editing systems are active as ribonucleoprotein complexes in a cell. Compositions for delivery of the protein and nucleic acid components of CRISPR/Cas to a cell, such as a cell in a patient, are needed.

We herein provide lipid nanoparticle-based compositions useful for delivery of CRISPR/Cas gene editing components.

In some embodiments, we herein provide a method of producing a genetically engineered liver cell, comprising contacting a cell with lipid nanoparticles (LNPs) comprising: a Class 2 Cas nuclease mRNA; a guide RNA nucleic acid; a CCD lipid; a helper lipid; a neutral lipid; and a stealth lipid. Lipid nanoparticles (LNPs) comprising a Class 2 Cas nuclease mRNA, a guide RNA nucleic acid, a CCD lipid, a helper lipid, a neutral lipid, and a stealth lipid are also provided.

Additional embodiments provide a method of gene editing, comprising delivering a Class 2 Cas nuclease mRNA and a guide RNA nucleic acid to a liver cell, wherein the Class 2 Cas mRNA and the guide RNA nucleic acid are formulated as at least one LNP composition comprising: a CCD lipid; a helper lipid; a neutral lipid; and a stealth lipid. Further embodiments provide a method of administering a CRISPR-Cas complex to a liver cell, comprising contacting a cell with LNPs comprising: a Class 2 Cas nuclease mRNA; a guide RNA nucleic acid; a CCD lipid; a helper lipid; a neutral lipid; and a stealth lipid.

In certain embodiments, a method of altering expression of a gene in a liver cell, comprising administering to the subject a therapeutically effective amount of a Class 2 Cas nuclease mRNA and a guide RNA nucleic acid as one or more LNP formulations, wherein at least one LNP formulation comprises: a guide RNA nucleic acid or a Class 2 Cas nuclease mRNA; a CCD lipid; a helper lipid; a neutral lipid; and a stealth lipid is provided.

In some embodiments, the method of producing a genetically engineered liver cell comprises contacting a cell with lipid nanoparticles (LNPs) comprising: a Class 2 Cas nuclease mRNA; a guide RNA nucleic acid that is or encodes a single-guide RNA (sgRNA); a CCD lipid; a helper lipid; a neutral lipid; and a stealth lipid.

In certain aspects, the Class 2 Cas nuclease mRNA is formulated in a first LNP composition and the guide RNA nucleic acid is formulation in a second LNP composition. In other aspects, the Class 2 Cas nuclease mRNA and the guide RNA nucleic acid are formulated together in a LNP composition.

## BRIEF DESCRIPTION OF DRAWINGS

Fig. 1 shows the expression of GFP after delivery of various LNP formulations to mouse hepatocyte cells (Hepa1.6) at amounts of 100 ng and 500 ng eGFP mRNA delivered per well.

Fig. 2 shows gLUC expression in mice after administration of various LNP formulations at varying doses, resulting in a dose-dependent response.

Fig. 3A shows the editing efficiency of targeting Factor VII in mice after administration of various LNP formulations.

Fig. 3B shows the editing efficiency of targeting TTR in mice after administration of various LNP formulations.

Fig. 4A shows the editing efficiency of targeting TTR in mice after delivery of various LNP formulations, according to various dosing regiments, where the gRNA and Cas9 mRNA are formulated separately.

Fig. 4B shows the editing efficiency of targeting TTR in mice after delivery of an LNP formulation where the gRNA and Cas9 mRNA are formulated separately.

Fig. 5 shows the editing efficiency of targeting Factor VII or TTR in cells after administration of various LNP formulations where the gRNA and Cas9 mRNA are formulated separately.

Fig. 6 shows the editing efficiency of targeting Factor VII or TTR in mice after administration of various LNP formulations where the gRNA and Cas9 mRNA are formulated separately.

Fig. 7 shows the editing efficiency in cells after administration of various LNP formulations where the gRNA and Cas9 mRNA are formulated together and delivered at various concentrations.

Fig. 8A shows the editing efficiency of targeting TTR in mice after administration of various LNP formulations.

Fig. 8B shows the editing efficiency of targeting Factor VII in mice after administration of various LNP formulations.

Fig. 9 shows PCR amplification of excision-site DNA collected from animals that were administered various LNP formulations.

Fig. 10 shows serum TTR levels of mice that were administered various LNP formulations where the gRNA and Cas9 mRNA are formulated together.

Fig. 11 shows relative Factor VII activity in mice after animals were administered various LNP formulations where the gRNA and Cas9 mRNA are formulated together.

Fig. 12A shows the editing efficiency of targeting TTR in mice after administering LNP-169 at various doses, resulting in a dose-dependent response.

Fig. 12B shows serum TTR levels in mice, on various days, after administering LNP-169 at various doses, resulting in a dose-dependent response.

Fig. 13A shows the editing efficiency of targeting TTR in mice after administration of various LNP formulations where the ratio of Cas9 mRNA to sgRNA was varied.

Fig. 13B shows the serum TTR levels in mice, on two separate days, after administration of various LNP formulations where the ratio of Cas9 mRNA to sgRNA was varied.

Fig. 14A shows the editing efficiency of targeting TTR in mice after administration of LNP-169 in one or two doses.

Fig. 14B shows the serum TTR levels in mice nine days after administration of LNP-169 in one or two doses.

Fig. 15 shows the editing efficiency in the spleen of targeting TTR in mice after administration of various LNP formulations.

Fig. 16 shows the editing efficiency of targeting TTR in mice after administration of various LNP formulations.

Fig. 17 shows the editing efficiency of targeting TTR in primary mouse hepatocytes after delivery of LNP-169 to cells, in various concentrations, in the presence of mouse serum.

Fig. 18 shows an increase in LNP-binding by ApoE as the amount of ApoE present increases.

Fig. 19 shows the editing efficiency of various LNP formulations wherein the guide RNA was delivered as a DNA expression cassette.

Fig. 20 shows that editing efficiency correlates between primary hepatocyte cultures and *in vivo* liver cells in mice.

Fig. 21 shows the distinctive repair spectrum of editing in the Neuro 2A *in vitro* cell line versus primary mouse hepatocytes.

Fig. 22 shows the similar repair spectrum of editing in primary mouse hepatocytes versus *in vivo* mouse liver cells.

Fig. 23 shows, as a function of time, the plasma concentration of Cas9 mRNA and guide RNA.

Fig. 24 shows, as a function of time, the concentration of Cas9 mRNA and guide RNA in liver tissue.

Fig. 25 shows, as a function of time, the concentration of Cas9 mRNA and guide RNA in spleen tissue.

Fig. 26A shows, as a function of time, the relative concentrations of Cas9 mRNA and guide RNA in plasma and in tissue.

Fig. 26B shows, as a function of time, the concentration of Lipid A in plasma and in tissue.

Fig. 27 shows, as a function of time after administration of an LNP, the change in plasma cytokine levels.

Fig. 28 shows mouse serum TTR levels over time after administration of an LNP.

Fig. 29A shows TTR editing over time in mice after administration of an LNP.

Fig. 29B shows TTR editing and serum TTR levels over time in mice after administration of an LNP.

Fig. 30 shows mouse serum cytokine levels after administration of LNPs containing different mRNA preparations.

Fig. 31 shows mouse serum TTR concentration levels after administration of LNPs containing different mRNA preparations.

Fig. 32 shows TTR editing levels over time in mice after administration of LNPs containing different mRNA preparations.

Fig. 33 shows mouse serum TTR concentration levels after administration of LNPs stored at -80° C or 4° C.

Fig. 34 shows mouse TTR editing levels after administration of LNPs stored at -80° C or 4° C.
Fig. 35 shows mouse serum concentration levels after administration of various formulations.
Fig. 36 shows mouse liver TTR editing levels after administration of various formulations.

## DETAILED DESCRIPTION

The present disclosure provides embodiments of lipid nanoparticle (LNP) compositions of CRISPR/Cas components (the "cargo") for delivery to a cell and methods for their use. The LNP may contain (i) a CCD lipid, (ii) a neutral lipid, (iii) a helper lipid, and (iv) a stealth lipid. In certain embodiments, the cargo includes an mRNA encoding a Cas nuclease, such as Cas9, and a guide RNA or a nucleic acid encoding a guide RNA.

## CRISPR/Cas Cargo

The CRISPR/Cas cargo delivered via LNP formulation includes an mRNA molecule encoding a Cas nuclease, allowing for expression of the Cas nuclease in a cell. The cargo further contains one or more guide RNAs or nucleic acids encoding guide RNAs. The cargo may further include a template nucleic acid for repair or recombination.

### Cas Nuclease

One component of the disclosed formulations is an mRNA encoding a Cas nuclease, also called a Cas nuclease mRNA. The mRNA may be modified for improved stability and/or immunogenicity properties. The modifications may be made to one or more nucleosides within the mRNA. Examples of chemical modifications to mRNA nucleobases include pseudouridine, 1-methyl-pseudouridine, and 5-methyl-cytidine. Additional known modifications to improve stability, expression, and immunogenicity are contemplated. The mRNA encoding a Cas nuclease may be codon optimized for expression in a particular cell type, such as a eukaryotic cell, a mammalian cell, or more specifically, a human cell. In some embodiments, the mRNA encodes a human codon optimized Cas9 nuclease or human codon optimized Cpf nuclease as the Cas nuclease. In some embodiments, the mRNA is purified. In some embodiments, the mRNA is purified using a precipation method (*e.g.,* LiCl precipitation, alcohol precipitation, or an equivalent method, *e.g.,* as described herein). In some embodiments, the mRNA is purified using a chromatography-based method, such as an HPLC-based method or an equivalent method (*e.g.,* as described herein). In some embodiments, the mRNA is purified using both a precipitation method (*e.g.,* LiCl precipitation) and an HPLC-based method.

In addition to the coding sequence for a Cas nuclease, the mRNA may comprise a 3' or 5' untranslated region (UTR). In some embodiments, the 3' or 5' UTR can be derived from a human gene sequence. Exemplary 3' and 5' UTRs include $\alpha$- and $\beta$-globin, albumin, HSD17B4, and eukaryotic elongation factor 1$\alpha$. In addition, viral-derived 5' and 3' UTRs can also be used and include orthopoxvirus and cytomegalovirus UTR sequences. In certain embodiments, the mRNA includes a 5' cap, such as m7G(5')ppp(5')N. In addition, this cap may be a cap-0 where nucleotide N does not contain 2'OMe, or cap-1 where nucleotide N contains 2'OMe, or cap-2 where nucleotides N and N+1 contain 2'OMe. This cap may also be of the structure $m_2^{7,3'\text{-}O}G(5')N$ as incorporated by the anti-reverse-cap analog (ARCA), and may also include similar cap-0, cap-1, and cap-2, etc., structures. In some embodiments, the 5' cap may regulate nuclear export; prevent degradation by exonucleases; promote translation; and promote 5' proximal intron excision. Stabilizing elements for caps include phosphorothioate linkages, boranophosphate modifications, and methylene bridges. In addition, caps may also contain a non-nucleic acid entity that acts as the binding element for eukaryotic translation initiation factor 4E, eIF4E. In certain embodiments, the mRNA includes a poly(A) tail. This tail may be about 40 to about 300 nucleotides in length. In some embodiments, the tail may be about 40 to about 100 nucleotides in length. In some embodiments, the tail may be about 100 to about 300 nucleotides in length. In some embodiments, the tail may be about 100 to about 300 nucleotides in length. In some embodiments, the tail may be about 50 to about 200 nucleotides in length. In some embodiments, the tail may be about 50 to about 250 nucleotides in length. In certain embodiments, the tail may be about 100, 150, or 200 nucleotides in length. The poly(A) tail may contain modifications to prevent exonuclease degradation including phosphorotioate linkages and modifications to the nucleobase. In addition, the poly(A) tail may contain a 3' "cap" which could include modified or non-natural nucleobases or other synthetic moieties.

The mRNAs described herein may comprise at least one element that is capable of modifying the intracellular half-life of the RNA. In some embodiments, the half-life of the RNA may be increased. In some embodiments, the half-life of the RNA may be decreased. In some embodiments, the element may be capable of increasing the stability of the RNA. In some embodiments, the element may be capable of decreasing the stability of the RNA. In some embodiments the element may promote RNA decay. In some embodiments, the element may activate translation. In some embodiments, the element may be within the 3' UTR of the RNA. For example, the element may be an mRNA decay signal. In some embodiments, the element may include a polyadenylation signal (PA). In some embodiments, the PA may be in the 3' UTR of the RNA. In some embodiments, the RNA may comprise no PA such that it is subject to quicker degradation in the cell

after transcription. In some embodiments, the element may include at least one AU-rich element (ARE). In some embodiments, the element does not include an ARE. The AREs may be bound by ARE binding proteins (ARE-BPs) in a manner that is dependent upon tissue type, cell type, timing, cellular localization, and environment. In some embodiments, the ARE may comprise 50 to 150 nucleotides in length. In some embodiments, the ARE may comprise at least one copy of the sequence AUUUA. In some embodiments, at least one ARE may be added to the 3' UTR of the RNA. In some embodiments, the element may be a Woodchuck Hepatitis Virus (WHV) Posttranscriptional Regulatory Element (WPRE), which creates a tertiary structure to enhance expression from the transcript. In some embodiments, the WPRE may be added to the 3' UTR of the RNA. In some embodiments, the element may be selected from other RNA sequence motifs that are present in fast- or slow-decaying transcripts. In some embodiments, each element can be used alone. In some embodiments, an element can be used in combination with one or more elements.

In some embodiments, the nuclease encoded by the delivered mRNA may include a Cas protein from a CRISPR/Cas system. The Cas protein may comprise at least one domain that interacts with a guide RNA ("gRNA"). Additionally, the Cas protein may be directed to a target sequence by a guide RNA. The guide RNA interacts with the Cas protein as well as the target sequence such that, it directs binding to the target sequence. In some embodiments, the guide RNA provides the specificity for the targeted cleavage, and the Cas protein may be universal and paired with different guide RNAs to cleave different target sequences. In certain embodiments, the Cas protein may cleave single or double-stranded DNA. In certain embodiments, the Cas protein may cleave RNA. In certain embodiments, the Cas protein may nick RNA. In some embodiments, the Cas protein comprises at least one DNA binding domain and at least one nuclease domain. In some embodiments, the nuclease domain may be heterologous to the DNA binding domain. In certain embodiments, the Cas protein may be modified to reduce or eliminate nuclease activity. The Cas protein may be used to bind to and modulate the expression or activity of a DNA sequence.

In some embodiments, the CRISPR/Cas system may comprise Class 1 or Class 2 system components, including ribonucleic acid protein complexes. See, *e.g.*, Makarova et al., Nat Rev Microbiol, 13(11): 722-36 (2015); Shmakov et al., Molecular Cell, 60:385-397 (2015). Class 2 CRISPR/Cas systems have single protein effectors. Cas proteins of Types II, V, and VI may be single-protein, RNA-guided endonucleases, herein called "Class 2 Cas nucleases." Class 2 Cas nucleases include, for example, Cas9, Cpfl, C2c1, C2c2, and C2c3 proteins. Cpfl protein, Zetsche et al., Cell, 163: 1-13 (2015), is homologous to Cas9, and contains a RuvC-like nuclease domain. Cpfl sequences of Zetsche are incorporated by reference in their entirety. See, *e.g.,* Zetsche, Tables S1 and S3.

In some embodiments, the Cas protein may be from a Type-II CRISPR/Cas system, *i.e.,* a Cas9 protein from a CRISPR/Cas9 system, or a Type-V CRISPR/Cas system, *e.g.*, a Cpf1 protein. In some embodiments, the Cas protein may be from a Class 2 CRISPR/Cas system, *i.e.,* a single-protein Cas nuclease such as a Cas9 protein or a Cpfl protein. The Class 2 Cas nuclease families of proteins are enzymes with DNA endonuclease activity, and they can be directed to cleave a desired nucleic acid target by designing an appropriate guide RNA, as described further herein.

A Class 2 CRISPR/Cas system component may be from a Type-IIA, Type-IIB, Type-IIC, Type V, or Type VI system. Cas9 and its orthologs are encompassed. Non-limiting exemplary species that the Cas9 protein or other components may be from include *Streptococcus pyogenes, Streptococcus thermophilus, Streptococcus sp., Staphylococcus aureus, Listeria innocua, Lactobacillus gasseri, Francisella novicida, Wolinella succinogenes, Sutterella wadsworthensis, Gamma proteobacterium, Neisseria meningitidis, Campylobacter jejuni, Pasteurella multocida, Fibrobacter succinogene, Rhodospirillum rubrum, Nocardiopsis dassonvillei, Streptomyces pristinaespiralis, Streptomyces viridochromogenes, Streptomyces viridochromogenes, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Lactobacillus buchneri, Treponema denticola, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionium, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Streptococcus pasteurianus, Neisseria cinerea, Campylobacter lari, Parvibaculum lavamentivorans, Corynebacterium diphtheria,* or *Acaryochloris marina.* In some embodiments, the Cas9 protein may be from *Streptococcus pyogenes.* In some embodiments, the Cas9 protein may be from *Streptococcus thermophilus.* In some embodiments, the Cas9 protein may be from *Staphylococcus aureus.* In further embodiments, a Cpfl protein may be from *Francisella tularensis, Lachnospiraceae bacterium, Butyrivibrio proteoclasticus, Peregrinibacteria bacterium, Parcubacteria bacterium, Smithella, Acidaminococcus, Candidatus Methanoplasma termitum, Eubacterium eligens, Moraxella bovoculi, Leptospira inadai, Porphyromonas crevioricanis, Prevotella disiens,* or *Porphyromonas macacae.* In certain embodiments the Cpfl protein may be from *Acidaminococcus* or *Lachnospiraceae.*

In some embodiments, a Class 2 Cas nuclease may comprise at least one RuvC-like nuclease domain, such as a

Cas9 or Cpfl protein. In some embodiments, a Class 2 Cas nuclease may comprise more than one nuclease domain. For example, a Class 2 Cas nuclease may comprise at least one RuvC-like nuclease domain and at least one HNH-like nuclease domain. In some embodiments, the Class 2 Cas nuclease may be capable of introducing a DSB in the target sequence. In some embodiments, the Class 2 Cas nuclease may be modified to contain only one functional nuclease domain. For example, the Class 2 Cas nuclease may be modified such that one of the nuclease domains is mutated or fully or partially deleted to reduce its nucleic acid cleavage activity. In some embodiments, the Class 2 Cas nuclease may be modified to contain no functional RuvC-like nuclease domain. In other embodiments, the Class 2 Cas nuclease, *e.g.* a Cas9 protein, may be modified to contain no functional HNH-like nuclease domain. In some embodiments in which only one nuclease domain is functional, the Class 2 Cas nuclease may be a nickase that is capable of introducing a single-stranded break (a "nick") into the target sequence. In some embodiments, a conserved amino acid within a nuclease domain of the Class 2 Cas nuclease is substituted to reduce or alter a nuclease activity. In some embodiments, the nuclease domain mutation may inactivate DNA cleavage activity. In some embodiments, the nuclease domain mutation may inactivate one nuclease domain of the Class 2 Cas nuclease, resulting in a nickase. In some embodiments, the nickase may comprise an amino acid substitution in the RuvC-like nuclease domain. Exemplary amino acid substitutions in the RuvC-like nuclease domain include D10A (based on the *S. pyogenes* Cas9 protein, see, *e.g.*, UniProtKB - Q99ZW2 (CASS STRP1)). Further exemplary amino acid substitutions include D917A, E1006A, and D1255A (based on the *Francisella novicida* U112 Cpf1 (FnCpf1) sequence (UniProtKB - A0Q7Q2 (CPF1_FRATN)). In some embodiments, the nickase may comprise an amino acid substitution in the HNH-like nuclease domain. Exemplary amino acid substitutions in the HNH-like nuclease domain include E762A, H840A, N863A, H983A, and D986A (based on the *S. pyogenes* Cas9 protein). Exemplary mutations alter conserved catalytic residues in the nuclease domain and alter nucleolytic activity of the domain. In some embodiments, the nuclease system described herein may comprise a nickase and a pair of guide RNAs that are complementary to the sense and antisense strands of the target sequence, respectively. The guide RNAs may direct the nickase to target and introduce a DSB by generating a nick on opposite strands of the target sequence (*i.e.,* double nicking). A chimeric Class 2 Cas nuclease may also be used, where one domain or region of the protein is replaced by a portion of a different protein. For example, a nuclease domain may be replaced with a domain from a different nuclease such as Fok1. In certain embodiments, the Class 2 Cas nuclease may be modified to reduce or eliminate nuclease activity. It may be used to bind to and modulate the expression or activity of a DNA sequence.

In alternative embodiments, the Cas protein may be a component of the Cascade complex of a Type-I CRISPR/Cas system. For example, the Cas protein may be a Cas3 protein. In some embodiments, the Cas protein may be from a Type-II CRISPR/Cas system. In some embodiments, the Cas protein may be from a Type-III CRISPR/Cas system. In some embodiments, the Cas protein may be from a Type-IV CRISPR/Cas system. In some embodiments, the Cas protein may be from a Type-V CRISPR/Cas system. In some embodiments, the Cas protein may be from a Type-VI CRISPR/Cas system. In some embodiments, the Cas protein may have an RNA cleavage activity.

In some embodiments, the nuclease may be fused with at least one heterologous protein domain. At least one protein domain may be located at the N-terminus, the C-terminus, or in an internal location of the nuclease. In some embodiments, two or more heterologous protein domains are at one or more locations on the nuclease.

In some embodiments, the protein domain may facilitate transport of the nuclease into the nucleus of a cell. For example, the protein domain may be a nuclear localization signal (NLS). In some embodiments, the nuclease may be fused with 1-10 NLS(s). In some embodiments, the nuclease may be fused with 1-5 NLS(s). In some embodiments, the nuclease may be fused with one NLS. Where one NLS is used, the NLS may be on the N-terminus or the C-terminus of the nuclease. In other embodiments, the nuclease may be fused with more than one NLS. In some embodiments, the nuclease may be fused with 2, 3, 4, or 5 NLSs. In some embodiments, the nuclease may be fused with two NLSs. In certain circumstances, the two NLSs may be the same (*e.g.*, two SV40 NLSs) or different. In some embodiments, the nuclease is fused to two SV40 NLS sequences at the carboxy terminus. In some embodiments, the nuclease may be fused with two NLSs, one on the N-terminus and one on the C-terminus. In some embodiments, the nuclease may be fused with 3 NLSs. In some embodiments, the nuclease may be fused with no NLS. In some embodiments, the NLS may be a monopartite sequence, such as, *e.g.,* the SV40 NLS, PKKKRKV or PKKKRRV. In some embodiments, the NLS may be a bipartite sequence, such as the NLS of nucleoplasmin, KRPAATKKAGQAKKKK. In a specific embodiment, a single PKKKRKV NLS may be at the C-terminus of the nuclease.

In some embodiments, the protein domain may be capable of modifying the intracellular half-life of the nuclease. In some embodiments, the half-life of the nuclease may be increased. In some embodiments, the half-life of the nuclease may be reduced. In some embodiments, the protein domain may be capable of increasing the stability of the nuclease. In some embodiments, the protein domain may be capable of reducing the stability of the nuclease. In some embodiments, the protein domain may act as a signal peptide for protein degradation. In some embodiments, the protein degradation may be mediated by proteolytic enzymes, such as, for example, proteasomes, lysosomal proteases, or calpain proteases. In some embodiments, the protein domain may comprise a PEST sequence. In some embodiments, the nuclease may be modified by addition of ubiquitin or a polyubiquitin chain. In some embodiments, the ubiquitin may be a ubiquitin-like protein (UBL). Non-limiting examples of ubiquitin-like proteins include small ubiquitin-like modifier (SUMO), ubiquitin cross-reactive protein (UCRP, also known as interferon-stimulated gene-15 (ISG15)), ubiquitin-related modifier-1 (URM1), neuronal-precursor-cell-expressed developmentally downregulated protein-8 (NEDD8, also called Rub1 in *S. cerevisiae*), human leukocyte antigen F-associated (FAT10), autophagy-8 (ATG8) and -12 (ATG12), Fau ubiquitin-like protein (FUB1), membrane-anchored UBL (MUB), ubiquitin fold-modifier-1 (UFM1), and ubiquitin-like protein-5 (UBL5).

In some embodiments, the protein domain may be a marker domain. Non-limiting examples of marker domains include fluorescent proteins, purification tags, epitope tags, and reporter gene sequences. In some embodiments, the marker domain may be a fluorescent protein. Non-limiting examples of suitable fluorescent proteins include green fluorescent proteins (*e.g.*, GFP, GFP-2, tagGFP, turboGFP, sfGFP, EGFP, Emerald, Azami Green, Monomeric Azami Green, CopGFP, AceGFP, ZsGreen1 ), yellow fluorescent proteins (*e.g.*, YFP, EYFP, Citrine, Venus, YPet, PhiYFP, ZsYellow1), blue fluorescent proteins (*e.g.*, EBFP, EBFP2, Azurite, mKalamal, GFPuv, Sapphire, T-sapphire,), cyan fluorescent proteins (*e.g.*, ECFP, Cerulean, CyPet, AmCyan1, Midoriishi-Cyan), red fluorescent proteins (*e.g.*, mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (mOrange, mKO, Kusabira-Orange, Monomeric Kusabira-Orange, mTangerine, tdTomato) or any other suitable fluorescent protein. In other embodiments, the marker domain may be a purification tag and/or an epitope tag. Non-limiting exemplary tags include glutathione-S-transferase (GST), chitin binding protein (CBP), maltose binding protein (MBP), thioredoxin (TRX), poly(NANP), tandem affinity purification (TAP) tag, myc, AcV5, AU1, AU5, E, ECS, E2, FLAG, HA, nus, Softag 1, Softag 3, Strep, SBP, Glu-Glu, HSV, KT3, S, S1, T7, V5, VSV-G, 6xHis, 8xHis, biotin carboxyl carrier protein (BCCP), poly-His, and calmodulin. Non-limiting exemplary reporter genes include glutathione-S-transferase (GST), horseradish peroxidase (HRP), chloramphenicol acetyltransferase (CAT), beta-galactosidase, beta-glucuronidase, luciferase, or fluorescent proteins.

In additional embodiments, the protein domain may target the nuclease to a specific organelle, cell type, tissue, or organ. In some embodiments, the protein domain may target the nuclease to mitochondria.

In further embodiments, the protein domain may be an effector domain. When the nuclease is directed to its target sequence, *e.g.*, when a Cas9 protein is directed to a target sequence by a guide RNA, the effector domain may modify or affect the target sequence. In some embodiments, the effector domain may be chosen from a nucleic acid binding domain, a nuclease domain, an epigenetic modification domain, a transcriptional activation domain, a methylation domain, or a transcriptional repressor domain. In certain embodiments, the DNA modification domain is a methylation domain, such as a demethylation or methyltransferase domain. In certain embodiments, the effector domain is a DNA modification domain, such as a base-editing domain. In particular embodiments, the DNA modification domain is a nucleic acid editing domain that introduces a specific modification into the DNA, such as a deaminase domain. *See* WO 2015/089406; US 2016/0304846. The nucleic acid editing domains, deaminase domains, and Cas9 variants described in WO 2015/089406 and US 2016/0304846 are hereby incorporated by reference.

## Guide RNA

In some embodiments of the present disclosure, the cargo for the LNP formulation includes at least one guide RNA. The guide RNA may guide the Class 2 Cas nuclease to a target sequence on a target nucleic acid molecule, where the guide RNA hybridizes with and the Cas nuclease cleaves or modulates the target sequence. In some embodiments, a guide RNA binds with and provides specificity of cleavage by a Class 2 nuclease. In some embodiments, the guide RNA and the Cas protein may form a ribonucleoprotein (RNP), *e.g.,* a CRISPR/Cas complex. In some embodiments, the CRISPR complex may be a Type-II CRISPR/Cas9 complex. In some embodiments, the CRISPR/Cas complex may be a Type-V CRISPR/Cas complex, such as a Cpf1/guide RNA complex. In some embodiments, the Cas nuclease may be a single-protein Cas nuclease, *e.g.* a Cas9 protein or a Cpf1 protein. In some embodiments, the guide RNA targets cleavage by a Cas9 protein.

A guide RNA for a CRISPR/Cas9 nuclease system comprises a CRISPR RNA (crRNA) and a tracr RNA (tracr). In some embodiments, the crRNA may comprise a targeting sequence that is complementary to and hybridizes with the target sequence on the target nucleic acid molecule. The crRNA may also comprise a flagpole that is complementary to and hybridizes with a portion of the tracrRNA. In some embodiments, the crRNA may parallel the structure of a naturally occurring crRNA transcribed from a CRISPR locus of a bacteria, where the targeting sequence acts as the spacer of the CRISPR/Cas9 system, and the flagpole corresponds to a portion of a repeat sequence flanking the spacers on the CRISPR locus.

The guide RNA may target any sequence of interest via the targeting sequence of the crRNA. In some embodiments, the degree of complementarity between the targeting sequence of the guide RNA and the target sequence on the target nucleic acid molecule may be about 60%, 65%, 70%, 75%, 80%, 85%, 90%, 95%, 97%, 98%, 99%, or 100%. In some embodiments, the targeting sequence of the guide RNA and the target sequence on the target nucleic acid molecule may be 100% complementary. In other embodiments, the targeting sequence of the guide RNA and the target sequence on the target nucleic acid molecule may contain at least one mismatch. For example, the targeting sequence of

the guide RNA and the target sequence on the target nucleic acid molecule may contain 1, 2, 3, 4, 5, 6, 7, 8, 9, or 10 mismatches. In some embodiments, the targeting sequence of the guide RNA and the target sequence on the target nucleic acid molecule may contain 1-6 mismatches. In some embodiments, the targeting sequence of the guide RNA and the target sequence on the target nucleic acid molecule may contain 5 or 6 mismatches.

The length of the targeting sequence may depend on the CRISPR/Cas system and components used. For example, different Cas proteins from different bacterial species have varying optimal targeting sequence lengths. Accordingly, the targeting sequence may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length. In some embodiments, the targeting sequence may comprise 18-24 nucleotides in length. In some embodiments, the targeting sequence may comprise 19-21 nucleotides in length. In some embodiments, the targeting sequence may comprise 20 nucleotides in length.

The flagpole may comprise any sequence with sufficient complementarity with a tracr RNA to promote the formation of a functional CRISPR/Cas complex. In some embodiments, the flagpole may comprise all or a portion of the sequence (also called a "tag" or "handle") of a naturally-occurring crRNA that is complementary to the tracr RNA in the same CRISPR/Cas system. In some embodiments, the flagpole may comprise all or a portion of a repeat sequence from a naturally-occurring CRISPR/Cas system. In some embodiments, the flagpole may comprise a truncated or modified tag or handle sequence. In some embodiments, the degree of complementarity between the tracr RNA and the portion of the flagpole that hybridizes with the tracr RNA along the length of the shorter of the two sequences may be about 40%, 50%, 60%, 70%, 80%, or higher, but lower than 100%. In some embodiments, the tracr RNA and the portion of the flagpole that hybridizes with the tracr RNA are not 100% complementary along the length of the shorter of the two sequences because of the presence of one or more bulge structures on the tracr and/or wobble base pairing between the tracr and the flagpole. The length of the flagpole may depend on the CRISPR/Cas system or the tracr RNA used. For example, the flagpole may comprise 10-50 nucleotides, or more than 50 nucleotides in length. In some embodiments, the flagpole may comprise 15-40 nucleotides in length. In other embodiments, the flagpole may comprise 20-30 nucleotides in length. In yet other embodiments, the flagpole may comprise 22 nucleotides in length. When a dual guide RNA is used, for example, the length of the flagpole may have no upper limit.

In some embodiments, the tracr RNA may comprise all or a portion of a wild-type tracr RNA sequence from a naturally-occurring CRISPR/Cas system. In some embodiments, the tracr RNA may comprise a truncated or modified variant of the wild-type tracr RNA. The length of the tracr RNA may depend on the CRISPR/Cas system used. In some embodiments, the tracr RNA may comprise 5, 6, 7, 8, 9, 10, 11, 12, 13, 14, 15, 16, 17, 18, 19, 20, 25, 30, 40, 50, 60, 70, 80, 90, 100, or more than 100 nucleotides in length. In certain embodiments, the tracr is at least 26 nucleotides in length. In additional embodiments, the tracr is at least 40 nucleotides in length. In some embodiments, the tracr RNA may comprise certain secondary structures, such as, *e.g.,* one or more hairpins or stem-loop structures, or one or more bulge structures.

In some embodiments, the guide RNA may comprise two RNA molecules and is referred to herein as a "dual guide RNA" or "dgRNA". In some embodiments, the dgRNA may comprise a first RNA molecule comprising a crRNA, and a second RNA molecule comprising a tracr RNA. The first and second RNA molecules may form a RNA duplex via the base pairing between the flagpole on the crRNA and the tracr RNA.

In additional embodiments, the guide RNA may comprise a single RNA molecule and is referred to herein as a "single guide RNA" or "sgRNA". In some embodiments, the sgRNA may comprise a crRNA covalently linked to a tracr RNA. In some embodiments, the crRNA and the tracr RNA may be covalently linked via a linker. In some embodiments, the single-molecule guide RNA may comprise a stem-loop structure via the base pairing between the flagpole on the crRNA and the tracr RNA. In some embodiments, the sgRNA is a "Cas9 sgRNA" capable of mediating RNA-guided DNA cleavage by a Cas9 protein. In some embodiments, the sgRNA is a "Cpfl sgRNA" capable of mediating RNA-guided DNA cleavage by a Cpfl protein. In certain embodiments, the guide RNA comprises a crRNA and tracr RNA sufficient for forming an active complex with a Cas9 protein and mediating RNA-guided DNA cleavage. In certain embodiments, the guide RNA comprises a crRNA sufficient for forming an active complex with a Cpfl protein and mediating RNA-guided DNA cleavage. *See* Zetsche 2015.

Certain embodiments of the invention also provide nucleic acids, *e.g.,* expression cassettes, encoding the guide RNA described herein. A "guide RNA nucleic acid" is used herein to refer to a guide RNA (*e.g.* an sgRNA or a dgRNA) and a guide RNA expression cassette, which is a nucleic acid that encodes one or more guide RNAs.

In some embodiments, the nucleic acid may be a DNA molecule. In some embodiments, the nucleic acid may comprise a nucleotide sequence encoding a crRNA. In some embodiments, the nucleotide sequence encoding the crRNA comprises a targeting sequence flanked by all or a portion of a repeat sequence from a naturally-occurring CRISPR/Cas system. In some embodiments, the nucleic acid may comprise a nucleotide sequence encoding a tracr RNA. In some embodiments, the crRNA and the tracr RNA may be encoded by two separate nucleic acids. In other embodiments, the crRNA and the tracr RNA may be encoded by a single nucleic acid. In some embodiments, the crRNA and the tracr RNA may be encoded by opposite strands of a single nucleic acid. In other embodiments, the crRNA and the tracr RNA may be encoded by the same strand of a single nucleic acid. In some embodiments, the expression cassette encodes an sgRNA. In some embodiments, the expression cassette encodes a Cas9 nuclease sgRNA. In come embodiments, the expression

cassette encodes a Cpfl nuclease sgRNA.

The nucleotide sequence encoding the guide RNA may be operably linked to at least one transcriptional or regulatory control sequence, such as a promoter, a 3' UTR, or a 5' UTR. In one example, the promoter may be a tRNA promoter, *e.g.*, $tRNA^{Lys3}$, or a tRNA chimera. *See* Mefferd et al., RNA. 2015 21:1683-9; Scherer et al., Nucleic Acids Res. 2007 35: 2620-2628. In certain embodiments, the promoter may be recognized by RNA polymerase III (Pol III). Non-limiting examples of Pol III promoters also include U6 and H1 promoters. In some embodiments, the nucleotide sequence encoding the guide RNA may be operably linked to a mouse or human U6 promoter. In some embodiments, the expression cassette is a modified nucleic acid. In certain embodiments, the expression cassette includes a modified nucleoside or nucleotide. In some embodiments, the expression cassette includes a 5' end modification, for example a modified nucleoside or nucleotide to stabilize and prevent integration of the expression cassette. In some embodiments, the expression cassette comprises a double-stranded DNA having a 5' end modification on each strand. In certain embodiments, the expression cassette includes an inverted dideoxy-T or an inverted abasic nucleoside or nucleotide as the 5' end modification. In some embodiments, the expression cassette includes a label such as biotin, desthiobioten-TEG, digoxigenin, and fluorescent markers, including, for example, FAM, ROX, TAMRA, and AlexaFluor.

In certain embodiments, more than one guide RNA can be used with a CRISPR/Cas nuclease system. Each guide RNA may contain a different targeting sequence, such that the CRISPR/Cas system cleaves more than one target sequence. In some embodiments, one or more guide RNAs may have the same or differing properties such as activity or stability within a CRISPR/Cas complex. Where more than one guide RNA is used, each guide RNA can be encoded on the same or on different expression cassettes. The promoters used to drive expression of the more than one guide RNA may be the same or different.

**Chemically Modified RNAs**

Modified nucleosides or nucleotides can be present in a guide RNA or mRNA. A guide RNA or Cas nuclease encoding mRNA comprising one or more modified nucleosides or nucleotides is called a "modified" RNA to describe the presence of one or more non-naturally and/or naturally occurring components or configurations that are used instead of or in addition to the canonical A, G, C, and U residues. In some embodiments, a modified RNA is synthesized with a non-canonical nucleoside or nucleotide, here called "modified." Modified nucleosides and nucleotides can include one or more of: (i) alteration, *e.g.*, replacement, of one or both of the non-linking phosphate oxygens and/or of one or more of the linking phosphate oxygens in the phosphodiester backbone linkage (an exemplary backbone modification); (ii) alteration, *e.g.,* replacement, of a constituent of the ribose sugar, *e.g.,* of the 2' hydroxyl on the ribose sugar (an exemplary sugar modification); (iii) wholesale replacement of the phosphate moiety with "dephospho" linkers (an exemplary backbone modification); (iv) modification or replacement of a naturally occurring nucleobase, including with a non-canonical nucleobase (an exemplary base modification); (v) replacement or modification of the ribose-phosphate backbone (an exemplary backbone modification); (vi) modification of the 3' end or 5' end of the oligonucleotide, *e.g.*, removal, modification or replacement of a terminal phosphate group or conjugation of a moiety, cap or linker (such 3' or 5' cap modifications may comprise a sugar and/or backbone modification); and (vii) modification or replacement of the sugar (an exemplary sugar modification).

The modifications listed above can be combined to provide modified RNAs comprising nucleosides and nucleotides (collectively "residues") that can have two, three, four, or more modifications. For example, a modified residue can have a modified sugar and a modified nucleobase. In some embodiments, every base of a gRNA is modified, *e.g.,* all bases have a modified phosphate group, such as a phosphorothioate group. In certain embodiments, all, or substantially all, of the phosphate groups of an sgRNA molecule are replaced with phosphorothioate groups. In some embodiments, modified RNAs comprise at least one modified residue at or near the 5' end of the RNA. In some embodiments, modified RNAs comprise at least one modified residue at or near the 3' end of the RNA.

In certain embodiments, modified residues can be incorporated into a guide RNA. In certain embodiments, modified residues can be incorporated into an mRNA. In some embodiments, the guide RNA comprises one, two, three or more modified residues. In some embodiments, the guide RNA comprises one, two, three or more modified residues at each of the 5' and the 3' ends of the guide RNA. In some embodiments the mRNA comprises 5, 10, 15, 50, 100, 200, 300, 400, 500, 600, 700, 800, 900, 1000, or more modified residues. In some embodiments, at least 5% *(e.g.,* at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%) of the positions in a modified guide RNA or mRNA are modified nucleosides or nucleotides.

Unmodified nucleic acids can be prone to degradation by, *e.g.,* cellular nucleases. For example, nucleases can hydrolyze nucleic acid phosphodiester bonds. Accordingly, in one aspect the guide RNAs described herein can contain one or more modified nucleosides or nucleotides, *e.g.*, to introduce stability toward nucleases. In certain embodiments, the mRNAs described herein can contain one or more modified nucleosides or nucleotides, *e.g.*, to introduce stability toward

nucleases. In some embodiments, the modified RNA molecules described herein can exhibit a reduced innate immune response when introduced into a population of cells, both *in vivo* and *ex vivo.* The term "innate immune response" includes a cellular response to exogenous nucleic acids, including single stranded nucleic acids, which involves the induction of cytokine expression and release, particularly the interferons, and cell death.

In some embodiments of a backbone modification, the phosphate group of a modified residue can be modified by replacing one or more of the oxygens with a different substituent. Further, the modified residue, *e.g.*, modified residue present in a modified nucleic acid, can include the wholesale replacement of an unmodified phosphate moiety with a modified phosphate group as described herein. In some embodiments, the backbone modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. The phosphorous atom in an unmodified phosphate group is achiral. However, replacement of one of the non-bridging oxygens with one of the above atoms or groups of atoms can render the phosphorous atom chiral. The stereogenic phosphorous atom can possess either the "R" configuration (herein Rp) or the "S" configuration (herein Sp). The backbone can also be modified by replacement of a bridging oxygen, (*i.e.,* the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

The phosphate group can be replaced by non-phosphorus containing connectors in certain backbone modifications. In some embodiments, the charged phosphate group can be replaced by a neutral moiety. Examples of moieties which can replace the phosphate group can include, without limitation, *e.g.*, methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

Scaffolds that can mimic nucleic acids can also be constructed wherein the phosphate linker and ribose sugar are replaced by nuclease resistant nucleoside or nucleotide surrogates. Such modifications may comprise backbone and sugar modifications. In some embodiments, the nucleobases can be tethered by a surrogate backbone. Examples can include, without limitation, the morpholino, cyclobutyl, pyrrolidine and peptide nucleic acid (PNA) nucleoside surrogates.

The modified nucleosides and modified nucleotides can include one or more modifications to the sugar group, *i.e.* at sugar modification. For example, the 2' hydroxyl group (OH) can be modified, *e.g.* replaced with a number of different "oxy" or "deoxy" substituents. In some embodiments, modifications to the 2' hydroxyl group can enhance the stability of the nucleic acid since the hydroxyl can no longer be deprotonated to form a 2'-alkoxide ion.

Examples of 2' hydroxyl group modifications can include alkoxy or aryloxy (OR, wherein "R" can be, *e.g.,* alkyl, cycloalkyl, aryl, aralkyl, heteroaryl or a sugar); polyethyleneglycols (PEG), $O(CH_2CH_2O)_nCH_2CH_2OR$ wherein R can be, *e.g.,* H or optionally substituted alkyl, and n can be an integer from 0 to 20 (*e.g.,* from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20). In some embodiments, the 2' hydroxyl group modification can be 2'-O-Me. In some embodiments, the 2' hydroxyl group modification can be a 2'-fluoro modification, which replaces the 2' hydroxyl group with a fluoride. In some embodiments, the 2' hydroxyl group modification can include "locked" nucleic acids (LNA) in which the 2' hydroxyl can be connected, *e.g.*, by a $C_1$-$_6$ alkylene or $C_{1-6}$ heteroalkylene bridge, to the 4' carbon of the same ribose sugar, where exemplary bridges can include methylene, propylene, ether, or amino bridges; O-amino (wherein amino can be, *e.g.,* $NH_2$; alkylamino, dialkylamino, heterocyclyl, arylamino, diarylamino, heteroarylamino, or diheteroarylamino, ethylenediamine, or polyamino) and aminoalkoxy, $O(CH_2)_n$-amino, (wherein amino can be, *e.g.,* $NH_2$; alkylamino, dialkylamino, heterocyclyl, arylamino, diarylamino, heteroarylamino, or diheteroarylamino, ethylenediamine, or polyamino). In some embodiments, the 2' hydroxyl group modification can included "unlocked" nucleic acids (UNA) in which the ribose ring lacks the C2'-C3' bond. In some embodiments, the 2' hydroxyl group modification can include the methoxyethyl group (MOE), ($OCH_2CH_2OCH_3$, *e.g.,* a PEG derivative).

"Deoxy" 2' modifications can include hydrogen (*i.e.* deoxyribose sugars, *e.g.,* at the overhang portions of partially dsRNA); halo (*e.g.*, bromo, chloro, fluoro, or iodo); amino (wherein amino can be, *e.g.,* -$NH_2$, alkylamino, dialkylamino, heterocyclyl, arylamino, diarylamino, heteroarylamino, diheteroarylamino, or amino acid); $NH(CH_2CH_2NH)_nCH2CH_2$-amino (wherein amino can be, *e.g.,* as described herein), - NHC(O)R (wherein R can be, *e.g.,* alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.,* an amino as described herein.

The sugar modification can comprise a sugar group which may also contain one or more carbons that possess the opposite stereochemical configuration than that of the corresponding carbon in ribose. Thus, a modified nucleic acid can include nucleotides containing *e.g.*, arabinose, as the sugar. The modified nucleic acids can also include abasic sugars. These abasic sugars can also be further modified at one or more of the constituent sugar atoms. The modified nucleic acids can also include one or more sugars that are in the L form, *e.g.* L- nucleosides.

The modified nucleosides and modified nucleotides described herein, which can be incorporated into a modified nucleic acid, can include a modified base, also called a nucleobase. Examples of nucleobases include, but are not limited to, adenine (A), guanine (G), cytosine (C), and uracil (U). These nucleobases can be modified or wholly replaced to provide modified residues that can be incorporated into modified nucleic acids. The nucleobase of the nucleotide can be independently selected from a purine, a pyrimidine, a purine analog, or pyrimidine analog. In some embodiments, the nucleobase can include, for example, naturally-occurring and synthetic derivatives of a base.

In embodiments employing a dual guide RNA, each of the crRNA and the tracr RNA can contain modifications. Such modifications may be at one or both ends of the crRNA and/or tracr RNA. In embodiments comprising an sgRNA, one or more residues at one or both ends of the sgRNA may be chemically modified, or the entire sgRNA may be chemically modified. Certain embodiments comprise a 5' end modification. Certain embodiments comprise a 3' end modification. In certain embodiments, one or more or all of the nucleotides in single stranded overhang of a guide RNA molecule are deoxynucleotides. The modified mRNA can contain 5' end and/or 3' end modifications.

**Template Nucleic Acid**

The formulations disclosed herein may include a template nucleic acid. The template may be used to alter or insert a nucleic acid sequence at or near a target site for a Cas nuclease.

In some embodiments, the template may be used in homologous recombination. In some embodiments, the homologous recombination may result in the integration of the template sequence or a portion of the template sequence into the target nucleic acid molecule. In some embodiments, a single template may be provided. In other embodiments, two or more templates may be provided such that homologous recombination may occur at two or more target sites. For example, different templates may be provided to repair a single gene in a cell, or two different genes in a cell. In some embodiments, multiple copies of at least one template are provided to a cell. In some embodiments, the different templates may be provided in independent copy numbers or independent amounts.

In other embodiments, the template may be used in homology-directed repair, which involves DNA strand invasion at the site of the cleavage in the nucleic acid. In some embodiments, the homology-directed repair may result in including the template sequence in the edited target nucleic acid molecule. In some embodiments, a single template may be provided. In other embodiments, two or more templates having different sequences may be used at two or more sites by homology-directed repair. For example, different templates may be provided to repair a single gene in a cell, or two different genes in a cell. In some embodiments, multiple copies of at least one template are provided to a cell. In some embodiments, the different templates may be provided in independent copy numbers or independent amounts.

In yet other embodiments, the template may be used in gene editing mediated by non-homologous end joining. In some embodiments, the template sequence has no similarity to the nucleic acid sequence near the cleavage site. In some embodiments, the template or a portion of the template sequence is incorporated. In some embodiments, a single template may be provided. In other embodiments, two or more templates having different sequences may be inserted at two or more sites by non-homologous end joining. For example, different templates may be provided to insert a single template in a cell, or two different templates in a cell. In some embodiments, the different templates may be provided in independent copy numbers. In some embodiments, the template includes flanking inverted terminal repeat (ITR) sequences.

In some embodiments, the template sequence may correspond to an endogenous sequence of a target cell. As used herein, the term "endogenous sequence" refers to a sequence that is native to the cell. The term "exogenous sequence" refers to a sequence that is not native to a cell, or a sequence whose native location in the genome of the cell is in a different location. In some embodiments, the endogenous sequence may be a genomic sequence of the cell. In some embodiments, the endogenous sequence may be a chromosomal or extrachromosomal sequence. In some embodiments, the endogenous sequence may be a plasmid sequence of the cell. In some embodiments, the template sequence may be substantially identical to a portion of the endogenous sequence in a cell at or near the cleavage site, but comprise at least one nucleotide change. In some embodiments, the repair of the cleaved target nucleic acid molecule with the template may result in a mutation comprising an insertion, deletion, or substitution of one or more nucleotides of the target nucleic acid molecule. In some embodiments, the mutation may result in one or more amino acid changes in a protein expressed from a gene comprising the target sequence. In some embodiments, the mutation may result in one or more nucleotide changes in an RNA expressed from the target gene. In some embodiments, the mutation may alter the expression level of the target gene. In some embodiments, the mutation may result in increased or decreased expression of the target gene. In some embodiments, the mutation may result in gene knockdown. In some embodiments, the mutation may result in gene knockout. In some embodiments, the mutation may result in restored gene function. In some embodiments, the repair of the cleaved target nucleic acid molecule with the template may result in a change in an exon sequence, an intron sequence, a regulatory sequence, a transcriptional control sequence, a translational control sequence, a splicing site, or a non-coding sequence of the target gene.

In other embodiments, the template sequence may comprise an exogenous sequence. In some embodiments, the exogenous sequence may comprise a protein or RNA coding sequence operably linked to an exogenous promoter

sequence such that, upon integration of the exogenous sequence into the target nucleic acid molecule, the cell is capable of expressing the protein or RNA encoded by the integrated sequence. In other embodiments, upon integration of the exogenous sequence into the target nucleic acid molecule, the expression of the integrated sequence may be regulated by an endogenous promoter sequence. In some embodiments, the exogenous sequence may be a chromosomal or extrachromosomal sequence. In some embodiments, the exogenous sequence may provide a cDNA sequence encoding a protein or a portion of the protein. In yet other embodiments, the exogenous sequence may comprise an exon sequence, an intron sequence, a regulatory sequence, a transcriptional control sequence, a translational control sequence, a splicing site, or a non-coding sequence. In some embodiments, the integration of the exogenous sequence may result in restored gene function. In some embodiments, the integration of the exogenous sequence may result in a gene knock-in. In some embodiments, the integration of the exogenous sequence may result in a gene knock-out.

The template may be of any suitable length. In some embodiments, the template may comprise 10, 15, 20, 25, 50, 75, 100, 150, 200, 500, 1000, 1500, 2000, 2500, 3000, 3500, 4000, 4500, 5000, 5500, 6000, or more nucleotides in length. The template may be a single-stranded nucleic acid. The template can be double-stranded or partially double-stranded nucleic acid. In certain embodiments, the single stranded template is 20, 30, 40, 50, 75, 100, 125, 150, 175, or 200 nucleotides in length. In some embodiments, the template may comprise a nucleotide sequence that is complementary to a portion of the target nucleic acid molecule comprising the target sequence (*i.e.,* a "homology arm"). In some embodiments, the template may comprise a homology arm that is complementary to the sequence located upstream or downstream of the cleavage site on the target nucleic acid molecule. In some embodiments, the template may comprise a first homology arm and a second homology arm (also called a first and second nucleotide sequence) that are complementary to sequences located upstream and downstream of the cleavage site, respectively. Where a template contains two homology arms, each arm can be the same length or different lengths, and the sequence between the homology arms can be substantially similar or identical to the target sequence between the homology arms, or it can be entirely unrelated. In some embodiments, the degree of complementarity between the first nucleotide sequence on the template and the sequence upstream of the cleavage site, and between the second nucleotide sequence on the template and the sequence downstream of the cleavage site, may permit homologous recombination, such as, *e.g*., high-fidelity homologous recombination, between the template and the target nucleic acid molecule. In some embodiments, the degree of complementarity may be about 50%, 55%, 60%, 65%, 70%, 75%, 80%, 85%, 90%, 95%, 97%, 98%, 99%, or 100%. In some embodiments, the degree of complementarity may be about 95%, 97%, 98%, 99%, or 100%. In some embodiments, the degree of complementarity may be at least 98%, 99%, or 100%. In some embodiments, the degree of complementarity may be 100%.

In some embodiments, the template contains ssDNA or dsDNA containing flanking invert-terminal repeat (ITR) sequences. In some embodiments, the template is supplied as a plasmid, minicircle, nanocircle, or PCR product.

**Purification of Nucleic Acids**

In some embodiments, the nucleic acid is purified. In some embodiments, the nucleic acid is purified using a precipation method (*e.g.,* LiCl precipitation, alcohol precipitation, or an equivalent method, *e.g.,* as described herein). In some embodiments, the nucleic acid is purified using a chromatography-based method, such as an HPLC-based method or an equivalent method (*e.g.,* as described herein). In some embodiments, the nucleic is purified using both a precipitation method (*e.g.,* LiCl precipitation) and an HPLC-based method.

**Target Sequences**

In some embodiments, a CRISPR/Cas system of the present disclosure may be directed to and cleave a target sequence on a target nucleic acid molecule. For example, the target sequence may be recognized and cleaved by the Cas nuclease. In some embodiments, a Class 2 Cas nuclease may be directed by a guide RNA to a target sequence of a target nucleic acid molecule, where the guide RNA hybridizes with and the Cas protein cleaves the target sequence. In some embodiments, the guide RNA hybridizes with and a Cas protein cleaves the target sequence comprising its cognate PAM. In some embodiments, the target sequence may be complementary to the targeting sequence of the guide RNA. In some embodiments, the degree of complementarity between a targeting sequence of a guide RNA and the portion of the corresponding target sequence that hybridizes to the guide RNA may be about 50%, 55%, 60%, 65%, 70%, 75%, 80%, 85%, 90%, 95%, 97%, 98%, 99%, or 100%. In some embodiments the homology region of the target is adjacent to a cognate PAM sequence. In some embodiments, the target sequence may comprise a sequence 100% complementary with the targeting sequence of the guide RNA. In other embodiments, the target sequence may comprise at least one mismatch, deletion, or insertion, as compared to the targeting sequence of the guide RNA. For example, the target sequence and the targeting sequence of the guide RNA may contain 1, 2, 3, 4, 5, 6, 7, 8, 9, or 10 mismatches, optionally in a portion of the target sequence adjacent to the PAM. In some embodiments, the target sequence and the targeting sequence of the guide RNA may contain 1-9 mismatches. In some embodiments, the target sequence and the targeting

sequence of the guide RNA may contain 3-6 mismatches. In some embodiments, the target sequence and the targeting sequence of the guide RNA may contain 5 or 6 mismatches.

The length of the target sequence may depend on the nuclease system used. For example, the targeting sequence of a guide RNA for a CRISPR/Cas system may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length and the target sequence is a corresponding length, optionally adjacent to a PAM sequence. In some embodiments, the target sequence may comprise 15-24 nucleotides in length. In some embodiments, the target sequence may comprise 17-21 nucleotides in length. In some embodiments, the target sequence may comprise 20 nucleotides in length. When nickases are used, the target sequence may comprise a pair of target sequences recognized by a pair of nickases that cleave opposite strands of the DNA molecule. In some embodiments, the target sequence may comprise a pair of target sequences recognized by a pair of nickases that cleave the same strands of the DNA molecule. In some embodiments, the target sequence may comprise a part of target sequences recognized by one or more Cas nucleases.

The target nucleic acid molecule may be any DNA or RNA molecule that is endogenous or exogenous to a cell. In some embodiments, the target nucleic acid molecule may be an episomal DNA, a plasmid, a genomic DNA, viral genome, mitochondrial DNA, or a chromosome from a cell or in the cell. In some embodiments, the target sequence of the target nucleic acid molecule may be a genomic sequence from a cell or in a cell. In other embodiments, the cell may be a mammalian cell. In some embodiments, the cell may be a rodent cell. In some embodiments, the cell may be a human cell. In some embodiments, the cell may be a liver cell. In certain embodiments, the cell may be a human liver cell. In some embodiments the liver cell is a hepatocyte. In some embodiments, the hepatocyte is a human hepatocyte. In some embodiments, the liver cell is a stem cell. In some embodiments, the human liver cell may be a liver sinusoidal endothelial cell (LSEC). In some embodiments, the human liver cell may be a Kupffer cell. In some embodiments, the human liver cell may be a hepatic stellate cell. In some embodiments, the human liver cell may be a tumor cell. In additional embodiments, the cell comprises ApoE-binding receptors. In some embodiments, the human liver cell may be a liver stem cell. *See, e.g.,* Wang, et al. Nature, 2015; Font-Burgada, et al. Cell, 2015, 162:766-799.

In further embodiments, the target sequence may be a viral sequence. In further embodiments, the target sequence may be a pathogen sequence. In yet other embodiments, the target sequence may be a synthesized sequence. In further embodiments, the target sequence may be a chromosomal sequence. In certain embodiments, the target sequence may comprise a translocation junction, *e.g.,* a translocation associated with a cancer. In some embodiments, the target sequence may be on a eukaryotic chromosome, such as a human chromosome. In certain embodiments, the target sequence is a liver-specific sequence, in that it is expressed in liver cells.

In some embodiments, the target sequence may be located in a coding sequence of a gene, an intron sequence of a gene, a regulatory sequence, a transcriptional control sequence of a gene, a translational control sequence of a gene, a splicing site or a non-coding sequence between genes. In some embodiments, the gene may be a protein coding gene. In other embodiments, the gene may be a non-coding RNA gene. In some embodiments, the target sequence may comprise all or a portion of a disease-associated gene. In certain cases, the gene is expressed in liver.

In some embodiments, the target sequence may be located in a non-genic functional site in the genome that controls aspects of chromatin organization, such as a scaffold site or locus control region.

In embodiments involving a Cas nuclease, such as a Class 2 Cas nuclease, the target sequence may be adjacent to a protospacer adjacent motif ("PAM"). In some embodiments, the PAM may be adjacent to or within 1, 2, 3, or 4, nucleotides of the 3' end of the target sequence. The length and the sequence of the PAM may depend on the Cas protein used. For example, the PAM may be selected from a consensus or a particular PAM sequence for a specific Cas9 protein or Cas9 ortholog, including those disclosed in Figure 1 of Ran et al., Nature, 520: 186-191 (2015), and Figure S5 of Zetsche 2015, the relevant disclosure of each of which is incorporated herein by reference. In some embodiments, the PAM may be 2, 3, 4, 5, 6, 7, 8, 9, or 10 nucleotides in length. Non-limiting exemplary PAM sequences include NGG, NGGNG, NG, NAAAAN, NNAAAAW, NNNNACA, GNNNCNNA, TTN, and NNNNGATT (wherein N is defined as any nucleotide, and W is defined as either A or T). In some embodiments, the PAM sequence may be NGG. In some embodiments, the PAM sequence may be NGGNG. In some embodiments, the PAM sequence may be TTN. In some embodiments, the PAM sequence may be NNAAAAW.

## Lipid Formulation

Disclosed herein are various embodiments of LNP formulations for CRISPR/Cas cargoes. Such LNP formulations may include a CCD lipid, along with a helper lipid, a neutral lipid, and a stealth lipid. By "lipid nanoparticle" is meant a particle that comprises a plurality of (*i.e.* more than one) lipid molecules physically associated with each other by intermolecular forces. The LNPs may be, *e.g.,* microspheres (including unilamellar and multilamellar vesicles, *e.g.*, "liposomes"-lamellar phase lipid bilayers that, in some embodiments, are substantially spherical-and, in more particular embodiments, can comprise an aqueous core, *e.g.*, comprising a substantial portion of RNA molecules), a dispersed phase in an emulsion, micelles, or an internal phase in a suspension. Emulsions, micelles, and suspensions may be

suitable compositions for local and/or topical delivery.

The LNP compositions provided herein are preferentially taken up by liver cells (*e.g.*, hepatocytes). Moreover, the LNP compositions are biodegradable, in that they do not accumulate to cytotoxic levels *in vivo* at a therapeutically effective dose. In some embodiments, the LNP compositions do not cause an innate immune response that leads to substantial adverse effects at a therapeutic dose level. In some embodiments, the LNP compositions provided herein do not cause toxicity at a therapeutic dose level. The LNP compositions specifically bind to apolipoproteins such as apolipoprotein E (ApoE) in the blood. Apolipoproteins are proteins circulating in plasma that are key in regulating lipid transport. ApoE represents one class of apolipoproteins which interacts with cell surface heparin sulfate proteoglycans in the liver during the uptake of lipoprotein. (See *e.g.,* Scherphof and Kamps, The role of hepatocytes in the clearance of liposomes from the blood circulation. Prog Lipid Res. 2001 May;40(3):149-66).

**CCD Lipids**

Lipid compositions for the delivery of biologically active agents can be adjusted to preferentially target a liver cell or organ. In certain embodiments, lipid compositions preferentially target apolipoprotein E (ApoE)-binding cells, such as cells expressing an ApoE receptor. Lipid compositions for delivery of CRISPR/Cas mRNA and guide RNA components to a liver cell comprise a CCD Lipid.

In some embodiments, the CCD lipid is Lipid A, which is (9Z,12Z)-3-((4,4-bis(octyloxy)butanoyl)oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate. Lipid A can be depicted as:

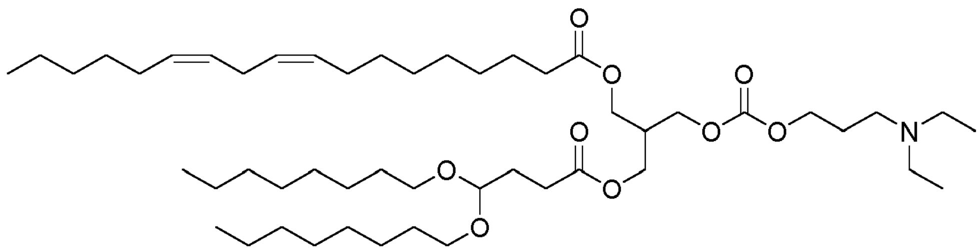

.

Lipid A may be synthesized according to WO2015/095340 *(e.g.,* pp. 84-86).

In some embodiments, the CCD lipid is Lipid B, which is ((5-((dimethylamino)methyl)-1,3-phenylene)bis(oxy))bis(octane-8,1-diyl)bis(decanoate), also called ((5-((dimethylamino)methyl)-1,3-phenylene)bis(oxy))bis(octane-8,1-diyl)bis(decanoate). Lipid B can be depicted as:

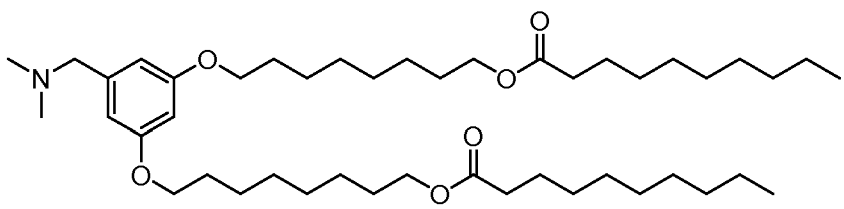

.

Lipid B may be synthesized according to WO2014/136086 *(e.g.,* pp. 107-09).

In some embodiments, the CCD lipid is Lipid C, which is 2-((4-(((3-(dimethylamino)propoxy)carbonyl)oxy)hexadecanoyl)oxy)propane-1,3-diyl (9Z,9'Z,12Z,12'Z)-bis(octadeca-9,12-dienoate). Lipid C can be depicted as:

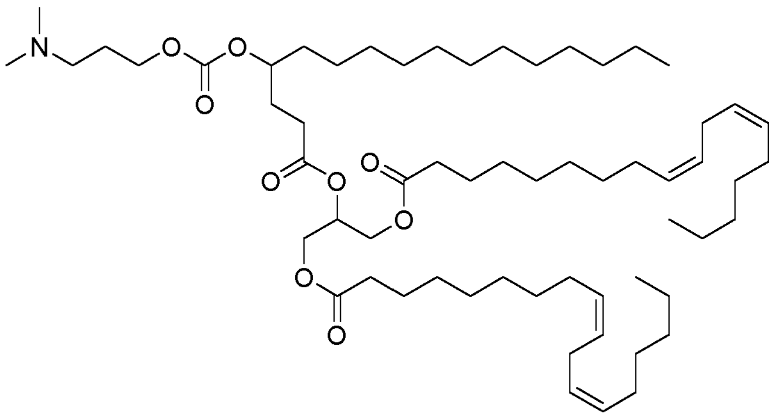

In some embodiments, the CCD lipid is Lipid D, which is 3-(((3-(dimethylamino)propoxy)carbonyl)oxy)-13-(octanoyloxy)tridecyl 3-octylundecanoate.

Lipid D can be depicted as:

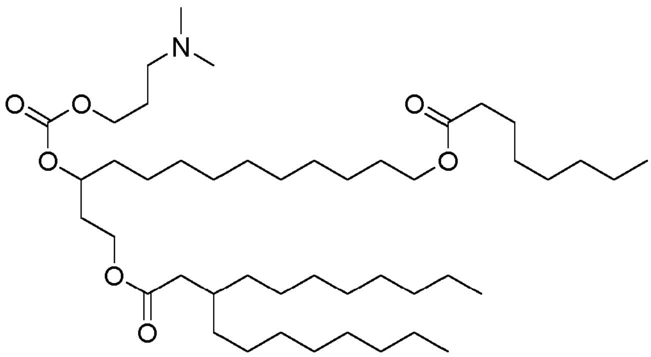

Lipid C and Lipid D may be synthesized according to WO2015/095340.

The CCD lipid can also be an equivalent to Lipid A, Lipid B, Lipid C, or Lipid D. In certain embodiments, the CCD lipid is an equivalent to Lipid A or an equivalent to Lipid B.

CCD lipids suitable for use in the LNPs described herein are biodegradable *in vivo.* The CCD lipids have low toxicity (*e.g.,* are tolerated in animal models without adverse effect in amounts of greater than or equal to 10 mg/kg). In certain embodiments, LNPs comprising a CCD lipid include those where at least 75% of the CCD lipid is cleared from the plasma within 8, 10, 12, 24, or 48 hours, or 3, 4, 5, 6, 7, or 10 days. In certain embodiments, LNPs comprising a CCD lipid include those where at least 50% of the mRNA or guide RNA is cleared from the plasma within 8, 10, 12, 24, or 48 hours, or 3, 4, 5, 6, 7, or 10 days. In certain embodiments, LNPs comprising a CCD lipid include those where at least 50% of the LNP is cleared from the plasma within 8, 10, 12, 24, or 48 hours, or 3, 4, 5, 6, 7, or 10 days, for example by measuring a lipid (e.g. CCD lipid), RNA (e.g. mRNA), or protein component. In certain embodiments, lipid-encapsulated versus free lipid, RNA, or protein component of the LNP is measured.

Lipid clearance may be measured as described in literature. *See* Maier, M.A., et al. Biodegradable Lipids Enabling Rapidly Eliminated Lipid Nanoparticles for Systemic Delivery of RNAi Therapeutics. Mol. Ther. 2013, 21(8), 1570-78 (*"Maier"*). For example, in *Maier,* LNP-siRNA systems containing luciferases-targeting siRNA were administered to six- to eight-week old male C57Bl/6 mice at 0.3 mg/kg by intravenous bolus injection *via* the lateral tail vein. Blood, liver, and spleen samples were collected at 0.083, 0.25, 0.5, 1, 2, 4, 8, 24, 48, 96, and 168 hours post-dose. Mice were perfused with saline before tissue collection and blood samples were processed to obtain plasma. All samples were processed and analyzed by LC-MS. Further, *Maier* describes a procedure for assessing toxicity after administration of LNP-siRNA formulations. For example, a luciferase-targeting siRNA was administered at 0, 1, 3, 5, and 10 mg/kg (5 animals/group) via single intravenous bolus injection at a dose volume of 5 mL/kg to male Sprague-Dawley rats. After 24 hours, about 1 mL of blood was obtained from the jugular vein of conscious animals and the serum was isolated. At 72 hours post-dose, all animals were euthanized for necropsy. Assessment of clinical signs, body weight, serum chemistry, organ weights and histopathology was performed. Although *Maier* describes methods for assessing siRNA-LNP formulations, these methods may be applied to assess clearance, pharmacokinetics, and toxicity of administration of formulations of the present disclosure.

The CCD lipids lead to an increased clearance rate. In some embodiments, the clearance rate is a lipid clearance

rate, for example the rate at which a CCD lipid is cleared from the blood, serum, or plasma. In some embodiments, the clearance rate is an RNA clearance rate, for example the rate at which an mRNA or a guide RNA is cleared from the blood, serum, or plasma. In some embodiments, the clearance rate is the rate at which LNP is cleared from the blood, serum, or plasma. In some embodiments, the clearance rate is the rate at which LNP is cleared from a tissue, such as liver tissue or spleen tissue. In certain embodiments, a high rate of clearance rate leads to a safety profile with no substantial adverse effects. The CCD lipids reduce LNP accumulation in circulation and in tissues. In some embodiments, a reduction in LNP accumulation in circulation and in tissues leads to a safety profile with no substantial adverse effects.

The CCD lipids of the present disclosure may be ionizable depending upon the pH of the medium they are in. For example, in a slightly acidic medium, the CCD lipids may be protonated and thus bear a positive charge. Conversely, in a slightly basic medium, such as, for example, blood where pH is approximately 7.35, the CCD lipids may not be protonated and thus bear no charge. In some embodiments, the CCD lipids of the present disclosure may be protonated at a pH of at least about 9. In some embodiments, the CCD lipids of the present disclosure may be protonated at a pH of at least about 9. In some embodiments, the CCD lipids of the present disclosure may be protonated at a pH of at least about 10.

The ability of a CCD lipid to bear a charge is related to its intrinsic pKa. For example, the CCD lipids of the present disclosure may each, independently, have a pKa in the range of from about 5.8 to about 6.2. This may be advantageous as it has been found that cationic lipids with a pKa ranging from about 5.1 to about 7.4 are effective for delivery of cargo to the liver. Further, it has been found that cationic lipids with a pKa ranging from about 5.3 to about 6.4 are effective for delivery to tumors. *See, e.g.,* WO 2014/136086.

## Additional Lipids

"Neutral lipids" suitable for use in a lipid composition of the disclosure include, for example, a variety of neutral, uncharged or zwitterionic lipids. Examples of neutral phospholipids suitable for use in the present disclosure include, but are not limited to, 5-heptadecylbenzene-1,3-diol (resorcinol), dipalmitoylphosphatidylcholine (DPPC), distearoylphosphatidylcholine (DSPC), pohsphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauryloylphosphatidylcholine (DLPC), dimyristoylphosphatidylcholine (DMPC), 1-myristoyl-2-palmitoyl phosphatidylcholine (MPPC), 1-palmitoyl-2-myristoyl phosphatidylcholine (PMPC), 1-palmitoyl-2-stearoyl phosphatidylcholine (PSPC), 1,2-diarachidoyl-sn-glycero-3-phosphocholine (DBPC), 1-stearoyl-2-palmitoyl phosphatidylcholine (SPPC), 1,2-dieicosenoyl-sn-glycero-3-phosphocholine (DEPC), palmitoyloleoyl phosphatidylcholine (POPC), lysophosphatidyl choline, dioleoyl phosphatidylethanolamine (DOPE), dilinoleoylphosphatidylcholine distearoylphosphatidylethanolamine (DSPE), dimyristoyl phosphatidylethanolamine (DMPE), dipalmitoyl phosphatidylethanolamine (DPPE), palmitoyloleoyl phosphatidylethanolamine (POPE), lysophosphatidylethanolamine and combinations thereof. In one embodiment, the neutral phospholipid may be selected from the group consisting of distearoylphosphatidylcholine (DSPC) and dimyristoyl phosphatidyl ethanolamine (DMPE). In another embodiment, the neutral phospholipid may be distearoylphosphatidylcholine (DSPC). Neutral lipids function to stabilize and improve processing of the LNPs.

"Helper lipids" are lipids that enhance transfection (e.g. transfection of the nanoparticle including the biologically active agent). The mechanism by which the helper lipid enhances transfection includes enhancing particle stability. In certain embodiments, the helper lipid enhances membrane fusogenicity. Helper lipids include steroids, sterols, and alkyl resorcinols. Helper lipids suitable for use in the present disclosure include, but are not limited to, cholesterol, 5-heptadecylresorcinol, and cholesterol hemisuccinate. In one embodiment, the helper lipid may be cholesterol. In one embodiment, the helper lipid may be cholesterol hemisuccinate.

"Stealth lipids" are lipids that alter the length of time the nanoparticles can exist *in vivo* (*e.g.,* in the blood). Stealth lipids may assist in the formulation process by, for example, reducing particle aggregation and controlling particle size. Stealth lipids used herein may modulate pharmacokinetic properties of the LNP. Stealth lipids suitable for use in a lipid composition of the disclosure include, but are not limited to, stealth lipids having a hydrophilic head group linked to a lipid moiety. Stealth lipids suitable for use in a lipid composition of the present disclosure and information about the biochemistry of such lipids can be found in Romberg et al., Pharmaceutical Research, Vol. 25, No. 1, 2008, pg. 55-71 and Hoekstra et al., Biochimica et Biophysica Acta 1660 (2004) 41-52. Additional suitable PEG lipids are disclosed, *e.g.,* in WO 2006/007712.

In one embodiment, the hydrophilic head group of stealth lipid comprises a polymer moiety selected from polymers based on PEG (sometimes referred to as poly(ethylene oxide)), poly(oxazoline), poly(vinyl alcohol), poly(glycerol), poly(N-vinylpyrrolidone), polyaminoacids and poly [N-(2-hydroxypropyl)methacrylamide].

Stealth lipids may comprise a lipid moiety. In some embodiments, the lipid moiety of the stealth lipid may be derived from diacylglycerol or diacylglycamide, including those comprising a dialkylglycerol or dialkylglycamide group having alkyl chain length independently comprising from about C4 to about C40 saturated or unsaturated carbon atoms, wherein the chain may comprise one or more functional groups such as, for example, an amide or ester. The dialkylglycerol or dialkylglycamide group can further comprise one or more substituted alkyl groups.

Unless otherwise indicated, the term "PEG" as used herein means any polyethylene glycol or other polyalkylene

ether polymer. In one embodiment, PEG is an optionally substituted linear or branched polymer of ethylene glycol or ethylene oxide. In one embodiment, PEG is unsubstituted. In one embodiment, the PEG is substituted, *e.g.*, by one or more alkyl, alkoxy, acyl, hydroxy, or aryl groups. In one embodiment, the term includes PEG copolymers such as PEG-polyurethane or PEG-polypropylene (see, *e.g.,* J. Milton Harris, Poly(ethylene glycol) chemistry: biotechnical and biomedical applications (1992)); in another embodiment, the term does not include PEG copolymers. In one embodiment, the PEG has a molecular weight of from about 130 to about 50,000, in a sub-embodiment, about 150 to about 30,000, in a sub-embodiment, about 150 to about 20,000, in a sub-embodiment about 150 to about 15,000, in a sub-embodiment, about 150 to about 10,000, in a sub-embodiment, about 150 to about 6,000, in a sub-embodiment, about 150 to about 5,000, in a sub-embodiment, about 150 to about 4,000, in a sub-embodiment, about 150 to about 3,000, in a sub-embodiment, about 300 to about 3,000, in a sub-embodiment, about 1,000 to about 3,000, and in a sub-embodiment, about 1,500 to about 2,500.

In certain embodiments, the PEG (*e.g.,* conjugated to a lipid, such as a stealth lipid), is a "PEG-2K," also termed "PEG 2000," which has an average molecular weight of about 2,000 daltons. PEG-2K is represented herein by the following formula (I), wherein n is 45, meaning that the number averaged degree of polymerization comprises about 45 subunits

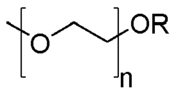
(I)

However, other PEG embodiments known in the art may be used, including, *e.g.*, those where the number-averaged degree of polymerization comprises about 23 subunits (n=23), and/or 68 subunits (n=68). In some embodiments, n may range from about 30 to about 60. In some embodiments, n may range from about 35 to about 55. In some embodiments, n may range from about 40 to about 50. In some embodiments, n may range from about 42 to about 48. In some embodiments, n may be 45. In some embodiments, R may be selected from H, substituted alkyl, and unsubstituted alkyl. In some embodiments, R may be unsubstituted alkyl. In some embodiments, R may be methyl.

In any of the embodiments described herein, the stealth lipid may be selected from PEG-dilauroylglycerol, PEG-dimyristoylglycerol (PEG-DMG) (catalog # GM-020 from NOF, Tokyo, Japan), PEG-dipalmitoylglycerol, PEG-distearoylglycerol (PEG-DSPE) (catalog # DSPE-020CN, NOF, Tokyo, Japan), PEG-dilaurylglycamide, PEG-dimyristylglycamide, PEG-dipalmitoylglycamide, and PEG-distearoylglycamide, PEG-cholesterol (1-[8'-(Cholest-5-en-3[beta]-oxy)carboxamido-3',6'-dioxaoctanyl]carbamoyl-[omega]-methyl-poly(ethylene glycol), PEG-DMB (3,4-ditetradecoxylbenzyl-[omega]-methyl-poly(ethylene glycol)ether), 1,2-dimyristoyl-sn-glycero-3-phosphoethanolamine-N-[methoxy(polyethylene glycol)-2000] (PEG2k-DMG) (cat. #880150P from Avanti Polar Lipids, Alabaster, Alabama, USA), 1,2-distearoyl-sn-glycero-3-phosphoethanolamine-N-[methoxy(polyethylene glycol)-2000] (PEG2k-DSPE) (cat. #880120C from Avanti Polar Lipids, Alabaster, Alabama, USA), 1,2-distearoyl-sn-glycerol, methoxypolyethylene glycol (PEG2k-DSG; GS-020, NOF Tokyo, Japan), poly(ethylene glycol)-2000-dimethacrylate (PEG2k-DMA), and 1,2-distearyloxypropyl-3-amine-N-[methoxy(polyethylene glycol)-2000] (PEG2k-DSA). In one embodiment, the stealth lipid may be PEG2k-DMG. In some embodiments, the stealth lipid may be PEG2k-DSG. In one embodiment, the stealth lipid may be PEG2k-DSPE. In one embodiment, the stealth lipid may be PEG2k-DMA. In one embodiment, the stealth lipid may be PEG2k-DSA. In one embodiment, the stealth lipid may be PEG2k-C11. In some embodiments, the stealth lipid may be PEG2k-C14. In some embodiments, the stealth lipid may be PEG2k-C16. In some embodiments, the stealth lipid may be PEG2k-C18.

### LNP Formulations

The LNP may contain (i) a CCD lipid for encapsulation and for endosomal escape, (ii) a neutral lipid for stabilization, (iii) a helper lipid, also for stabilization, and (iv) a stealth lipid.

In certain embodiments, the cargo includes an mRNA encoding a Cas nuclease, such as Cas9, and a guide RNA or a nucleic acid encoding a guide RNA. In one embodiment, an LNP composition may comprise a CCD lipid, such as Lipid A, Lipid B, Lipid C, or Lipid D. In some aspects, the CCD lipid is Lipid A. In some aspects, the CCD lipid is Lipid B. In various embodiments, an LNP composition comprises a CCD lipid, a neutral lipid, a helper lipid, and a stealth lipid. In certain embodiments, the helper lipid is cholesterol. In certain embodiments, the neutral lipid is DSPC. In specific embodiments, stealth lipid is PEG2k-DMG. In some embodiments, an LNP composition may comprise a Lipid A, a helper lipid, a neutral lipid, and a stealth lipid. In some embodiments, an LNP composition comprises a CCD lipid, DSPC, cholesterol, and a stealth lipid. In some embodiments, the LNP composition comprises a stealth lipid comprising PEG. In certain embodiments, the CCD lipid is selected from Lipid A, Lipid B, Lipid C, or Lipid D. In additional embodiments, an LNP composition comprises a CCD lipid selected from Lipid A or Lipid B, cholesterol, DSPC, and PEG2k-DMG.

In one embodiment, an LNP composition may comprise a CCD lipid and an mRNA encoding a Cas nuclease. In one embodiment, an LNP composition may comprise a CCD lipid, an mRNA encoding a Cas nuclease, and at least one

other lipid component. In some compositions comprising an mRNA encoding a Cas nuclease, the LNP includes at least one other lipid component chosen from a helper lipid, a neutral lipid, or a stealth lipid. In certain compositions comprising an mRNA encoding a Cas nuclease, the helper lipid is cholesterol. In other compositions comprising an mRNA encoding a Cas nuclease, the neutral lipid is DSPC. In additional embodiments comprising an mRNA encoding a Cas nuclease, the stealth lipid is PEG2k-DMG. In certain embodiments, an LNP composition may comprise a CCD lipid, a helper lipid, a neutral lipid, a stealth lipid, and an mRNA encoding a Cas nuclease. In specific compositions comprising an mRNA encoding a Cas nuclease, the CCD lipid is selected from Lipid A, Lipid B, Lipid C, or Lipid D. In additional compositions comprising an mRNA encoding a Cas nuclease, the CCD lipid is selected from Lipid A, Lipid B, Lipid C, or Lipid D, the helper lipid is cholesterol, the neutral lipid is DSPC, and the stealth lipid is PEG2k-DMG. In some embodiments, the CCD lipid in compositions comprising an mRNA encoding a Cas nuclease is Lipid A. In some embodiments, the CCD lipid in compositions comprising an mRNA encoding a Cas nuclease is Lipid B. In some embodiments, the CCD lipid in compositions comprising an mRNA encoding a Cas nuclease is Lipid C. In some embodiments, the CCD lipid in compositions comprising an mRNA encoding a Cas nuclease is Lipid D.

In one embodiment, an LNP composition may comprise a CCD lipid and a Class 2 Cas nuclease mRNA. In one embodiment, an LNP composition may comprise a CCD lipid, a Class 2 Cas nuclease mRNA, and at least one other lipid component. In some compositions comprising a Class 2 Cas nuclease mRNA, the LNP includes at least one other lipid component chosen from a helper lipid, a neutral lipid, or a stealth lipid. In certain compositions comprising a Class 2 Cas nuclease mRNA, the helper lipid is cholesterol. In other compositions comprising a Class 2 Cas nuclease mRNA, the neutral lipid is DSPC. In additional embodiments comprising a Class 2 Cas nuclease mRNA, the stealth lipid is PEG2k-DMG. In certain embodiments, an LNP composition may comprise a CCD lipid, a helper lipid, a neutral lipid, a stealth lipid, and a Class 2 Cas nuclease mRNA. In specific compositions comprising a Class 2 Cas nuclease mRNA, the CCD lipid is selected from Lipid A, Lipid B, Lipid C, or Lipid D. In additional compositions comprising a Class 2 Cas nuclease mRNA, the CCD lipid is selected from Lipid A, Lipid B, Lipid C, or Lipid D, the helper lipid is cholesterol, the neutral lipid is DSPC, and the stealth lipid is PEG2k-DMG. In some embodiments, the CCD lipid in compositions comprising a Class 2 Cas nuclease mRNA is Lipid A. In some embodiments, the CCD lipid in compositions comprising a Class 2 Cas nuclease mRNA is Lipid B. In some embodiments, the CCD lipid in compositions comprising a Class 2 Cas nuclease mRNA is Lipid C. In some embodiments, the CCD lipid in compositions comprising a Class 2 Cas nuclease mRNA is Lipid D.

In some embodiments, an LNP composition may comprise a guide RNA. In certain embodiments, an LNP composition may comprise a CCD lipid, a guide RNA, a helper lipid, a neutral lipid, and a stealth lipid. In certain LNP compositions comprising a guide RNA, the helper lipid is cholesterol. In other compositions comprising a guide RNA, the neutral lipid is DSPC. In additional embodiments comprising a guide RNA, the stealth lipid is PEG2k-DMG or PEG2k-C11. In certain embodiments, the LNP composition comprises Lipid A, Lipid B, Lipid C, or Lipid D; a helper lipid; a neutral lipid; a stealth lipid; and a guide RNA. In certain compositions comprising a guide RNA, the CCD lipid Lipid A. In certain compositions comprising a guide RNA, the CCD lipid is Lipid B. In certain compositions comprising a guide RNA, the CCD lipid is Lipid C. In certain compositions comprising a guide RNA, the CCD lipid is Lipid D. In additional compositions comprising a guide RNA, the CCD lipid is Lipid A, Lipid B, Lipid C, or Lipid D; the helper lipid is cholesterol; the neutral lipid is DSPC; and the stealth lipid is PEG2k-DMG.

In certain embodiments, the LNP formulation includes a ratio of Class 2 Cas nuclease mRNA to gRNA nucleic acid ranging from about 25:1 to about 1:25. In certain embodiments, the LNP formulation includes a ratio of Class 2 Cas nuclease mRNA to gRNA nucleic acid ranging from about 10:1 to about 1:10. As measured herein, the ratios are by weight. In some embodiments, the LNP formulation includes a ratio of Class 2 Cas nuclease mRNA to gRNA nucleic acid ranging from about 5:1 to about 1:5. In some embodiments, the LNP formulation includes a ratio of Class 2 Cas nuclease mRNA to gRNA nucleic acid of about 1:1. In some embodiments, the LNP formulation includes a ratio of Class 2 Cas nuclease mRNA to gRNA nucleic acid from about 1:1 to about 1:5. In some embodiments, the LNP formulation includes a ratio of Class 2 Cas nuclease mRNA to gRNA nucleic acid of about 10:1. In some embodiments, the LNP formulation includes a ratio of Class 2 Cas nuclease mRNA to gRNA nucleic acid of about 1:10. The ratio may be about 25:1, 10:1, 5:1, 3:1, 1:1, 1:3, 1:5, 1:10, or 1:25.

In one embodiment, an LNP composition may comprise an sgRNA. In one embodiment, an LNP composition may comprise a Cas9 sgRNA. In one embodiment, an LNP composition may comprise a Cpf1 sgRNA. In some compositions comprising an sgRNA, the LNP includes a CCD lipid, a helper lipid, a neutral lipid, and a stealth lipid. In certain compositions comprising an sgRNA, the helper lipid is cholesterol. In other compositions comprising an sgRNA, the neutral lipid is DSPC. In additional embodiments comprising an sgRNA, the stealth lipid is PEG2k-DMG or PEG2k-C11. In certain embodiments, an LNP composition may comprise a CCD lipid, a helper lipid, a neutral lipid, a stealth lipid, and an sgRNA. In specific compositions comprising an sgRNA, the CCD lipid is Lipid A, Lipid B, Lipid C, or Lipid D. In additional compositions comprising an sgRNA, the CCD lipid is Lipid A, Lipid B, Lipid C, or Lipid D; the helper lipid is cholesterol; the neutral lipid is DSPC; and the stealth lipid is PEG2k-DMG.

In certain embodiments, an LNP composition comprises an mRNA encoding a Cas nuclease and a guide RNA, which may be an sgRNA. In one embodiment, an LNP composition may comprise a CCD lipid, an mRNA encoding a Cas

nuclease, a guide RNA, a helper lipid, a neutral lipid, and a stealth lipid. In certain compositions comprising an mRNA encoding a Cas nuclease and a guide RNA, the helper lipid is cholesterol. In some compositions comprising an mRNA encoding a Cas nuclease and a guide RNA, the neutral lipid is DSPC. In additional embodiments comprising an mRNA encoding a Cas nuclease and a guide RNA, the stealth lipid is PEG2k-DMG or PEG2k-C11. In certain embodiments, an LNP composition may comprise a CCD lipid, a helper lipid, a neutral lipid, a stealth lipid, an mRNA encoding a Cas nuclease, and a guide RNA. In specific compositions comprising an mRNA encoding a Cas nuclease and a guide RNA, the CCD lipid is Lipid A, Lipid B, Lipid C, or Lipid D. In additional compositions comprising an mRNA encoding a Cas nuclease and a guide RNA, the CCD lipid is Lipid A, Lipid B, Lipid C, or Lipid D; the helper lipid is cholesterol; the neutral lipid is DSPC; and the stealth lipid is PEG2k-DMG.

The LNP compositions disclosed herein may include a template nucleic acid. The template nucleic acid may be co-formulated with an mRNA encoding a Cas nuclease, such as a Class 2 Cas nuclease mRNA. In some embodiments, the template nucleic acid may be co-formulated with a guide RNA. In some embodiments, the template nucleic acid may be co-formulated with both an mRNA encoding a Cas nuclease and a guide RNA. In some embodiments, the template nucleic acid may be formulated separately from an mRNA encoding a Cas nuclease or a guide RNA. In such formulations, the template nucleic acid may be single- or double-stranded, depending on the desired repair mechanism. The template may have regions of homology to the target DNA, or to sequences adjacent to the target DNA.

Embodiments of the present disclosure also provide lipid compositions described according to the respective molar ratios of the component lipids in the formulation. In one embodiment, the mol-% of the CCD lipid may be from about 30 mol-% to about 60 mol-%. In one embodiment, the mol-% of the CCD lipid may be from about 35 mol-% to about 55 mol-%. In one embodiment, the mol-% of the CCD lipid may be from about 40 mol-% to about 50 mol-%. In one embodiment, the mol-% of the CCD lipid may be from about 42 mol-% to about 47 mol-%. In one embodiment, the mol-% of the CCD lipid may be about 45%. In some embodiments, the CCD lipid mol-% of the LNP batch will be ±30%, ±25%, ±20%, ±15%, ±10%, ±5%, or ±2.5% of the target mol-%. In certain embodiments, LNP inter-lot variability will be less than 15%, less than 10% or less than 5%.

In one embodiment, the mol-% of the helper lipid may be from about 30 mol-% to about 60 mol-%. In one embodiment, the mol-% of the helper lipid may be from about 35 mol-% to about 55 mol-%. In one embodiment, the mol-% of the helper lipid may be from about 40 mol-% to about 50 mol-%. In one embodiment, the mol-% of the helper lipid may be from about 41 mol-% to about 46 mol-%. In one embodiment, the mol-% of the helper lipid may be about 44 mol-%. In some embodiments, the helper mol-% of the LNP batch will be ±30%, ±25%, ±20%, ±15%, ±10%, ±5%, or ±2.5% of the target mol-%. In certain embodiments, LNP inter-lot variability will be less than 15%, less than 10% or less than 5%.

In one embodiment, the mol-% of the neutral lipid may be from about 1 mol-% to about 20 mol-%. In one embodiment, the mol-% of the neutral lipid may be from about 5 mol-% to about 15 mol-%. In one embodiment, the mol-% of the neutral lipid may be from about 7 mol-% to about 12 mol-%. In one embodiment, the mol-% of the neutral lipid may be about 9 mol-%. In some embodiments, the neutral lipid mol-% of the LNP batch will be ±30%, ±25%, ±20%, ±15%, ±10%, ±5%, or ±2.5% of the target mol-%. In certain embodiments, LNP inter-lot variability will be less than 15%, less than 10% or less than 5%.

In one embodiment, the mol-% of the stealth lipid may be from about 1 mol-% to about 10 mol-%. In one embodiment, the mol-% of the stealth lipid may be from about 1 mol-% to about 5 mol-%. In one embodiment, the mol-% of the stealth lipid may be from about 1 mol-% to about 3 mol-%. In one embodiment, the mol-% of the stealth lipid may be about 2 mol-%. In one embodiment, the mol-% of the stealth lipid may be about 1 mol-%. In some embodiments, the stealth lipid mol-% of the LNP batch will be ±30%, ±25%, ±20%, ±15%, ±10%, ±5%, or ±2.5% of the target mol-%. In certain embodiments, LNP inter-lot variability will be less than 15%, less than 10% or less than 5%.

Embodiments of the present disclosure also provide lipid compositions described according to the ratio between the positively charged amine groups of the CCD lipid (N) and the negatively charged phosphate groups (P) of the nucleic acid to be encapsulated. This may be mathematically represented by the equation N/P. In one embodiment, the N/P ratio may be from about 0.5 to about 100. In one embodiment, the N/P ratio may be from about 1 to about 50. In one embodiment, the N/P ratio may be from about 1 to about 25. In one embodiment, the N/P ratio may be from about 1 to about 10. In one embodiment, the N/P ratio may be from about 1 to about 7. In one embodiment, the N/P ratio may be from about 3 to about 5. In one embodiment, the N/P ratio may be from about 4 to about 5. In one embodiment, the N/P ratio may be about 4. In one embodiment, the N/P ratio may be about 4.5. In one embodiment, the N/P ratio may be about 5.

In some embodiments, LNPs are formed by mixing an aqueous RNA solution with an organic solvent-based lipid solution, e.g., 100% ethanol. Suitable solutions or solvents include or may contain: water, PBS, Tris buffer, NaCl, citrate buffer, ethanol, chloroform, diethylether, cyclohexane, tetrahydrofuran, methanol, isopropanol. A pharmaceutically acceptable buffer, e.g., for *in vivo* administration of LNPs, may be used. In certain embodiments, a buffer is used to maintain the pH of the composition comprising LNPs at or above pH 7.0. In additional embodiments, the composition has a pH ranging from about 7.3 to about 7.7 or ranging from about 7.4 to about 7.6. In further embodiments, the composition has a pH of about 7.3, 7.4, 7.5, 7.6, or 7.7. The pH of a composition may be measured with a micro pH probe. In certain embodiments, a cryoprotectant is included in the composition. Non-limiting examples of cryoprotectants include sucrose,

trehalose, glycerol, DMSO, and ethylene glycol. Exemplary compositions may include up to 10% cryoprotectant, such as, for example, sucrose. In certain embodiments, the LNP composition may include about 1, 2, 3, 4, 5, 6, 7, 8, 9, or 10% cryoprotectant. In certain embodiments, the LNP composition may include about 1, 2, 3, 4, 5, 6, 7, 8, 9, or 10% sucrose. In some embodiments, the LNP composition may include a buffer. In some embodiments, the buffer may comprise a phosphate buffer (PBS), a Tris buffer, a citrate buffer, and mixtures thereof. In certain exemplary embodiments, the buffer comprises NaCl. Exemplary amounts of NaCl may range from about 40 mM to about 50 mM. In some embodiments, the amount of NaCl is about 45 mM. In some embodiments, the buffer is a Tris buffer. Exemplary amounts of Tris may range from about 40 mM to about 60 mM. In some embodiments, the amount of Tris is about 50 mM. In some embodiments, the buffer comprises NaCl and Tris. Certain exemplary embodiments of the LNP compositions contain 5% sucrose and 45 mM NaCl in Tris buffer. In other exemplary embodiments, compositions contain sucrose in an amount of about 5% w/v, about 45 mM NaCl, and about 50 mM Tris. The salt, buffer, and cryoprotectant amounts may be varied such that the osmolality of the overall formulation is maintained. For example, the final osmolality may be maintained at less than 450 mOsm/L. In further embodiments, the osmolality is between 350 and 250 mOsm/L. Certain embodiments have a final osmolality of 300 +/- 20 mOsm/L.

In some embodiments, microfluidic mixing, T-mixing, or cross-mixing is used. In certain aspects, flow rates, junction size, junction geometry, junction shape, tube diameter, solutions, and/or RNA and lipid concentrations may be varied. LNPs or LNP compositions may be concentrated or purified, *e.g.,* via dialysis or chromatography. The LNPs may be stored as a suspension, an emulsion, or a lyophilized powder, for example. In some embodiments, the LNP compositions are stored at 2-8° C, in certain aspects, the LNP compositions are stored at room temperature. In additional embodiments, the LNP composition is stored frozen, for example at -20° C or -80° C. In other embodiments, the LNP compositionis stored at a temperature ranging from about 0° C to about -80° C. Frozen LNP compositions may be thawed before use, for example on ice, at room temperature, or at 25° C

Dynamic Light Scattering ("DLS") can be used to characterize the polydispersity index ("pdi") and size of the LNPs of the present disclosure. DLS measures the scattering of light that results from subjecting a sample to a light source. PDI, as determined from DLS measurements, represents the distribution of particle size (around the mean particle size) in a population, with a perfectly uniform population having a PDI of zero. In some embodiments, the pdi may range from about 0.005 to about 0.75. In some embodiments, the pdi may range from about 0.01 to about 0.5. In some embodiments, the pdi may range from about 0.02 to about 0.4. In some embodiments, the pdi may range from about 0.03 to about 0.35. In some embodiments, the pdi may range from about 0.1 to about 0.35.

The LNPs disclosed herein have a size of about 1 to about 250 nm. In some embodiments, the LNPs have a size of about 10 to about 200 nm. In further embodiments, the LNPs have a size of about 20 to about 150 nm. In some embodiments, the LNPs have a size of about 50 to about 150 nm. In some embodiments, the LNPs have a size of about 50 to about 100 nm. In some embodiments, the LNPs have a size of about 50 to about 120 nm. In some embodiments, the LNPs have a size of about 75 to about 150 nm. In some embodiments, the LNPs have a size of about 30 to about 200 nm. Unless indicated otherwise, all sizes referred to herein are the average sizes (diameters) of the fully formed nanoparticles, as measured by dynamic light scattering on a Malvern Zetasizer. The nanoparticle sample is diluted in phosphate buffered saline (PBS) so that the count rate is approximately 200-400 kcts. The data is presented as a weighted-average of the intensity measure. In some embodiments, the LNPs are formed with an average encapsulation efficiency ranging from about 50% to about 100%. In some embodiments, the LNPs are formed with an average encapsulation efficiency ranging from about 50% to about 70%. In some embodiments, the LNPs are formed with an average encapsulation efficiency ranging from about 70% to about 90%. In some embodiments, the LNPs are formed with an average encapsulation efficiency ranging from about 90% to about 100%. In some embodiments, the LNPs are formed with an average encapsulation efficiency ranging from about 75% to about 95%.

## Methods of Engineering Cells; Engineered Cells

The LNP compositions disclosed herein may be used in methods for engineering cells through gene editing, both *in vivo* and *in vitro.* In some embodiments, the methods involve contacting a cell with an LNP composition described herein. In some embodiments, the cell may be a mammalian cell. In some embodiments, the cell may be a rodent cell. In some embodiments, the cell may be a human cell. In some embodiments, the cell may be a liver cell. In certain embodiments, the cell may be a human liver cell. In some embodiments the liver cell is a hepatocyte. In some embodiments, the hepatocyte is a human hepatocyte. In some embodiments, the liver cell is a stem cell. In some embodiments, the human liver cell may be a liver sinusoidal endothelial cell (LSEC). In some embodiments, the human liver cell may be a Kupffer cell. In some embodiments, the human liver cell may be a hepatic stellate cell. In some embodiments, the human liver cell may be a tumor cell. In some embodiments, the human liver cell may be a liver stem cell. In additional embodiments, the cell comprises ApoE-binding receptors.

In some embodiments, engineered cells are provided, for example an engineered cell derived from any one of the cell types in the preceding paragraph. Such engineered cells are produced according to the methods described herein. In

some embodiments, the engineered cell resides within a tissue or organ, e.g., a liver within a subject.

In some of the methods and cells described herein, a cell comprises a modification, for example an insertion or deletion ("indel") or substitution of nucleotides in a target sequence. In some embodiments, the modification comprises an insertion of 1, 2, 3, 4 or 5 or more nucleotides in a target sequence. In some embodiments, the modification comprises an insertion of either 1 or 2 nucleotides in a target sequence. In other embodiments, the modification comprises a deletion of 1, 2, 3, 4, 5, 6, 7, 8, 9, 10, 15, 20 or 25 or more nucleotides in a target sequence. In some embodiments, the modification comprises a deletion of either 1 or 2 nucleotides in a target sequence. In some embodiments, the modification comprises an indel which results in a frameshift mutation in a target sequence. In some embodiments, the modification comprises a substitution of 1, 2, 3, 4, 5, 6, 7, 8, 9, 10, 15, 20 or 25 or more nucleotides in a target sequence. In some embodiments, the modification comprises a substitution of either 1 or 2 nucleotides in a target sequence. In some embodiments, the modification comprises one or more of an insertion, deletion, or substitution of nucleotides resulting from the incorporation of a template nucleic acid, for example any of the template nucleic acids described herein.

In some embodiments, a population of cells comprising engineered cells is provided, for example a population of cells comprising cells engineered according to the methods described herein. In some embodiments, the population comprises engineered cells cultured *in vitro.* In some embodiments, the population resides within a tissue or organ, e.g., a liver within a subject. In some embodiments, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% or more of the cells within the population is engineered. In certain embodiments, a method disclosed herein results in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% editing efficiency (or "percent editing"), defined by detetion of indels. In other embodiments, a method disclosed herein, results in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% DNA modification efficiency, defined by detecting a change in sequence, whether by insertion, deletion, substitution or otherwise. In certain embodiments, a method disclosed herein results in an editing efficiency level or a DNA modification efficiency level of between about 5% to about 100%, about 10% to about 50%, about 20 to about 100%, about 20 to about 80%, about 40 to about 100%, or about 40 to about 80%.

In some of the methods and cells described herein, cells within the population comprise a modification, e.g., an indel or substitution at a target sequence. In some embodiments, the modification comprises an insertion of 1, 2, 3, 4 or 5 or more nucleotides in a target sequence. In some embodiments, the modification comprises an insertion of either 1 or 2 nucleotides in a target sequence. In other embodiments, the modification comprises a deletion of 1, 2, 3, 4, 5, 6, 7, 8, 9, 10, 15, 20 or 25 or more nucleotides in a target sequence. In some embodiments, the modification comprises a deletion of either 1 or 2 nucleotides in a target sequence. In some embodiments, the modification comprises an indel which results in a frameshift mutation in a target sequence. In some embodiments, at least 80%, at least 85%, at least 90%, at least 91%, at least 92%, at least 93%, at least 94%, at least 95%, at least 96%, at least 97%, at least 98%, or at least 99% or more of the engineered cells in the population comprise a frameshift mutation. In some embodiments, the modification comprises a substitution of 1, 2, 3, 4, 5, 6, 7, 8, 9, 10, 15, 20 or 25 or more nucleotides in a target sequence. In some embodiments, the modification comprises a substitution of either 1 or 2 nucleotides in a target sequence. In some embodiments, the modification comprises one or more of an insertion, deletion, or substitution of nucleotides resulting from the incorporation of a template nucleic acid, for example any of the template nucleic acids described herein.

## Methods of Treatment

The LNP compositions disclosed herein may be used for gene editing *in vivo* and *in vitro.* In one embodiment, one or more LNP compositions described herein may be administered to a subject in need thereof. In one embodiment, a therapeutically effective amount of a composition described herein may contact a cell of a subject in need thereof. In one embodiment, a genetically engineered cell may be produced by contacting a cell with an LNP composition described herein.

In some embodiments the methods involve administering the LNP composition to a cell associated with a liver disorder. In some embodiments, the methods involve treating a liver disorder. In certain embodiments, the methods involve contacting a hepatic cell with the LNP composition. In certain embodiments, the methods involve contacting a hepatocyte with the LNP composition. In some embodiments, the methods involve contacting an ApoE binding cell with the LNP composition.

In one embodiment, an LNP composition comprising an mRNA encoding a Cas nuclease, a gRNA, and a template may be administered to a cell, such as an ApoE binding cell. In certain instances, an LNP composition comprising a Cas nuclease and an sgRNA may be administered to a cell, such as an ApoE binding cell. In one embodiment, an LNP composition comprising an mRNA encoding a Cas nuclease, a gRNA, and a template may be administered to a liver cell. In

certain instances, an LNP composition comprising a Cas nuclease and an sgRNA may be administered to a liver cell. In some cases, the liver cell is in a subject. In certain embodiments, a subject may receive a single dose of an LNP composition. In other examples, a subject may receive multiple doses of an LNP composition. Where more than one dose is administered, the doses may be administered about 1, 2, 3, 4, 5, 6, 7, 14, 21, or 28 days apart; about 2, 3, 4, 5, or 6 months apart; or about 1, 2, 3, 4, or 5 years apart.

In one embodiment, an LNP composition comprising an mRNA encoding a Cas nuclease may be administered to a liver cell (also called a hepatic cell), followed by the administration of a composition comprising a gRNA and optionally a template. In one embodiment, an LNP composition comprising an mRNA encoding a Cas nuclease and a gRNA may be administered to a liver cell, followed by the administration of a composition comprising a template to the cell. In one embodiment, an LNP composition comprising an mRNA encoding a Cas nuclease may be administered to a liver cell, followed by the sequential administration of an LNP composition comprising a gRNA and then an LNP composition comprising a template to the cell. In embodiments where an LNP composition comprising an mRNA encoding a Cas nuclease is administered before an LNP composition comprising a gRNA, the administrations may be separated by about 4, 6, 8, 12, or 24 hours; or 2, 3, 4, 5, 6, or 7 days.

In one embodiment, the LNP compositions may be used to edit a gene resulting in a gene knockout. In one embodiment, the LNP compositions may be used to edit a gene resulting in a gene correction. In one embodiment, the LNP compositions may be used to edit a cell resulting in gene insertion.

In one embodiment, administration of the LNP compositions may result in gene editing which results in persistent response. For example, administration may result in a duration of response of a day, a month, a year, or longer. As used herein, "duration of response" means that, after cells have been edited using an LNP composition disclosed herein, the resulting modification is still present for a certain period of time after administration of the LNP composition. The modification may be detected by measuring target protein levels. The modification may be detected by detecting the target DNA. In some embodiments, the duration of response may be at least 1 week. In other embodiments, the duration of response may be at least 2 weeks. In one embodiment, the duration of response may be at least 1 month. In some embodiments, the duration of response may be at least 2 months. In one embodiment, the duration of response may be at least 4 months. In one embodiment, the duration of response may be at least 6 months. In certain embodiments, the duration of response may be about 26 weeks. In some embodiments, the duration of response may be at least 1 year. In some embodiments, the duration of response may be at least 5 years. In some embodiments, the duration of response may be at least 10 years. A persistent response is detectable after at least 6 months, either by measuring target protein levels or by detection of the target DNA.

The LNP compositions can be administered parenterally. The LNP compositions may be administered directly into the blood stream, into tissue, into muscle, or into an internal organ. Administration may be systemic, *e.g.*, to injection or infusion. Administration may be local. Suitable means for administration include intravenous, intraarterial, intrathecal, intraventricular, intraurethral, intrasternal, intracranial, subretinal, intravitreal, intra-anterior chamber, intramuscular, intrasynovial and subcutaneous. Suitable devices for administration include needle (including microneedle) injectors, needle-free injectors and infusion techniques.

The LNP compositions will generally, but not necessarily, be administered as a formulation in association with one or more pharmaceutically acceptable excipients. The term "excipient" includes any ingredient other than the compound(s) of the disclosure, the other lipid component(s) and the biologically active agent. An excipient may impart either a functional (*e.g.* drug release rate controlling) and/or a non-functional (*e.g.* processing aid or diluent) characteristic to the formulations. The choice of excipient will to a large extent depend on factors such as the particular mode of administration, the effect of the excipient on solubility and stability, and the nature of the dosage form.

Parenteral formulations are typically aqueous or oily solutions or suspensions. Where the formulation is aqueous, excipients such as sugars (including but not restricted to glucose, mannitol, sorbitol, *etc.*) salts, carbohydrates and buffering agents (preferably to a pH of from 3 to 9), but, for some applications, they may be more suitably formulated with a sterile non-aqueous solution or as a dried form to be used in conjunction with a suitable vehicle such as sterile, pyrogen-free water (WFI).

In some embodiments, the methods of gene editing modify a Factor VII target gene. In certain embodiments, the LNP compositions are administered to a liver cell to modify a Factor VII gene. The LNP compositions may be used for treating a liver disorder, such as Factor VII deficiency. The methods may modulate aberrant Factor VII activity. In certain embodiments, the LNP composition may be administered to treat or prevent hemophilia, or the inability to control blood clotting. *See, e.g.,* Lapecorella, M. and Mariani, G. Factor VII deficiency: defining the clinical picture and optimizing therapeutic options. Haemophilia (2008), 14, 1170-1175. In certain embodiments, the LNP compositions may be administered to treat or prevent thrombophilia, a condition where blood has an increased tendency to form clots.

When an injury to a tissue occurs, the formation of an equimolar complex between Factor VII zymogen and Tissue Factor, resulting in a cleavage at position 152 of the Factor VII sequence, leading to the formation of activated Factor VII, or Factor VIIa. The Factor VIIa/Tissue Factor complex leads to coagulation. The methods of treatment of a Factor VII-associated disorder include methods of increasing Factor VIIa coagulation, methods of improving blood clotting, or

methods of improving a blood coagulation profile. In certain embodiments, the methods administer an LNP composition to a subject with a Factor VII deficiency. In some embodiments, the methods administer an LNP composition to a subject previously treated for Factor VII deficiency, *e.g.* with recombinant Factor VIIa.

In some embodiments, the methods of gene editing modify a TTR target gene. In certain embodiments, the LNP compositions may be used for treating a disorder associated with TTR expression in the liver, such as amyloidosis. In certain embodiments, the LNP composition may be administered to treat or prevent amyloidosis, including transthyretin type amyloidosis. *See, e.g.,* Patel, K. and Hawkins, P. Cardiac amyloidosis: where are we today? J. Intern. Med. (2008), 278, 126-144.

The TTR-associated disorder can lead to accumulation of amyloid deposits. Therefore, the methods to treat or prevent a TTR-associated disorder include methods of reducing TTR levels, methods of reducing TTR production, methods of reducing amyloid deposits, methods of treating inherited transthyretin type amyloidosis, methods of treating nonhereditary transthyretin type amyloidosis, or methods of affecting amyloid deposits in the heart, and autonomic and peripheral nerves. In some embodiments, the methods of treating or preventing a TTR-associated disorder comprise administering an LNP composition to a subject diagnosed amyloid deposits. In certain embodiments, the methods administer an LNP composition to a subject in need of reduced TTR production

In some embodiments, the methods of gene editing target a gene selected from *SERPINA1, FVIII, FIX, SERPING1, KLKB1, KNG1, FXII, ASS1, ASL, BCKDHA, BCKDHB, G6PC, GO*/*HAO1, AGXT, PCCA, PCCB, OTC, LIPA, ABCB11, GALT, ATP7B,* and *PAH.* In some embodiments, the methods of gene editing may be used to treat a subject afflicted with a disease selected from Alpha 1 Antitrypsin Deficiency, Hemophilia A, Hemophilia B, HAE, Type 1 Citrullinemia, Arginiosuccinic aciduria, Maple syrup urine disease, Glycogen storage disease, Primary hyperoxaluria type 1, Propionic academia, Ornithine transcarbamylase deficiency, Cholesteryl ester storage disease, Progressive familial intrahepatic cholestasis, Galactosemia, Wilson's disease, and Phenylketonuria.

The words "a", "an" or "the" when used in conjunction with the term "comprising" in the claims and/or the specification may mean "one," but each is also consistent with the meaning of "one or more," "at least one," and "one or more than one." The use of "or" means "and/or" unless stated otherwise. The use of the term "including" and "containing," as well as other forms, such as "includes," "included," "contains," and "contained" is not limiting. All ranges given in the application encompass the endpoints unless stated otherwise.

## EXAMPLES

### Example 1. Materials and Methods.

#### *Lipid Nanoparticle ("LNP") Formulation*

The LNPs were formulated with a CCD lipid amine to RNA phosphate (N:P) molar ratio of about 4.5. The lipid nanoparticle components were dissolved in 100% ethanol with the following molar ratios: 45 mol-% (12.7 mM) CCD lipid (*e.g.,* Lipid A or Lipid B); 44 mol-% (12.4 mM) helper lipid (*e.g.*, cholesterol); 9 mol-% (2.53 mM) neutral lipid (*e.g.,* DSPC); and 2 mol-% (.563 mM) PEG (*e.g.,* PEG2k-DMG or PEG2k-C11). The RNA cargo were dissolved in 50 mM acetate buffer, pH 4.5, resulting in a concentration of RNA cargo of approximately 0.45 mg/mL.

The LNPs were formed by microfluidic mixing of the lipid and RNA solutions using a Precision Nanosystems NanoAssemblr™ Benchtop Instrument, according to the manufacturer's protocol. A 2:1 ratio of aqueous to organic solvent was maintained during mixing using differential flow rates. After mixing, the LNPs were collected, diluted in phosphate buffered saline (PBS, approximately 1:1), and then remaining buffer was exchanged into PBS (100-fold excess of sample volume), overnight at 4°C under gentle stirring using a 10 kDa Slide-a-Lyzer™ G2 Dialysis Cassette (ThermoFisher Scientific). The resulting mixture was then filtered using a 0.2 μm sterile filter. The resulting filtrate was stored at 2-8 °C. The isolated LNPs were characterized to determine the encapsulation efficiency, polydispersity index, and average particle size, as described below.

#### *In vitro transcription ("IVT") of nuclease mRNA and single guide RNA (sgRNA)*

Capped and polyadenylated Cas9 mRNA containing N1-methyl pseudo-U was generated by *in vitro* transcription using a linearized plasmid DNA template and T7 RNA polymerase. Plasmid DNA containing a T7 promoter and a 100 nt poly(A/T) region was linearized by incubating at 37 °C for 2 hrs with XbaI with the following conditions: 200 ng/μL plasmid, 2 U/μL XbaI (NEB), and 1x reaction buffer. The XbaI was inactivated by heating the reaction at 65 °C for 20 min. The linearized plasmid was purified from enzyme and buffer salts using a silica maxi spin column (Epoch Life Sciences) and analyzed by agarose gel to confirm linearization. The IVT reaction to generate Cas9 modified mRNA was incubated at 37 °C for 4 hours in the following conditions: 50 ng/μL linearized plasmid; 2 mM each of GTP, ATP, CTP, and N1-methyl pseudo-UTP (Trilink); 10 mM ARCA (Trilink); 5 U/μL T7 RNA polymerase (NEB); 1 U/μL Murine RNase inhibitor (NEB);

0.004 U/μL Inorganic *E. coli* pyrophosphatase (NEB); and 1x reaction buffer. After the 4 hr incubation, TURBO DNase (ThermoFisher) was added to a final concentration of 0.01 U/μL, and the reaction was incubated for an additional 30 minutes to remove the DNA template. The Cas9 mRNA was purified from enzyme and nucleotides using a MegaClear Transcription Clean-up kit according to the manufacturer's protocol (ThermoFisher). Alternatively, for example as shown in Example 15, the mRNA was purified through a precipitation protocol, which in some cases was followed by HPLC-based purification. Briefly, after the DNase digestion, the mRNA was precipitated by adding 0.21x vol of a 7.5 M LiCl solution and mixing, and the precipitated mRNA was pelleted by centrifugation. Once the supernatant was removed, the mRNA was reconstituted in water. The mRNA was precipitated again using ammonium acetate and ethanol. 5M Ammonium acetate was added to the mRNA solution for a final concentration of 2M along with 2x volume of 100% EtOH. The solution was mixed and incubated at -20 °C for 15 min. The precipitated mRNA was again pelleted by centrifugation, the supernatant was removed, and the mRNA was reconstituted in water. As a final step, the mRNA was precipitated using sodium acetate and ethanol. 1/10 volume of 3 M sodium acetate (pH 5.5) was added to the solution along with 2x volume of 100% EtOH. The solution was mixed and incubated at -20 °C for 15 min. The precipitated mRNA was again pelleted by centrifugation, the supernatant was removed, the pellet was washed with 70% cold ethanol and allowed to air dry. The mRNA was reconstituted in water. For HPLC purified mRNA, after the LiCl precipitation and reconstitution, the mRNA was purified by RP-IP HPLC (see, e.g., Kariko, et al. Nucleic Acids Research, 2011, Vol. 39, No. 21 e142). The fractions chosen for pooling were combined and deslated by sodium acetate/ethanol precipitation as described above.

For all methods, the transcript concentration was determined by measuring the light absorbance at 260 nm (Nanodrop), and the transcript was analyzed by capillary electrophoresis by Bioanlayzer (Agilent).

IVT was also used to generate sgRNA in a similar process. DNA template for sgRNA was generated by annealing a top oligo composed of only the T7 RNA polymerase promoter sequence and a bottom strand containing the sgRNA template and the complementary sequence to the promoter site. The annealed template was used directly in an IVT reaction in the following conditions: 125 nM template; 7.5 mM each of GTP, ATP, CTP, and UTP; 5 U/μL T7 RNA polymerase (NEB); 1 U/μL Murine RNase inhibitor (NEB); 0.004 U/μL Inorganic *E. coli* pyrophosphatase (NEB); and 1x reaction buffer. The reaction was incubated at 37 °C for 8 hours, after which TURBO DNase (ThermoFisher) was added to a final concentration of 0.01 U/μL, and the reaction was incubated another 30 minutes to remove the DNA template. The sgRNA transcript was purified by a MegaClear Transcription Clean-up kit according to the manufacturer's protocol (ThermoFisher). The transcript concentration was determined by absorbance at 260 nm (Nanodrop), and the transcript was analyzed by PAGE.

***Formulation Analytics***

LNP formulations were analyzed for average particle size, polydispersity (pdi), total RNA content and encapsulation efficiency of RNA. Average particle size and polydispersity were measured by dynamic light scattering (DLS) using a Malvern Zetasizer DLS instrument. LNP samples were diluted 30X in PBS prior to being measured by DLS. Z-average diameter which is intensity based measurement of average particle size was reported along with pdi.

A fluorescence-based assay was used to determine total RNA concentration and encapsulation efficiency. LNPs were diluted 75X with 1x TE buffer to be within the linear range of the RiboGreen® dye (ThermoFisher Scientific, catalog number R11491). 50 μl of diluted LNP were further mixed with either 50μl 1x TE buffer or 1x TE buffer with 0.2% Triton X-100 in duplicate. Samples were incubated at 37 °C for 10 minutes to allow Triton to completely disrupt the LNPs and expose total RNA to interact with the RiboGreen® dye. Samples for standard curve were prepared by utilizing the starting RNA solution used to make the LNPs and following the same steps as above. Diluted RiboGreen® dye (100 μL, 100X in 1xTE buffer, according to the manufacturer's instructions) was then added to each of the samples and allowed to incubate for 10 minutes at room temperature, in the absence of light. A SpectraMax M5 Microplate Reader (Molecular Devices) was used to read the samples with excitation, auto cutoff and emission wavelengths set to 488 nm, 515 nm, and 525 nm respectively. Encapsulation efficiency (%EE) was calculated using the following equation:

$$\%EE = \left(1 - \frac{Fluorescence\ @525nm - triton}{Fluorescence\ @525 + triton}\right) * 100$$

where Fluorescence @ 525 nm - triton is average fluorescence reading for sample without Triton, and Fluorescence @ 525 nm + triton is average fluorescence reading for sample with Triton. Total RNA concentration was determined using a liner standard curve and average fluorescence reading for sample with triton value.

The same procedure may be used for determining the encapsulation efficiency of a DNA-based cargo component. For single-strand DNA Oligreen Dye may be used, and for double-strand DNA, Picogreen Dye.

The values for average particle size, polydispersity, and %EE are reported in Table 1, below, for various LNP compositions.

**Table 1. Summary of LNP Formulation Data**

| LNP # | Target | RNA Cargo | CCD Lipid | Stealth Lipid | Avg. Particle Size (nm) | pdi | EE (%) |
|---|---|---|---|---|---|---|---|
| LNP002 | N/A | eGFP mRNA | Lipid A | PEG2k-DMG | 71.8 | 0.073 | 80% |
| LNP006 | N/A | eGFP mRNA | Lipid A | PEG2k-C11 | 83.2 | 0.130 | 92% |
| LNP007 | N/A | eGFP mRNA | Lipid A | PEG2k-C11 | 94.5 | 0.122 | 90% |
| LNP010 | N/A | eGFP mRNA | Lipid A | PEG2k-DMG | 71.0 | 0.135 | 96% |
| LNP011 | N/A | eGFP mRNA | Lipid A | PEG2k-C11 | 78.9 | 0.138 | 96% |
| LNP012 | N/A | eGFP mRNA | Lipid B | PEG2k-DMG | 88.8 | 0.029 | 94% |
| LNP013 | N/A | eGFP mRNA | Lipid B | PEG2k-C11 | 88.1 | 0.056 | 95% |
| LNP014 | N/A | gLUC mRNA | Lipid A | PEG2k-DMG | 66.6 | 0.129 | 92% |
| LNP015 | N/A | gLUC mRNA | Lipid B | PEG2k-DMG | 110.4 | 0.191 | 90% |
| LNP093 | FVII | cr004* + tr002* | Lipid A | PEG2k-DMG | 97.67 | 0.173 | 79% |
| LNP094 | FVII | cr004* + tr002* | Lipid A | PEG2k-DMG | 83.09 | 0.159 | 92% |
| LNP095 | TTR | cr005* + tr002* | Lipid A | PEG2k-DMG | 131 | 0.219 | 86% |
| LNP096 | TTR | cr005* + tr002* | Lipid A | PEG2k-DMG | 77.66 | 0.138 | 96% |
| LNP097 | N/A | Cas9 mRNA | Lipid A | PEG2k-DMG | 90.02 | 0.118 | 88% |
| LNP116 | TTR | cr005* + tr002* | Lipid A | PEG2k-DMG | 136.3 | 0.202 | 56% |
| LNP120 | N/A | Cas9 mRNA | Lipid A | PEG2k-DMG | 85.8 | 0.123 | 94% |
| LNP121 | TTR | cr005* + tr002* | Lipid A | PEG2k-DMG | 77.8 | 0.150 | 94% |
| LNP123 | TTR | sg003 | Lipid A | PEG2k-DMG | 93.4 | 0.215 | 86% |
| LNP136 | TTR | cr005* + tr002* | Lipid A | PEG2k-DMG | 72.2 | 0.043 | 96% |
| LNP137 | TTR | cr005* + tr002* | Lipid A | PEG2k-DMG | 76.1 | 0.090 | 96% |
| LNP 13 8 | FVII | sg008** | Lipid A | PEG2k-DMG | 86.9 | 0.305 | 96% |
| LNP139 | TTR | sg003 | Lipid A | PEG2k-DMG | 84.5 | 0.324 | 97% |
| LNP 140 | N/A | Cas9 mRNA | Lipid A | PEG2k-DMG | 71.95 | 0.183 | 95% |
| LNP152 | FVII | sg013** + Cas9 mRNA | Lipid A | PEG2k-DMG | 97.5 | 0.092 | 95% |

(continued)

| LNP # | Target | RNA Cargo | CCD Lipid | Stealth Lipid | Avg. Particle Size (nm) | pdi | EE (%) |
|---|---|---|---|---|---|---|---|
| LNP153 | FVII | sg014** + Cas9 mRNA | Lipid A | PEG2k-DMG | 96.5 | 0.057 | 97% |
| LNP154 | TTR | sg015** + Cas9 mRNA | Lipid A | PEG2k-DMG | 96.4 | 0.060 | 97% |
| LNP155 | TTR | sg016** + Cas9 mRNA | Lipid A | PEG2k-DMG | 92.9 | 0.060 | 97% |
| LNP 169 | TTR | sg017** + Cas9 mRNA | Lipid A | PEG2k-DMG | 81.8 | 0.098 | 98% |
| LNP170 | TTR | sg017** + Cas9 mRNA | Lipid A | PEG2k-DMG | 75.3 | 0.088 | 99% |
| LNP171 | TTR | sg017** + Cas9 mRNA | Lipid A | PEG2k-DMG | 100.7 | 0.062 | 97% |
| LNP172 | N/A | Cas9 mRNA | Lipid A | PEG2k-DMG | 111.4 | 0.028 | 98% |
| LNP173 | TTR | cr005* + tr002* | Lipid A | PEG2k-DMG | 58.3 | 0.087 | 98% |
| LNP174 | TTR | cr005* + tr002* + Cas9 mRNA | Lipid A | PEG2k-DMG | 85.5 | 0.079 | 98% |
| LNP175 | TTR | cr005* + tr002* | Lipid A | PEG2k-DMG | 82.6 | 0.065 | 98% |
| LNP176 | TTR | sg004 (DNA) | Lipid A | PEG2k-DMG | 65.82 | 0.064 | 100% |
| LNP178 | N/A | Cas9 mRNA | Lipid A | PEG2k-DMG | 115.8 | 0.072 | 97% |
| LNP294 | TTR | sg009* + Cas9 mRNA | Lipid A | PEG2k-DMG | 83.6 | 0.115 | 92% |
| *** = phosphorothioate linkages between 3 terminal nucleotides at the 5' and 3' ends<br>** = 2'-O-methyl modifications and phosphorothioate linkages at and between the three terminal nucleotides at the 5' and 3' ends** | | | | | | | |

***T7E1 Assay***

A T7E1 assay was used in some Examples to detect mutation events in genomic DNA such as insertions, deletions and substitutions created through non-homologous end joining (NHEJ) following DNA cleavage by Cas9 (See, *e.g.,* Cho et al., Targeted genome engineering in human cells with the Cas9 RNA-guided endonuclease. Nature Biotechnology. 2013; 31, 230-232).

The genomic DNA regions targeted by CRISPR/Cas9 were amplified by PCR, denatured at 95° C for 10 minutes, and then re-annealed by ramping down the temperature from 95° C to 25° C at a rate of 0.5° C/second. The combination of DNA to form heteroduplexes indicated the presence of mutations in the amplified region. The re-annealed heteroduplexes were then digested with bacteriophage resolvase T7E1 (New England Biolabs) at 37° C for 25 minutes or longer to generate double-stranded breaks where the T7E1 nuclease recognized mismatches. The resulting DNA fragments were analyzed using a Fragment Analyzer and quantified to determine an approximation of editing efficiency. For quantitative analysis of editing efficiency, Next-Generation Sequencing was used as described herein.

***Next-Generation Sequencing ("NGS") and Analysis for On-Target Cleavage Efficiency***

To quantitatively determine the efficiency of editing at the target location in the genome, deep sequencing was

utilized to identify the presence of insertions and deletions introduced by gene editing.

PCR primers were designed around the target site (*e.g.,* TTR, FVII), and the genomic area of interest was amplified. Primer sequences are provided below. Additional PCR was performed according to the manufacturer's protocols (Illumina) to add the necessary chemistry for sequencing. The amplicons were sequenced on an Illumina MiSeq instrument. The reads were aligned to the human reference genome (*e.g.,* hg38) after eliminating those having low quality scores. The resulting files containing the reads were mapped to the reference genome (BAM files), where reads that overlapped the target region of interest were selected and the number of wild type reads versus the number of reads which contain an insertion, substitution, or deletion was calculated.

The editing percentage (*e.g.*, the "editing efficiency" or "percent editing") is defined as the total number of sequence reads with insertions or deletions over the total number of sequence reads, including wild type.

***LNP Delivery in vitro***

Mouse cells lines (Neuro2A and Hepa1.6 ) were cultured in DMEM media supplemented with 10% fetal bovine serum and were plated at a density of 15,000 cells/well 24 hours prior to transfection with LNPs for 18-24 hours prior to lysis and analysis as described herein (*e.g.*, reporter expression, T7E1 assay, NGS). Mouse primary hepatocytes (Invitrogen) were cultured at 15,000 cells per well in hepatocyte plating media (Invitrogen) using collagen coated 96 well plates. After 5 hours, the plating media was removed and replaced with hepatocyte maintenance media containing LNPs and 3% mouse serum (pre-incubated for 5 min at 37° C). Cells were transfected for 42-48 hours prior to lysis and analysis as described herein (*e.g.,* T7E1 assay, NGS). For both cell lines and primary hepatocytes the LNPs were diluted and added to cells starting at 100 ng Cas9 mRNA and approximately 30 nM guide RNA per well, carrying out serial dilutions in a semi-log manner down to 0.1 ng Cas9 mRNA and 0.03 nM guide RNA per well.

***LNP Delivery in vivo***

CD-1 female mice, ranging from 6-10 weeks of age were used in each study. Animals were weighed and grouped according to body weight for preparing dosing solutions based on group average weight. LNPs were dosed via the lateral tail vein in a volume of 0.2 mL per animal (approximately 10 mL per kilogram body weight). The animals were observed at approximately 6 hours post dose for adverse effects. Body weight was measured at twenty-four hours post-administration, and animals were euthanized at various time points by exsanguination via cardiac puncture under isoflourane anesthesia. Blood was collected into serum separator tubes or into tubes containing buffered sodium citrate for plasma as described herein. For studies involving *in vivo* editing, liver tissue was collected from the median lobe or from three independent lobes (*e.g.,* the right median, left median, and left lateral lobes) from each animal for DNA extraction and analysis. For some studies, spleen tissue was also collected.

***Genomic DNA Isolation***

Genomic DNA was extracted from 10 mg of tissue using Invitrogen PureLink Genomic DNA Kit (Cat. K1820-02) according to manufacturer's protocol, which includes homogenizing the tissue in lysis buffer (approximately 200 μL/10 mg tissue) and precipitating the DNA. All DNA samples were normalized to 100 ng/μL concentration for PCR and subsequent NGS analysis, as described herein.

***Transthyretin (TTR) ELISA analysis***

Blood was collected and the serum was isolated as indicated. The total TTR serum levels were determined using a Mouse Prealbumin (Transthyretin) ELISA Kit (Aviva Systems Biology, Cat. OKIA00111). Kit reagents and standards were prepared according to the manufacture's protocol. Mouse serum was diluted to a final dilution of 10,000-fold with 1x assay diluent. This was done by carrying out two sequential 50-fold dilutions resulting in a 2,500-fold dilution. A final 4-fold dilution step was carried out for a total sample dilution of 10,000-fold. Both standard curve dilutions (100 μL each) and diluted serum samples were added to each well of the ELISA plate pre-coated with capture antibody. The plate was incubated at room temperature for 30 minutes before washing. Enzyme-antibody conjugate (100 μL per well) was added for a 20 minute incubation. Unbound antibody conjugate was removed and the plate was washed again before the addition of the chromogenic substrate solution. The plate was incubated for 10 minutes before adding 100 μL of the stop solution, *e.g.*, sulfuric acid (approximately 0.3 M). The plate was read on a SpectraMax M5 plate reader at an absorbance of 450 nm. Serum TTR levels were calculated by SoftMax Pro software ver. 6.4.2 using a four parameter logistic curve fit off the standard curve. Final serum values were adjusted for the assay dilution.

***Factor-VII (FVII) Activity Assay***

Blood was collected for the plasma as indicated. Plasma Factor VII activity levels were measured using BIOPHEN FVII assay kit (Anaria Diagnostics, Cat. A221304). Kit reagents were prepared according to the manufacturer's protocol. Plasma was diluted 10,000-fold with the kit sample dilution buffer by carrying out two sequential 50-fold dilutions resulting in a 2,500-fold dilution. A final 4-fold dilution step was carried out for a total sample dilution of 10,000-fold. Diluted sample (30 μL) was added to kit reagent 1 (30 μL). Next, kit reagent 2 (60 μL) was added to the plate, which was subsequently incubated at 37° C for 7 minutes. Kit reagent 3 (60 μL) was then added to the plate and the plate was incubated for an additional 5 minutes at 37° C, before adding acetic acid (20% v/v in water, 60 μL) to stop the enzyme reaction. The plate was read on a SoftMax M5 plate reader at 405 nM. The relative values of FVII activity were calculated based upon a calibration curve prepared from plasma of control animals and reported as a percent of vehicle control.

Example 2. *In vitro* delivery of eGFP mRNA encapsulated LNPs.

LNPs comprising mRNA encoding eGFP (TriLink, Cat. L-6101) were prepared as described in Example 1. The components of each LNP preparation include a CCD lipid (45 mol-%), cholesterol (44 mol-%), DSPC (9 mol-%), and PEG2k-DMG or PEG2k-C11 (2 mol-%). LNP-002, -006, -007, -010, and -011 include Lipid A as the CCD lipid, whereas LNP-012 and -013 include Lipid B as the CCD lipid. LNP-002, - 010, and -012 include PEG2k-DMG, and LNP-006, -007, -011, and -013 include PEG2k-C11. LNP details are provided in Table 1, including average particle size, polydispersity, and encapsulation efficiency. LNPs were delivered to a mouse hepatocyte cell line (Hepa1.6) as described in Example 1, with total amounts of eGFP mRNA delivered being either 100 ng or 500 ng per well, for each LNP. Cells were incubated with LNPs for approximately 18 hours, and eGFP expression was measured using a CytoFLEX Cell Analyzer (Beckman Coulter).

As shown in Fig. 1, eGFP expression was observed for each formulation. LNP formulations comprising Lipid A (LNP-002, -006, -007, -010, and -011) successfully delivered eGFP mRNA. LNP formulations comprising Lipid B (LNP-012 and -013) also delivered eGFP mRNA. LNPs that include PEG2k-C11 and PEG2k-DMG stealth lipids both deliver mRNA effectively in these experiments, demonstrating delivery of mRNA to a mouse hepatocyte cell line using LNPs *in vitro.*

Example 3. *In vivo* delivery of gLUC mRNA encapsulated LNPs.

LNPs comprising mRNA encoding *Gaussia* luciferase (gLUC) (TriLink, Cat. L-6123) were prepared as described in Example 1 and tested for mRNA delivery to animals *in vivo.* The components of each LNP preparation include a CCD lipid (45 mol-%), cholesterol (44 mol-%), DSPC (9 mol-%), and PEG2k-DMG (2 mol-%). LNP-014 included Lipid A, whereas LNP-015 included Lipid B. Details for these formulations are provided in Table 1, such as average particle size, polydispersity, and encapsulation efficiency. gLUC mRNA doses of 0.1 mg/kg and 0.3 mg/kg were delivered with each LNP formulation. The animals were euthanized 24 hours later with blood collection and serum isolation performed as described in Example 1. Serum luciferase expression was measured using a Pierce™ Gaussia Luciferase Flash Assay Kit (ThermoFisher Scientific, catalog number 16158) according to the manufacturer's protocol.

As shown in Fig. 2, dose dependent increases in gLUC expression were observed for each animal (n=5 for each group) as compared to a PBS control. LNPs comprising either Lipid A or Lipid B showed effective *in vivo* delivery and expression of mRNA as measured by luciferase activity.

Example 4. *In vivo* delivery and editing using Cas9 mRNA encapsulated LNPs (mRNA-LNP) with dual guide RNA encapsulated LNPs (dgRNA-LNP).

LNPs for delivering CRISPR/Cas RNA components (*e.g.*, gRNA and mRNA encoding Cas9) for *in vivo* editing in the liver were tested in CD-1 mice. In these experiments, dgRNA and mRNA were formulated separately.

LNPs were formulated with *in vitro* transcribed Cas9 mRNA and chemically modified dgRNA (targeting either TTR or FVII), separately, as described in Example 1. The dgRNAs used in this Example were chemically synthesized and sourced from commercial suppliers, with phosphorothioate linkages between the three terminal nucleotides at both the 5' and 3' ends of the crRNA and the trRNA making up the dual guide. The components of each LNP preparation (LNP-093, -094, -095, -096, and -097) include a CCD lipid (Lipid A) (45 mol-%), cholesterol (44 mol-%), DSPC (9 mol-%), and PEG2k-DMG (2 mol-%). Details for these formulations are provided in Table 1, including average particle size, polydispersity, and encapsulation efficiency. Two different dosing regimens were employed: (1) combining the mRNA-LNP formulation (LNP-097) and a dgRNA-LNP formulation (LNP-093, -094, -095 or -096) together in equal parts (by weight of RNA) and dosing the combined formulation on two consecutive days (each day dosed at 1 mg /kg of each RNA component formulation, for a total of 2 mg/kg); or (2) dosing the mRNA-LNP (LNP-097) four hours prior to dosing a dgRNA-LNP

(LNP-093, -094, -095, and -096), on two consecutive days (each formulation dosed at 1 mg/kg). The animals were euthanized 5 days following the first dose in each group. In addition to control group comparisons (animals receiving PBS), each experimental group had an internal sequencing control, and PCR reactions for NGS analysis, as described in Example 1, were run for both targets in each animal (n=3 for each group). Genomic DNA from liver was isolated and analyzed by NGS, as described in Example 1.

As shown in Figs. 3A and 3B, *in vivo* editing (approximately 1.8% editing - 2.8% editing) was observed in the livers of animals that received LNPs targeting FVII using either a co-dosing (A1 (LNP-093/-097) or A2 (LNP-094/-097)) or pre-dosing (A3 (LNP-093/-097)) dosing regimen. Animals that received LNPs targeting TTR showed approximately 2% - 4.5% editing in the livers of animals receiving dgRNA co-dosed with Cas9 mRNA (B1 (LNP-095/LNP-097) or B2 (LNP-096/-097)) or when pre-dosed (B3 (LNP-095/-097) or B4 (LNP-096/-097)). Serum and plasma analyses were conducted for all of the animals, as described in Example 1, with none of the animals displaying statistically significant differences (as compared to animals administered PBS) in either total serum levels of TTR or plasma FVII activity (not shown).

Example 5. *In vitro* and *in vivo* delivery and editing using dgRNA-LNPs and IVT sgRNA-LNPs.

The efficacy of LNPs comprising chemically modified dgRNA and LNPs comprising *in vitro* transcribed (IVT) sgRNA were tested in the context of co-dosing with Cas9 mRNA-LNPs.

LNP-115, -116, -117, -120, -121, and -123 were formulated according to Example 1, and the details about the specific formulations are provided in Table 1. The formulations of this Example were tested for delivery to Neuro2A cells, using the procedure as described in Example 1.

LNP-121 (gRNA) and LNP-120 (Cas9 mRNA) were mixed together and administered at gRNA concentrations of 152 nM, 76 nM, and 38 nM, plus mRNA at 570 ng, 285 ng, and 142 ng per well, respectively; LNP-123 (gRNA) and LNP-120 were mixed together and administered at gRNA concentrations of 156 nM, 78 nM, and 39 nM, plus mRNA at 528 ng, 264 ng, and 132 ng per well, respectively; and LNP-116 (gRNA) was mixed with LNP-120 (Cas9 mRNA) and administered at gRNA concentrations of 124 nM, 62 nM, and 31 nM, plus mRNA at 460 ng, 229 ng, and 114 ng per well, respectively. LNP-121 was administered at gRNA concentrations of 198 nM, 99 nM, and 49.5 nM; LNP-123 was administered at gRNA concentrations of 189 nM, 94.5 nM, and 47 nM; and LNP-116 was administered at gRNA concentrations of 124 nM, 62 nM, and 31 nM, and the Cas9 mRNA (100 ng per well) was added by LF2K to the experiments according to the manufacturer's instructions. Editing was observed in samples involving both co-dosing IVT sgRNA-LNP (LNP-123) with Cas9 mRNA using either an LNP (LNP-120) or LF2K, as well as with chemically modified dgRNA at the tested concentrations of gRNA (LNP-121).

The formulations in this Example were then tested *in vivo.* Animals were administered, as described in Example 1, a mixture of Cas9 mRNA-LNP and one of the gRNA-LNPs (animals were administered 1 mg/kg of each formulation, each day) on two consecutive days, with one formulation being dosed on one day only (n=5 for each group). The animals were euthanized 6 days following the first dose (or 7 days with the group receiving only a single dose), and liver tissues were collected and analyzed by NGS, as described in Example 1.

As shown in Fig. 4A, single and dual doses were effective for delivery. There is no statistical difference between the group that received one dose on a single day (LNP-121 and LNP-120; C in Fig. 4A) and the group that received two doses on consecutive days when co-dosing Cas9 mRNA-LNP with chemically modified dgRNA-LNPs (LNP-116 and LNP-120, A in Fig. 4A; LNP-121 and LNP-120, B in Fig. 4B). Animals that received Cas9 mRNA-LNP co-dosed with unmodified IVT sgRNA-LNPs (LNP-123 and LNP-120, D in Fig. 4B) displayed relatively lower levels of editing as compared to the dgRNA-LNPs used in this Example (Fig. 4B). These experiments establish that LNPs comprising modified dgRNA or IVT sgRNA allow for *in vitro* and *in vivo* editing when co-dosed with Cas9 mRNA-LNPs. The levels of *in vivo* editing observed when using LNPs comprising IVT sgRNA in this experiment may be affected by impurities in the isolated IVT sgRNA.

Example 6. *In vitro* and *in vivo* delivery and editing using modified dgRNA-LNPs or modified sgRNA-LNPs.

LNPs comprising chemically modified dgRNA and LNPs comprising chemically modified sgRNA were also tested by co-dosing with Cas9 mRNA-LNPs.

LNPs were formulated with chemically modified dgRNA (targeting TTR or FVII), chemically modified sgRNA (targeting TTR or FVII), and IVT Cas9 mRNA, as described in Example 1. The dgRNA in this Example were chemically synthesized and sourced from commercial suppliers, with phosphorothioate linkages between the three terminal nucleotides at both the 5' and 3' ends of both the crRNA and the trRNA making up the dual guide. The sgRNA in this Example was also chemically synthesized and sourced from a commercial supplier with 2'-O-methyl modifications and phosphorothioate linkages at and between the three terminal nucleotides at both the 5' and 3' ends of the sgRNA. The components of each LNP preparation include a CCD lipid (Lipid A, 45 mol-%), cholesterol (44 mol-%), DSPC (9 mol-%),

and PEG2k-DMG (2 mol-%). LNP-136, -137, -138, -139, and -140 were used in these experiments. Details are provided in Table 1, including average particle size, polydispersity, and encapsulation efficiency.

The formulations of this Example were tested for delivery to Neuro2A cells, as described in Example 1. Cells were co-transfected with guide LNP and Cas9 mRNA LNP by adding each formulation directly to the cell culture media, resulting in the concentrations listed in Table 2, and percent editing was determined using the T7E1 assay, as described in Example 1. In Fig. 5, the labels represent the formulations, as described in Table 2.

**Table 2. Formulations Employed in Example 6**

| Figure Label | guide LNP | Guide Concentration (nM) | Cas9 mRNA (ng) LNP 140 |
|---|---|---|---|
| A1 | LNP-136 | 66 | 245 |
| A2 | | 33 | 122.5 |
| A3 | | 16.5 | 61 |
| B1 | LNP-139 | 54 | 175 |
| B2 | | 27 | 87.5 |
| B3 | | 13.5 | 43 |
| C1 | LNP-137 | 49 | 343 |
| C2 | | 24.5 | 171.5 |
| C3 | | 12 | 85 |
| D1 | LNP-138 | 74 | 239 |
| D2 | | 37 | 119.5 |
| D3 | | 18.5 | 60 |

Large increases in editing were measured for both targets when using chemically modified sgRNA-LNPs co-transfected with Cas9 mRNA-LNPs, when compared to the dgRNA-LNP formulations that were tested (Fig. 5). The chemically modified sgRNA-LNPs co-transfected with Cas9 mRNA-LNPs (LNP-138 and -139, Fig. 5), resulted in approximately 35-50% and 65-70% editing in cells when targeting FVII and TTR, respectively.

The formulations in this Example were then tested *in vivo.* Animals were administered, as described in Example 1, a mixture of Cas9 mRNA-LNP (LNP-140) and one of the gRNA-LNPs tested (LNP-136, -137, -138, and -139), where each component formulation was dosed at 1 mg/kg/day (for a total of 2 mg/kg/day), on two consecutive days (n=5 for each group). The animals were euthanized 6 days following the first dose, and liver tissues were collected and analyzed by NGS, as described in Example 1.

In Fig. 6, A1 and A2 represent administration of the mixture of formulations LNP-136 and LNP-140; B1 and B2 represent administration of the mixture of formulations LNP-139 and LNP-140; C1 and C2 represent administration of the mixture of formulations LNP-137 and LNP-140; and D1 and D2 represent administration of the mixture of formulations LNP-138 and LNP-140. As shown in Fig. 6, increases in editing (approximately 10% editing-32% editing) were measured for both targets when using chemically modified sgRNA-LNPs co-dosed with Cas9 mRNA-LNPs, as compared to the amount of editing the use of dgRNA-LNP formulations resulted in (approximately 2% editing-5% editing). Animals receiving the dgRNA-LNP formulations targeting TTR resulted in less than 5% editing across two liver biopsies, while sgRNA-LNP formulations resulted in average percent editing of over 20% (with a peak of over 30% in one animal). Similarly, animals receiving the dgRNA-LNP formulations targeting FVII displayed less than 3% editing across two liver biopsies, while sgRNA-LNP formulations resulted in average percent editing of approximately 10% (with a peak of over 12% in one animal).

These results established that LNPs separately formulated with Cas9 mRNA and gRNA (both dgRNA and sgRNA) achieve editing *in vivo* when co-dosed together, and the LNPs achieve editing *in vivo* when Cas9 mRNA-LNPs are dosed prior to gRNA-LNPs.

Example 7. *In vitro* and *in vivo* delivery and editing using LNPs comprising sgRNA co-formulated with Cas9 mRNA.

LNPs formulated for delivery of Cas9 mRNA and sgRNA encapsulated together in an LNP composition also effectively deliver the CRISPR/Cas components.

LNPs were formulated with IVT Cas9 mRNA together with chemically modified sgRNA (targeting TTR or FVII), as described in Example 1. The ratio of mRNA:sgRNA was approximately 1:1, by weight of the RNA component. The sgRNA

in this Example was chemically synthesized and sourced from a commercial supplier, with 2'-O-methyl modifications and phosphorothioate linkages at and between the three terminal nucleotides at both the 5' and 3' ends of the sgRNA, respectively. The components of each LNP preparation include a CCD lipid (Lipid A, 45 mol-%), cholesterol (44 mol-%), DSPC (9 mol-%), and PEG2k-DMG (2 mol-%). LNP-152, - 153, -154, and -155 were used in these experiments, and details of these formulations are provided in Table 1, including average particle size, polydispersity, and encapsulation efficiency.

The formulations of this Example were tested for delivery to Neuro2A cells, as described in Example 1. Cells were transfected with the formulations and percent editing was determined using NGS, as described in Example 1.

In Fig. 7, A represents administration of LNP-152; B represents administration of LNP-153; C represents administration of LNP-154; D represents administration of LNP-155; and E represents administration of a combination of LNP-152 and LNP-153. Each formulation was administered at 300 ng Cas9 mRNA and 93 nM gRNA; 100 ng Cas9 mRNA and 31 nM gRNA; 30 ng Cas9 mRNA and 10 nM gRNA; and 10 ng Cas9 mRNA and 3 nM gRNA. As shown in Fig. 7, administration of each LNP formulation resulted in robust editing efficiency, with some formulations resulting in more than 80% of cells being edited (LNP-153 and -155). Cells were treated with a combination of two of the LNP formulations (LNP-152 and LNP-153) targeting FVII, which also resulted in efficient editing (approximately 70-90% editing), as well as excision of a portion of the FVII gene lying between the two sgRNAs delivered (Fig. 7, and data not shown).

The formulations in this Example were also tested *in vivo.* Animals were dosed as described in Example 1 (n=4 for each group). Treatment groups receiving LNPs targeting FVII received a single dose (at 2 mg/kg), with one of the treatment groups having received a single, combined dose (LNP-152 and LNP-153) of 2 mg/kg *(e.g.,* 1 mg/kg of each of LNP-152 and LNP-153). The treatment groups receiving LNPs targeting TTR received two doses (each at 2 mg/kg), wherein the second dose was delivered four days after the first dose (*i.e.,* dose 1 on day 1, dose 2 on day 5). Animals in all groups were euthanized 8 days following the first dose with blood and liver tissues collected and analyzed as described in Example 1. Each formulation was administered to four animals.

In Figs. 8A, 8B, and 9, A represents administration of LNP-152; B represents administration of LNP-153; and C represents administration of a combination of LNP-152 and LNP-153. Each formulation was tested in four animals. As shown in Fig. 8A and 8B, each LNP formulation that was tested resulted in robust *in vivo* editing efficiencies. For animals treated with LNP formulations targeting a TTR sequence, more than 50% of liver cells from each biopsy for some animals displayed indels at the target site, with overall averages (across all biopsies of all animals) for each treatment group of 45.2 $\pm$ 6.4% (LNP-154) and 51.1 $\pm$ 3.7% (LNP-155) (Fig. 8A).

Animals treated with LNPs targeting an FVII sequence displayed a range of percentage editing in liver biopsies, with a maximum observed editing of greater than 70% of liver cells being edited from biopsy samples (*e.g.*, having either an indel or excision event at or between the target site(s)) for one animal receiving both LNP formulations targeting an FVII sequence. Overall averages (across all biopsies of all animals) for each treatment group (LNP-152, LNP-153, and LNP-152 and LNP-153) were 16.9 $\pm$ 6.5%, 38.6 $\pm$ 13.2%, and 50.7 $\pm$ 15.0%, respectively (Fig. 8B). For animals receiving both FVII-targeting LNPs, excision of the intervening genomic DNA between the target sites for each sgRNA was detected by PCR, as were indels at one or both of the target sites (Fig. 9).

In Figs. 10 and 11, A represents administration of LNP-152; B represents administration of LNP-153; C represents administration of LNP-154; D represents administration of LNP-155; and E represents administration of the combination of LNP-152 and LNP-153. The robust *in vivo* editing that was observed when the LNP formulations were administered in this Example also resulted in phenotypic changes. As shown in Fig. 10, large decreases (of up to approximately 75%) in serum TTR levels were observed in animals treated with LNPs targeting a TTR sequence (but not in controls or animals treated with LNPs targeting FVII). Similarly, reduced levels of plasma FVII activity were observed in animals treated with LNPs targeting FVII (but not in controls or animals treated with LNPs targeting TTR) (Fig. 11).

## Example 8. Variation of Formulation Parameters.

LNPs formulated for delivery of Cas9 mRNA and gRNA together in one formulation were tested (1) across a range of doses; (2) with altered ratios of mRNA:gRNA; (3) for efficacy with a single dose versus two doses; and (4) whether the LNPs are taken up by and result in editing in the spleen.

LNPs were formulated with IVT Cas9 mRNA together with chemically modified sgRNA (targeting TTR), as described in Example 1. The ratios tested (by weight of RNA component) of mRNA:sgRNA were approximately 1:1 (LNP-169), approximately 10:1 (LNP-170), or approximately 1:10 (LNP-171). The sgRNA used in this Example comprises 2'-O-methyl modifications and phosphorothioate linkages at and between the three terminal nucleotides at both the 5' and 3' ends of the sgRNA, respectively. The components of each LNP preparation included Lipid A (45 mol-%), cholesterol (44 mol-%), DSPC (9 mol-%), and PEG2k-DMG (2 mol-%). LNP-169, - 170 and LNP-171 were used in these experiments. Details are provided in Table 1, including average particle size, polydispersity, and encapsulation efficiency.

***Dose Response Study***

In this study, animals were dosed on day 1, as described in Example 1, with LNP-169 at doses of 0.1 mg/kg, 0.5 mg/kg, or 2 mg/kg (n=5 for each group). On days 5 and 9 blood was collected for TTR serum level analysis. Liver and spleen were collected at necropsy on day 9 for NGS analysis, as described in Example 1.

As shown in Fig. 12A, administration of all three doses resulted in significant editing efficiency in the liver, with a linear dose response observed having an $r^2$ value of 0.9441. In the highest dose group (2 mg/kg), nearly 60% of liver cells in one animal were edited at the TTR target site, with the group having an average of about 50% of liver cells edited. Each animal that was administered the highest dose also displayed statistically significant reductions in serum TTR levels when measured at days 5 and 9 post-administration, with an average reduction 75% of serum TTR levels (as compared to animals that were administered PBS; Fig. 12B).

***Altering Ratios of mRNA:gRNA***

On day 1, animals were administered, as described in Example 1, LNP-169 at a mRNA:gRNA ratio of 1:1 (*i.e.,* 1 mg/kg mRNA, 1 mg/kg gRNA, for a total RNA dose of 2 mg/kg), LNP-170 at a ratio of 10:1 (*i.e.,* 1.8 mg/kg of mRNA, 0.18 mg/kg of gRNA, for a total RNA dose of 1.98 mg/kg) or LNP-171 at a ratio of 1:10 (*i.e., 0.18* mg/kg mRNA, 1.8 mg/kg gRNA, for a total RNA dose of 1.98 mg/kg) (n=5 for each group). (Note: The group and data receiving a dose with a 1:1 mRNA:gRNA ratio is the same group and data as described in the dose response study in this Example, *supra.*) Blood was collected on days 5 and 9, and the serum TTR levels were measured. Liver and spleen were collected at necropsy on day 9 for NGS analysis, as described in Example 1.

As shown in Fig. 13A, administration of LNP-169 (mRNA:gRNA ratio of 1:1) resulted in editing of nearly 60% in one animal at the TTR target site, with the group having an average of about 50% editing. Animals that received 1:10 and 10:1 LNP formulations also demonstrated editing, with the average percent editing for the group receiving LNP-171 showing approximately 32% editing and the group receiving LNP-170 showing approximately 17% editing in this experiment. Additionally, as shown in Fig. 13B, statistically significant reductions in serum TTR levels were detected for each treatment group at day 5 (as compared to PBS control). By day 9, the groups receiving 1:1 mRNA:sgRNA and 1:10 mRNA:sgRNA retained statistically significant reductions in serum TTR levels.

*Single Dose versus Two Doses*

In this study, one group of animals received a single dose of LNP-169 (at 2 mg/kg) on day 1, while another group received two doses of LNP-169 (each at 2 mg/kg) with the first dose administered on day 1 and the second dose on day 5, administered as described in Example 1 (n=5 for both groups). (Note: The group and data receiving a single dose of LNP-169 is the same group and data as described in the dose response and mRNA:gRNA ratio studies in this Example, *supra*). Blood was collected for TTR serum levels from both groups at day 5 (prior to administration of the second dose for the group receiving the second dose), and again at necropsy on day 9, as described in Example 1.

As shown in Fig. 14A, in the group receiving a single dose of LNP-169, nearly 60% editing of the TTR target site was observed in one animal, with the group having an average of about 50% editing. Similar average numbers were achieved in animals receiving two doses of LNP-169, with lower standard deviation and with the group averaging approximately 55% editing of the TTR target site. As shown in Fig. 14B, both groups displayed significant reductions in serum TTR levels.

***Evaluating Uptake by and Editing in the Spleen***

The spleen from each animal in the above studies (within this Example) were collected at necropsy in order to determine whether the LNPs were directed to and taken up by the spleen, thereby resulting in gene editing. Genomic DNA was extracted from spleen tissues and subjected to NGS analysis as described in Example 1.

In Fig. 15, A represents LNP-169 administered at 2 mg/kg for 2 doses; B represents LNP-169 with a 1:1 ratio of mRNA:gRNA at 0.1 mg/kg as a single dose; C represents LNP-169 with a 1:1 ratio of mRNA:gRNA at 0.5 mg/kg as a single dose; D represents LNP-169 with a 1:1 ratio of mRNA:gRNA at 2 mg/kg as a single dose; E represents LNP-170 with a 10:1 ratio of mRNA:gRNA at 2 mg/kg as a single dose; and F represents LNP-171 with a 1:10 ratio of mRNA:gRNA at 2 mg/kg as a single dose. As shown in Fig. 15, significantly less editing (less than approximately 2% of cells) was observed in the spleens of these animals as compared to their livers. Editing of approximately 50% in the liver was observed (e.g., in those groups receiving LNP-169) in these studies. These results indicate that the LNPs provided herein are largely targeted to the liver, as opposed to the spleen.

Example 9. Comparative *in vivo* study between (1) modified dgRNA-LNPs co-dosed with Cas9 mRNA-LNPs and (2) LNPs comprising Cas9 mRNA and modified dgRNA together in one formulation.

LNPs formulated for delivery of Cas9 mRNA and modified dgRNA either as separate LNPs or together in one formulation effectively deliver the CRISPR/Cas components.

LNPs were formulated with IVT Cas9 mRNA either together with (LNP-174, - 175) or separately from (LNP-172, -173) chemically modified dgRNA (targeting TTR), as described in Example 1. Both the crRNA and the trRNA making up the dgRNA in this Example comprised phosphorothioate linkages between the three terminal nucleotides at both the 5' and 3' ends of each RNA. The components of each LNP preparation include a CCD lipid (Lipid A, 45 mol-%), cholesterol (44 mol-%), DSPC (9 mol-%), and PEG2k-DMG (2 mol-%). LNP-172, -173, -174, and -175 were used in these experiments. The compositions of LNP-174 and LNP-175 were identical, except that the crRNA and trRNA making up the dgRNA in LNP-175 were first pre-annealed to one another prior to being formulated with the LNP. This was accomplished by first incubating the crRNA and trRNA together at 95° C for 10 minutes before cooling to room temperature and proceeding to formulation, as previously described. Other details concerning the LNPs are provided in Table 1, including average particle size, polydispersity, and encapsulation efficiency.

Animals were dosed with each LNP at 2 mg/kg as described in Example 1 (n=5 for each group). Livers were collected at necropsy 8 days post-administration, and genomic DNA was isolated and subjected to NGS analysis, as described in Example 1.

In Fig. 16, A represents administration of the dgRNA split-formulation (LNP-172 and LNP-173; B represents administration of the dgRNA co-formulation (LNP-174); and C represents administration of the formulation wherein the dgRNA was pre-annealed (LNP-175). As shown in Fig. 16, editing was detected in livers from each group (with approximately 4-6% editing). Animals that received LNP that was co-formulated with Cas9 mRNA and dgRNA together and animals that received the mRNA and dgRNA from separately formulated LNPs showed editing. The editing efficiencies measured using LNPs formulated with dgRNA (either together with or separately from Cas9 mRNA) are substantially lower than those detected using LNPs formulated with sgRNA (*see, e.g.,* Examples 6-8).

Example 10. ApoE binding of LNPs and transfection of primary hepatocytes.

As demonstrated in Example 8, LNPs provided herein are effectively taken up by the liver, and only to a minor extent by the spleen. This Example provides data regarding ApoE-mediated uptake in primary hepatocytes and provides an assay for testing LNP-ApoE binding which demonstrated that the LNPs bind ApoE.

***LNP delivery to primary hepatocytes***

In addition to other proteins, serum provides a source of ApoE in culture media, and therefore whether the LNPs require serum (*e.g.*, as a source of ApoE) for uptake into primary hepatocytes was tested. This was accomplished by adding LNPs to primary hepatocytes *in vitro,* with and without the presence of serum.

LNPs were delivered to mouse primary hepatocytes as described in Example 1. In the absence of any serum, no editing was detected by T7E1 assay for any LNP tested (data not shown). However, when LNPs were incubated with 3% mouse serum prior to transfection, LNPs were taken up by the hepatocytes resulting in editing. A representative data set is shown in Fig. 17. In this experiment, LNP-169 (targeting TTR) was pre-incubated in 3% mouse serum, and then added to mouse primary hepatocytes at various concentrations. The labels in Fig. 17 are defined in Table 3 and describe the concentration of the LNP-169 that was administered. As shown in Fig. 17, the addition of serum resulted in a dose dependent increase in editing at the TTR target site as measured by NGS. These results suggest that ApoE present in the serum mediates LNP uptake in hepatocytes.

**Table 3. Concentration of LNP-169 Administered**

| Label | nM gRNA | ng Cas9 mRNA |
|---|---|---|
| **A** | 30.8 | 99.9 |
| **B** | 10.3 | 33.3 |
| **C** | 3.4 | 11.1 |
| **D** | 1.1 | 3.7 |
| **E** | 0.4 | 1.2 |
| **F** | 0.1 | 0.4 |

(continued)

| Label | nM gRNA | ng Cas9 mRNA |
|---|---|---|
| G | 0.0 | 0.1 |

*ApoE binding assay*

LNPs were incubated with recombinant ApoE3, the most common form of ApoE, and then separated with a heparin affinity column using a salt gradient on an HPLC. There were two peak groups in the HPLC run, corresponding to LNPs bound to ApoE3 and unbound LNPs. Un-bound is free LNP that did not bind with ApoE3 and flowed freely though the heparin column. Bound was a peak with a longer retention time representing the LNP/ApoE3 complex that was bound to the heparin column and was eluted in the salt gradient. To calculate the binding, the percentage of the bound peak area was calculated by dividing the peak area corresponding to the LNPs bound to ApoE3 and dividing that number by the sum of the area of both peaks.

LNPs were formulated with Cas9 mRNA and chemically modified sgRNA, as described in Example 1. The sgRNAs used in this Example were chemically synthesized and sourced from commercial suppliers, with 2'-O-methyl modifications and phosphorothioate linkages at and between the three terminal nucleotides at both the 5' and 3' ends of the sgRNA, respectively. The components of each LNP preparation (LNP-169 and LNP-171) include Lipid A (45 mol-%), cholesterol (44 mol-%), DSPC (9 mol-%), and PEG2k-DMG (2 mol-%). Details for these formulations are provided in Table 1, including average particle size, polydispersity, and encapsulation efficiency. Using a stock ApoE3 (Recombinant Human Apolipoprotein E3 , R&D Systems, cat #4144-AE-500) solution at 0.5 mg/mL, ApoE3 was added to LNP samples at 25 µg/mL, 50 µg/mL, 100 µg/mL, 200 µg/mL, and 300 µg/mL. The samples were incubated overnight at room temperature.

Two buffers were prepared (500 mL each); Buffer A is a 20 mM Tris buffer, adjusted to pH 8.0 and Buffer B is a 20 mM Tris buffer, with 1 M NaCl, adjusted to pH 8.0. The gradient and flow rate for the HPLC analysis is as described below.

| No | Time | Flow [ml/min] | %B | Curve |
|---|---|---|---|---|
| 1 | 0.000 | Run | | |
| 2 | 0.000 | 0.200 | 0.0 | 5 |
| 3 | 8.000 | 0.200 | 0.0 | 5 |
| 4 | 10.000 | 0.200 | 80.0 | 5 |
| 5 | 13.000 | 0.200 | 80.0 | 5 |
| 6 | 15.000 | 0.200 | 100.0 | 5 |
| 7 | 18.000 | 0.200 | 100.0 | 5 |
| 8 | 19.000 | 0.200 | 0.0 | 5 |
| 9 | 25.000 | 0.200 | 0.0 | 5 |
| 10 | *New Row* | | | |
| 11 | 25.000 | Stop Run | | |

After incubating the samples overnight, each sample was analyzed by HPLC and the percent area of the bound peak was calculated as previously described.

As shown in Fig. 18, with increasing amounts of ApoE3, more LNP (both LNP-169 (represented by the dashed line) and -171 (represented by the solid line)) was bound to the heparin column, *e.g.,* as a result of being bound to ApoE3. These results indicate that the LNPs bind ApoE3.

Example 11. *In vitro* and *in vivo* delivery and editing using LNPs with sgRNA expressed from DNA expression cassettes.

This example demonstrates gene editing using LNPs loaded with Cas9 mRNA and an expression cassette encoding an sgRNA.

***LNP delivery in vitro***

Amplicons encoding sgRNA were prepared by PCR amplification of a DNA sequence containing a U6 promoter linked to as sgRNA targeting mouse TTR. Each primer contained an inverted dideoxyT nucleotide at the 5' end to prevent integration of the DNA amplicon into genomic DNA. PCR product was purified by phenol/chloroform extraction followed by ethanol precipitation. The DNA pellet was dried and resuspended in TE buffer.

LNPs were formulated with IVT Cas9 mRNA ("mRNA-LNP" or LNP-178) or the sgRNA expression cassette ("DNA-LNP" or LNP-176) as described in Example 1. IVT Cas9 mRNA and the sgRNA expression cassette were also separately formulated with Lipofectamine 2000 (Thermo Fisher) according to manufacturer's instructions ("mRNA LF2K" or "DNA LF2K", respectively). Formulations were applied to mouse Neuro2A cells (100 ng Cas9 mRNA and 100 ng sgRNA expression cassette) by diluting directly into the cell culture media in each well according to the following regimens:

- Co-delivery of Cas9 mRNA and sgRNA expression cassette;
- sgRNA expression cassette administered 2 hours prior to Cas9 mRNA; and
- Cas9 mRNA administered 2 hours prior to sgRNA expression cassette.

Cells were incubated for 48 hours post transfection, and cell lysates were analyzed by T7E1 analysis as described in Example 1. As shown in Fig. 19, higher percentages of TTR editing were observed when both the mRNA and DNA components were formulated in LNPs, compared to when one component or the other was formulated with Lipofectamine 2000.

Example 12. Editing *in vitro* vs. *in vivo.*

Cas9 mRNA and chemically modified sgRNA targeting different mouse TTR sequences were formulated and dosed to mice (2 mg/kg) as described in Example 1. The same LNP preparations were used to transfect mouse primary hepatocytes *in vitro.* The sgRNA in this Example was chemically synthesized and sourced from a commercial supplier, with 2'-O-methyl modifications and phosphorothioate linkages at and between the three terminal nucleotides at both the 5' and 3' ends of the sgRNA, respectively.

**Table 4. Formulations Employed in Example 12**

| LNP # | Target | RNA Cargo | CCD Lipid | Stealth Lipid | Avg. Particle Size (nm) | pdi | EE (%) |
|---|---|---|---|---|---|---|---|
| LNP257 (TTR686) | TTR | sg009 + Cas9 mRNA | Lipid A | PEG2k-DMG | 77.86 | 0.015 | 99% |
| LNP258 (TTR705) | TTR | sg016 + Cas9 mRNA | Lipid A | PEG2k-DMG | 88.24 | 0.033 | 99% |
| LNP259 (TTR268) | TTR | cr013*** + Cas9 mRNA | Lipid A | PEG2k-DMG | 81.74 | 0.070 | 99% |
| LNP260 (TTR269) | TTR | cr018*** + Cas9 mRNA | Lipid A | PEG2k-DMG | 86.94 | 0.049 | 99% |
| LNP262 (TTR271) | TTR | cr021*** + Cas9 mRNA | Lipid A | PEG2k-DMG | 86.48 | 0.078 | 98% |
| LNP263 (TTR272) | TTR | cr009*** + Cas9 mRNA | Lipid A | PEG2k-DMG | 86.81 | 0.047 | 98% |
| LNP264 (TTR273) | TTR | cr010*** + Cas9 mRNA | Lipid A | PEG2k-DMG | 86.86 | 0.032 | 98% |
| LNP265 (TTR274) | TTR | cr007*** + Cas9 mRNA | Lipid A | PEG2k-DMG | 86.85 | 0.049 | 97% |
| LNP266 (TTR275) | TTR | cr019*** + Cas9 mRNA | Lipid A | PEG2k-DMG | 87.77 | 0.050 | 97% |
| LNP267 (TTR276) | TTR | cr008*** + Cas9 mRNA | Lipid A | PEG2k-DMG | 81.29 | 0.081 | 98% |

(continued)

| LNP # | Target | RNA Cargo | CCD Lipid | Stealth Lipid | Avg. Particle Size (nm) | pdi | EE (%) |
|---|---|---|---|---|---|---|---|
| LNP268 (TTR277) | TTR | cr011*** + Cas9 mRNA | Lipid A | PEG2k-DMG | 83.80 | 0.053 | 97% |
| *** = single guide format with 2'-O-methyl modifications and phosphorothioate linkages at and between the three terminal nucleotides at the 5' and 3' ends | | | | | | | |

For the *in vitro* studies, a 7 point semi-log dose response was performed (starting at 100 ng/well). 48 hours post transfection, genomic DNA was harvested and editing percent was measured by NGS. Figure 20 shows the editing percentages for these *in vitro* and *in vivo* experiments, demonstrating that editing efficiency is correlated between primary hepatocytes in culture and *in vivo.*

Because NGS provides specific sequencing results in addition to overall editing efficiency, sequence-specific editing patterns were compared to Neuro 2A cells. Figure 21 shows representative data demonstrating that insertion and deletion patterns differ significantly between mouse Neuro2A cells (transfected with Cas9 mRNA and gRNA) and mouse primary hepatocytes (transfected with LNPs containing Cas9 mRNA and gRNA). Mouse primary hepatocytes yielded editing patterns very similar to those observed *in vivo* (transfected with LNPs containing Cas9 mRNA and gRNA) (Figure 22). As shown in Figure 22, 53.2% of the edits measured in mouse primary hepatocytes were deletions (primarily 1 bp deletions) and 16.8% were insertions (primarily 1 bp insertions), for a total of 70% editing. Out of the total of 70% editing, 64.5% of the edits resulted in a frameshift mutation, which represents ~92% of the total edits measured (not shown). Similarly, representative data is shown for the editing percentages and edit types as observed from LNP-based delivery of Cas9 mRNA and gRNA to mouse liver cells *in vivo:* 46.6% of the edits measured in mouse liver cells *in vivo* were deletions (again, primarily 1 bp deletions) and 12.9% were insertions (again, primarily 1 bp insertions), for a total of 59.5% editing. Out of the total of 59.5% editing, 57.4% of the edits resulted in a frameshift mutation, representing ~96% of the edits measured *in vivo* (not shown).

Example 13. Pharmacokinetics of CRISPR/Cas9 components delivered by LNP.

LNP-294, containing Cas9 mRNA and sgRNA targeting mouse TTR, was formulated as described in Example 1. The ratio of mRNA to guideRNA was confirmed by HPLC. Animals were dosed with each LNP at 2 mg/kg as described in Example 1 (n=3 for each group), and taken down at the following time points: 5 min, 15 min, 30 min, 60 min, 2 hr, 4 hr, 6 hr, 12 hr, 24 hr, 72 hr, and 7 days. At necropsy, plasma, liver, and spleen were collected for qPCR analysis of levels of Cas9 mRNA and guideRNA. Figure 23 shows plasma concentrations of these components, Figure 24 shows concentrations in liver, and Figure 25 shows concentrations in spleen. The following pharmacokinetic parameters were calculated for plasma concentrations:

**Table 5. Pharmacokinetic parameters**

| Parameter | sg009 (sgRNA) | Cas9 (mRNA) |
|---|---|---|
| Dose (mg/kg) | 1 (25mcg/ms) | 1 (25mcg/ms) |
| $C_{max}$ (mcg/mL) | 39.049 | 18.15 |
| $T_{max}$ (hr) | 0.083 | 0.5 |
| $T_{1/2}$ (hr) | 2.32 | 2.54 |
| Vd (mL/kg) | 195.6 | 208.4 |
| Cl (mL/hr*kg) | 58.4 | 56.7 |
| $AUC_{last}$ (mcg*hr/mL) | 21.99 | 18.39 |

Figure 26A shows the relative ratios of the sgRNA to Cas9 mRNA in plasma and tissue.

Cytokine induction in the treated mice was also measured. For this analysis, approximately 50-100 µL of blood was collected by tail vein nick for serum cytokine measurements. Blood was allowed to clot at room temperature for approximately 2 hours, and then centrifuged at 1000xg for 10 minutes before collecting the serum. A Luminex based magnetic bead multiplex assay (Affymetrix ProcartaPlus, catalog number Exp040-00000-801) measuring IL-6, TNF-alpha, IFN-alpha, and MCP-1 was used for cytokine analysis in collected in samples. Kit reagents and standards were prepared as directed in the manufacturer's protocol. Mouse serum was diluted 4-fold using the sample diluent provided and 50 μL was added to wells containing 50 μL of the diluted antibody coated magnetic beads. The plate was incubated for 2 hours at room temperature and then washed. Diluted biotin antibody (50 μL) was added to the beads and incubated for 1 hour at room temperature. The beads were washed again before adding 50 μL of diluted streptavidin-PE to each well, followed by incubation for 30 minutes. The beads were washed once again and then suspended in 100 μL of wash buffer and read on the Bio-Plex 200 instrument (Bio-Rad). The data was analyzed using Bioplex Manager ver. 6.1 analysis package with cytokine concentrations calculated off a standard curve using a five parameter logistic curve fit. Figure 27 shows plasma cytokine levels for the treated mice over time. As shown in Figure 27, each of the cytokines had a measureable increase between 2-4 hours post treatment, and each returned to baseline by 12-24 hours.

Three different guide sequences were separately formulated, according to Example 1, and injected into mice (n=3) to determine the pharmacokinetic profile of Lipid A. Levels of Lipid A in mouse liver and plasma were measured by LC/MS. Figure 26B shows the plasma and liver concentrations of Lipid A over time. $T_{max}$ in liver was achieved within 30 minutes of administration, whereas $T_{1/2}$ in plasma and liver were achieved within approximately 5-6 hours of LNP administration.

Example 14. Duration of Response for *in vivo* editing

Cas9 mRNA and chemically modified sgRNA targeting a mouse TTR sequence were formulated as described in Example 1:

**Table 6. Formulation information for LNP 402.**

| LNP # | Target | RNA Cargo | CCD Lipid | Stealth Lipid | Avg. Particle Size (nm) | pdi | EE (%) |
|---|---|---|---|---|---|---|---|
| LNP402 | TTR | sg282 + Cas9 mRNA | Lipid A | PEG2k-DMG | 82.3 | 0.171 | 97.43 |

The LNPs were dosed to mice (single dose at 3 mg/kg, 1 mg/kg, or 0.3 mg/kg) as described in Example 1. Cohorts of mice were measured for serum TTR levels at 1, 2, 4, 9, 13, and 16 weeks post-dosing, and liver TTR editing at 1, 2, 9, and 16 weeks post-dosing. To measure liver TTR editing, tissue sample from the liver was collected from the median lobe from each animal of the particular cohort for DNA extraction and analysis. The genomic DNA was extracted from 10 mg of tissue using a bead-based extraction kit, MagMAX-96 DNA Multi-Sample Kit (ThermoFisher, Catalog No. 4413020) according to the manufacturer's protocol, which includes homogenizing tissue in lysis buffer (approximately 400 μL/10 mg tissue) and precipitating the DNA. All DNA samples were normalized to 100 ng/μL concentration for PCR and subsequent NGS analysis.

The sgRNA in this example was chemically synthesized and sourced from a commercial supplier, with 2'-O-methyl modifications and phosphorothioate linkages as represented below (m = 2'-OMe; * = phosphorothioate):
sg282:

mU*mU*mA*CAGCCACGUCUACAGCAGUUUUAGAmGmCmUmAmGmAmAm

AmUmAmGmCAAGUUAAAAUAAGGCUAGUCCGUUAUCAmAmCmUmUmGm

AmAmAmAmAmGmUmGmGmCmAmCmCmGmAmGmUmCmGmGmUmGmCmU*

mU*mU*mU.

Figure 28 shows mouse serum TTR levels over time, and Figure 29A shows corresponding editing percentages as measured by NGS. Figure 29B shows both mouse serum TTR levels over time and the corresponding editing percentages as measured by NGS, through 16 weeks post-dosing.

Example 15. Formulations using mRNA preparations

Cas9 mRNA was prepared as described in Example 1 using both the precipitation-only and HPLC purification protocols LNP was formulated using the HPLC purified mRNA (LNP492), and compared to LNP formulated using the

precipitation-only processed mRNA (LNP490, LNP494). The Cas9 mRNA cargo of LNP494 was prepared using a differnent synthesis lot of precipitation-only mRNA.

**Table 7. Formulations employed in Example 15.**

| LNP # | Target | RNA Cargo | CCD Lipid | Stealth Lipid | Avg. Particle Size (nm) | pdi | EE (%) |
|---|---|---|---|---|---|---|---|
| LNP490 | TTR | sg282 + Cas9 mRNA | Lipid A | PEG2k-DMG | 81.9 | 0.194 | 98.24 |
| LNP492 | TTR | sg282 + Cas9 mRNA | Lipid A | PEG2k-DMG | 85.9 | 0.207 | 96.33 |
| LNP494 | TTR | sg282 + Cas9 mRNA | Lipid A | PEG2k-DMG | 70.2 | 0.153 | 96.48 |

Mice were dosed with 0.5 or 1 mg/kg of each formulation as described in Example 1, LNP Delivery *in vivo.* The sgRNA used in this Example was sg282, as described in Example 14.

Figure 30 shows mouse serum cytokine activity at 4 hours post dosing. Figure 31 shows mouse serum TTR concentration levels, and Figure 32 shows mouse liver TTR editing levels.

**Table 8. Figure Labels in Figures 30, 31, and 32.**

| Figure Label | LNP | Dose (mg/kg) |
|---|---|---|
| Control | N/A (PBS) | N/A |
| A1 | LNP490 | 1 |
| A2 | | 0.5 |
| B1 | LNP492 | 1 |
| B2 | | 0.5 |
| C1 | LNP494 | 1 |
| C2 | | 0.5 |

Example 16. Frozen formulations

LNPs were formulated with a Lipid A to RNA phosphate (N:P) molar ratio of about 4.5. The lipid nanoparticle components were dissolved in 100% ethanol with the following molar ratios: 45 mol-% (12.7 mM) Lipid A; 44 mol-% (12.4 mM) cholesterol; 9 mol-% (2.53 mM) DSPC; and 2 mol-% (0.563 mM) PEG2k-DMG. The RNA cargo were dissolved in 50 mM acetate buffer, pH 4.5, resulting in a concentration of RNA cargo of approximately 0.45 mg/mL. For this study, sg282 described in Example 14 was used.

**Table 9. LNP formulations employed in Example 16.**

| LNP # | Target | RNA Cargo | CCD Lipid | Stealth Lipid | Avg. Particle Size (nm) | pdi | EE (%) |
|---|---|---|---|---|---|---|---|
| LNP493 | TTR | sg282 + Cas9 mRNA | Lipid A | PEG2k-DMG | 69.1 | 0.013 | 97.93 |
| LNP496 | PCSK9 | sg396 + Cas9 mRNA | Lipid A | PEG2k-DMG | 78.6 | 0.150 | 94.45 |

The LNPs (LNP493, LNP496) were formed by microfluidic mixing of the lipid and RNA solutions using a Precision Nanosystems NanoAssemblr™ Benchtop Instrument, according to the manufacturer's protocol. A 2:1 ratio of aqueous to organic solvent was maintained during mixing using differential flow rates. After mixing, the LNPs were collected, diluted in 50 mM Tris buffer, pH 7.5 The formulated LNPs were filtered using a 0.2 μm sterile filter. The resulting filtrate was mixed 1: 1 with 10% w/v sucrose 90 mM NaCl prepared in 50 mM Tris buffer at pH 7.5. The final LNP formulation at 5% w/v sucrose, 45 mM NaCl, 50 mM Tris buffer was stored at 4° C and -80° C for 1.5 days until the day of dosing.

The LNPs were administered to mice at 0.5 and 1 mg/kg (frozen formulation was thawed at 25° C one hour prior to administration). Figure 33 shows mouse serum TTR concentration levels, and Figure 34 shows mouse liver TTR editing levels after dosing.

**Table 10. Figure Labels in Figures 33 and 34.**

| Figure Label | LNP | Dose (mg/kg) |
|---|---|---|
| Control | N/A (PBS) | N/A |
| A1 | LNP493 (4° C storage) | 1 |
| A2 | | 0.5 |
| B1 | LNP493 (-80° C storage) | 1 |
| B2 | | 0.5 |
| C1 | LNP494 (4° C storage) | 1 |
| C2 | | 0.5 |
| D | LNP496 (non-TTR targeting control, targeting mouse PCSK9) | 2 |

The sgRNA in this example was chemically synthesized and sourced from a commercial supplier, with 2'-O-methyl modifications and phosphorothioate linkages as represented below (m = 2'-OMe; * = phosphorothioate): sg396:

mG*mC*mU*GCCAGGAACCUACAUUGGUUUUAGAmGmCmUmAmGmAmAm

AmUmAmGmCAAGUUAAAAUAAGGCUAGUCCGUUAUCAmAmCmUmUmGm

AmAmAmAmAmGmUmGmGmCmAmCmCmGmAmGmUmCmGmGmUmGmCmU*

mU*mU*mU.

Example 17: Alternative LNP formulation processes

LNPs were formulated with a Lipid A to RNA phosphate (N:P) molar ratio of about 4.5. The lipid nanoparticle components were dissolved in 100% ethanol with the following molar ratios: 45 mol-% (12.7 mM) Lipid A; 44 mol-% (12.4 mM) cholesterol; 9 mol-% (2.53 mM) DSPC; and 2 mol-% (0.563 mM) PEG2k-DMG. The RNA cargo were dissolved in either acetate buffer (in a final concentration of 25 mM sodium acetate, pH 4.5), or citrate buffer (in a final concentration of 25mM sodium citrate, 100 mM NaCl, pH 5) resulting in a concentration of RNA cargo of approximately 0.45 mg/mL. For this study, sg282 described in Example 14 was used.

The LNPs were formed either by by microfluidic mixing of the lipid and RNA solutions using a Precision Nanosystems NanoAssemblr™ Benchtop Instrument, per the manufacturer's protocol, or cross-flow mixing. LNP563 and LNP564 were prepared using the NanoAssemblr preparation, where a 2:1 ratio of aqueous to organic solvent was maintained during mixing using differential flow rates, 8 mL/min for aqueous and 4 mL/min for the organic phase. After mixing, the LNPs were collected and 1: 1 diluted in 50 mM Tris buffer, pH 7.5. The LNPs were dialyzed in 50 mM Tris, pH 7.5 overnight and the next day filtered using a 0.2 μm sterile filter. The resulting filtrate was concentrated and mixed 1: 1 with 10% w/v sucrose 90 mM NaCl prepared in 50 mM Tris buffer at pH 7.5. The final LNP formulation at 5% w/v sucrose, 45 mM NaCl, 50 mM Tris buffer was stored at 4° C and -80° C for 1.5 days until the day of dosing.

**Table 11. Formulation information for LNPs used in Example 17.**

| LNP # | Target | RNA Cargo | CCD Lipid | Stealth Lipid | Avg. Particle Size (nm) | pdi | EE (%) |
|---|---|---|---|---|---|---|---|
| LNP561 | TTR | sg282 + Cas9 mRNA* | Lipid A | PEG2k-DMG | 111.0 | 0.058 | 94.73 |
| LNP562 | TTR | sg282 + Cas9 mRNA* | Lipid A | PEG2k-DMG | 106.2 | 0.047 | 93.68 |
| LNP563 | TTR | sg282 + Cas9 mRNA* | Lipid A | PEG2k-DMG | 72.8 | 0.065 | 94.68 |

(continued)

| LNP # | Target | RNA Cargo | CCD Lipid | Stealth Lipid | Avg. Particle Size (nm) | pdi | EE (%) |
|---|---|---|---|---|---|---|---|
| LNP564 | TTR | sg282 + Cas9 mRNA* | Lipid A | PEG2k-DMG | 123.0 | 0.105 | 88.03 |
| *Cas9 1xNLS, no HA tag. | | | | | | | |

LNP561 and LNP562 were prepared using the cross-flow technique a syringe pump was used with two syringes of RNA at 0.45 mg/mL, one syringe of organice phase containing lipids and one syringe of water. These were mixed at 40 mL/min with variable tubing lengths, aqueous and organic phases were pushed through a 0.5 mm peek cross and this output was introduced into a 1 mm tee connected to the water tubing. LNPs were incubated at room temperature for one hour and then diluted 1: 1 with water. Briefly, LNPs and water were introduced at 25 mL/min in a 1 mm tee by a syringe pump.

For purification and concentration, tangential flow filtration was used. Generally for this procedure, Vivaflow 50 cartridges from Sartorius are primed with 500 mL water and then LNPs are introduced using Pall Minimate systems at feed rate of 60 mL/min. The permeate line is clamped to maintain a fixed flow rate of around 1.7 mL/min. Once the LNPs are concentrated a 15 times volume of either PBS or 5% sucrose, 45 mM NaCl, 50 mM Tris at pH 7.5 is introduced under vacuum at a feed rate of 80 mL/min. The permeate line is clamped to maintain a flow rate of 1.9 mL/min. Once the diafiltration is complete, LNPs are concentrated and collected in a sterile DNase RNase free collection tube and stored at 4° C for PBS formulations, or 4° C or -80° C for TSS (*i.e.,* Tris, sucrose, and salt) formulations until the day of dosing.

The LNPs were administered to mice at 1.0 and 2 mg/kg (frozen formulation was thawed at 25° C one hour prior to administration). Figure 35 shows mouse serum TTR concentration levels, while Figure 36 shows mouse liver TTR editing levels after dosing with the different formulations.

**Table 12. Figure Labels in Figures 35 and 36.**

| Figure Label | LNP | Dose (mg/kg) |
|---|---|---|
| Control | N/A (TSS buffer) | N/A |
| A1 | LNP561 | 2 |
| A2 | | 1 |
| B1 | LNP 562 (LNPs stored at 2-8° C) | 2 |
| B2 | | 1 |
| C1 | LNP562 (LNPs stored at -80° C) | 2 |
| C2 | | 1 |
| D1 | LNP563 | 2 |
| D2 | | 1 |
| E1 | LNP564 | 2 |
| E2 | | 1 |

Example 18 Delivery of LNPs to higher species.

Formulations were prepared similar to those described in Example 14. In certain experiments, the sgRNA was modified with the same chemical modifications as in sg282, but with targeting sequences specific to rat TTR sequences. Efficient editing in rat liver was observed. A 2 mg/kg (total cargo) dose and a 5 mg/kg (total cargo) dose were well tolerated in the experiment. Similar formulations containing mRNA encoding GFP were also well-tolerated by non-human primates at doses of 1 mg/kg and 3 mg/kg.

***Sequences***

Sequences described in the above examples are listed as follows (polynucleotide sequences from 5' to 3'):

Cas9 mRNA (Cas9 coding sequence in bold; HA tag in bold underlined; 2xNLS in underlined):

GGGUCCCGCAGUCGGCGUCCAGCGGCUCUGCUUGUUCGUGUGUGUGUCGUUGCAGGCCUUAUUCGGAUCC**AUGGAUAAGAAGUACUCAAUCGGGCUGGAUAUCGGAACUAAUUCCGUGGGUUGGGCAGUGAUCACGGAUGAAUACAAAGUGCCGUCCAAGAAGUUCAAGGUCCUGGGGAACACCGAUAGACACAGCAUCAAGAAAAAUCUCAUCGGAGCCCUGCUGUUUGACUCCGGCGAAA**

CCGCAGAAGCGACCCGGCUCAAACGUACCGCGAGGCGACGCUACACCC
GGCGGAAGAAUCGCAUCUGCUAUCUGCAAGAGAUCUUUUCGAACGAAA
UGGCAAAGGUCGACGACAGCUUCUUCCACCGCCUGGAAGAAUCUUUCC
UGGUGGAGGAGGACAAGAAGCAUGAACGGCAUCCUAUCUUUGGAAAC
AUCGUCGACGAAGUGGCGUACCACGAAAAGUACCCGACCAUCUACCAU
CUGCGGAAGAAGUUGGUUGACUCAACUGACAAGGCCGACCUCAGAUUG
AUCUACUUGGCCCUCGCCCAUAUGAUCAAAUUCCGCGGACACUUCCUG
AUCGAAGGCGAUCUGAACCCUGAUAACUCCGACGUGGAUAAGCUUUUC
AUUCAACUGGUGCAGACCUACAACCAACUGUUCGAAGAAAACCCAAUC
AAUGCUAGCGGCGUCGAUGCCAAGGCCAUCCUGUCCGCCCGGCUGUC
GAAGUCGCGGCGCCUCGAAAACCUGAUCGCACAGCUGCCGGGAGAGA
AAAAGAACGGACUUUUCGGCAACUUGAUCGCUCUCUCACUGGGACUCA
CUCCCAAUUUCAAGUCCAAUUUUGACCUGGCCGAGGACGCGAAGCUGC
AACUCUCAAAGGACACCUACGACGACGACUUGGACAAUUUGCUGGCAC
AAAUUGGCGAUCAGUACGCGGAUCUGUUCCUUGCCGCUAAGAACCUUU
CGGACGCAAUCUUGCUGUCCGAUAUCCUGCGCGUGAACACCGAAAUAA
CCAAAGCGCCGCUUAGCGCCUCGAUGAUUAAGCGGUACGACGAGCAUC
ACCAGGAUCUCACGCUGCUCAAAGCGCUCGUGAGACAGCAACUGCCUG
AAAAGUACAAGGAGAUCUUCUUCGACCAGUCCAAGAAUGGGUACGCAG
GGUACAUCGAUGGAGGCGCUAGCCAGGAAGAGUUCUAUAAGUUCAUCA
AGCCAAUCCUGGAAAAGAUGGACGGAACCGAAGAACUGCUGGUCAAGC
UGAACAGGGAGGAUCUGCUCCGGAAACAGAGAACCUUUGACAACGGAU
CCAUUCCCCACCAGAUCCAUCUGGGUGAGCUGCACGCCAUCUUGCGGC
GCCAGGAGGACUUUUACCCAUUCCUCAAGGACAACCGGGAAAAGAUCG
AGAAAAUUCUGACGUUCCGCAUCCCGUAUUACGUGGGCCCACUGGCGC
GCGGCAAUUCGCGCUUCGCGUGGAUGACUAGAAAAUCAGAGGAAACCA
UCACUCCUUGGAAUUUCGAGGAAGUUGUGGAUAAGGGAGCUUCGGCA
CAAAGCUUCAUCGAACGAAUGACCAACUUCGACAAGAAUCUCCCAAAC
GAGAAGGUGCUUCCUAAGCACAGCCUCCUUUACGAAUACUUCACUGUC
UACAACGAACUGACUAAAGUGAAAUACGUUACUGAAGGAAUGAGGAAG
CCGGCCUUUCUGUCCGGAGAACAGAAGAAAGCAAUUGUCGAUCUGCUG
UUCAAGACCAACCGCAAGGUGACCGUCAAGCAGCUUAAAGAGGACUAC
UUCAAGAAGAUCGAGUGUUUCGACUCAGUGGAAAUCAGCGGGGUGGA

GGACAGAUUCAACGCUUCGCUGGGAACCUAUCAUGAUCUCCUGAAGAU
CAUCAAGGACAAGGACUUCCUUGACAACGAGGAGAACGAGGACAUCCU
GGAAGAUAUCGUCCUGACCUUGACCCUUUUCGAGGAUCGCGAGAUGAU
CGAGGAGAGGCUUAAGACCUACGCUCAUCUCUUCGACGAUAAGGUCAU
GAAACAACUCAAGCGCCGCCGGUACACUGGUUGGGGCCGCCUCUCCCG
CAAGCUGAUCAACGGUAUUCGCGAUAAACAGAGCGGUAAAACUAUCCU
GGAUUUCCUCAAAUCGGAUGGCUUCGCUAAUCGUAACUUCAUGCAAUU
GAUCCACGACGACAGCCUGACCUUUAAGGAGGACAUCCAAAAAGCACA
AGUGUCCGGACAGGGAGACUCACUCCAUGAACACAUCGCGAAUCUGGC
CGGUUCGCCGGCGAUUAAGAAGGGAAUUCUGCAAACUGUGAAGGUGG
UCGACGAGCUGGUGAAGGUCAUGGGACGGCACAAACCGGAGAAUAUC
GUGAUUGAAAUGGCCCGAGAAAACCAGACUACCCAGAAGGGCCAGAAA
AACUCCCGCGAAAGGAUGAAGCGGAUCGAAGAAGGAAUCAAGGAGCU
GGGCAGCCAGAUCCUGAAAGAGCACCCGGUGGAAAACACGCAGCUGC
AGAACGAGAAGCUCUACCUGUACUAUUUGCAAAAUGGACGGGACAUGU
ACGUGGACCAAGAGCUGGACAUCAAUCGGUUGUCUGAUUACGACGUG
GACCACAUCGUUCCACAGUCCUUUCUGAAGGAUGACUCGAUCGAUAAC
AAGGUGUUGACUCGCAGCGACAAGAACAGAGGGAAGUCAGAUAAUGU
GCCAUCGGAGGAGGUCGUGAAGAAGAUGAAGAAUUACUGGCGGCAGC
UCCUGAAUGCGAAGCUGAUUACCCAGAGAAAGUUUGACAAUCUCACUA
AAGCCGAGCGCGGCGGACUCUCAGAGCUGGAUAAGGCUGGAUUCAUC
AAACGGCAGCUGGUCGAGACUCGGCAGAUUACCAAGCACGUGGCGCA
GAUCUUGGACUCCCGCAUGAACACUAAAUACGACGAGAACGAUAAGCU
CAUCCGGGAAGUGAAGGUGAUUACCCUGAAAAGCAAACUUGUGUCGGA
CUUUCGGAAGGACUUUCAGUUUUACAAAGUGAGAGAAAUCAACAACUA
CCAUCACGCGCAUGACGCAUACCUCAACGCUGUGGUCGGUACCGCCCU
GAUCAAAAAGUACCCUAAACUUGAAUCGGAGUUUGUGUACGGAGACUA
CAAGGUCUACGACGUGAGGAAGAUGAUAGCCAAGUCCGAACAGGAAAU
CGGGAAAGCAACUGCGAAAUACUUCUUUUACUCAAACAUCAUGAACUU
UUUCAAGACUGAAAUUACGCUGGCCAAUGGAGAAAUCAGGAAGAGGCC
ACUGAUCGAAACUAACGGAGAAACGGGCGAAAUCGUGUGGGACAAGG
GCAGGGACUUCGCAACUGUUCGCAAAGUGCUCUCUAUGCCGCAAGUCA
AUAUUGUGAAGAAAACCGAAGUGCAAACCGGCGGAUUUUCAAGGAAU

**CGAUCCUCCCAAAGAGAAAUAGCGACAAGCUCAUUGCACGCAAGAAAG**
**ACUGGGACCCGAAGAAGUACGGAGGAUUCGAUUCGCCGACUGUCGCA**
**UACUCCGUCCUCGUGGUGGCCAAGGUGGAGAAGGGAAAGAGCAAAAA**
**GCUCAAAUCCGUCAAAGAGCUGCUGGGGAUUACCAUCAUGGAACGAUC**
**CUCGUUCGAGAAGAACCCGAUUGAUUUCCUCGAGGCGAAGGGUUACAA**
**GGAGGUGAAGAAGGAUCUGAUCAUCAAACUCCCCAAGUACUCACUGUU**
**CGAACUGGAAAAUGGUCGGAAGCGCAUGCUGGCUUCGGCCGGAGAAC**
**UCCAAAAAGGAAAUGAGCUGGCCUUGCCUAGCAAGUACGUCAACUUCC**
**UCUAUCUUGCUUCGCACUACGAAAAACUCAAAGGGUCACCGGAAGAUA**
**ACGAACAGAAGCAGCUUUUCGUGGAGCAGCACAAGCAUUAUCUGGAUG**
**AAAUCAUCGAACAAAUCUCCGAGUUUCAAAGCGCGUGAUCCUCGCCG**
**ACGCCAACCUCGACAAAGUCCUGUCGGCCUACAAUAAGCAUAGAGAUA**
**AGCCGAUCAGAGAACAGGCCGAGAACAUUAUCCACUUGUUCACCCUGA**
**CUAACCUGGGAGCCCCAGCCGCCUUCAAGUACUUCGAUACUACUAUCG**
**AUCGCAAAAGAUACACGUCCACCAAGGAAGUUCUGGACGCGACCCUGA**
**UCCACCAAAGCAUCACUGGACUCUACGAAACUAGGAUCGAUCUGUCGC**
**AGCUGGGUGGCGAUGGCUCGGCUUACCCAUACGACGUGCCUGACUAC**
**GCCUCGCUCGGAUCGGGCUCC**CCCAAAAAGAAACGGAAGGUG**GACGGA**
**UCC**CCGAAAAAGAAGAGAAAGGUG**GACUCCGGAUGA**GAAUUAUGCAGUC
UAGCCAUCACAUUUAAAAGCAUCUCAGCCUACCAUGAGAAUAAGAGAAA
GAAAAUGAAGAUCAAUAGCUUAUUCAUCUCUUUUUCUUUUUCGUUGGUG
UAAAGCCAACACCCUGUCUAAAAAACAUAAAUUUCUUUAAUCAUUUUGCC
UCUUUUCUCUGUGCUUCAAUUAAUAAAAAAUGGAAAGAACCUCGAGAAA
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAU
CUAG

’Cas9 1xNLS, no HA tag’ referenced in Table 11 and used in Example 17:

GGGUCCCGCAGUCGGCGUCCAGCGGCUCUGCUUGUUCGUGUGUGUGUCGU
UGCAGGCCUUAUUCGGAUCCAUGGAUAAGAAGUACUCAAUCGGGCUGGA
UAUCGGAACUAAUUCCGUGGGUUGGGCAGUGAUCACGGAUGAAUACAAA
GUGCCGUCCAAGAAGUUCAAGGUCCUGGGGAACACCGAUAGACACAGCAU
CAAGAAAAAUCUCAUCGGAGCCCUGCUGUUUGACUCCGGCGAAACCGCAG

AAGCGACCCGGCUCAAACGUACCGCGAGGCGACGCUACACCCGGCGGAAG AAUCGCAUCUGCUAUCUGCAAGAGAUCUUUUCGAACGAAAUGGCAAAGG UCGACGACAGCUUCUUCCACCGCCUGGAAGAAUCUUUCCUGGUGGAGGAG GACAAGAAGCAUGAACGGCAUCCUAUCUUUGGAAACAUCGUCGACGAAG UGGCGUACCACGAAAAGUACCCGACCAUCUACCAUCUGCGGAAGAAGUUG GUUGACUCAACUGACAAGGCCGACCUCAGAUUGAUCUACUUGGCCCUCGC CCAUAUGAUCAAAUUCCGCGGACACUUCCUGAUCGAAGGCGAUCUGAACC CUGAUAACUCCGACGUGGAUAAGCUUUUCAUUCAACUGGUGCAGACCUAC AACCAACUGUUCGAAGAAAACCCAAUCAAUGCUAGCGGCGUCGAUGCCAA GGCCAUCCUGUCCGCCCGGCUGUCGAAGUCGCGGCGCCUCGAAAACCUGA UCGCACAGCUGCCGGGAGAGAAAAAGAACGGACUUUUCGGCAACUUGAUC GCUCUCUCACUGGGACUCACUCCCAAUUUCAAGUCCAAUUUUGACCUGGC CGAGGACGCGAAGCUGCAACUCUCAAAGGACACCUACGACGACGACUUGG ACAAUUUGCUGGCACAAAUUGGCGAUCAGUACGCGGAUCUGUUCCUUGCC GCUAAGAACCUUUCGGACGCAAUCUUGCUGUCCGAUAUCCUGCGCGUGAA CACCGAAAUAACCAAAGCGCCGCUUAGCGCCUCGAUGAUUAAGCGGUACG ACGAGCAUCACCAGGAUCUCACGCUGCUCAAAGCGCUCGUGAGACAGCAA CUGCCUGAAAAGUACAAGGAGAUCUUCUUCGACCAGUCCAAGAAUGGGU ACGCAGGGUACAUCGAUGGAGGCGCUAGCCAGGAAGAGUUCUAUAAGUU CAUCAAGCCAAUCCUGGAAAAGAUGGACGGAACCGAAGAACUGCUGGUCA AGCUGAACAGGGAGGAUCUGCUCCGGAAACAGAGAACCUUUGACAACGG AUCCAUUCCCCACCAGAUCCAUCUGGGUGAGCUGCACGCCAUCUUGCGGC GCCAGGAGGACUUUUACCCAUUCCUCAAGGACAACCGGGAAAAGAUCGAG AAAAUUCUGACGUUCCGCAUCCCGUAUUACGUGGGCCCACUGGCGCGCGG CAAUUCGCGCUUCGCGUGGAUGACUAGAAAAUCAGAGGAAACCAUCACUC CUUGGAAUUUCGAGGAAGUUGUGGAUAAGGGAGCUUCGGCACAAAGCUU CAUCGAACGAAUGACCAACUUCGACAAGAAUCUCCCAAACGAGAAGGUGC UUCCUAAGCACAGCCUCCUUUACGAAUACUUCACUGUCUACAACGAACUG ACUAAAGUGAAAUACGUUACUGAAGGAAUGAGGAAGCCGGCCUUUCUGU CCGGAGAACAGAAGAAAGCAAUUGUCGAUCUGCUGUUCAAGACCAACCGC AAGGUGACCGUCAAGCAGCUUAAAGAGGACUACUUCAAGAAGAUCGAGU GUUUCGACUCAGUGGAAAUCAGCGGGGUGGAGGACAGAUUCAACGCUUC GCUGGGAACCUAUCAUGAUCUCCUGAAGAUCAUCAAGGACAAGGACUUCC

UUGACAACGAGGAGAACGAGGACAUCCUGGAAGAUAUCGUCCUGACCUU
GACCCUUUUCGAGGAUCGCGAGAUGAUCGAGGAGAGGCUUAAGACCUAC
GCUCAUCUCUUCGACGAUAAGGUCAUGAAACAACUCAAGCGCCGCCGGUA
CACUGGUUGGGGCCGCCUCUCCCGCAAGCUGAUCAACGGUAUUCGCGAUA
AACAGAGCGGUAAAACUAUCCUGGAUUUCCUCAAAUCGGAUGGCUUCGCU
AAUCGUAACUUCAUGCAAUUGAUCCACGACGACAGCCUGACCUUUAAGGA
GGACAUCCAAAAAGCACAAGUGUCCGGACAGGGAGACUCACUCCAUGAAC
ACAUCGCGAAUCUGGCCGGUUCGCCGGCGAUUAAGAAGGGAAUUCUGCAA
ACUGUGAAGGUGGUCGACGAGCUGGUGAAGGUCAUGGGACGGCACAAAC
CGGAGAAUAUCGUGAUUGAAAUGGCCCGAGAAAACCAGACUACCCAGAA
GGGCCAGAAAAACUCCCGCGAAAGGAUGAAGCGGAUCGAAGAAGGAAUC
AAGGAGCUGGGCAGCCAGAUCCUGAAAGAGCACCCGGUGGAAAACACGCA
GCUGCAGAACGAGAAGCUCUACCUGUACUAUUUGCAAAAUGGACGGGAC
AUGUACGUGGACCAAGAGCUGGACAUCAAUCGGUUGUCUGAUUACGACG
UGGACCACAUCGUUCCACAGUCCUUUCUGAAGGAUGACUCGAUCGAUAAC
AAGGUGUUGACUCGCAGCGACAAGAACAGAGGGAAGUCAGAUAAUGUGC
CAUCGGAGGAGGUCGUGAAGAAGAUGAAGAAUUACUGGCGGCAGCUCCU
GAAUGCGAAGCUGAUUACCCAGAGAAAGUUUGACAAUCUCACUAAAGCC
GAGCGCGGCGGACUCUCAGAGCUGGAUAAGGCUGGAUUCAUCAAACGGCA
GCUGGUCGAGACUCGGCAGAUUACCAAGCACGUGGCGCAGAUCUUGGACU
CCCGCAUGAACACUAAAUACGACGAGAACGAUAAGCUCAUCCGGGAAGUG
AAGGUGAUUACCCUGAAAAGCAAACUUGUGUCGGACUUUCGGAAGGACU
UUCAGUUUUACAAAGUGAGAGAAAUCAACAACUACCAUCACGCGCAUGAC
GCAUACCUCAACGCUGUGGUCGGUACCGCCCUGAUCAAAAAGUACCCUAA
ACUUGAAUCGGAGUUUGUGUACGGAGACUACAAGGUCUACGACGUGAGG
AAGAUGAUAGCCAAGUCCGAACAGGAAAUCGGGAAAGCAACUGCGAAAU
ACUUCUUUUACUCAAACAUCAUGAACUUUUUCAAGACUGAAAUUACGCU
GGCCAAUGGAGAAAUCAGGAAGAGGCCACUGAUCGAAACUAACGGAGAA
ACGGGCGAAAUCGUGUGGGACAAGGGCAGGGACUUCGCAACUGUUCGCA
AAGUGCUCUCUAUGCCGCAAGUCAAUAUUGUGAAGAAAACCGAAGUGCA
AACCGGCGGAUUUUCAAAGGAAUCGAUCCUCCCAAAGAGAAAUAGCGACA
AGCUCAUUGCACGCAAGAAAGACUGGGACCCGAAGAAGUACGGAGGAUU
CGAUUCGCCGACUGUCGCAUACUCCGUCCUCGUGGUGGCCAAGGUGGAGA

AGGGAAAGAGCAAAAAGCUCAAAUCCGUCAAAGAGCUGCUGGGGAUUACCAUCAUGGAACGAUCCUCGUUCGAGAAGAACCCGAUUGAUUUCCUCGAGGCGAAGGGUUACAAGGAGGUGAAGAAGGAUCUGAUCAUCAAACUCCCCAAGUACUCACUGUUCGAACUGGAAAAUGGUCGGAAGCGCAUGCUGGCUUCGGCCGGAGAACUCCAAAAAGGAAAUGAGCUGGCCUUGCCUAGCAAGUACGUCAACUUCCUCUAUCUUGCUUCGCACUACGAAAAACUCAAAGGGUCACCGGAAGAUAACGAACAGAAGCAGCUUUUCGUGGAGCAGCACAAGCAUUAUCUGGAUGAAAUCAUCGAACAAAUCUCCGAGUUUUCAAAGCGCGUGAUCCUCGCCGACGCCAACCUCGACAAAGUCCUGUCGGCCUACAAUAAGCAUAGAGAUAAGCCGAUCAGAGAACAGGCCGAGAACAUUAUCCACUUGUUCACCCUGACUAACCUGGGAGCCCCAGCCGCCUUCAAGUACUUCGAUACUACUAUCGAUCGCAAAAGAUACACGUCCACCAAGGAAGUUCUGGACGCGACCCUGAUCCACCAAAGCAUCACUGGACUCUACGAAACUAGGAUCGAUCUGUCGCAGCUGGGUGGCGAUGGCGGUGGAUCUCCGAAAAAGAAGAGAAAGGUGUAAUGAGCUAGCCAUCACAUUUAAAAGCAUCUCAGCCUACCAUGAGAAUAAGAGAAAGAAAAUGAAGAUCAAUAGCUUAUUCAUCUCUUUUUCUUUUUCGUUGGUGUAAAGCCAACACCCUGUCUAAAAAACAUAAAUUUCUUUAAUCAUUUUGCCUCUUUUCUCUGUGCUUCAAUUAAUAAAAAAUGGAAAGAACCUCGAGAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAUCUAG

tr001 (trRNA):

AACAGCAUAGCAAGUUAAAAUAAGGCUAGUCCGUUAUCAACUUGAAAAAGUGGCACCGAGUCGGUGCUUUUUUU

cr002 (crRNA targeting FVII; targeting sequence underlined):
AGGGCUCUUGAAGAUCUCCCGUUUUAGAGCUAUGCUGUUUUG

sg001 (sgRNA targeting FVII; targeting sequence underlined):

AGGGCUCUUGAAGAUCUCCCGUUUUAGAGCUAGAAAUAGCAAGUUAAAAUAAGGCUAGUCCGUUAUCAACUUGAAAAAGUGGCACCGAGUCGGUGCUUUUUUU

cr003 (crRNA targeting TTR; targeting sequence underlined):
CCAGUCCAGCGAGGCAAAGGGUUUUAGAGCUAUGCUGUUUUG

sg006 (sgRNA targeting TTR made by IVT; targeting sequence underlined):

GGCCAGUCCAGCGAGGCAAAGGGUUUUAGAGCUAGAAAUAGCAAGUUAAAAUAAGGCUAGUCCGUUAUCAACUUGAAAAAGUGGCACCGAGUCGGUGCUUUUUUU

sg003 (sgRNA targeting TTR; targeting sequence underlined):

CCAGUCCAGCGAGGCAAAGGGUUUUAGAGCUAGAAAUAGCAAGUUAAAAUAAGGCUAGUCCGUUAUCAACUUGAAAAAGUGGCACCGAGUCGGUGCUUUU

sg007 (sgRNA targeting FVII; targeting sequence underlined):

CUCAGUUUUCAUAACCCAGGGUUUUAGAGCUAGAAAUAGCAAGUUAAAAUAAGGCUAGUCCGUUAUCAACUUGAAAAAGUGGCACCGAGUCGGUGCUUUU

sg002 (sgRNA targeting FVII; targeting sequence underlined):

CAGGGCUCUUGAAGAUCUCCGUUUUAGAGCUAGAAAUAGCAAGUUAAAAUAAGGCUAGUCCGUUAUCAACUUGAAAAAGUGGCACCGAGUCGGUGCUUUU

sg004 (sgRNA targeting TTR; targeting sequence underlined):

CUUUCUACAAGCUUACCCAGGUUUUAGAGCUAGAAAUAGCAAGUUAAAAUAAGGCUAGUCCGUUAUCAACUUGAAAAAGUGGCACCGAGUCGGUGCUUUU

sg005 (sgRNA targeting TTR; targeting sequence underlined):

UUACAGCCACGUCUACAGCAGUUUUAGAGCUAGAAAUAGCAAGUUAAAAUAAGGCUAGUCCGUUAUCAACUUGAAAAAGUGGCACCGAGUCGGUGCUUUU

* = Phosphorothioate linkage
m = 2'OMe

tr002 (trRNA):

A*A*C*AGCAUAGCAAGUUAAAAUAAGGCUAGUCCGUUAUCAACUUGAAAAAGUGGCACCGAGUCGGUGCUUUU*U*U*U

cr004 (crRNA targeting FVII; targeting sequence underlined):
A*G*G*GCUCUUGAAGAUCUCCCGUUUUAGAGCUAUGCUGUU*U*U*G

cr005 (crRNA targeting TTR; targeting sequence underlined):
C*C*A*GUCCAGCGAGGCAAAGGGUUUUAGAGCUAUGCUGUU*U*U*G

sg008 (sgRNA targeting FVII; targeting sequence underlined):

mA*mG*mG*GCUCUUGAAGAUCUCCCGUUUUAGAGCUAGAAAUAGCAAGUUAAAAUAAGGCUAGUCCGUUAUCAACUUGAAAAAGUGGCACCGAGUCGGUGCmU*mU*mU*U

sg009 (sgRNA targeting TTR; targeting sequence underlined):

mC*mC*mA*GUCCAGCGAGGCAAAGGGUUUUAGAGCUAGAAAUAGCAAGUUAAAAUAAGGCUAGUCCGUUAUCAACUUGAAAAAGUGGCACCGAGUCGGUGCmU*mU*mU*U

sg010 (sgRNA targeting FVII; targeting sequence underlined):

mC*mU*mC*AGUUUUCAUAACCCAGGGUUUUAGAGCUAGAAAUAGCAAGUUAAAAUAAGGCUAGUCCGUUAUCAACUUGAAAAAGUGGCACCGAGUCGGUGCmU*mU*mU*U

sg002 (sgRNA targeting FVII; targeting sequence underlined):

mC*mA*mG*GGCUCUUGAAGAUCUCCGUUUUAGAGCUAGAAAUAGCAAGUUAAAAUAAGGCUAGUCCGUUAUCAACUUGAAAAAGUGGCACCGAGUCGGUGCmU*mU*mU*U

sg011 (sgRNA targeting TTR; targeting sequence underlined):

mC*mU*mU*UCUACAAGCUUACCCAGGUUUUAGAGCUAGAAAUAGCAAGUUAAAAUAAGGCUAGUCCGUUAUCAACUUGAAAAAGUGGCACCGAGUCGGUGCmU*mU*mU*U

sg012 (sgRNA targeting TTR; targeting sequence underlined):

mU*mU*mA*CAGCCACGUCUACAGCAGUUUUAGAGCUAGAAAUAGCAAGUUAAAAUAAGGCUAGUCCGUUAUCAACUUGAAAAAGUGGCACCGAGUCGGUGCmU*mU*mU*U

ec001 (expression cassette - amplicon for expressing sgRNA targeting TTR; U6 promoter in bold, targeting sequence underlined; construct contains inverted dideoxy T at each 5' end):

GCTGCAAGGCGATTAAGTTGGGTAACGCCAGGGTTTTCCCAGTCACGACGTTGTAAAACGACGGCCAGTGAATTC**GAGGGCCTATTTCCCATGATTCCTTCATATTTGCATATACGATACAAGGCTGTTAGAGAGATAATTGGAATTAATTTGACTGTAAACACAAAGATATTAGTACAAAATACGTGACGTAGAAAGTAATAATTTCTTGGGTAGTTTGCAGTTTTAAAATTATGTTTTAAAATGGACTATCATATGCTTACCGTAACTTGAAAGTATTTCGATTTCTTGGCTTTATATATCTTGTGGAAAGGACGAAACACC**GCTTTCTACAAGCTTACCCAGGTTTTAGAGCTAGAAATAGCAAGTTAAAATAAGGCTAGTCCGTTATCAACTTGAAAAAGTGGCACCGAGTCGGTGCTTTTTTTGGTACCCGGGGATCCTCTAGAGTCG

ACCTGCAGGCATGCAAGCTTGGCGTAATCATGGTCATAGCTGTTTCCTGTGTGAAATTGTTATCCGCTCACAATTCCACACAACATACGAGCCGGAAGCATAAAGTGTAAAGCCTGGGGTGCCTAATGAGTGAGCTA

Primer pairs for NGS analysis of FVII target site targeted by cr001:

Forward:

CACTCTTTCCCTACACGACGCTCTTCCGATCTGATCCAGTGTGGCTGTTTCCATTC

Reverse:

GGAGTTCAGACGTGTGCTCTTCCGATCTTTACACAAGAGCAGGCACGAGATG

Primer pairs for NGS analysis of FVII target site targeted by cr002 and sg001: Forward:

CACTCTTTCCCTACACGACGCTCTTCCGATCTAGCACATGAGACCTTCTGTTTCTC

Reverse:
GGAGTTCAGACGTGTGCTCTTCCGATCTGACATAGGTGTGACCCTCACAATC

Primer pairs for NGS analysis of FVII target site targeted by sg002:

CACTCTTTCCCTACACGACGCTCTTCCGATCTAGCACATGAGACCTTCTGTTTCTC

Reverse:
GGAGTTCAGACGTGTGCTCTTCCGATCTGACATAGGTGTGACCCTCACAATC

Primer pairs for NGS analysis of TTR target site targeted by cr003 and sg003:

Forward:

CACTCTTTCCCTACACGACGCTCTTCCGATCTAGTCAATAATCAGAATCAGCAGGT

Reverse:

GGAGTTCAGACGTGTGCTCTTCCGATCTAGAAGGCACTTCTTCTTTATCTAAGGT

Primer pairs for NGS analysis of TTR target site targeted by sg004:

Forward:

CACTCTTTCCCTACACGACGCTCTTCCGATCTTGCTGGAGAATCCAAATGTCCTC

Reverse:

GGAGTTCAGACGTGTGCTCTTCCGATCTGCTAGGAATTAAACCTGTGTCTCTTAC

Primer pairs for NGS analysis of TTR target site targeted by sg005:

Forward:

CACTCTTTCCCTACACGACGCTCTTCCGATCTGTTTTGTTCCAGAGTCTATCACCG

Reverse:

GGAGTTCAGACGTGTGCTCTTCCGATCTACACGAATAAGAGCAAATGGGAAC

Primer pairs for PCR amplification of sg004 expression cassette:
/5InvddT/ = inverted dideoxyT

Forward:
/5InvddT/GCTGCAAGGCGATTAAGTTG
Reverse:
/5InvddT/TAGCTCACTCATTAGGCACC

**Table 13. Mouse TTR Guide Sequences.**

| Guide Name | Locations | Guide |
|---|---|---|
| cr006 | Chr18:20666429-20666451 | UCUUGUCUCCUCUGUGCCCA |
| cr007 | Chr18:20666435-20666457 | CUCCUCUGUGCCCAGGGUGC |
| cr008 | Chr18:20666458-20666480 | AGAAUCCAAAUGUCCUCUGA |
| cr009 | Chr18:20666533-20666555 | AGUGUUCAAAAAGACCUCUG |
| cr010 | Chr18:20666541-20666563 | AAAAGACCUCUGAGGGAUCC |

(continued)

| Guide Name | Locations | Guide |
|---|---|---|
| cr011 | Chr18:20666558-20666580 | UCCUGGGAGCCCUUUGCCUC |
| cr012 | Chr18:20666500-20666522 | CGUCUACAGCAGGGCUGCCU |
| cr013 | Chr18:20666559-20666581 | CCCAGAGGCAAAGGGCUCCC |
| cr014 | Chr18:20670008-20670030 | UUCUACAAACUUCUCAUCUG |
| cr015 | Chr18:20670086-20670108 | AUCCGCGAAUUCAUGGAACG |
| cr016 | Chr18:20673606-20673628 | UGUCUCUCCUCUCUCCUAGG |
| cr017 | Chr18:20673628-20673650 | GUUUUCACAGCCAACGACUC |
| cr018 | Chr18:20673684-20673706 | CCCAUACUCCUACAGCACCA |
| cr019 | Chr18:20673657-20673679 | GCAGGGCUGCGAUGGUGUAG |
| cr020 | Chr18:20673675-20673697 | UGUAGGAGUAUGGGCUGAGC |
| cr021 | Chr18:20673685-20673707 | GCCGUGGUGCUGUAGGAGUA |
| cr022 | Chr18:20673723-20673745 | UGGGCUGAGUCUCUCAAUUC |
| cr023 | Chr18:20665448-20665470 | CUCUUCCUCCUUUGCCUCGC |
| cr024 | Chr18:20665472-20665494 | CUGGUAUUUGUGUCUGAAGC |
| cr025 | Chr18:20665453-20665475 | CCUCCUUUGCCUCGCUGGAC |
| cr026 | Chr18:20665496-20665518 | CUCACAGGAUCACUCACCGC |
| cr027 | Chr18:20665414-20665436 | UCCACAAGCUCCUGACAGGA |
| cr028 | Chr18:20665441-20665463 | GGCAAAGGAGGAAGAGUCGA |
| cr003 | Chr18:20665453-20665475 | CCAGUCCAGCGAGGCAAAGG |
| cr029 | Chr18:20665456-20665478 | AUACCAGUCCAGCGAGGCAA |
| cr030 | Chr18:20665497-20665519 | GCUCACAGGAUCACUCACCG |
| cr031 | Chr18:20665462-20665484 | ACACAAAUACCAGUCCAGCG |
| cr032 | Chr18:20665495-20665517 | UCACAGGAUCACUCACCGCG |
| cr033 | Chr18:20665440-20665462 | GCAAAGGAGGAAGAGUCGAA |
| cr034 | Chr18:20666463-20666485 | UUUGACCAUCAGAGGACAUU |
| cr035 | Chr18:20666488-20666510 | GGCUGCCUCGGACAGCAUCC |
| cr036 | Chr18:20666470-20666492 | UCCUCUGAUGGUCAAAGUCC |
| cr037 | Chr18:20666542-20666564 | AAAGACCUCUGAGGGAUCCU |
| cr038 | Chr18:20666510-20666532 | UUUACAGCCACGUCUACAGC |
| cr039 | Chr18:20666534-20666556 | GUGUUCAAAAAGACCUCUGA |
| cr040 | Chr18:20666567-20666589 | CAAGCUUACCCAGAGGCAAA |
| cr041 | Chr18:20666503-20666525 | AGGCAGCCCUGCUGUAGACG |
| cr042 | Chr18:20666471-20666493 | UCCAGGACUUUGACCAUCAG |
| cr043 | Chr18:20666547-20666569 | GGGCUCCCAGGAUCCCUCAG |
| cr044 | Chr18:20666483-20666505 | AAAGUCCUGGAUGCUGUCCG |
| cr045 | Chr18:20666568-20666590 | ACAAGCUUACCCAGAGGCAA |
| cr046 | Chr18:20666574-20666596 | CUUUCUACAAGCUUACCCAG |
| cr047 | Chr18:20666509-20666531 | UUACAGCCACGUCUACAGCA |
| cr048 | Chr18:20669968-20669990 | UCCAGGAAGACCGCGGAGUC |

(continued)

| Guide Name | Locations | Guide |
|---|---|---|
| cr049 | Chr18:20670093-20670115 | CACUUACAUCCGCGAAUUCA |
| cr050 | Chr18:20670056-20670078 | AAGUGUCUUCCAGUACGAUU |
| cr051 | Chr18:20670087-20670109 | CAUCCGCGAAUUCAUGGAAC |
| cr052 | Chr18:20670058-20670080 | AAAUCGUACUGGAAGACACU |
| cr053 | Chr18:20669981-20670003 | CGGAGUCUGGAGAGCUGCAC |
| cr054 | Chr18:20670030-20670052 | AGGAGUGUACAGAGUAGAAC |
| cr055 | Chr18:20670084-20670106 | UUCCCCGUUCCAUGAAUUCG |
| cr056 | Chr18:20670010-20670032 | ACAGAUGAGAAGUUUGUAGA |
| cr057 | Chr18:20670047-20670069 | AACUGGACACCAAAUCGUAC |
| cr058 | Chr18:20670088-20670110 | ACAUCCGCGAAUUCAUGGAA |
| cr059 | Chr18:20669980-20670002 | GCGGAGUCUGGAGAGCUGCA |
| cr060 | Chr18:20669978-20670000 | GUGCAGCUCUCCAGACUCCG |
| cr061 | Chr18:20669961-20669983 | UGUGCCCUCCAGGAAGACCG |
| cr062 | Chr18:20673723-20673745 | UGGGCUGAGUCUCUCAAUUC |
| cr063 | Chr18:20673675-20673697 | UGUAGGAGUAUGGGCUGAGC |
| cr064 | Chr18:20673665-20673687 | UGGGCUGAGCAGGGCUGCGA |
| cr065 | Chr18:20673638-20673660 | GUGGCGAUGGCCAGAGUCGU |
| cr066 | Chr18:20673651-20673673 | CUGCGAUGGUGUAGUGGCGA |
| cr067 | Chr18:20673685-20673707 | GCCGUGGUGCUGUAGGAGUA |

**Table 14. Human TTR Guide Sequences.**

| Guide Name | Guide | Locations | Exon | Strand |
|---|---|---|---|---|
| cr700 | CUGCUCCUCCUCUGCCUUGC | Chr18:31591918-31591940 | 1 | + |
| cr701 | CCUCCUCUGCCUUGCUGGAC | Chr18:31591923-31591945 | 1 | + |
| cr702 | CCAGUCCAGCAAGGCAGAGG | Chr18:31591923-31591945 | 1 | - |
| cr703 | AUACCAGUCCAGCAAGGCAG | Chr18:31591926-31591948 | 1 | - |
| cr704 | ACACAAAUACCAGUCCAGCA | Chr18:31591932-31591954 | 1 | - |
| cr705 | UGGACUGGUAUUUGUGUCUG | Chr18:31591938-31591960 | 1 | + |
| cr706 | CUGGUAUUUGUGUCUGAGGC | Chr18:31591942-31591964 | 1 | + |
| cr707 | CUUCUCUACACCCAGGGCAC | Chr18:31592881-31592903 | 2 | + |
| cr708 | CAGAGGACACUUGGAUUCAC | Chr18:31592900-31592922 | 2 | - |
| cr709 | UUUGACCAUCAGAGGACACU | Chr18:31592909-31592931 | 2 | - |
| cr710 | UCUAGAACUUUGACCAUCAG | Chr18:31592917-31592939 | 2 | - |
| cr711 | AAAGUUCUAGAUGCUGUCCG | Chr18:31592929-31592951 | 2 | + |
| cr712 | CAUUGAUGGCAGGACUGCCU | Chr18:31592946-31592968 | 2 | - |
| cr713 | AGGCAGUCCUGCCAUCAAUG | Chr18:31592949-31592971 | 2 | + |
| cr714 | UGCACGGCCACAUUGAUGGC | Chr18:31592956-31592978 | 2 | - |
| cr715 | CACAUGCACGGCCACAUUGA | Chr18:31592960-31592982 | 2 | - |
| cr716 | AGCCUUUCUGAACACAUGCA | Chr18:31592972-31592994 | 2 | - |

(continued)

| Guide Name | Guide | Locations | Exon | Strand |
|---|---|---|---|---|
| cr717 | GAAAGGCUGCUGAUGACACC | Chr18:31592987-31593009 | 2 | + |
| cr718 | AAAGGCUGCUGAUGACACCU | Chr18:31592988-31593010 | 2 | + |
| cr719 | ACCUGGGAGCCAUUUGCCUC | Chr18:31593004-31593026 | 2 | + |
| cr720 | CCCAGAGGCAAAUGGCUCCC | Chr18:31593005-31593027 | 2 | - |
| cr721 | GCAACUUACCCAGAGGCAAA | Chr18:31593013-31593035 | 2 | - |
| cr722 | UUCUUUGGCAACUUACCCAG | Chr18:31593020-31593042 | 2 | - |
| cr723 | AUGCAGCUCUCCAGACUCAC | Chr18:31595125-31595147 | 3 | - |
| cr724 | AGUGAGUCUGGAGAGCUGCA | Chr18:31595127-31595149 | 3 | + |
| cr725 | GUGAGUCUGGAGAGCUGCAU | Chr18:31595128-31595150 | 3 | + |
| cr726 | GCUGCAUGGGCUCACAACUG | Chr18:31595141-31595163 | 3 | + |
| cr727 | GCAUGGGCUCACAACUGAGG | Chr18:31595144-31595166 | 3 | + |
| cr728 | ACUGAGGAGGAAUUUGUAGA | Chr18:31595157-31595179 | 3 | + |
| cr729 | CUGAGGAGGAAUUUGUAGAA | Chr18:31595158-31595180 | 3 | + |
| cr730 | UGUAGAAGGGAUAUACAAAG | Chr18:31595171-31595193 | 3 | + |
| cr731 | AAAUAGACACCAAAUCUUAC | Chr18:31595194-31595216 | 3 | + |
| cr732 | AGACACCAAAUCUUACUGGA | Chr18:31595198-31595220 | 3 | + |
| cr733 | AAGUGCCUUCCAGUAAGAUU | Chr18:31595203-31595225 | 3 | - |
| cr734 | CUCUGCAUGCUCAUGGAAUG | Chr18:31595233-31595255 | 3 | - |
| cr735 | CCUCUGCAUGCUCAUGGAAU | Chr18:31595234-31595256 | 3 | - |
| cr736 | ACCUCUGCAUGCUCAUGGAA | Chr18:31595235-31595257 | 3 | - |
| cr737 | UACUCACCUCUGCAUGCUCA | Chr18:31595240-31595262 | 3 | - |
| cr738 | GUAUUCACAGCCAACGACUC | Chr18:31598571-31598593 | 4 | + |
| cr739 | GCGGCGGGGGCCGGAGUCGU | Chr18:31598581-31598603 | 4 | - |
| cr740 | AAUGGUGUAGCGGCGGGGGC | Chr18:31598590-31598612 | 4 | - |
| cr741 | CGGCAAUGGUGUAGCGGCGG | Chr18:31598594-31598616 | 4 | - |
| cr742 | GCGGCAAUGGUGUAGCGGCG | Chr18:31598595-31598617 | 4 | - |
| cr743 | GGCGGCAAUGGUGUAGCGGC | Chr18:31598596-31598618 | 4 | - |
| cr744 | GGGCGGCAAUGGUGUAGCGG | Chr18:31598597-31598619 | 4 | - |
| cr745 | GCAGGGCGGCAAUGGUGUAG | Chr18:31598600-31598622 | 4 | - |
| cr746 | GGGGCUCAGCAGGGCGGCAA | Chr18:31598608-31598630 | 4 | - |
| cr747 | GGAGUAGGGGCUCAGCAGGG | Chr18:31598614-31598636 | 4 | - |
| cr748 | AUAGGAGUAGGGGCUCAGCA | Chr18:31598617-31598639 | 4 | - |
| cr749 | AAUAGGAGUAGGGGCUCAGC | Chr18:31598618-31598640 | 4 | - |
| cr750 | CCCCUACUCCUAUUCCACCA | Chr18:31598627-31598649 | 4 | + |
| cr751 | CCGUGGUGGAAUAGGAGUAG | Chr18:31598627-31598649 | 4 | - |
| cr752 | GCCGUGGUGGAAUAGGAGUA | Chr18:31598628-31598650 | 4 | - |
| cr753 | GACGACAGCCGUGGUGGAAU | Chr18:31598635-31598657 | 4 | - |
| cr754 | AUUGGUGACGACAGCCGUGG | Chr18:31598641-31598663 | 4 | - |
| cr755 | GGGAUUGGUGACGACAGCCG | Chr18:31598644-31598666 | 4 | - |

(continued)

| Guide Name | Guide | Locations | Exon | Strand |
|---|---|---|---|---|
| cr756 | GGCUGUCGUCACCAAUCCCA | Chr18:31598648-31598670 | 4 | + |
| cr757 | AGUCCCUCAUUCCUUGGGAU | Chr18:31598659-31598681 | 4 | - |

## EMBODIMENTS

The present invention provides the following embodiments:

1. A method of producing a genetically engineered liver cell, comprising contacting a cell with lipid nanoparticles (LNPs) comprising:

a Class 2 Cas nuclease mRNA;
a guide RNA nucleic acid;
a CCD lipid;
a helper lipid;
a neutral lipid; and
a stealth lipid.

2. A method of gene editing, comprising delivering a Class 2 Cas nuclease mRNA and a guide RNA nucleic acid to a liver cell, wherein the Class 2 Cas mRNA and the guide RNA nucleic acid are formulated as at least one LNP composition comprising:

a CCD lipid;
a helper lipid;
a neutral lipid; and
a stealth lipid.

3. A method of gene editing, comprising administering a Class 2 Cas nuclease mRNA and a guide RNA nucleic acid to ApoE-binding cells in a subject wherein the Class 2 Cas mRNA and the guide RNA nucleic acid are formulated as at least one LNP composition comprising:

a CCD lipid;
a helper lipid;
a neutral lipid; and
a stealth lipid.

4. A method gene editing, comprising contacting a liver cell with LNPs comprising:

an mRNA encoding a Cas nuclease;
a guide RNA nucleic acid;
a CCD lipid;
a helper lipid;
a neutral lipid; and
a stealth lipid.

5. A method of altering expression of a gene in a liver cell, comprising administering to a subject a therapeutically effective amount of a Class 2 Cas nuclease mRNA and a guide RNA nucleic acid as one or more LNP formulations, wherein at least one LNP formulation comprises:

a guide RNA nucleic acid or a Class 2 Cas nuclease mRNA;
a CCD lipid;
a helper lipid;
a neutral lipid; and
a stealth lipid.

6. A method of producing a genetically engineered liver cell, comprising contacting a cell with lipid nanoparticles (LNPs) comprising:

a Class 2 Cas nuclease mRNA;
a guide RNA nucleic acid that is or encodes a single-guide RNA (sgRNA);
a CCD lipid;
a helper lipid;
a neutral lipid; and
a stealth lipid.

7. A method of producing a genetically engineered liver cell, comprising contacting a cell with an LNP comprising:

a Class 2 Cas nuclease mRNA;
a guide RNA nucleic acid that is or encodes an sgRNA;
a CCD lipid;
a helper lipid;
a neutral lipid; and
a stealth lipid.

8. A method of producing a genetically engineered liver cell, comprising contacting a cell with a lipid nanoparticle composition comprising:

a Class 2 Cas nuclease mRNA;
a guide RNA nucleic acid;
a means for delivering the RNA in a liver-specific manner.

9. A method of producing a genetically engineered liver cell, comprising contacting a cell with a lipid nanoparticle comprising:

a Class 2 Cas nuclease mRNA;
a guide RNA nucleic acid that is or encodes an sgRNA;
a means for delivering the RNA to a liver cell.

10. A method of administering a CRISPR-Cas complex to a liver cell, comprising administering to a subject an LNP composition for gene editing in a liver cell comprising:

a Cas9 nuclease mRNA
a guide RNA that is or encodes an sgRNA;
a biodegradable means for delivering the RNA to a liver cell.

11. The method of any of embodiments 1-10, wherein the liver cell is a hepatocyte.
12. The method of embodiment 11, wherein the hepatocyte is a primary hepatocyte.
13. The method of embodiment 11, wherein the liver cell is a stem cell.
14. The method of any of embodiments 1-13, wherein the cell is in a subject.
15. The method of embodiment 14, wherein the subject is human.
16. The method of any of embodiments 1-15, wherein the mRNA is formulated in a first LNP composition and the guide RNA nucleic acid is formulated in a second LNP composition.
17. The method of embodiment 16, wherein the first and second LNP compositions are administered simultaneously.
18. The method of embodiment 16, wherein the first and second LNP compositions are administered sequentially.
19. The method of any of embodiments 1-15, wherein the mRNA and the guide RNA nucleic acid are formulated in a single LNP composition.
20. The method of any of embodiments 1-19, further comprising at least one template.
21. The method of any one of embodiments 1-20, wherein the mRNA is a Cas9 nuclease mRNA.
22. The method of embodiment 16, wherein the Cas9 mRNA is a human codon-optimized Cas9 nuclease.
23. The method of any of embodiments 1-22, wherein the guide RNA nucleic acid is an expression cassette that encodes a guide RNA.
24. The method of embodiment 23, wherein the expression cassette further comprises a regulatory element.
25. The method of any of embodiments 1-22, wherein the guide RNA nucleic acid is a guide RNA.

26. The method of any of embodiments 23-25, wherein the guide RNA is an sgRNA.

27. The method of any of embodiments 23-25, wherein the guide RNA is a dual-guide RNA (dgRNA).

28. The method of any of embodiments 1-27, wherein the guide RNA nucleic acid comprises a modified residue.

29. The method of embodiment 28, wherein the modified residue comprises a modification selected from a backbone modification, a sugar modification, and a base modification.

30. The method of any of embodiments 1-29, wherein the CCD lipid is Lipid A.

31. The method of any of embodiments 1-29, wherein the CCD lipid chosen from Lipid A, Lipid B, Lipid C, and Lipid D.

32. The method of any of embodiments 1-31, wherein the helper lipid is selected from cholesterol, 5-heptadecylresorcinol, and cholesterol hemisuccinate.

33. The method of embodiment 32, wherein the helper lipid is cholesterol.

34. The method of any of embodiments 1-33, wherein the neutral lipid is selected from DSPC and DMPE.

35. The method of embodiment 34, wherein the neutral lipid is DSPC.

36. The method of any of embodiments 1-35, wherein the stealth lipid is selected from PEG2k-DMG and PEG2k-C11.

37. The method of embodiment 36, wherein the stealth lipid is PEG2k-DMG.

38. The method of any of embodiments 1-37, wherein at least one LNP comprises Lipid A, cholesterol, DSPC, and PEG2k-DMG.

39. The method of any of embodiments 1-37, wherein at least one LNP comprises Lipid B, cholesterol, DSPC, and PEG2k-DMG.

40. The method of any of embodiments 1-39, wherein the composition comprises the CCD lipid in an amount ranging from about 30 mol-% to about 60 mol-%.

41. The method of any of embodiments 1-40, wherein the composition comprises the helper lipid in an amount ranging from about 30 mol-% to about 60 mol-%.

42. The method of any of embodiments 1-41, wherein the composition comprises the neutral lipid in an amount ranging from about 1 mol-% to about 20 mol-%.

43. The method of any of embodiments 1-42, wherein the composition comprises the stealth lipid in an amount ranging from about 1 mol-% to about 10 mol-%.

44. The method of any of embodiments 1-43, wherein the composition comprises the CCD lipid in an amount of about 45 mol-%.

45. The method of any of embodiments 1-44, wherein the composition comprises the helper lipid in an amount of about 44 mol-%.

46. The method of any of embodiments 1-45, wherein the composition comprises the neutral lipid in an amount of about 9 mol-%.

47. The method of any of embodiments 1-46, wherein the composition comprises the stealth lipid in an amount of about 2 mol-%.

48. The method of any of embodiments 1-47, wherein the Class 2 Cas nuclease mRNA and the guide RNA nucleic acid are present in a ratio ranging from about 10:1 to about 1:10 by weight.

49. The method of any of embodiments 1-48, wherein the Class 2 Cas nuclease mRNA and the guide RNA are present in a ratio of about 1: 1 by weight.

50. The method of any of embodiments 1-49, wherein the ratio of the CCD lipid amine to the RNA phosphate ranges from about 3 to about 5.

51. The method of any of embodiments 1-50, wherein the ratio of the CCD lipid amine to the RNA phosphate is about 4.5.

52. The method of any of embodiments 1-51, wherein the particle size of the composition ranges from about 50 nm to about 120 nm.

53. The method of any of embodiments 1-52, wherein the particle size of the composition ranges from about 75 nm to about 150 nm.

54. The method of any of embodiments 1-53, wherein the encapsulation efficiency of the composition ranges from about 70% to about 100%.

55. The method of any of embodiments 1-54, wherein the polydispersity index of the composition ranges from about .005 to about 0.5.

56. The method of any of embodiments 1-55, wherein the polydispersity index of the composition ranges from about 0.02 to about 0.35.

57. The method of any of embodiments 1-56, wherein administration of the composition results in gene editing.

58. The method of embodiment 57, wherein the gene editing results in a gene knockout.

59. The method of embodiment 58, wherein the gene editing results in a gene correction.

60. The method of any of embodiments 57-59, wherein the gene editing results in a persistent response.

61. The method of any of embodiments 57-59, wherein the gene editing results in a duration of response from about 1 day to about 1 year.

62. The method of any of embodiments 57-59, wherein the gene editing results in a duration of response of at least 1 week.

63. The method of any of embodiments 57-59, wherein the gene editing results in a duration of response of at least 2 weeks.

64. The method of any of embodiments 57-59, wherein the gene editing results in a duration of response of at least one month.

65. The method of any of embodiments 57-59, wherein the gene editing results in a duration of response of at least 4 months.

66. The method of any of embodiments 57-59, wherein the gene editing results in a duration of response of at least 1 year.

67. An LNP composition comprising:

an mRNA encoding a Cas nuclease;
a guide RNA nucleic acid;
a CCD lipid;
a helper lipid;
a neutral lipid; and
a stealth lipid.

68. An LNP composition comprising:

a Class 2 Cas nuclease mRNA;
a guide RNA nucleic acid that is or encodes an sgRNA;
a CCD lipid;
a helper lipid;
a neutral lipid; and
a stealth lipid.

69. An LNP composition for gene editing in a liver cell comprising:

a Class 2 Cas nuclease mRNA
a guide RNA nucleic acid that is or encodes an sgRNA;
a CCD lipid;
a helper lipid;
a neutral lipid; and
a stealth lipid.

70. An LNP composition comprising:

a Class 2 Cas nuclease mRNA;
a guide RNA nucleic acid;
a means for delivering the RNA in a liver-specific manner.

71. An LNP composition comprising:

a Class 2 Cas nuclease mRNA;
a guide RNA nucleic acid that is or encodes an sgRNA;
a means for delivering the RNA to a liver cell.

72. An LNP composition for gene editing in a liver cell comprising:

a Cas9 nuclease mRNA
a guide RNA that is or encodes an sgRNA;
a biodegradable means for delivering the RNA to a liver cell.

73. The composition of any of embodiments 67-72, wherein the mRNA and the guide RNA nucleic acid are separately encapsulated in LNPs, and the LNPs are combined to form the LNP composition.

74. The composition of any of embodiments 67-72, wherein the mRNA and the guide RNA nucleic acid are co-encapsulated in the LNP composition.

75. The composition of any of embodiments 67-74, further comprising at least one template.

76. The composition of any of embodiments 67-75, wherein the mRNA is a Cas9 nuclease mRNA.

77. The composition embodiment 76, wherein the Cas9 nuclease mRNA is a human codon-optimized Cas9 nuclease.

78. The composition of any of embodiments 67-77, wherein the guide RNA nucleic acid is an expression cassette that encodes a guide RNA.

79. The composition of embodiment 78, wherein the expression cassette further comprises a regulatory element.

80. The composition of any of embodiments 67-77, wherein the guide RNA nucleic acid is a guide RNA.

81. The composition of any of embodiments 78-80, wherein the guide RNA is an sgRNA.

82. The composition of any of embodiments 73-80, wherein the guide RNA is a dual-guide RNA (dgRNA).

83. The composition of any of embodiments 67-82, wherein the guide RNA nucleic acid comprises a modified residue.

84. The composition of embodiment 83, wherein the modified residue comprises a modification selected from a backbone modification, a sugar modification, and a base modification.

85. The composition of any of embodiments 67-74, wherein the CCD lipid is Lipid A.

86. The composition of any of embodiments 67-85, wherein the CCD lipid is selected from Lipid A, Lipid B, Lipid, C, and Lipid D.

87. The composition of any of embodiments 67-86, wherein the helper lipid is selected from cholesterol, 5-heptadecylresorcinol, and cholesterol hemisuccinate.

88. The composition of embodiment 87, wherein the helper lipid is cholesterol.

89. The composition of any of embodiments 67-88, wherein the neutral lipid is selected from DSPC and DMPE.

90. The composition of embodiment 89, wherein the neutral lipid is DSPC.

91. The composition of any of embodiments 67-90, wherein the stealth lipid is selected from PEG2k-DMG and PEG2k-C11.

92. The composition of embodiment 91, wherein the stealth lipid is PEG2k-DMG.

93. The composition of any of embodiments 67-92, wherein at least one LNP comprises Lipid A, cholesterol, DSPC, and PEG2k-DMG.

94. The composition of any of embodiments 67-93, wherein at least one LNP comprises Lipid B, cholesterol, DSPC, and PEG2k-DMG

95. The composition of any of embodiments 67-94, wherein the composition comprises the CCD lipid in an amount ranging from about 30 mol-% to about 60 mol-%.

96. The composition of any of embodiments 67-95, wherein the composition comprises the helper lipid in an amount ranging from about 30 mol-% to about 60 mol-%.

97. The composition of any of embodiments 67-96, wherein the composition comprises the neutral lipid in an amount ranging from about 1 mol-% to about 20 mol-%.

98. The composition of any of embodiments 67-97, wherein the composition comprises the stealth lipid in an amount ranging from about 1 mol-% to about 10 mol-%.

99. The composition of any of embodiments 67-98, wherein the composition comprises the CCD lipid in an amount of about 45 mol-%.

100. The composition of any of embodiments 67-99, wherein the composition comprises the helper lipid in an amount of about 44 mol-%.

101. The composition of any of embodiments 67-100, wherein the composition comprises the neutral lipid in an amount of about 9 mol-%.

102. The composition of any of embodiments 67-101, wherein the composition comprises the stealth lipid in an amount of about 2 mol-%.

103. The composition of any of embodiments 67-102, wherein the Class 2 Cas nuclease mRNA and the guide RNA nucleic acid are present in a ratio ranging from about 10:1 to about 1:10 by weight.

104. The composition of any of embodiments 67-103, wherein the Class 2 Cas nuclease mRNA and the guide RNA nucleic acid are present in a molar ratio of about 1:1 by weight.

105. The composition of any of embodiments 67-104, wherein the ratio of the CCD lipid amine to the RNA phosphate ranges from about 3 to about 5.

106. The composition of any of embodiments 67-105, wherein the ratio of the CCD lipid amine to the RNA phosphate is about 4.5.

107. The composition of any of embodiments 67-106, wherein the particle size of the composition ranges from about 50 nm to about 120 nm.

108. The composition of any of embodiments 67-107, wherein the particle size of the composition ranges from about 75 nm to about 150 nm.

109. The composition of any of embodiments 67-108, wherein the encapsulation efficiency of the composition ranges from about 70% to about 100%.

110. The composition of any of embodiments 67-109, wherein the polydispersity index of the composition ranges from about .005 to about 0.5.

111. The composition of any of embodiments 67-110, wherein the polydispersity index of the composition ranges from about .02 to about 0.35.

112. The composition of any of embodiments 67-111, wherein the composition is liver-selective.

113. The composition of embodiment 112, wherein the composition is hepatocyte-selective.

114. The composition of embodiment 112, wherein the composition is ApoE receptor selective.

115. A genetically engineered liver cell, made by a process of any of embodiments 1-59.

116. A genetically engineered liver cell made with a composition of any of embodiments 67-114.

117. The genetically engineered liver cell of embodiment 115 or 116, wherein the liver cell is a primary hepatocyte.

118. The composition of any of embodiments 67-114 further comprising a cryoprotectant.

119. The composition of embodiment 118, wherein the cryoprotectant is present in an amount ranging from about 1% to about 10% w/v.

120. The composition of embodiment 118 or 119, wherein the cryoprotectant is chosen from sucrose, trehalose, glycerol, DMSO, and ethylene glycol.

121. The composition of any of embodiments 118-120, wherein the cryoprotectant is sucrose.

122. The composition of any of embodiments 67-114 or 118-121 further comprising a buffer.

123. The composition of embodiment 122, wherein the buffer is chosen from a phosphate buffer (PBS), a Tris buffer, a citrate buffer, and mixtures thereof.

124. The composition of embodiment 122 or 123, further comprising NaCl.

125. The composition of embodiment 124, wherein:

the cryoprotectant is sucrose;
the sucrose is present in an amount ranging from about 1% to about 10% w/v;
the buffer is a mixture of the Tris buffer and the NaCl buffer;
the NaCl buffer is present in an amount ranging from about 40 mM to about 50 mM; and
the Tris buffer is present in an amount ranging from about 40 mM to about 60 mM.

126. The composition of embodiment 125, wherein:

the sucrose is present in an amount of about 5% w/v;
the NaCl buffer is present in an amount of about 45 mM; and
the Tris buffer is present in an amount of about 50 mM.

127. The composition of embodiment 125 or 126, wherein the composition has a pH ranging from about 7.3 to about 7.7.

128. The composition of embodiment 127, wherein the composition has a pH of about 7.3, about 7.4, about 7.5, or about 7.6.

129. The composition of embodiment 127 or 128, wherein the composition has a pH ranging from about 7.4 to about 7.6.

130. The composition of embodiment 129, wherein the composition has a pH of about 7.5.

131. The method of any of embodiments 1-66, further comprising achieving at least 20% editing efficiency.

132. The method of any of embodiments 1-66, further comprising achieving at least 50% editing efficiency.

133. The method of any of embodiments 1-66, further comprising achieving at least 80% editing efficiency.

134. The method of any of embodiments 1-66, further comprising achieving at least 20% DNA modification efficiency.

135. The method of any of embodiments 1-66, further comprising achieving at least 50% DNA modification efficiency.

136. The method of any of embodiments 1-66, further comprising achieving at least 80% DNA modification efficiency.

## Claims

**1.** An LNP composition comprising:

an mRNA encoding a Cas nuclease;
a guide RNA nucleic acid, optionally wherein the guide RNA is a single guide RNA (sgRNA);
a CCD lipid, wherein the CCD lipid is Lipid A;
a helper lipid;
a neutral lipid; and

a stealth lipid.

2. The LNP composition of claim 1, wherein the mRNA encoding the Cas nuclease is:
a Class 2 Cas nuclease mRNA, optionally wherein the Class 2 Cas nuclease is a Cas9 nuclease, further optionally wherein the Cas9 nuclease mRNA is a human codon-optimized Cas9 nuclease.

3. The LNP composition of claim 1 or 2, further comprising:
a means for delivering the RNA in a liver-specific manner a means for delivering the RNA to a liver cell, or a biodegradable means for delivering the RNA to a liver cell.

4. The composition of any of the preceding claims, wherein the mRNA and the guide RNA nucleic acid are separately encapsulated in LNPs, and the LNPs are combined to form the LNP composition.

5. The composition of any of claims 1-3, wherein the mRNA and the guide RNA nucleic acid are co-encapsulated in the LNP composition.

6. The composition of any of the preceding claims, further comprising at least one template.

7. The composition of any of the preceding claims, wherein the guide RNA nucleic acid is an expression cassette that encodes a guide RNA, optionally wherein the expression cassette further comprises a regulatory element.

8. The composition of any of the preceding claims, wherein the guide RNA nucleic acid is an sgRNA or a dual-guide RNA, optionally wherein the guide RNA nucleic acid comprises a modified residue, optionally wherein the modified residue comprises a modification selected from a backbone modification, a sugar modification, and a base modification.

9. The composition of any of the preceding claims, wherein the helper lipid is selected from cholesterol, 5-heptadecylresorcinol, and cholesterol hemisuccinate, optionally wherein the helper lipid is cholesterol.

10. The composition of any of the preceding claims, wherein the neutral lipid is selected from DSPC and DMPE, optionally wherein the neutral lipid is DSPC.

11. The composition of any of the preceding claims, wherein the stealth lipid is selected from PEG2k-DMG and PEG2k-C11, optionally wherein the stealth lipid is PEG2k-DMG.

12. The composition of any of the preceding claims, wherein at least one LNP comprises Lipid A, cholesterol, DSPC, and PEG2k-DMG.

13. The composition of any of the preceding claims, wherein the composition comprises Lipid A in an amount ranging from about 30 mol-% to about 60 mol-%, optionally wherein the composition comprises Lipid A in an amount of about 45 mol-%.

14. The composition of any of the preceding claims, wherein the composition comprises the helper lipid in an amount ranging from about 30 mol-% to about 60 mol-%, optionally wherein the composition comprises the helper lipid in an amount of about 44 mol-%.

15. The composition of any of the preceding claims, wherein the composition comprises the neutral lipid in an amount ranging from about 1 mol-% to about 20 mol-%, optionally wherein the composition comprises the neutral lipid in an amount of about 9 mol-%.

16. The composition of any of the preceding claims, wherein the composition comprises the stealth lipid in an amount ranging from about 1 mol-% to about 10 mol-%, optionally wherein the composition comprises the stealth lipid in an amount of about 2 mol-%.

17. The composition of any of the preceding claims, wherein the Class 2 Cas nuclease mRNA and the guide RNA nucleic acid are present in a ratio ranging from about 10:1 to about 1:10 by weight, or about 5:1 to 1:5 by weight.

18. The composition of any of the preceding claims, wherein the Class 2 Cas nuclease mRNA and the guide RNA nucleic acid are present in a molar ratio of about 1: 1 by weight.

**19.** The composition of any of the preceding claims, wherein the ratio of Lipid A to the RNA phosphate ranges from about 3 to about 5, or wherein the ratio of Lipid A to the RNA phosphate is about 4.5.

**20.** The composition of any of the preceding claims, wherein the particle size of the composition ranges from about 50 nm to about 120 nm, or from about 75 nm to about 150 nm.

**21.** The composition of any of the preceding claims, wherein the encapsulation efficiency of the composition ranges from about 70% to about 100%.

**22.** The composition of any of the preceding claims, wherein the polydispersity index of the composition ranges from about .005 to about 0.5, such as wherein the polydispersity index of the composition ranges from about .02 to about 0.35.

**23.** The composition of any of the preceding claims, wherein the composition is liver-selective, optionally wherein the composition is hepatocyte-selective, optionally wherein the composition is ApoE receptor selective.

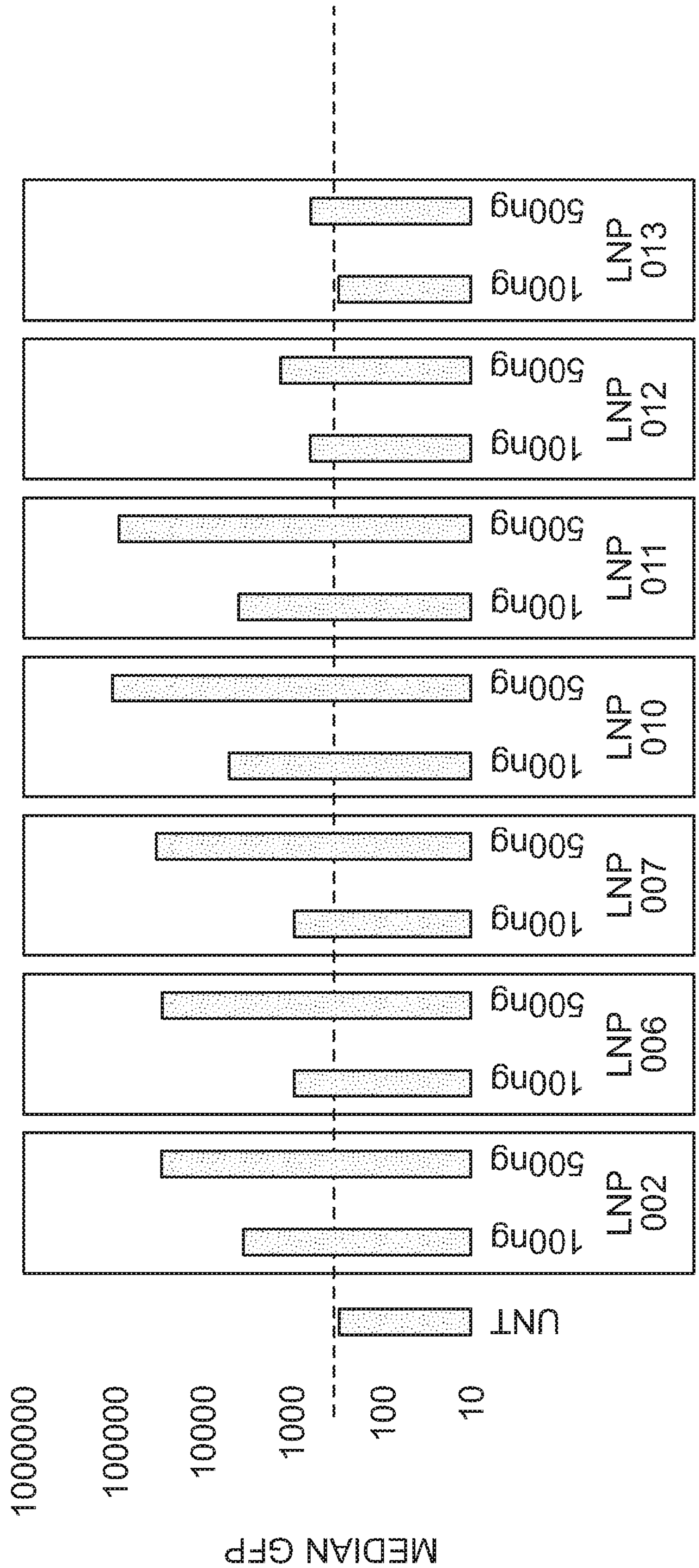
MEDIAN GFP
1000000
100000
10000
1000
100
10
UNT
100ng
500ng
LNP 002
LNP 006
LNP 007
LNP 010
LNP 011
LNP 012
LNP 013

FIG. 1

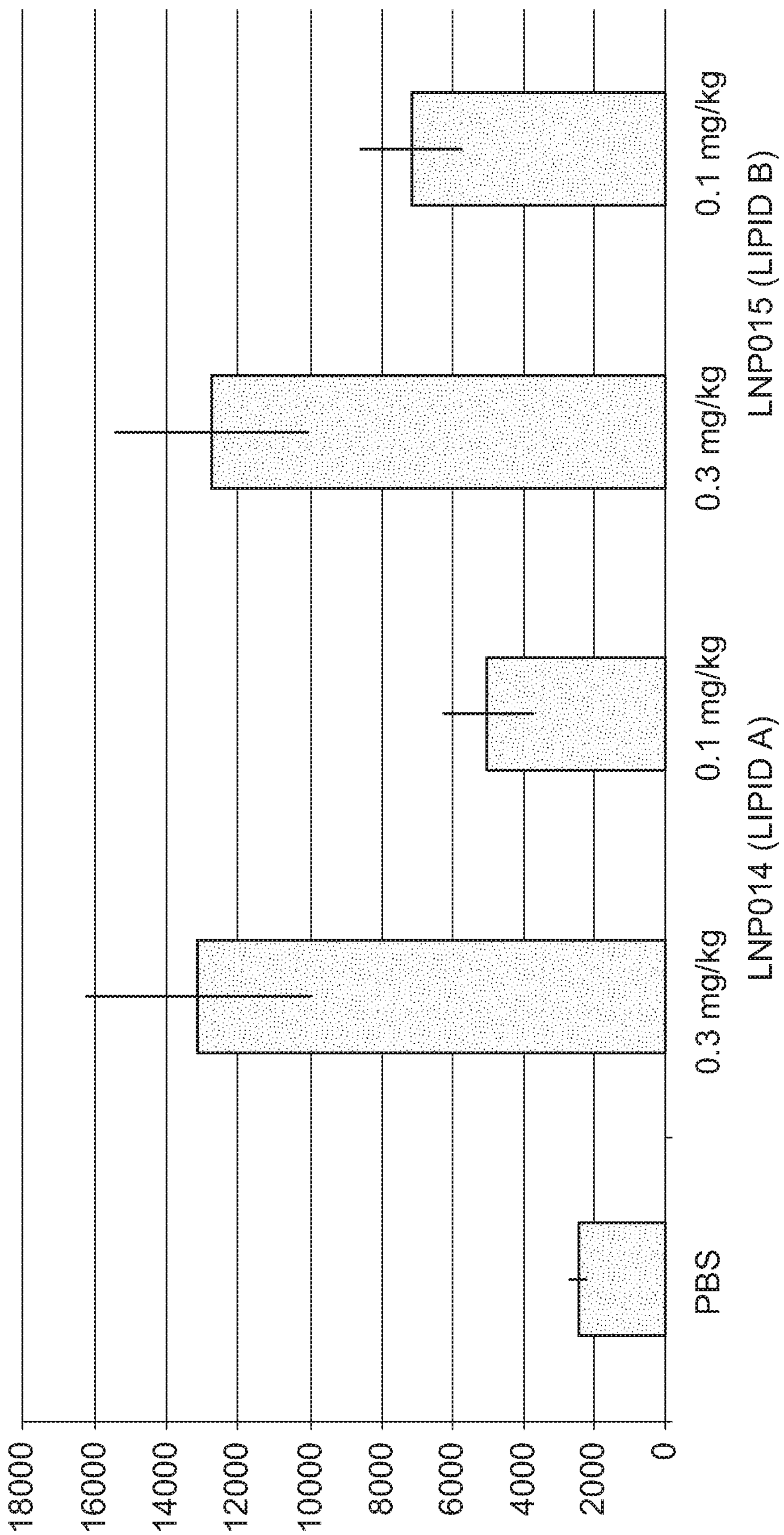
LUm
18000
16000
14000
12000
10000
8000
6000
4000
2000
0
PBS
0.3 mg/kg
0.1 mg/kg
LNP014 (LIPID A)
0.3 mg/kg
0.1 mg/kg
LNP015 (LIPID B)

FIG. 2

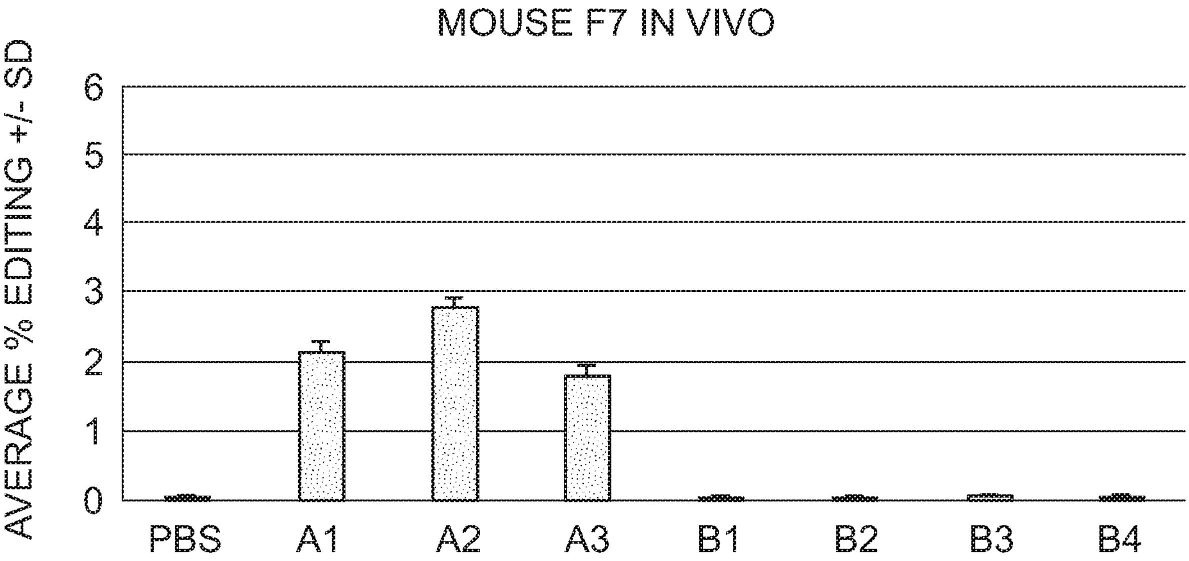
MOUSE F7 IN VIVO
AVERAGE % EDITING +/- SD
6
5
4
3
2
1
0
PBS
A1
A2
A3
B1
B2
B3
B4

FIG. 3A

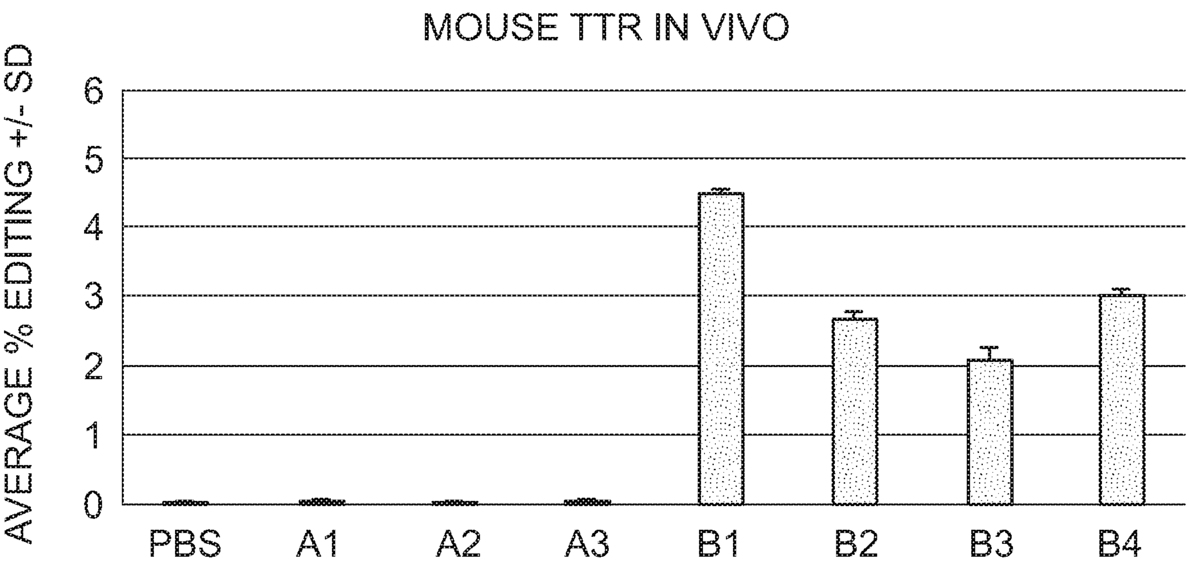
MOUSE TTR IN VIVO
AVERAGE % EDITING +/- SD
6
5
4
3
2
1
0
PBS
A1
A2
A3
B1
B2
B3
B4

FIG. 3B

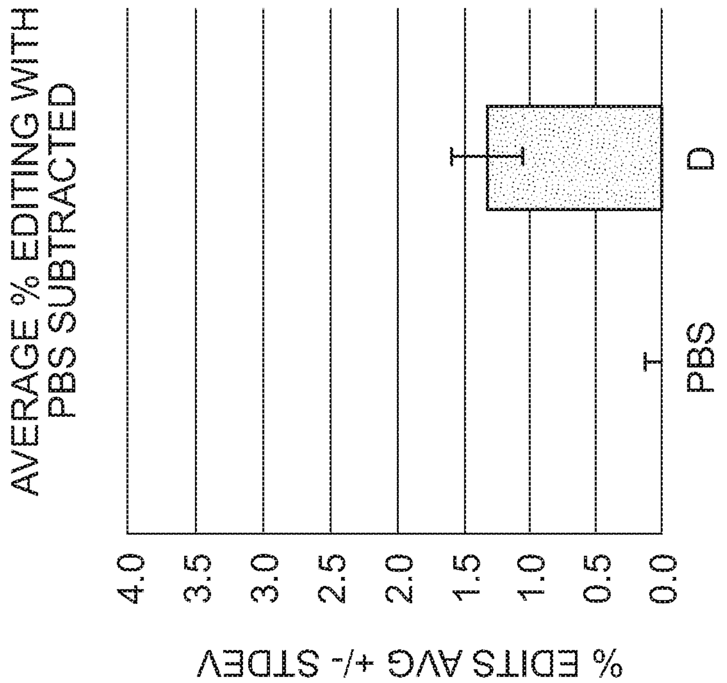
AVERAGE % EDITING WITH
PBS SUBTRACTED
% EDITS AVG +/- STDEV
4.0
3.5
3.0
2.5
2.0
1.5
1.0
0.5
0.0
PBS
D

FIG. 4B

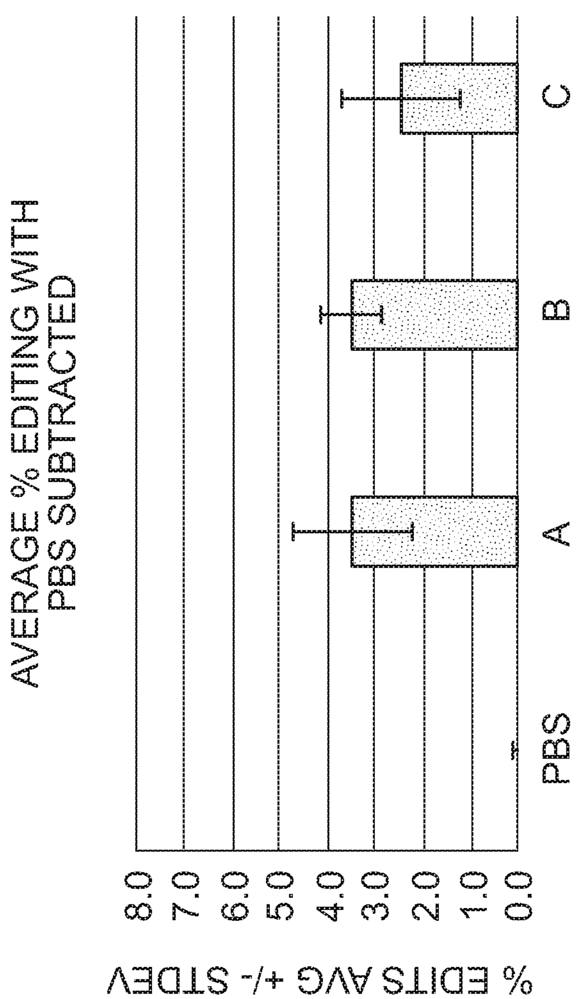
AVERAGE % EDITING WITH
PBS SUBTRACTED
% EDITS AVG +/- STDEV
8.0
7.0
6.0
5.0
4.0
3.0
2.0
1.0
0.0
PBS
A
B
C

FIG. 4A

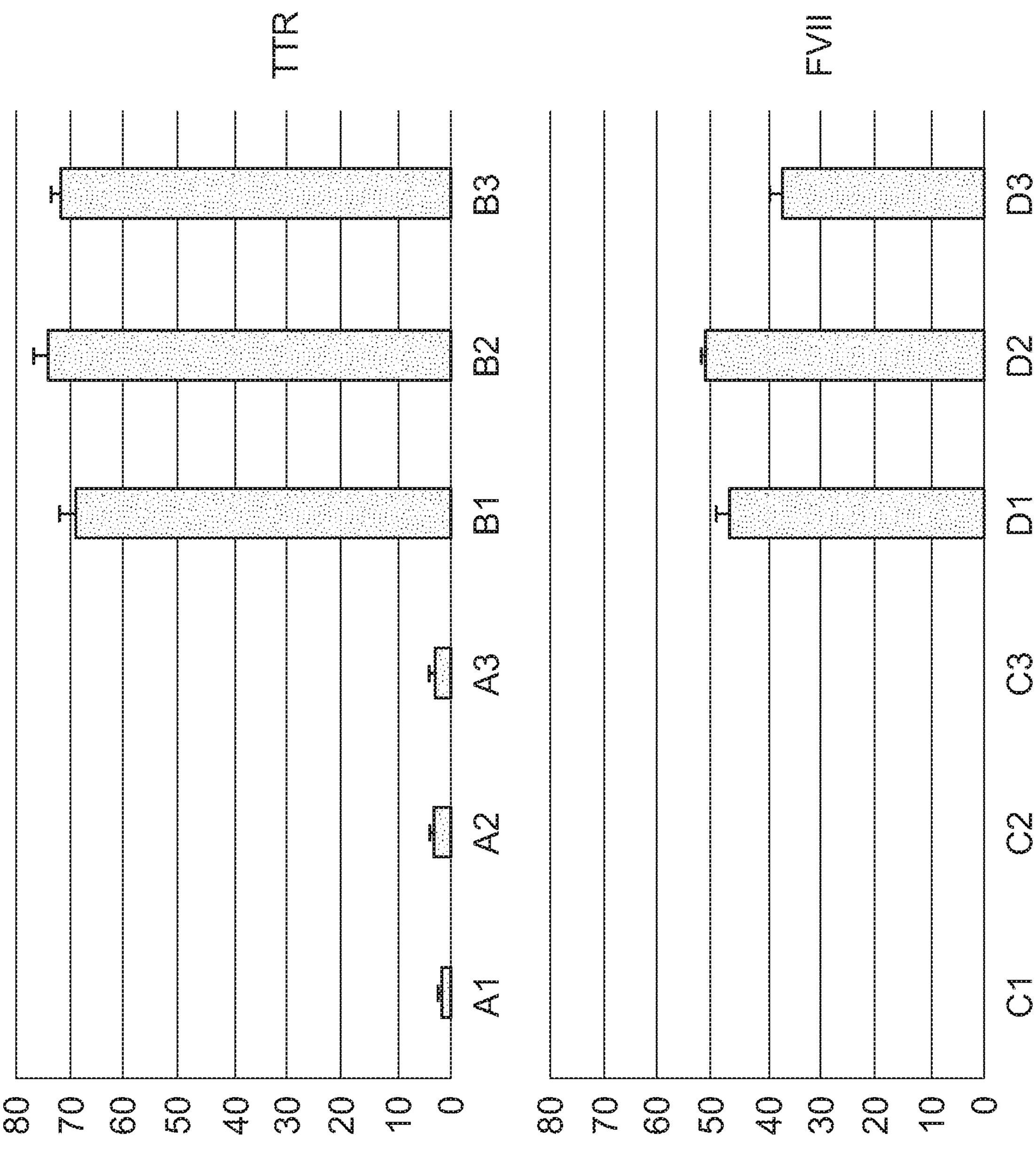
TTR
AVERAGE % EDITING
0
10
20
30
40
50
60
70
80
A1
A2
A3
B1
B2
B3
FVII
AVERAGE % EDITING
0
10
20
30
40
50
60
70
80
C1
C2
C3
D1
D2
D3

FIG. 5

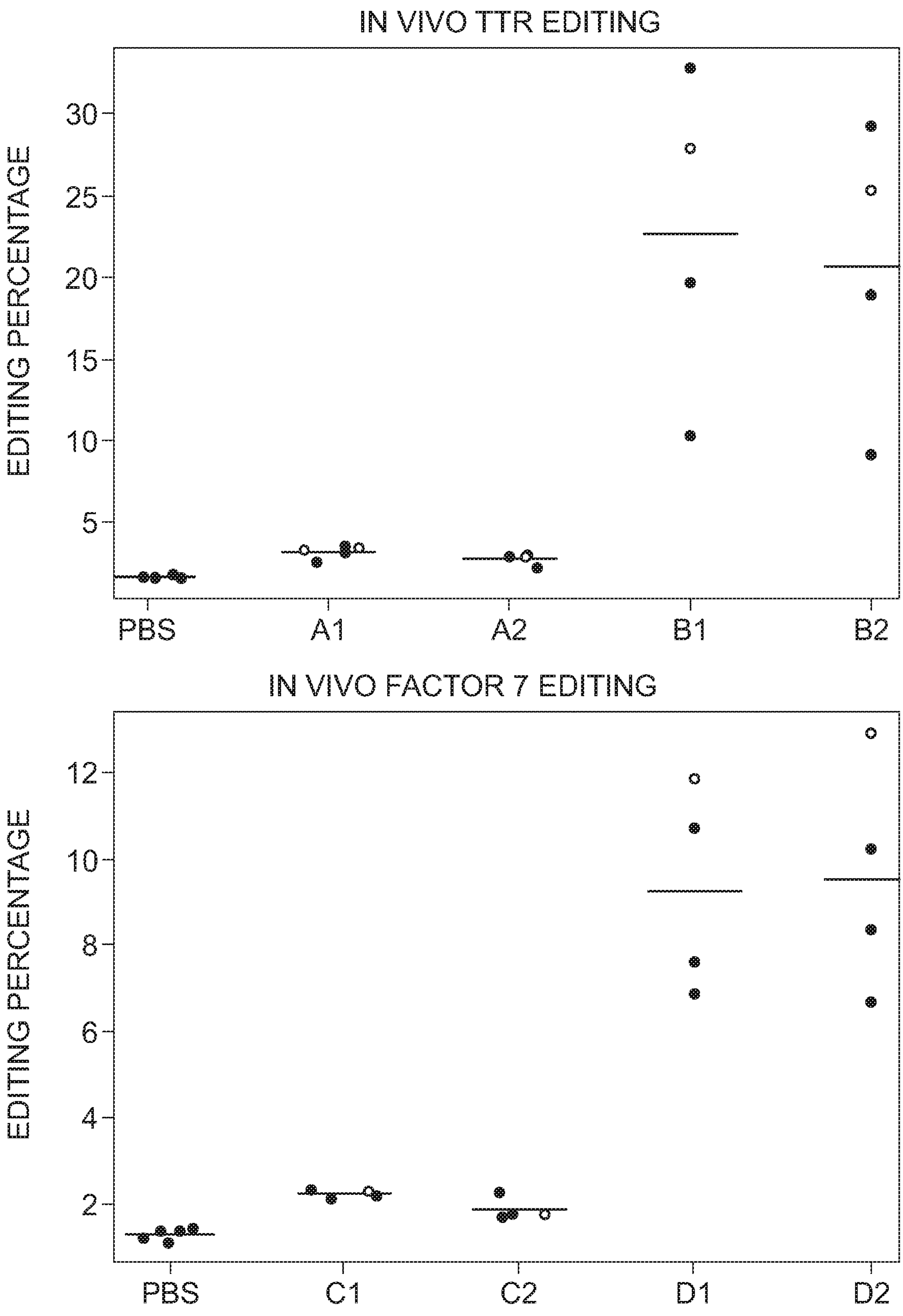
IN VIVO TTR EDITING
EDITING PERCENTAGE
30
25
20
15
10
5
PBS
A1
A2
B1
B2
IN VIVO FACTOR 7 EDITING
EDITING PERCENTAGE
12
10
8
6
4
2
PBS
C1
C2
D1
D2

FIG. 6

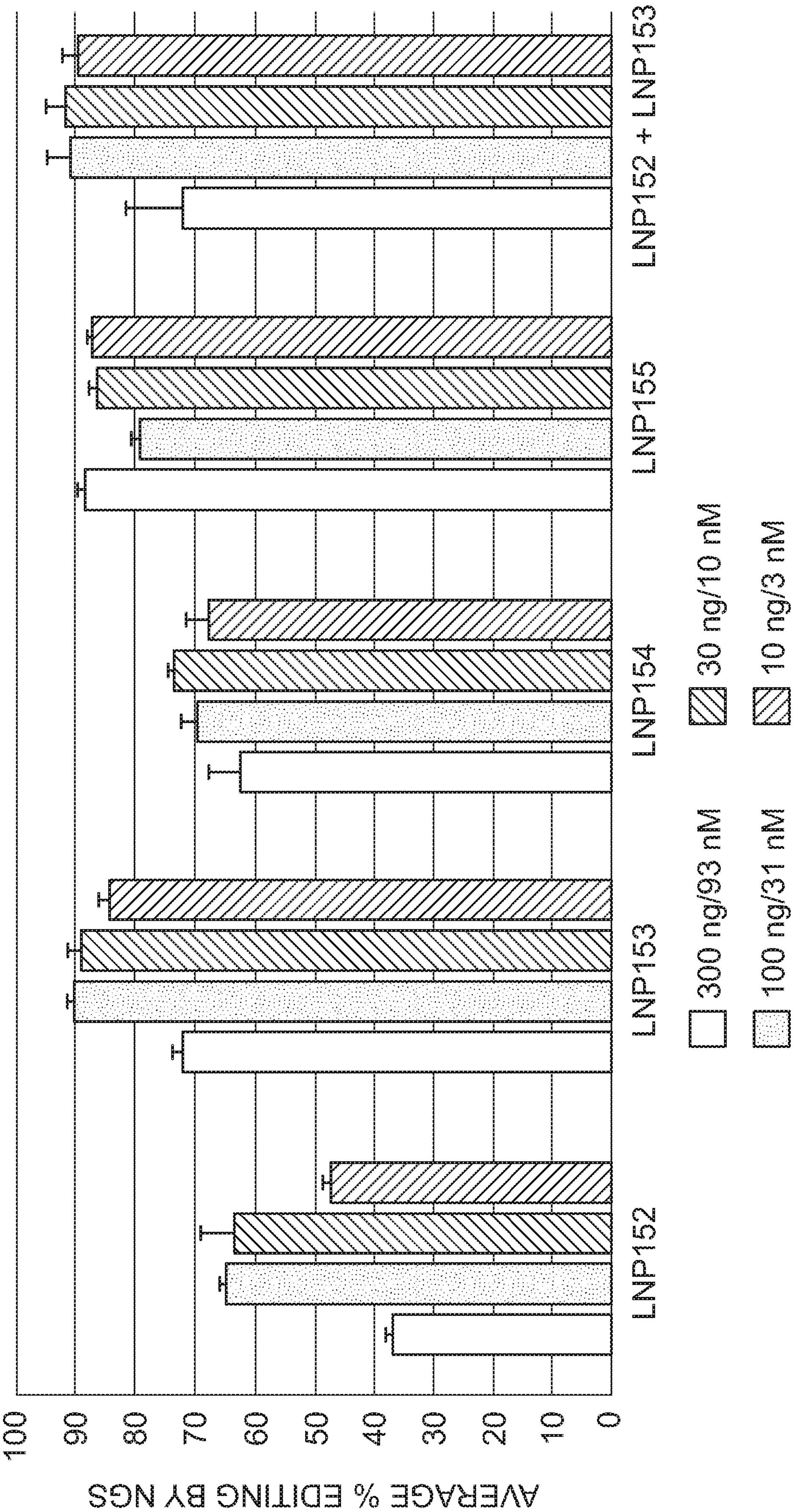
AVERAGE % EDITING BY NGS
0
10
20
30
40
50
60
70
80
90
100
LNP152
LNP153
LNP154
LNP155
LNP152 + LNP153
300 ng/93 nM
30 ng/10 nM
100 ng/31 nM
10 ng/3 nM

FIG. 7

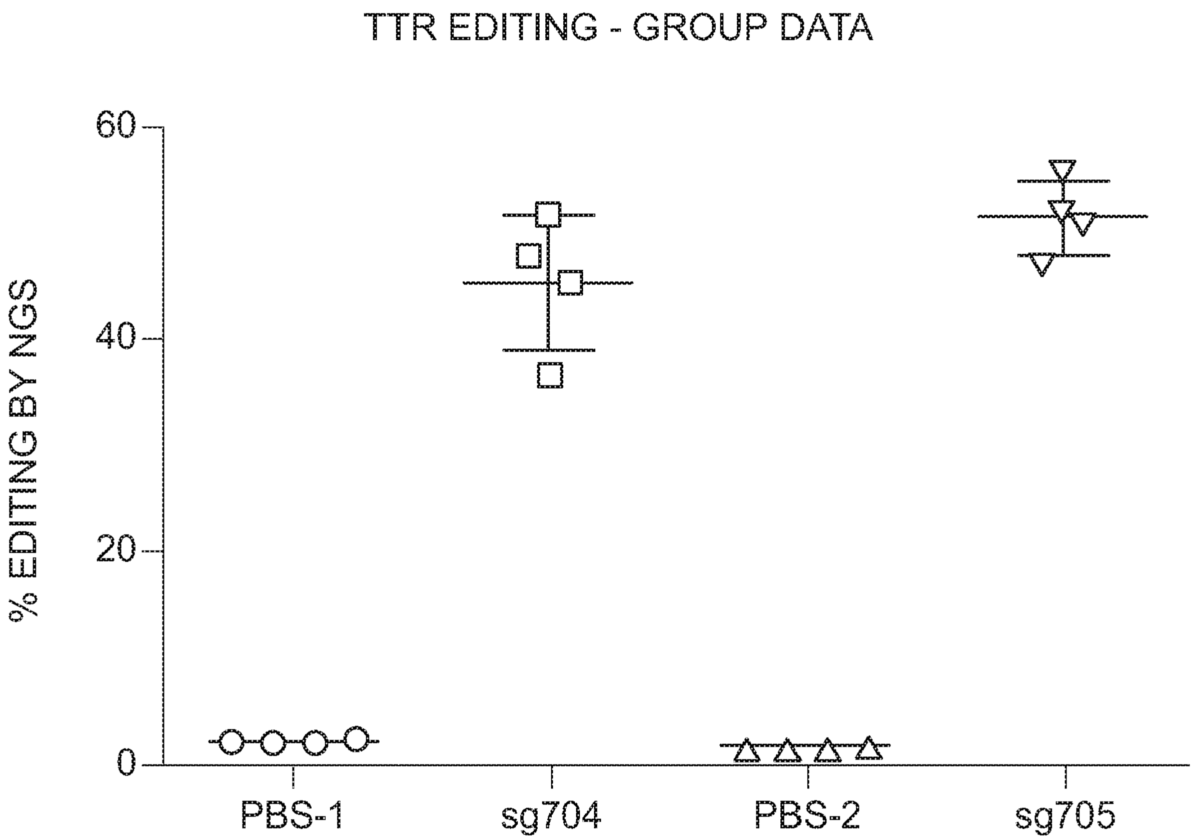
TTR EDITING - GROUP DATA
60
40
20
0
% EDITING BY NGS
PBS-1
sg704
PBS-2
sg705

FIG. 8A

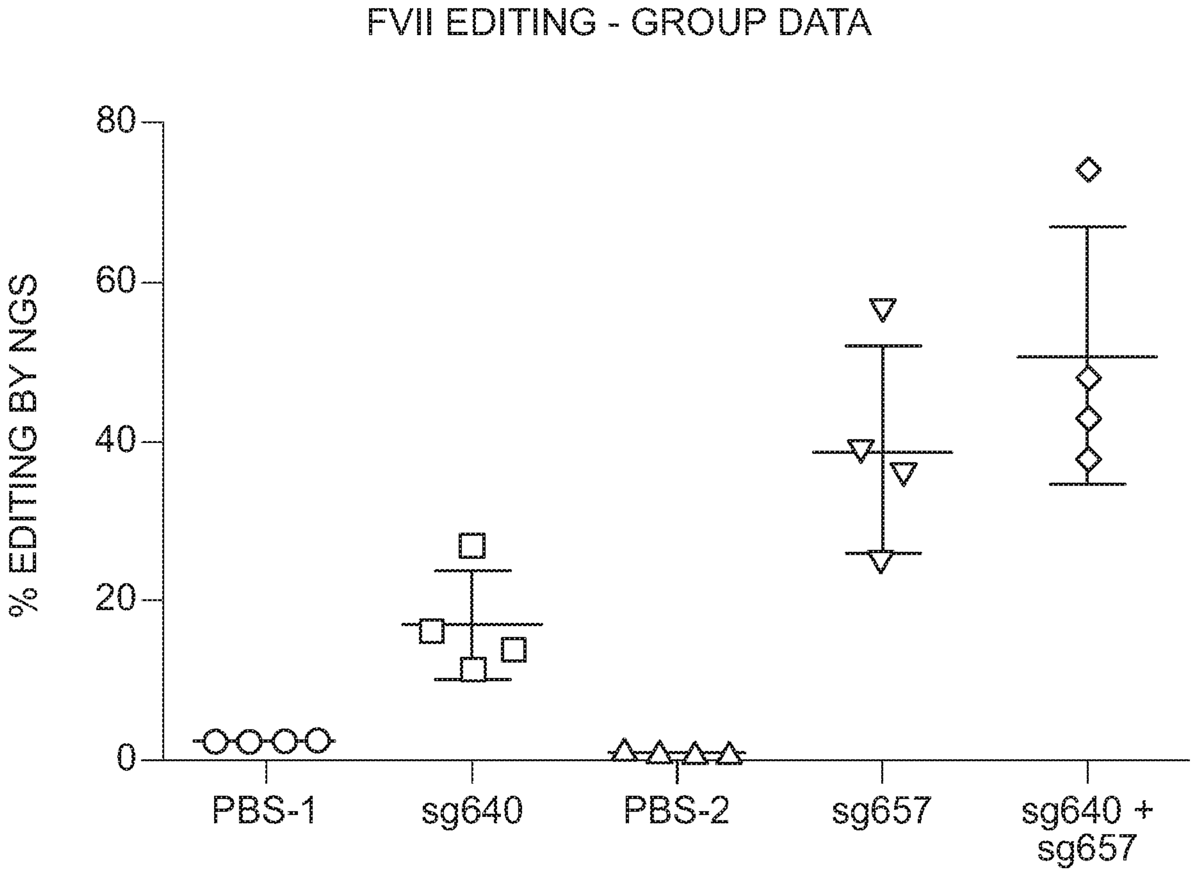
FVII EDITING - GROUP DATA
% EDITING BY NGS
80
60
40
20
0
PBS-1
sg640
PBS-2
sg657
sg640 + sg657

*FIG. 8B*

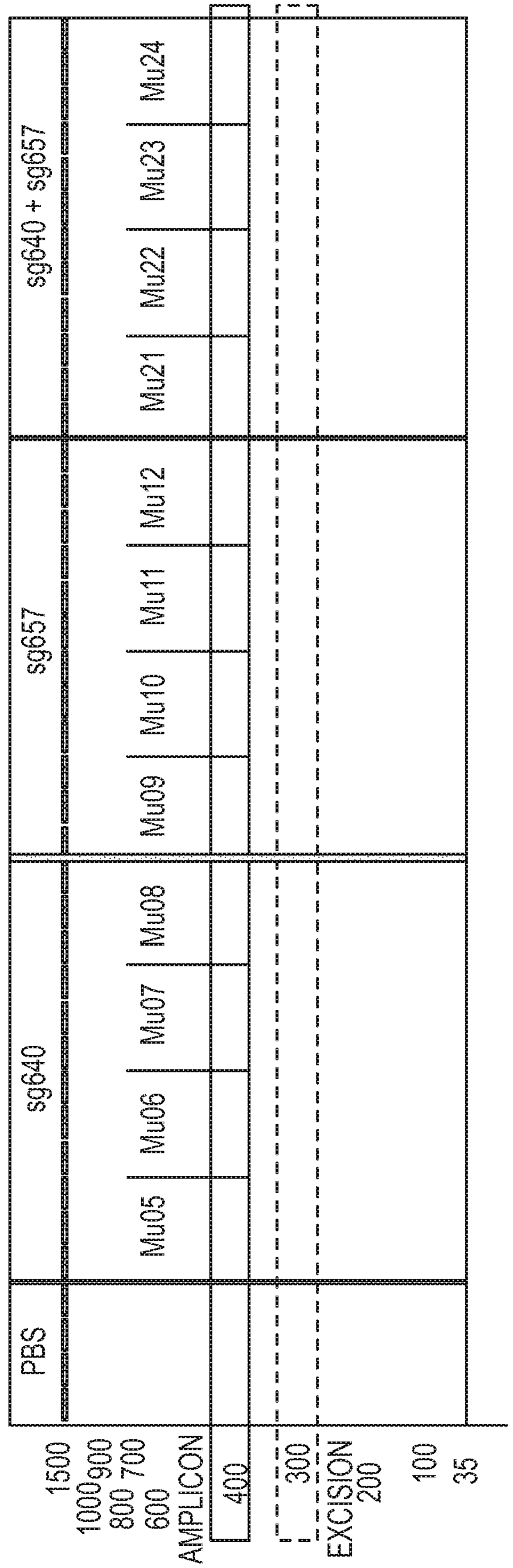
PBS
sg640
sg657
sg640 + sg657
Mu05
Mu06
Mu07
Mu08
Mu09
Mu10
Mu11
Mu12
Mu21
Mu22
Mu23
Mu24
1500
1000
900
800
700
600
AMPLICON
400
300
EXCISION
200
100
35

FIG. 9

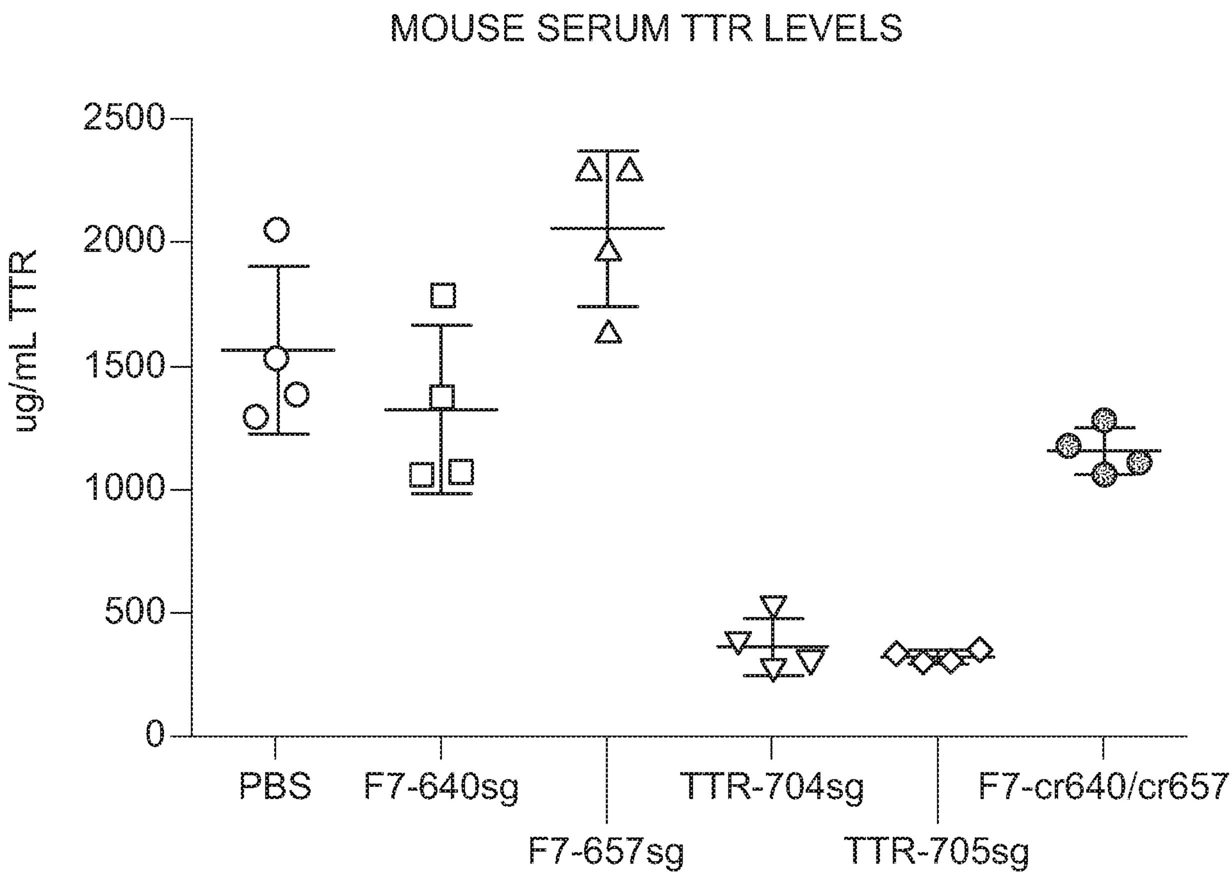
MOUSE SERUM TTR LEVELS
ug/mL TTR
2500
2000
1500
1000
500
0
PBS
F7-640sg
F7-657sg
TTR-704sg
TTR-705sg
F7-cr640/cr657

*FIG. 10*

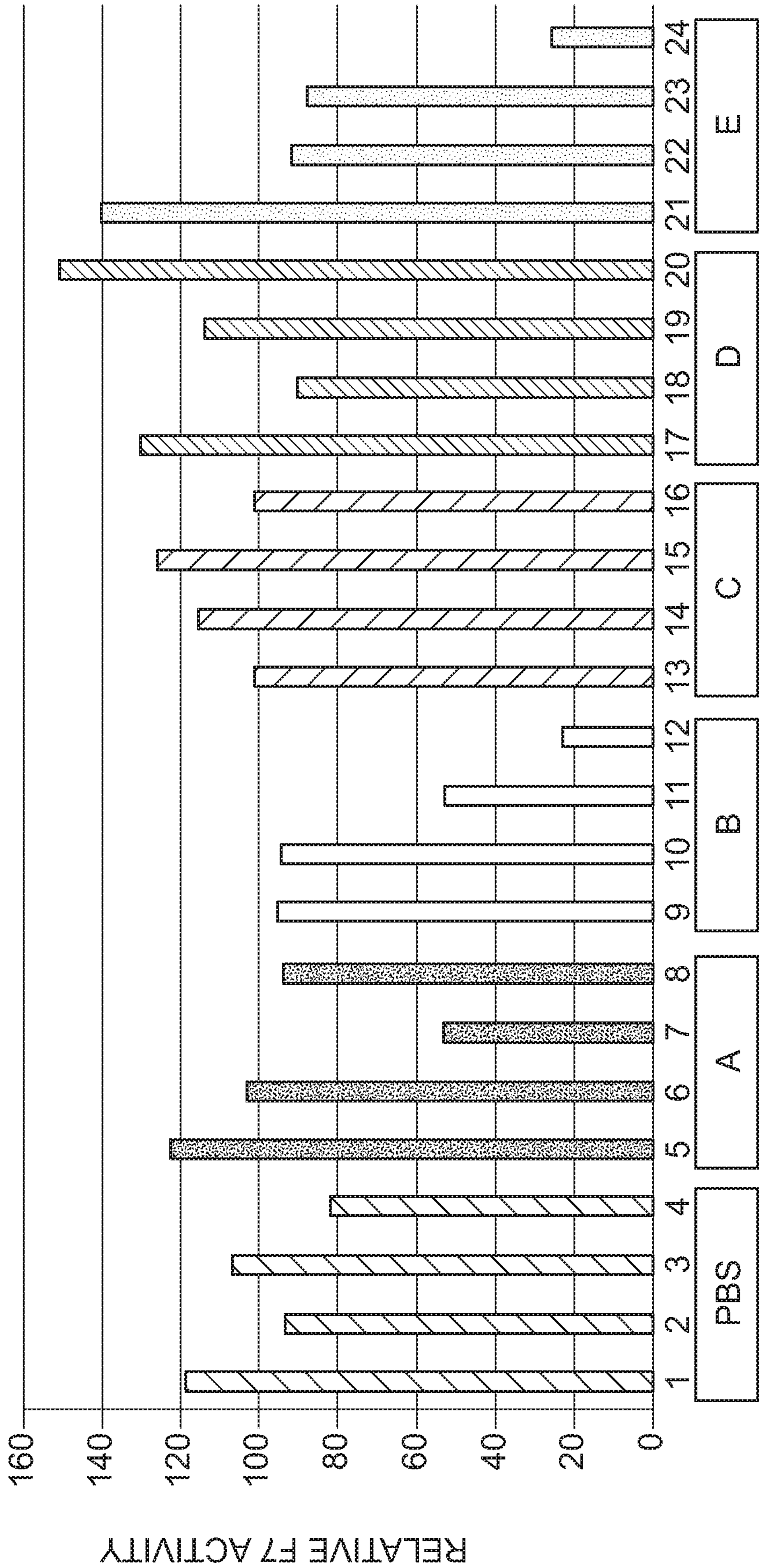
RELATIVE F7 ACTIVITY
160
140
120
100
80
60
40
20
0
1
2
3
4
5
6
7
8
9
10
11
12
13
14
15
16
17
18
19
20
21
22
23
24
PBS
A
B
C
D
E

FIG. 11

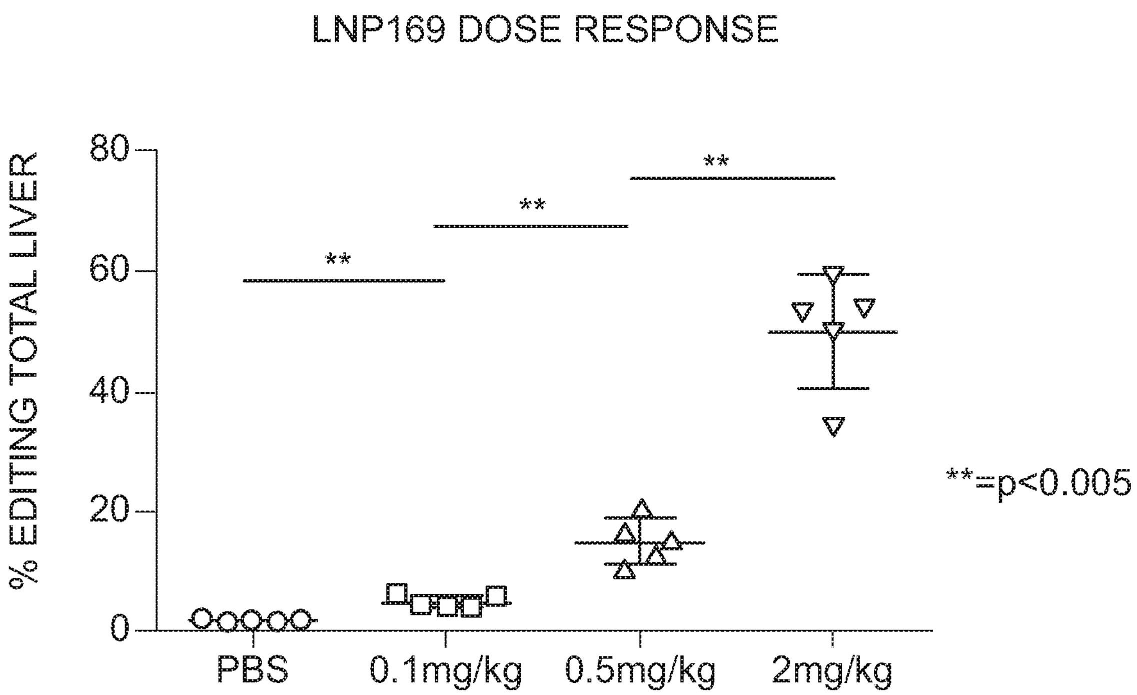
LNP169 DOSE RESPONSE
% EDITING TOTAL LIVER
80
60
40
20
0
**
**
**
**=p<0.005
PBS
0.1mg/kg
0.5mg/kg
2mg/kg

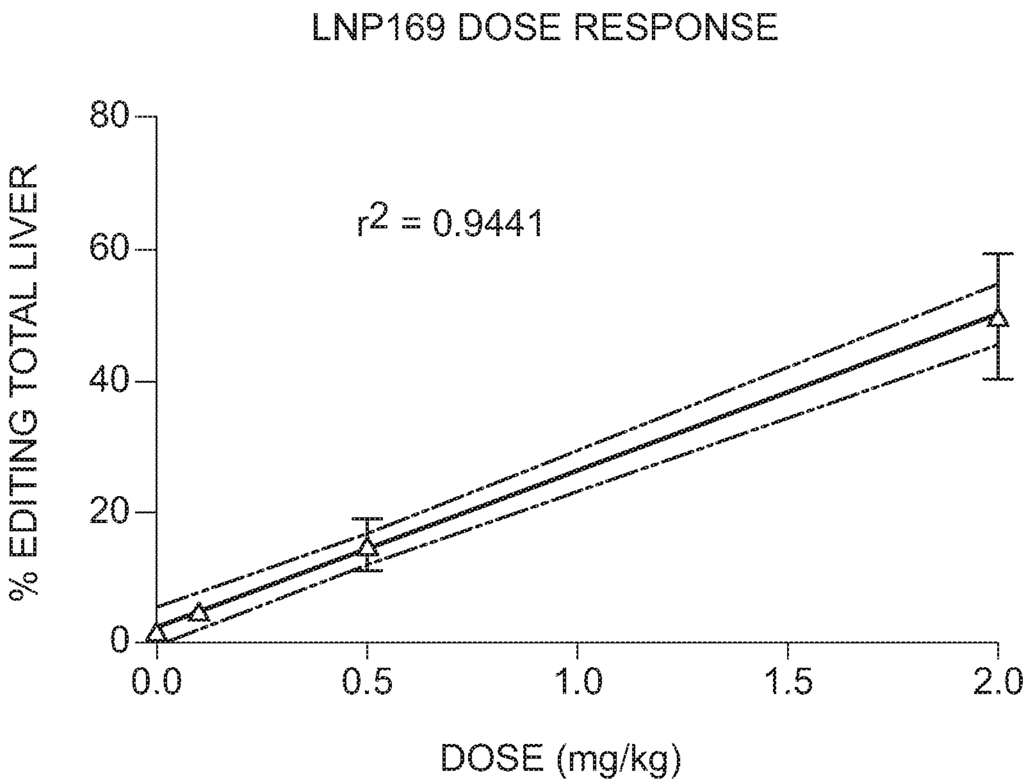
LNP169 DOSE RESPONSE
% EDITING TOTAL LIVER
r2 = 0.9441
80
60
40
20
0
0.0
0.5
1.0
1.5
2.0
DOSE (mg/kg)

FIG. 12A

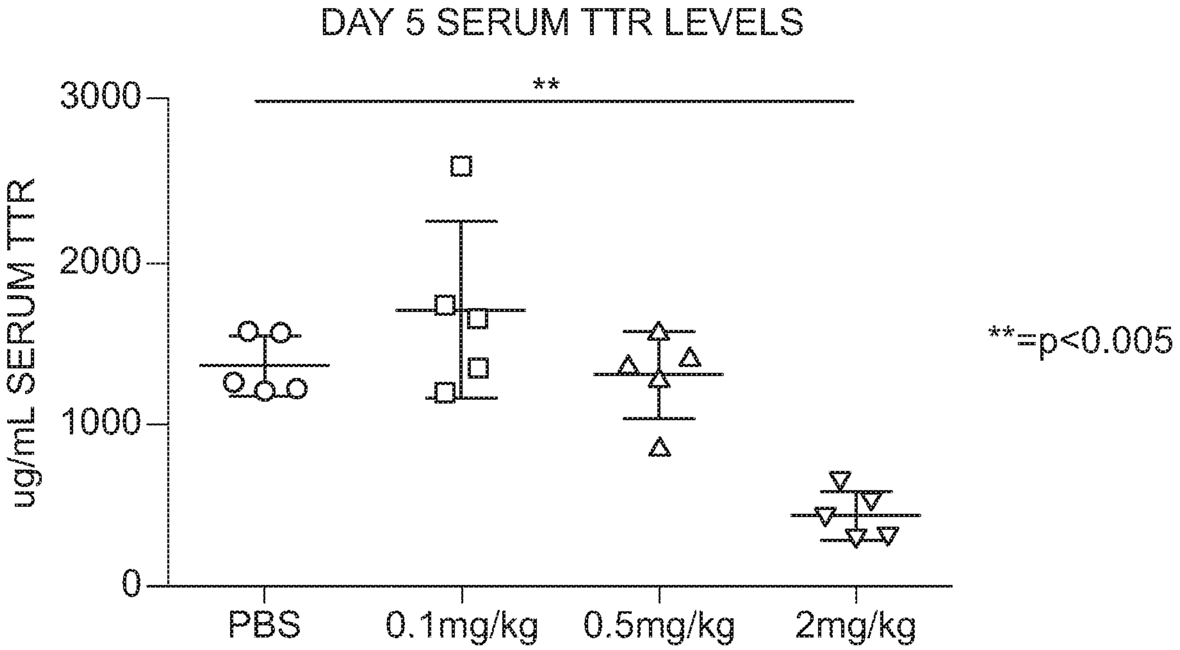
DAY 5 SERUM TTR LEVELS
**
3000
2000
1000
0
ug/mL SERUM TTR
PBS
0.1mg/kg
0.5mg/kg
2mg/kg
**=p<0.005

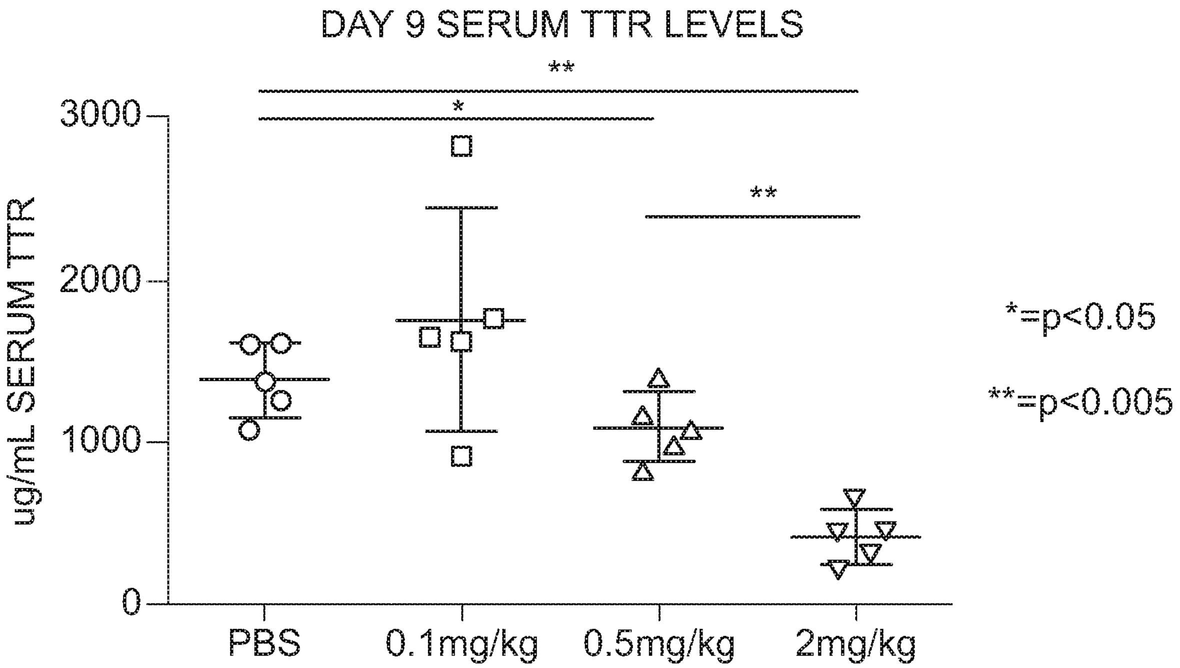
DAY 9 SERUM TTR LEVELS
**
*
**
3000
2000
1000
0
ug/mL SERUM TTR
PBS
0.1mg/kg
0.5mg/kg
2mg/kg
*=p<0.05
**=p<0.005

*FIG. 12B*

mRNA:sgRNA RATIO

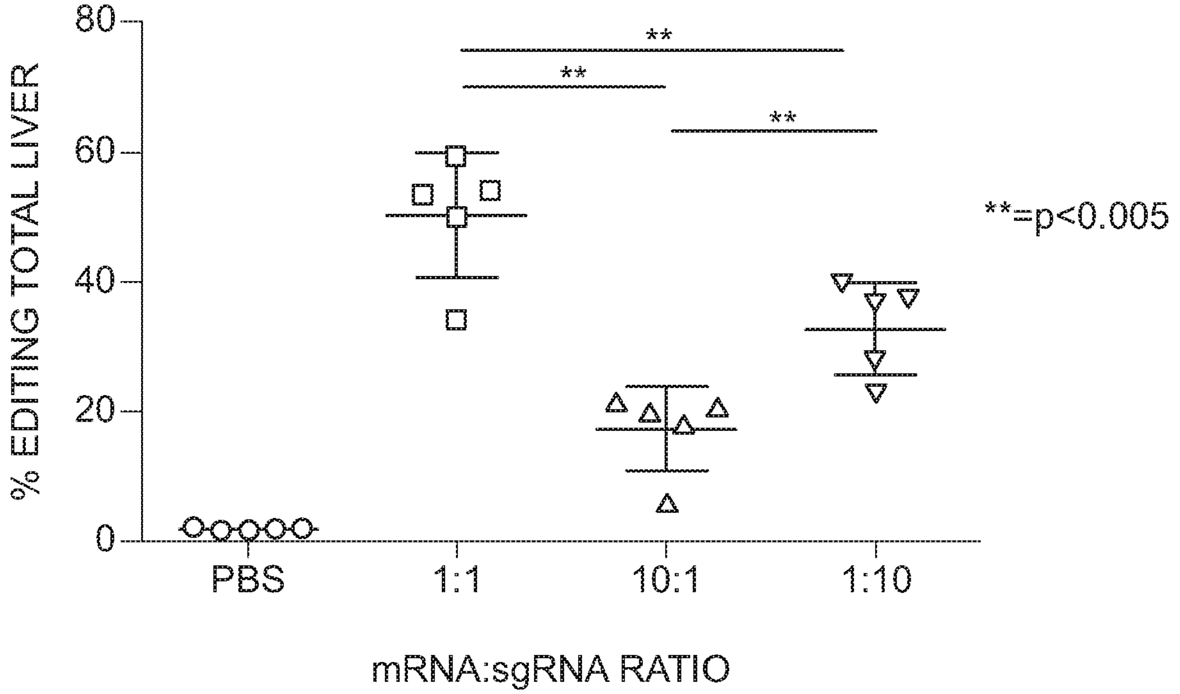


*FIG. 13A*

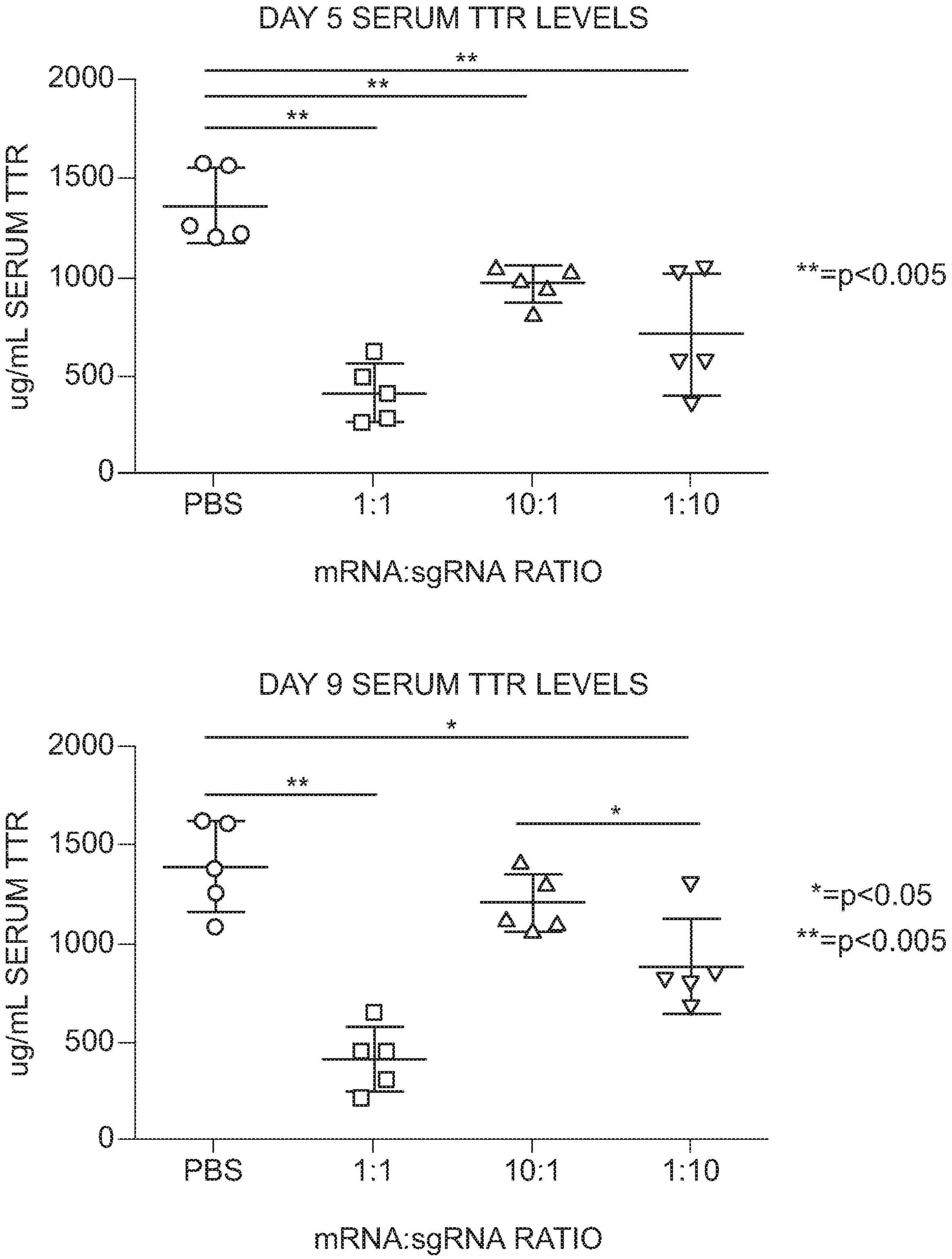
DAY 5 SERUM TTR LEVELS
**
**
**
2000
1500
1000
500
0
ug/mL SERUM TTR
PBS
1:1
10:1
1:10
mRNA:sgRNA RATIO
**=p<0.005
DAY 9 SERUM TTR LEVELS
*
**
*
2000
1500
1000
500
0
ug/mL SERUM TTR
PBS
1:1
10:1
1:10
mRNA:sgRNA RATIO
*=p<0.05
**=p<0.005

*FIG. 13B*

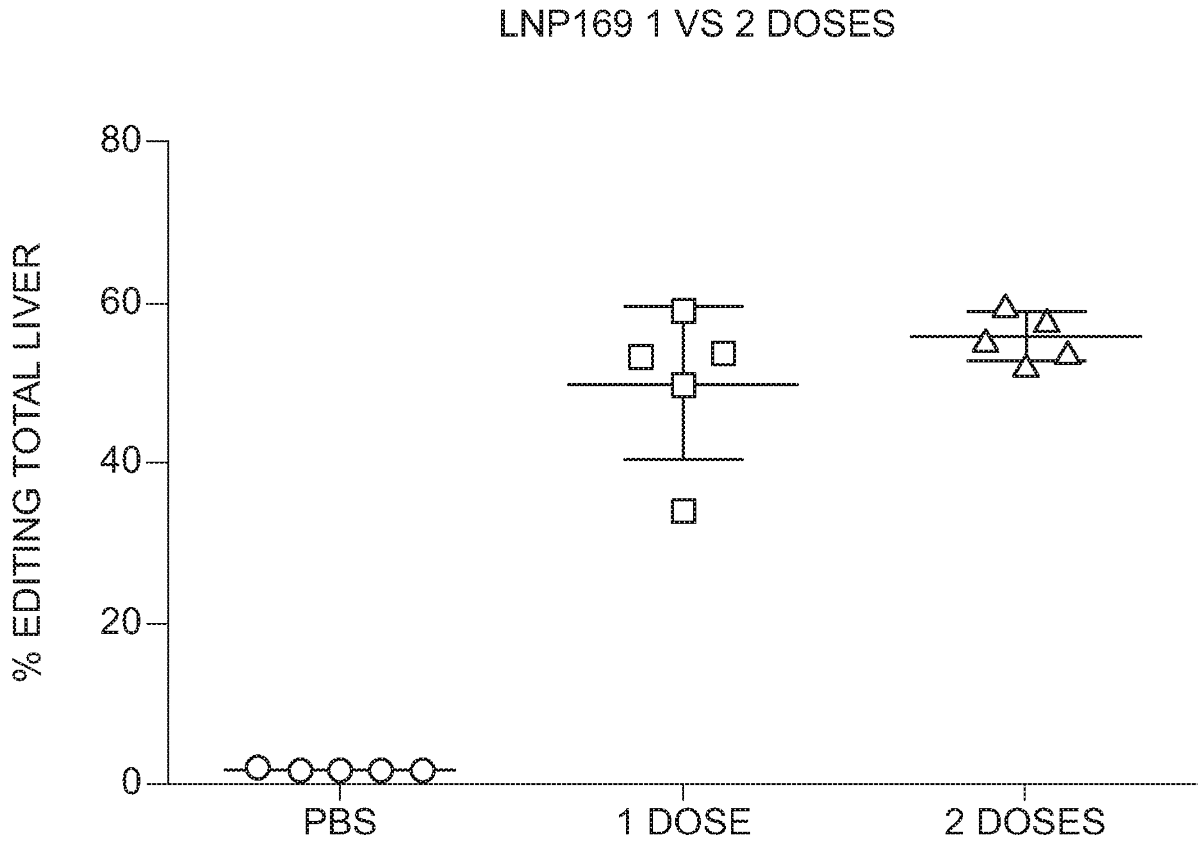
LNP169 1 VS 2 DOSES
% EDITING TOTAL LIVER
80
60
40
20
0
PBS
1 DOSE
2 DOSES

FIG. 14A

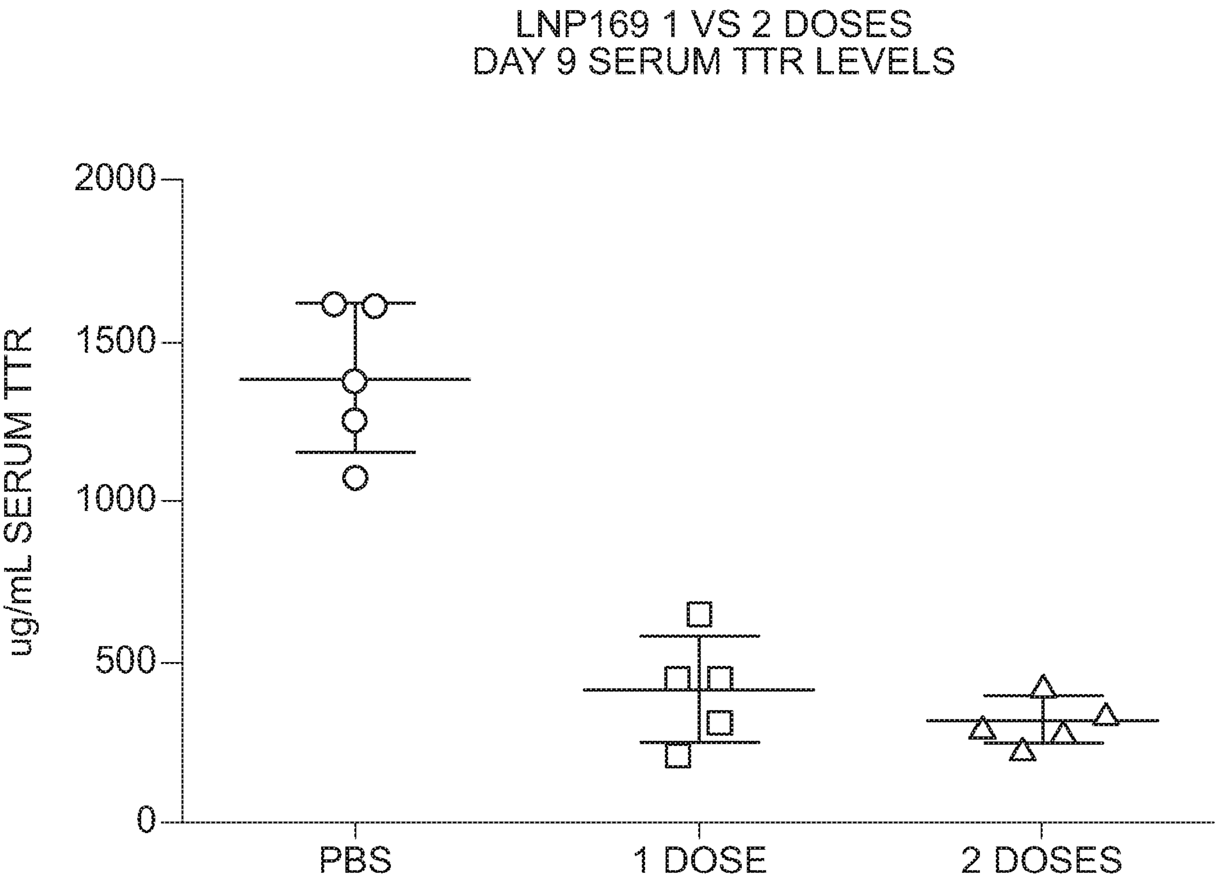
LNP169 1 VS 2 DOSES
DAY 9 SERUM TTR LEVELS
ug/mL SERUM TTR
2000
1500
1000
500
0
PBS
1 DOSE
2 DOSES

FIG. 14B

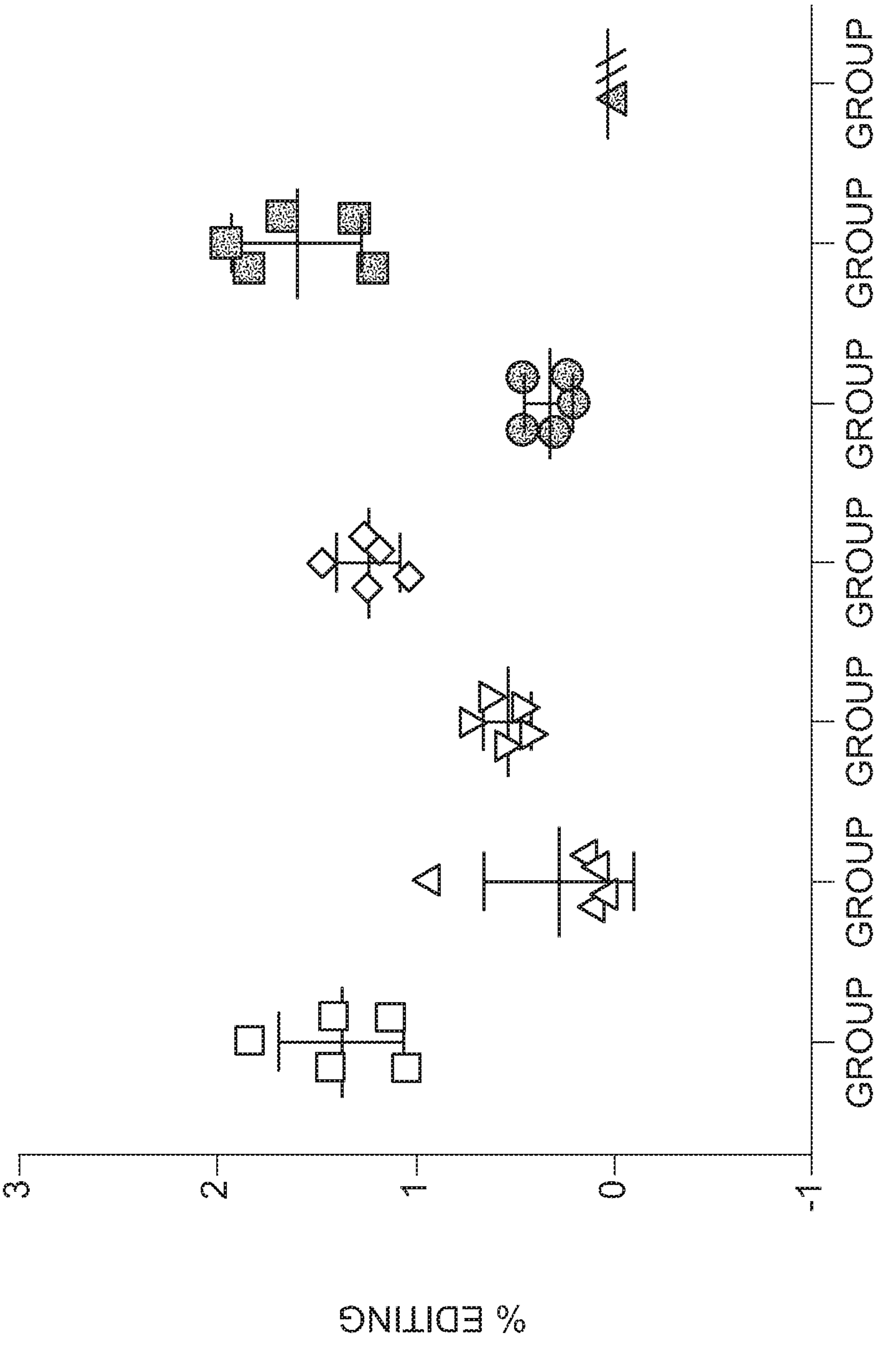
% EDITING
3
2
1
0
-1
GROUP 1
GROUP 2
GROUP 3
GROUP 4
GROUP 5
GROUP 6
GROUP 7

FIG. 15

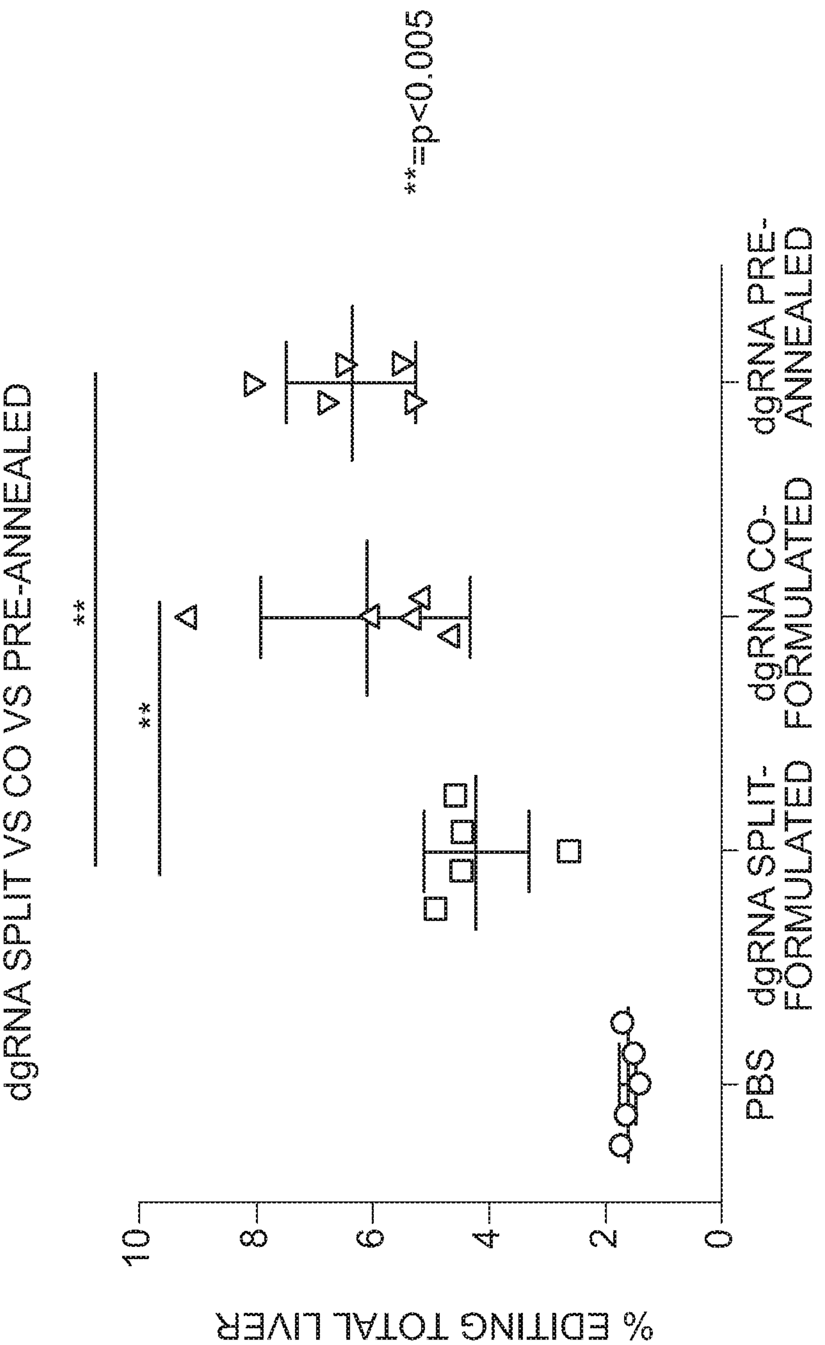
dgRNA SPLIT VS CO VS PRE-ANNEALED
**
**
**=p<0.005
% EDITING TOTAL LIVER
10
8
6
4
2
0
PBS
dgRNA SPLIT-FORMULATED
dgRNA CO-FORMULATED
dgRNA PRE-ANNEALED

*FIG. 16*

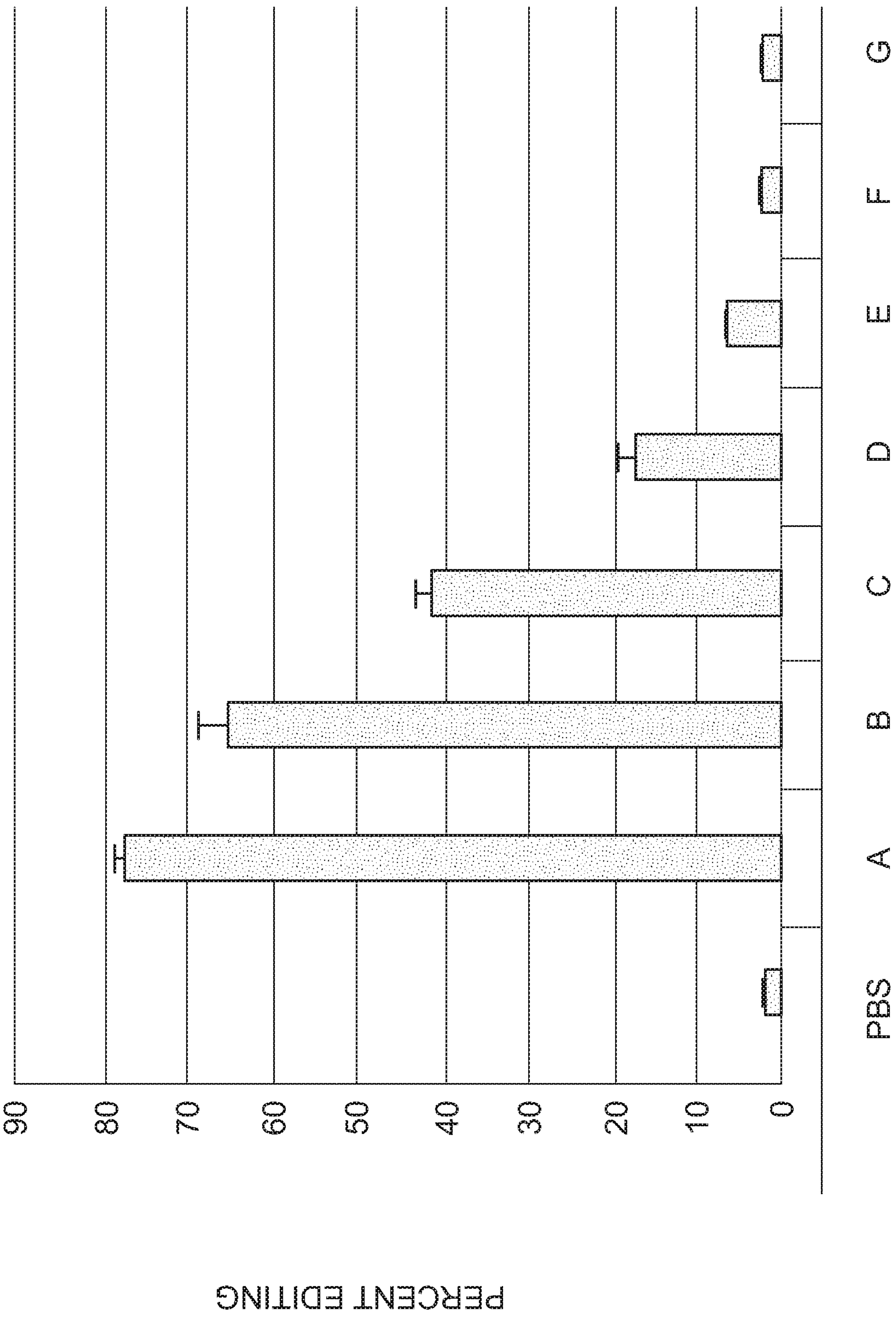


FIG. 17

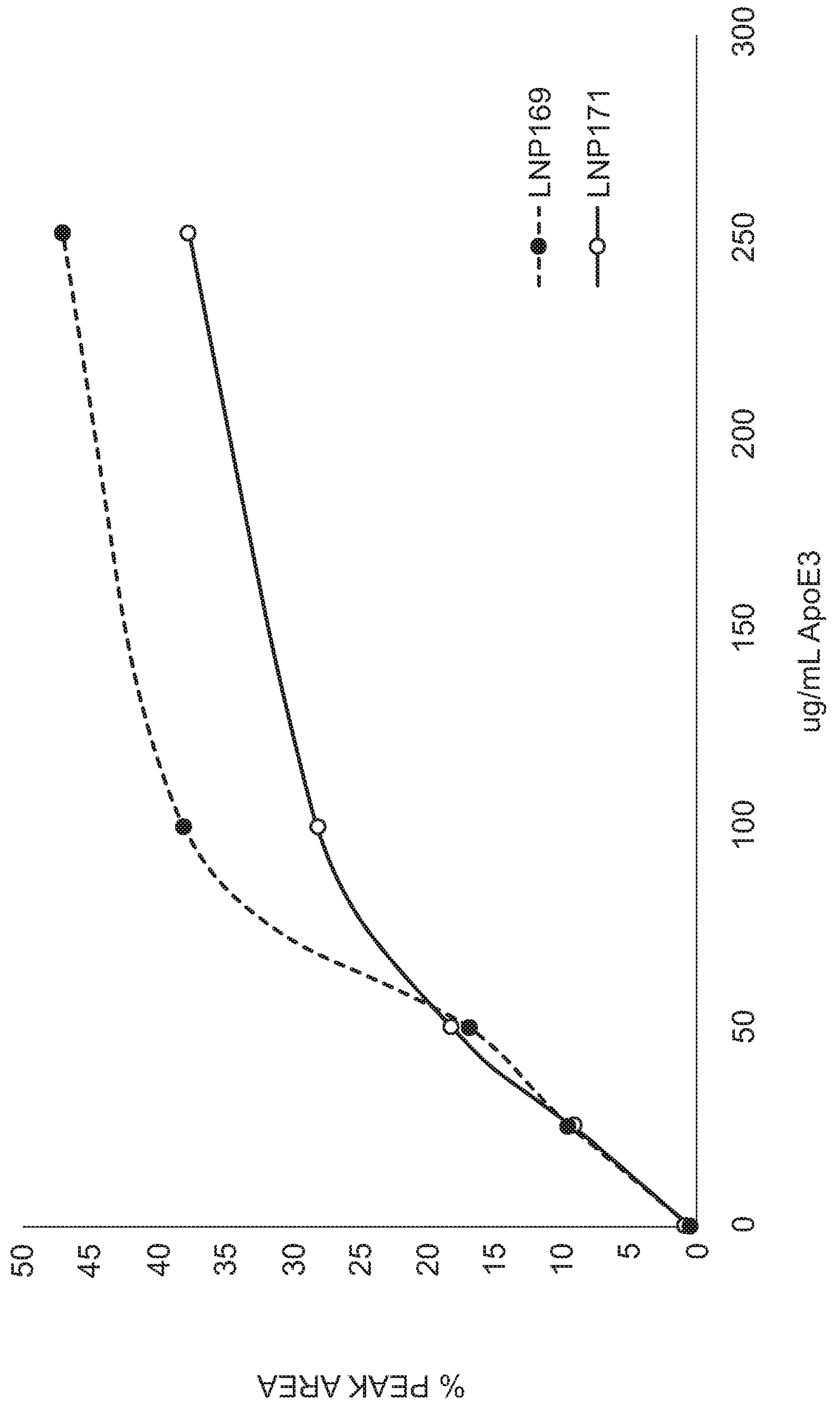
% PEAK AREA
0
5
10
15
20
25
30
35
40
45
50
0
50
100
150
200
250
300
ug/mL ApoE3
LNP169
LNP171

FIG. 18

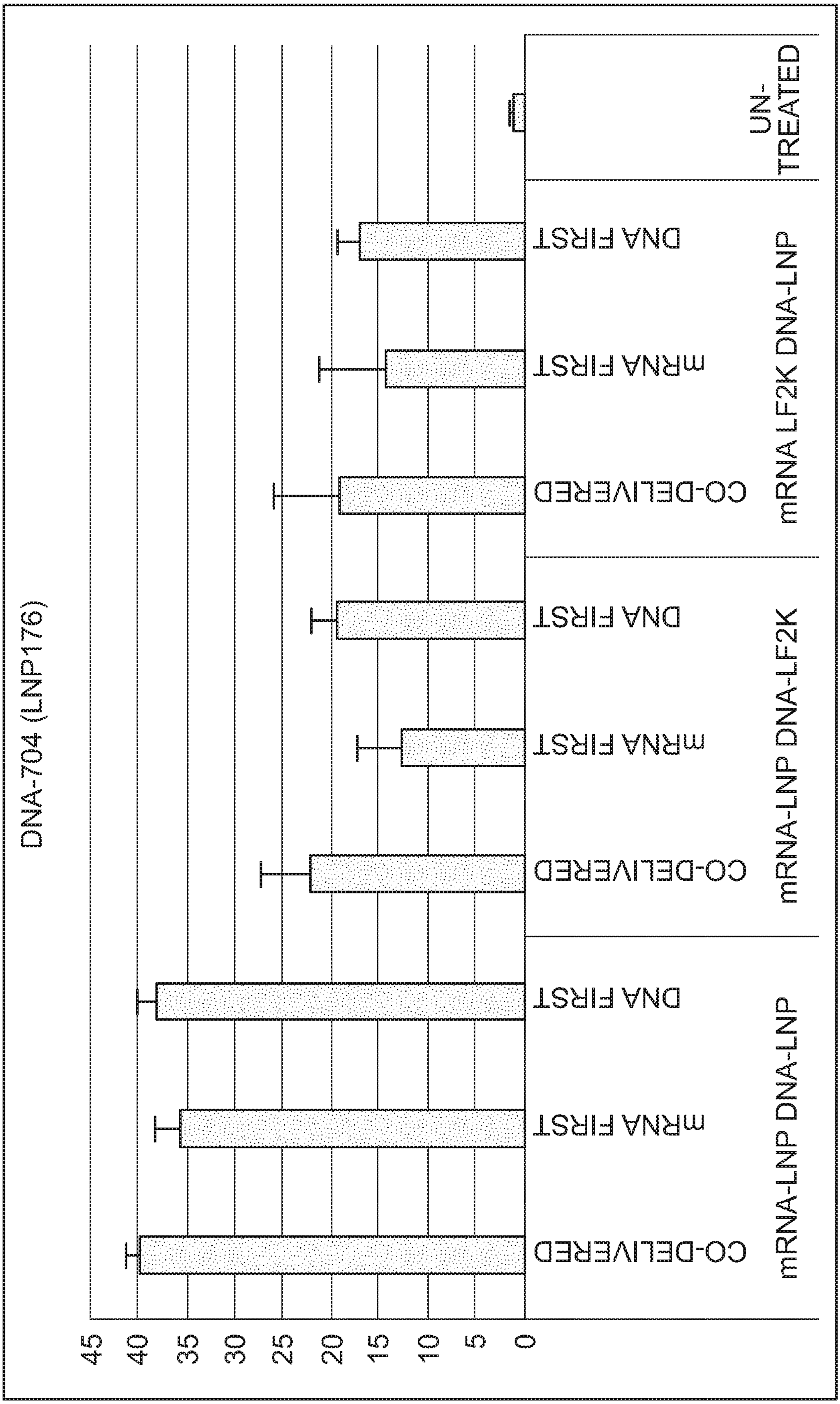
DNA-704 (LNP176)
45
40
35
30
25
20
15
10
5
0
CO-DELIVERED
mRNA FIRST
DNA FIRST
mRNA-LNP DNA-LNP
CO-DELIVERED
mRNA FIRST
DNA FIRST
mRNA-LNP DNA-LF2K
CO-DELIVERED
mRNA FIRST
DNA FIRST
mRNA LF2K DNA-LNP
UN-
TREATED

FIG. 19

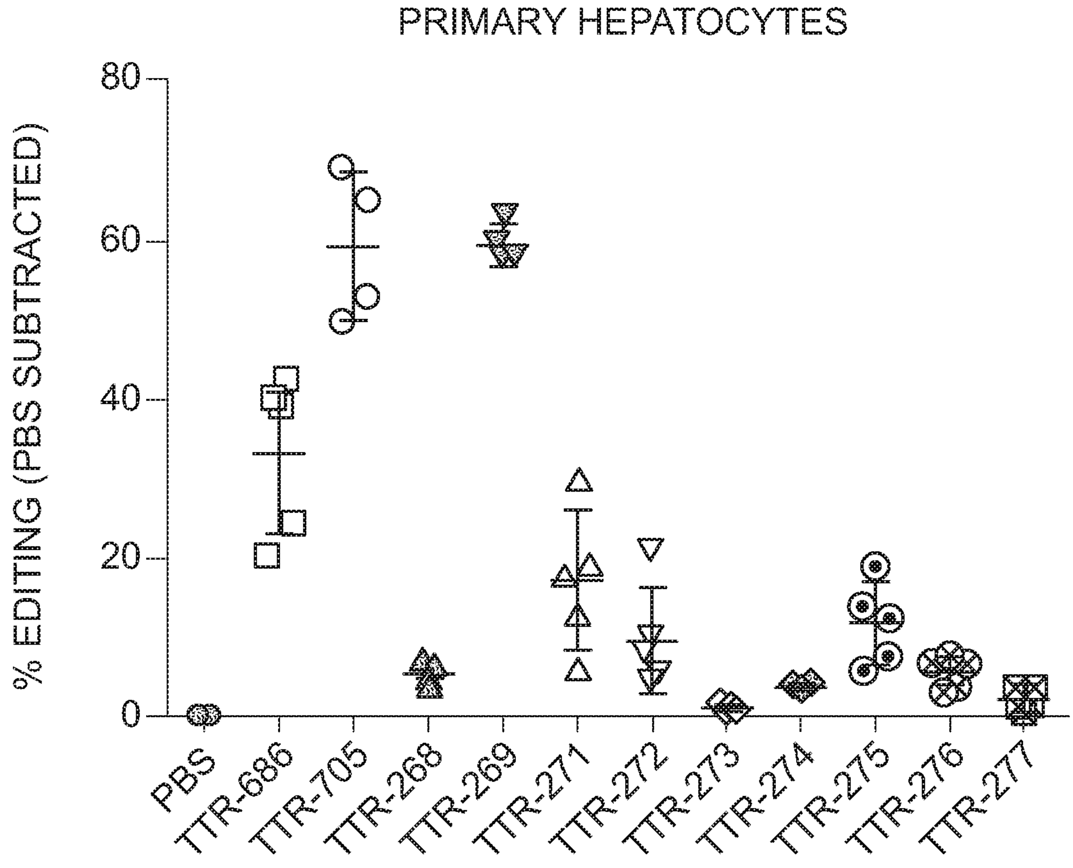
PRIMARY HEPATOCYTES
% EDITING (PBS SUBTRACTED)
80
60
40
20
0
PBS
TTR-686
TTR-705
TTR-268
TTR-269
TTR-271
TTR-272
TTR-273
TTR-274
TTR-275
TTR-276
TTR-277

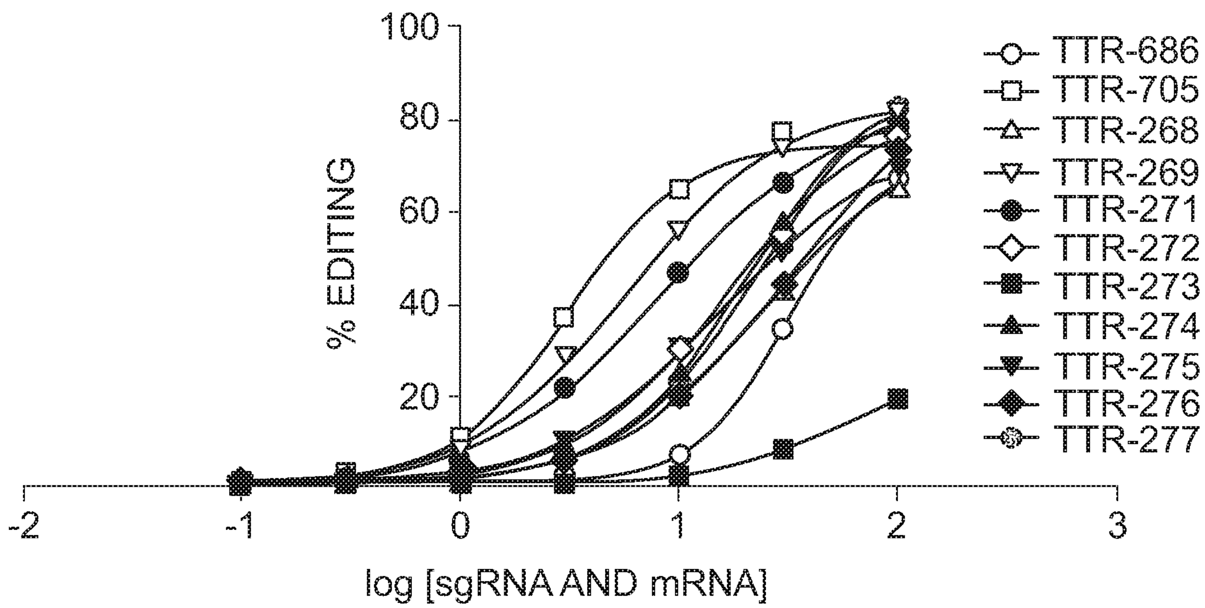
% EDITING
100
80
60
40
20
-2
-1
0
1
2
3
log [sgRNA AND mRNA]
TTR-686
TTR-705
TTR-268
TTR-269
TTR-271
TTR-272
TTR-273
TTR-274
TTR-275
TTR-276
TTR-277

*FIG. 20*

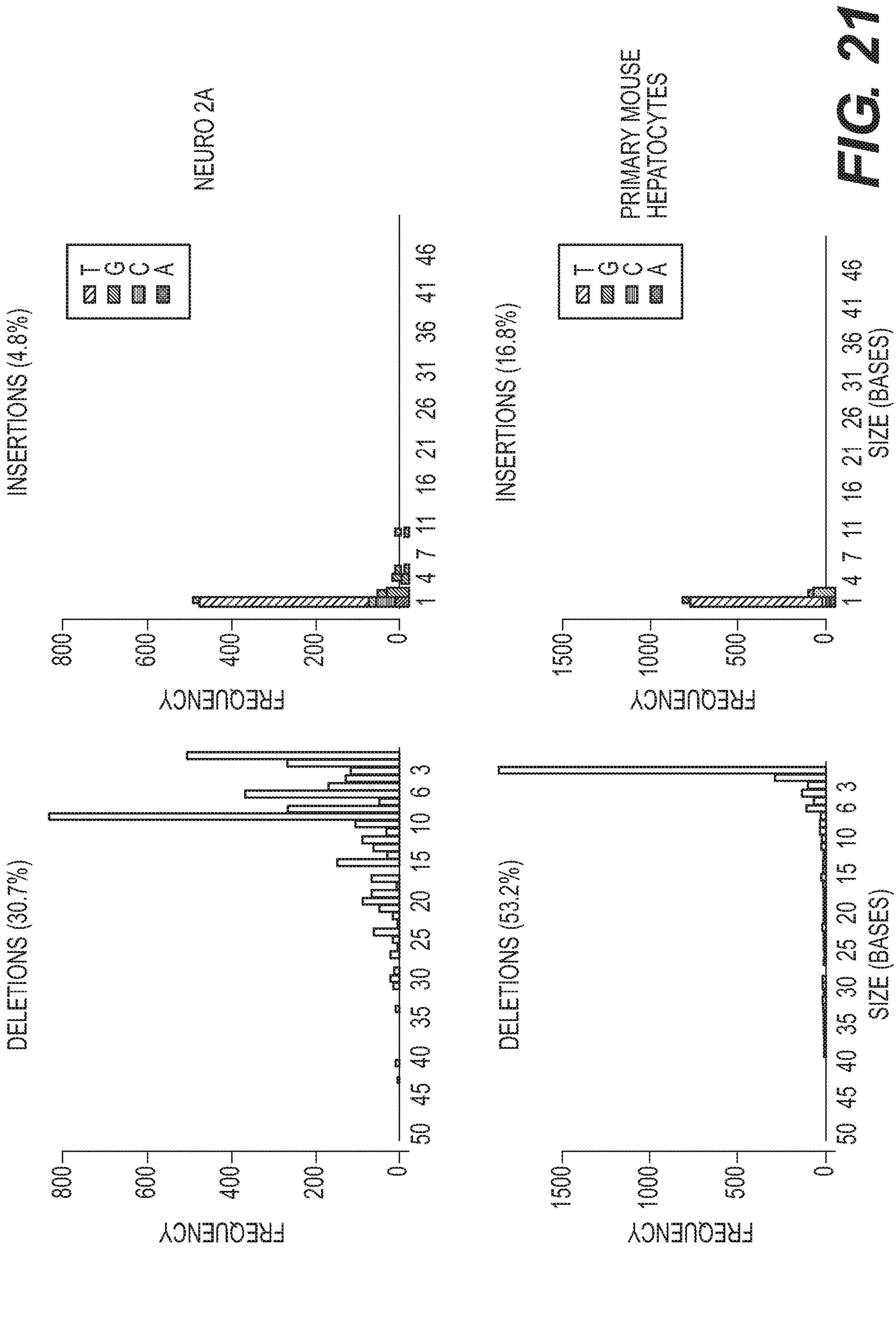

NEURO 2A
DELETIONS (30.7%)
INSERTIONS (4.8%)
FREQUENCY
T
G
C
A
PRIMARY MOUSE HEPATOCYTES
DELETIONS (53.2%)
INSERTIONS (16.8%)
SIZE (BASES)


FIG. 21

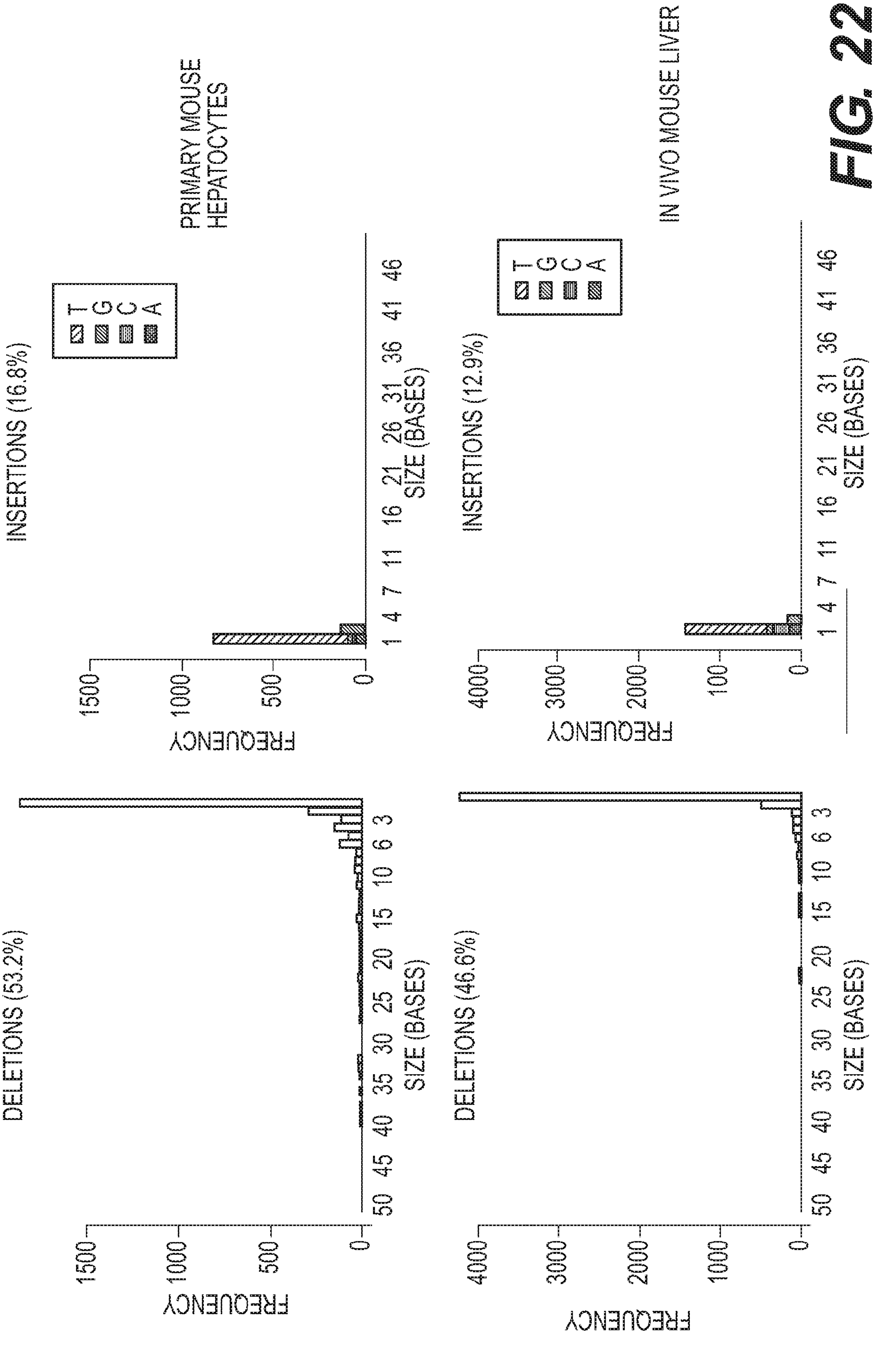

DELETIONS (53.2%)
FREQUENCY
1500
1000
500
0
50 45 40 35 30 25 20 15 10 6 3
SIZE (BASES)
INSERTIONS (16.8%)
FREQUENCY
1500
1000
500
0
1 4 7 11 16 21 26 31 36 41 46
SIZE (BASES)
T
G
C
A
PRIMARY MOUSE HEPATOCYTES
DELETIONS (46.6%)
FREQUENCY
4000
3000
2000
1000
0
50 45 40 35 30 25 20 15 10 6 3
SIZE (BASES)
INSERTIONS (12.9%)
FREQUENCY
4000
3000
2000
100
0
1 4 7 11 16 21 26 31 36 41 46
SIZE (BASES)
T
G
C
A
IN VIVO MOUSE LIVER


FIG. 22

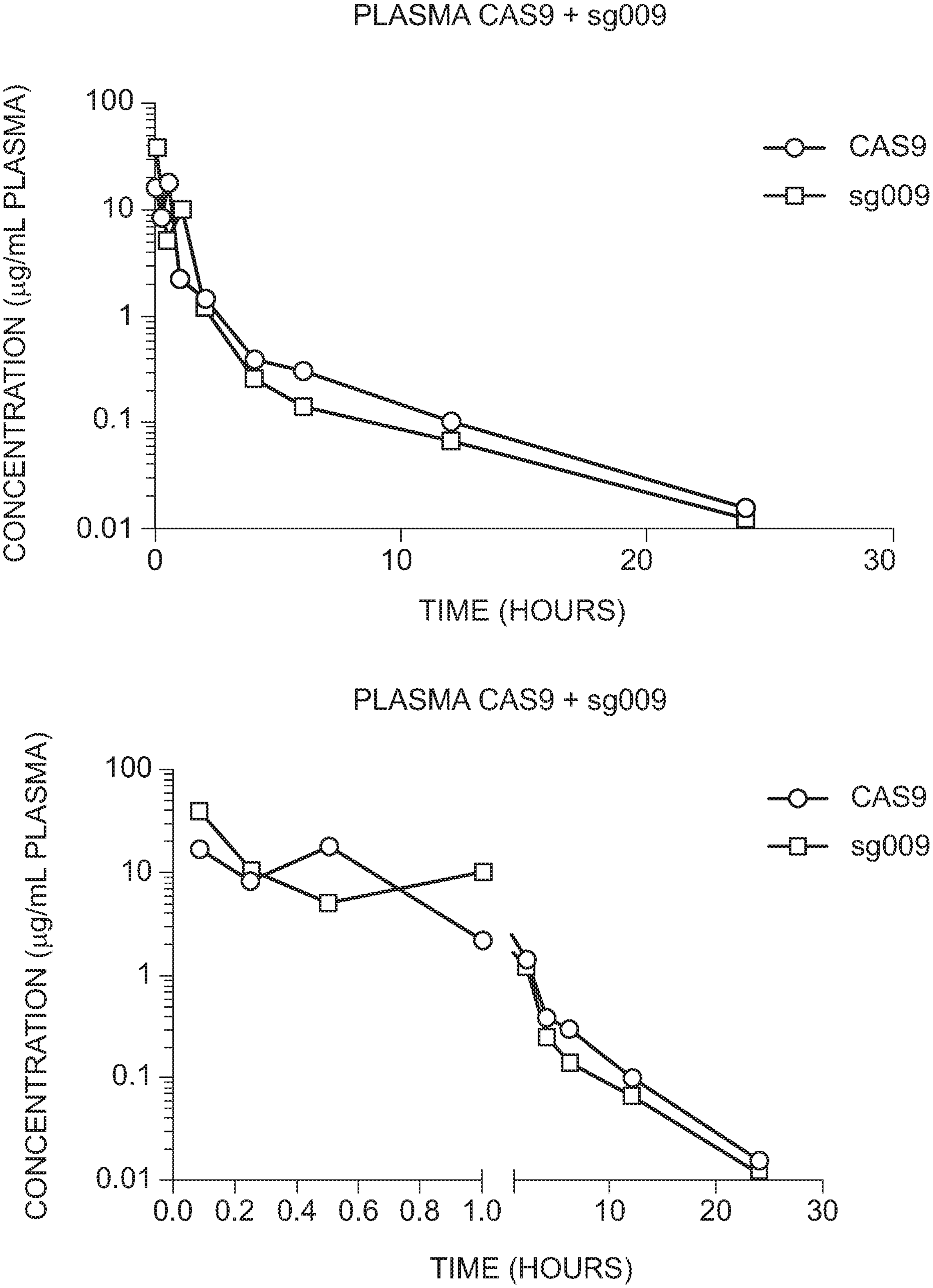
PLASMA CAS9 + sg009
CONCENTRATION (μg/mL PLASMA)
100
10
1
0.1
0.01
0
10
20
30
TIME (HOURS)
CAS9
sg009
PLASMA CAS9 + sg009
CONCENTRATION (μg/mL PLASMA)
100
10
1
0.1
0.01
0.0
0.2
0.4
0.6
0.8
1.0
10
20
30
TIME (HOURS)
CAS9
sg009

*FIG. 23*

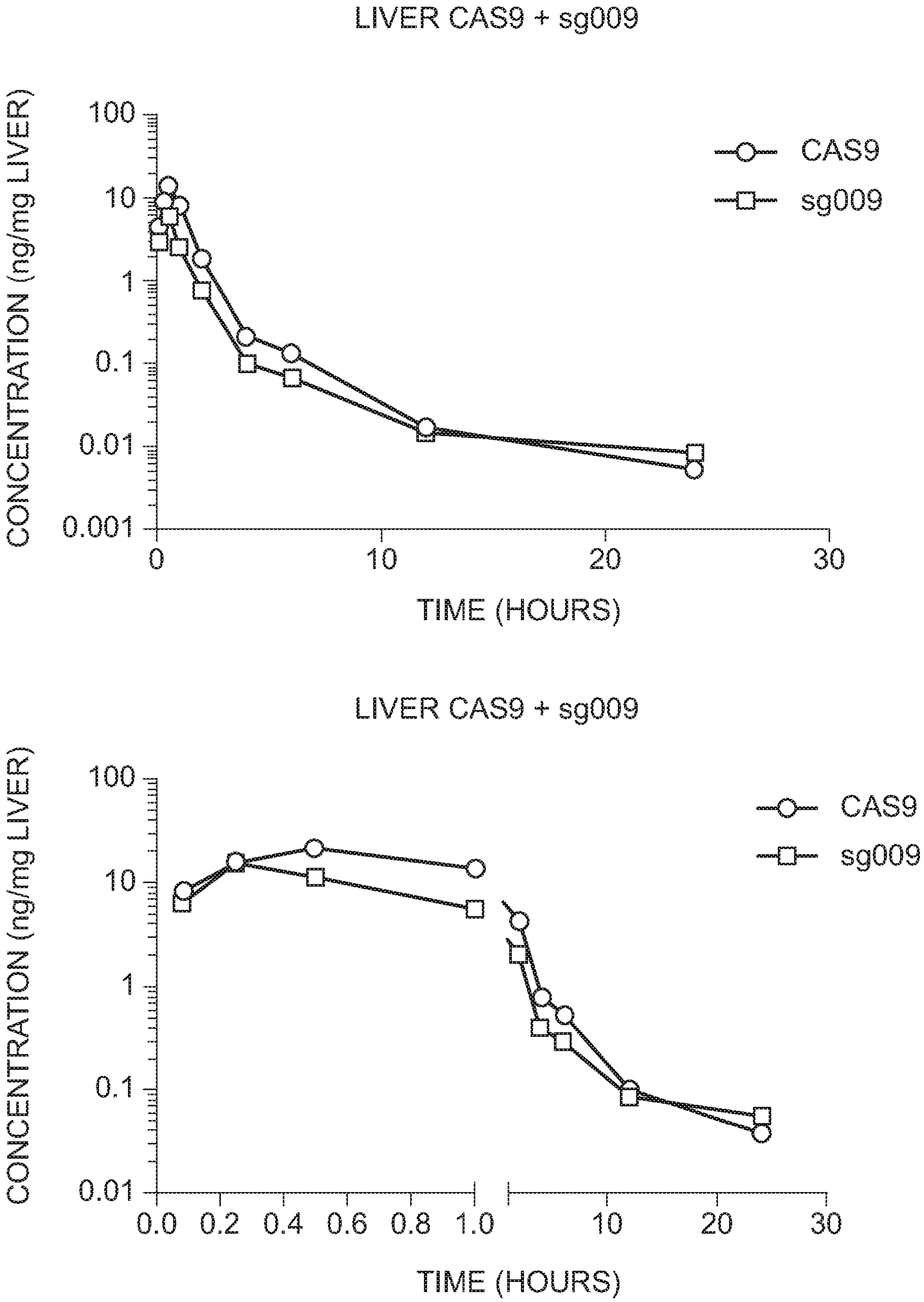
LIVER CAS9 + sg009
CONCENTRATION (ng/mg LIVER)
100
10
1
0.1
0.01
0.001
0
10
20
30
TIME (HOURS)
CAS9
sg009
LIVER CAS9 + sg009
CONCENTRATION (ng/mg LIVER)
100
10
1
0.1
0.01
0.0
0.2
0.4
0.6
0.8
1.0
10
20
30
TIME (HOURS)
CAS9
sg009

*FIG. 24*

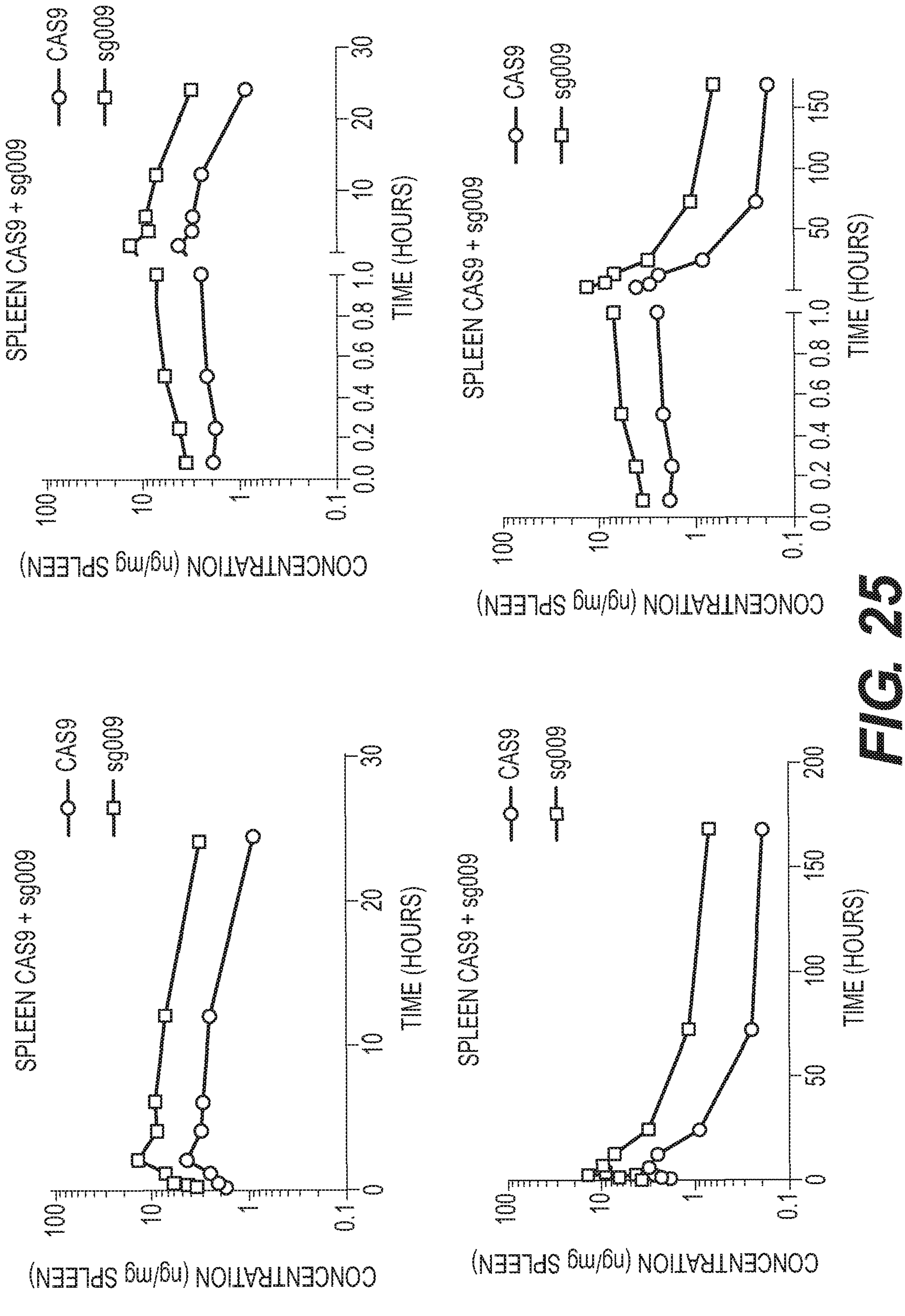
SPLEEN CAS9 + sg009
CAS9
sg009
CONCENTRATION (ng/mg SPLEEN)
TIME (HOURS)
SPLEEN CAS9 + sg009
CAS9
sg009
CONCENTRATION (ng/mg SPLEEN)
TIME (HOURS)
SPLEEN CAS9 + sg009
CAS9
sg009
CONCENTRATION (ng/mg SPLEEN)
TIME (HOURS)
SPLEEN CAS9 + sg009
CAS9
sg009
CONCENTRATION (ng/mg SPLEEN)
TIME (HOURS)

FIG. 25

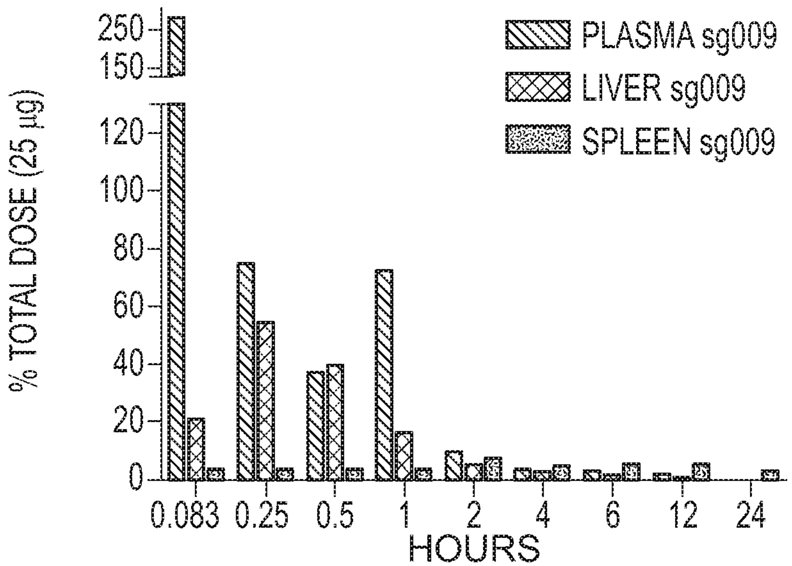


sg009 % DOSE

| TIME (h) | PLASMA | LIVER | SPLEEN | TOTAL % |
|---|---|---|---|---|
| 0.083 | 273.3 | 18.5 | 1.7 | 293.6 |
| 0.25 | 73.6 | 53.6 | 2 | 129.1 |
| 0.5 | 35.9 | 37.8 | 2.8 | 76.6 |
| 1 | 71.9 | 15.6 | 3.4 | 90.9 |
| 2 | 8.5 | 4.7 | 6.5 | 19.7 |
| 4 | 1.8 | 0.6 | 4.1 | 6.5 |
| 6 | 1 | 0.4 | 4.3 | 5.7 |
| 12 | 0.5 | 0.1 | 3.3 | 3.9 |
| 24 | 0.1 | 0.1 | 1.5 | 1.6 |

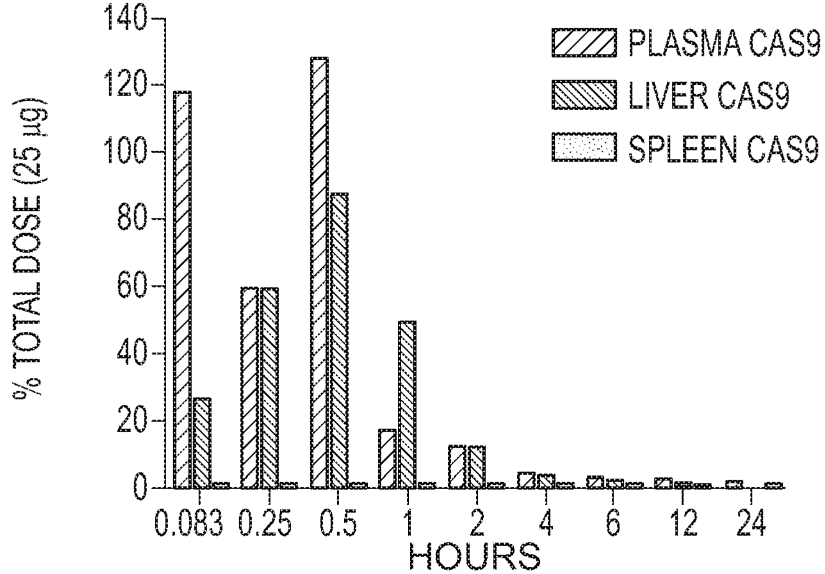


Cas9 % DOSE

| TIME (h) | PLASMA | LIVER | SPLEEN | TOTAL % |
|---|---|---|---|---|
| 0.083 | 117.1 | 25.4 | 0.9 | 143.4 |
| 0.25 | 57.5 | 57 | 0.8 | 115.3 |
| 0.5 | 127 | 86.3 | 1 | 214.4 |
| 1 | 15.9 | 48.7 | 1.2 | 65.8 |
| 2 | 10.6 | 11.3 | 2.1 | 24 |
| 4 | 2.8 | 1.3 | 1.5 | 5.5 |
| 6 | 2.2 | 0.8 | 1.4 | 4.4 |
| 12 | 0.7 | 0.1 | 1.2 | 2 |
| 24 | 0.1 | 0 | 0.4 | 0.6 |

FIG. 26A

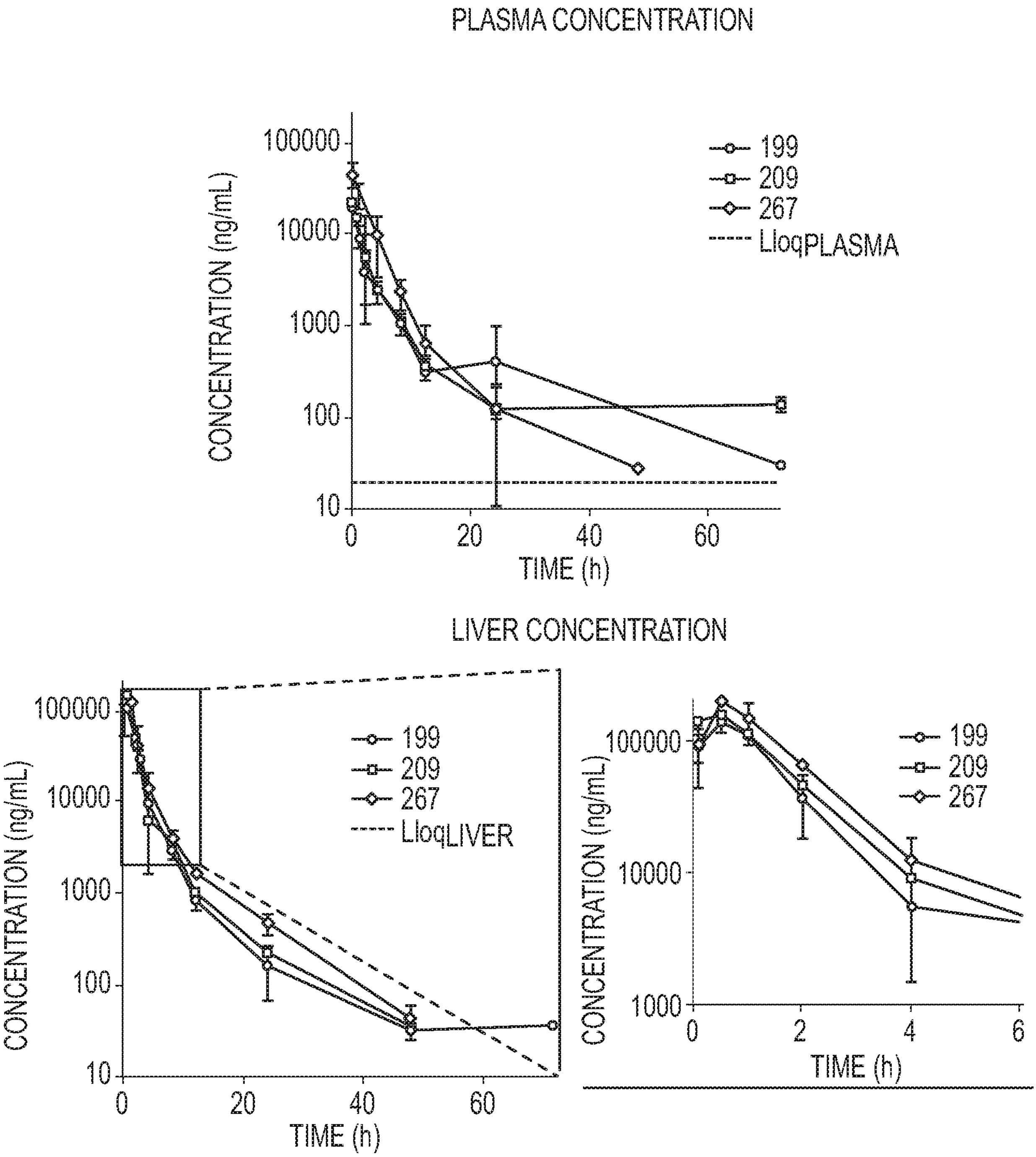
PLASMA CONCENTRATION
CONCENTRATION (ng/mL)
100000
10000
1000
100
10
0
20
40
60
TIME (h)
199
209
267
LloqPLASMA
LIVER CONCENTRATION
CONCENTRATION (ng/mL)
100000
10000
1000
100
10
0
20
40
60
TIME (h)
199
209
267
LloqLIVER
CONCENTRATION (ng/mL)
100000
10000
1000
0
2
4
6
TIME (h)
199
209
267

FIG. 26B

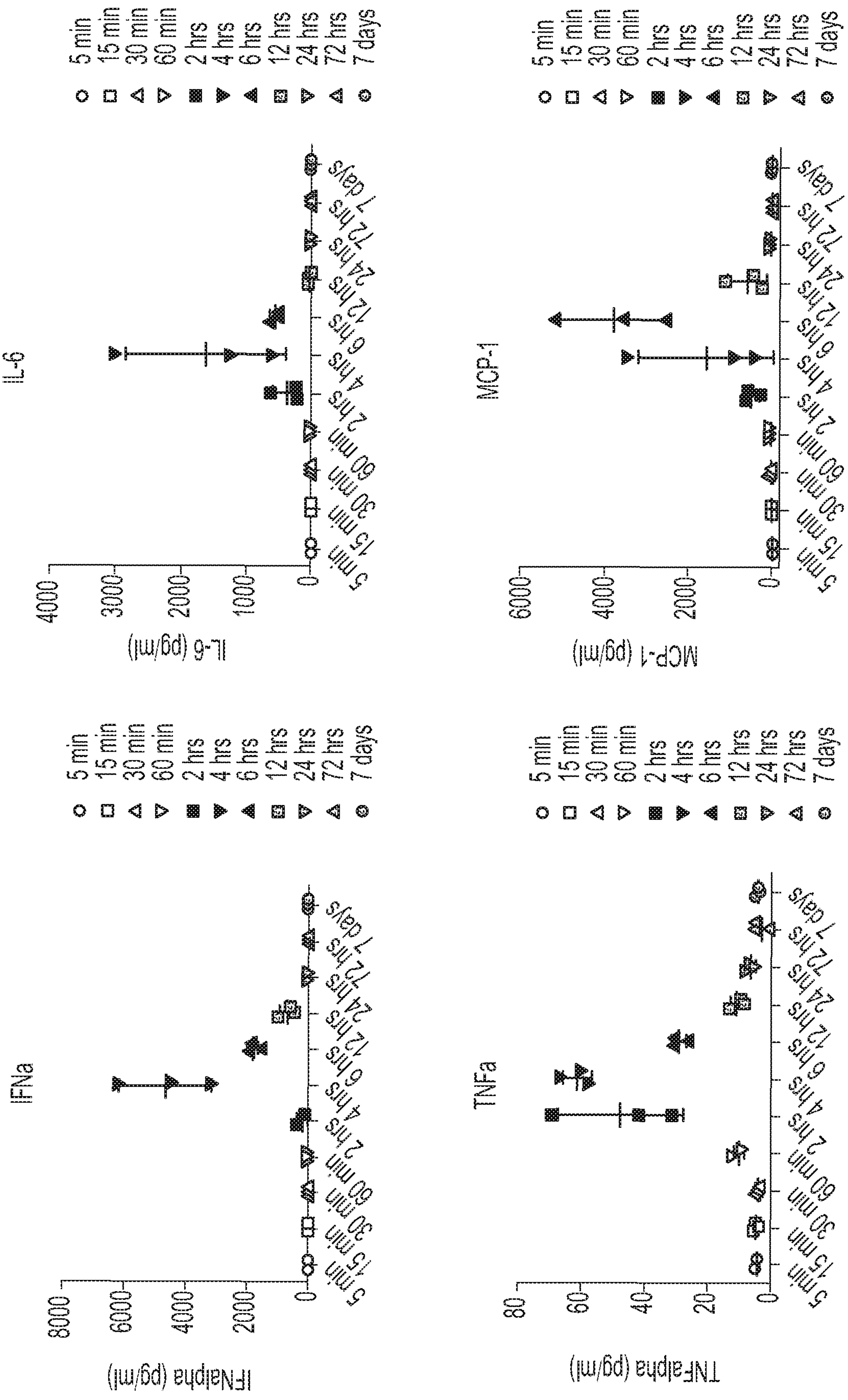
IFNa
IFNalpha (pg/ml)
8000
6000
4000
2000
0
TNFa
TNFalpha (pg/ml)
80
60
40
20
0
IL-6
IL-6 (pg/ml)
4000
3000
2000
1000
0
MCP-1
MCP-1 (pg/ml)
6000
4000
2000
0
5 min
15 min
30 min
60 min
2 hrs
4 hrs
6 hrs
12 hrs
24 hrs
72 hrs
7 days

FIG. 27

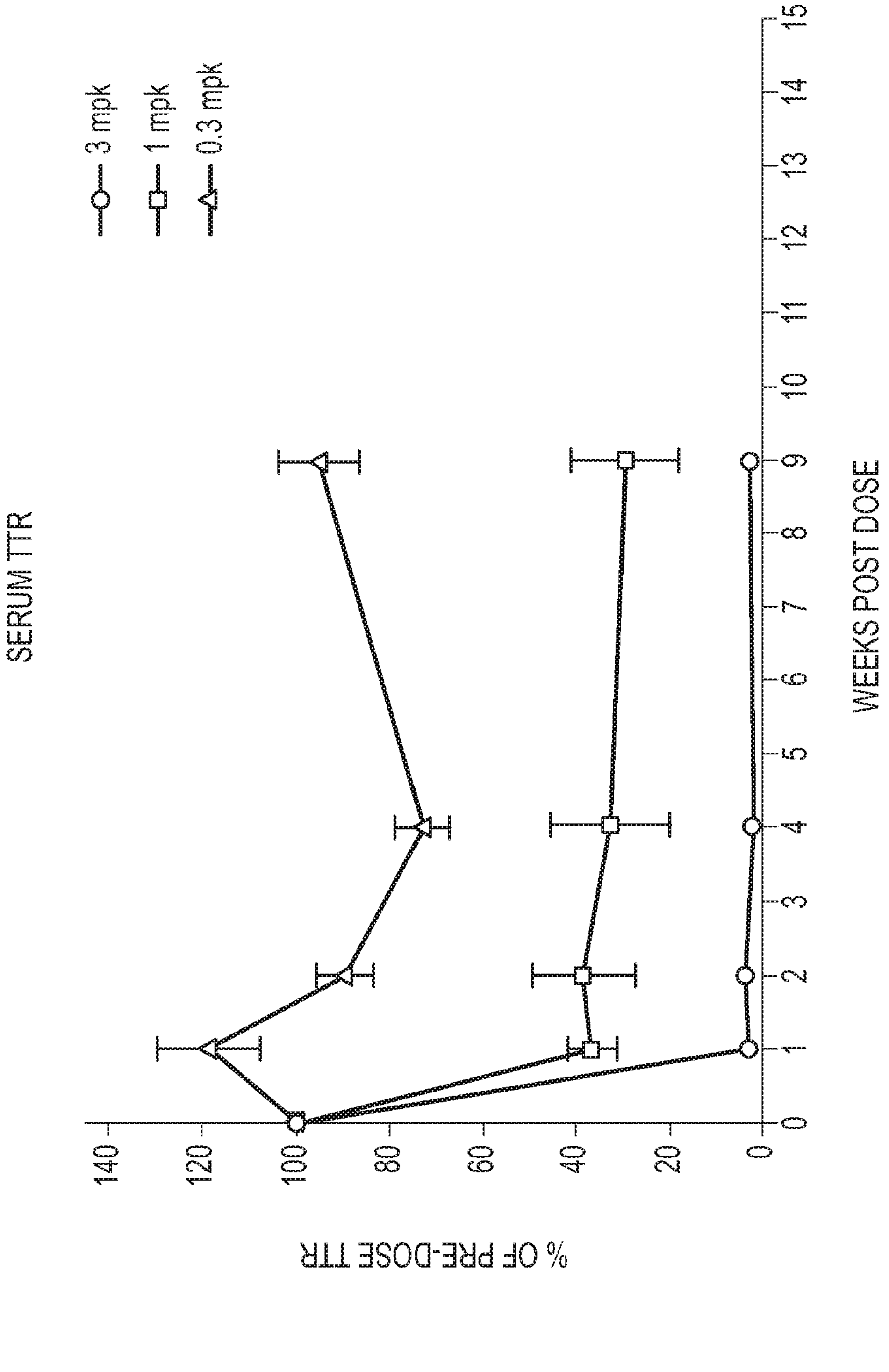
SERUM TTR
3 mpk
1 mpk
0.3 mpk
% OF PRE-DOSE TTR
0
20
40
60
80
100
120
140
0
1
2
3
4
5
6
7
8
9
10
11
12
13
14
15
WEEKS POST DOSE

FIG. 28

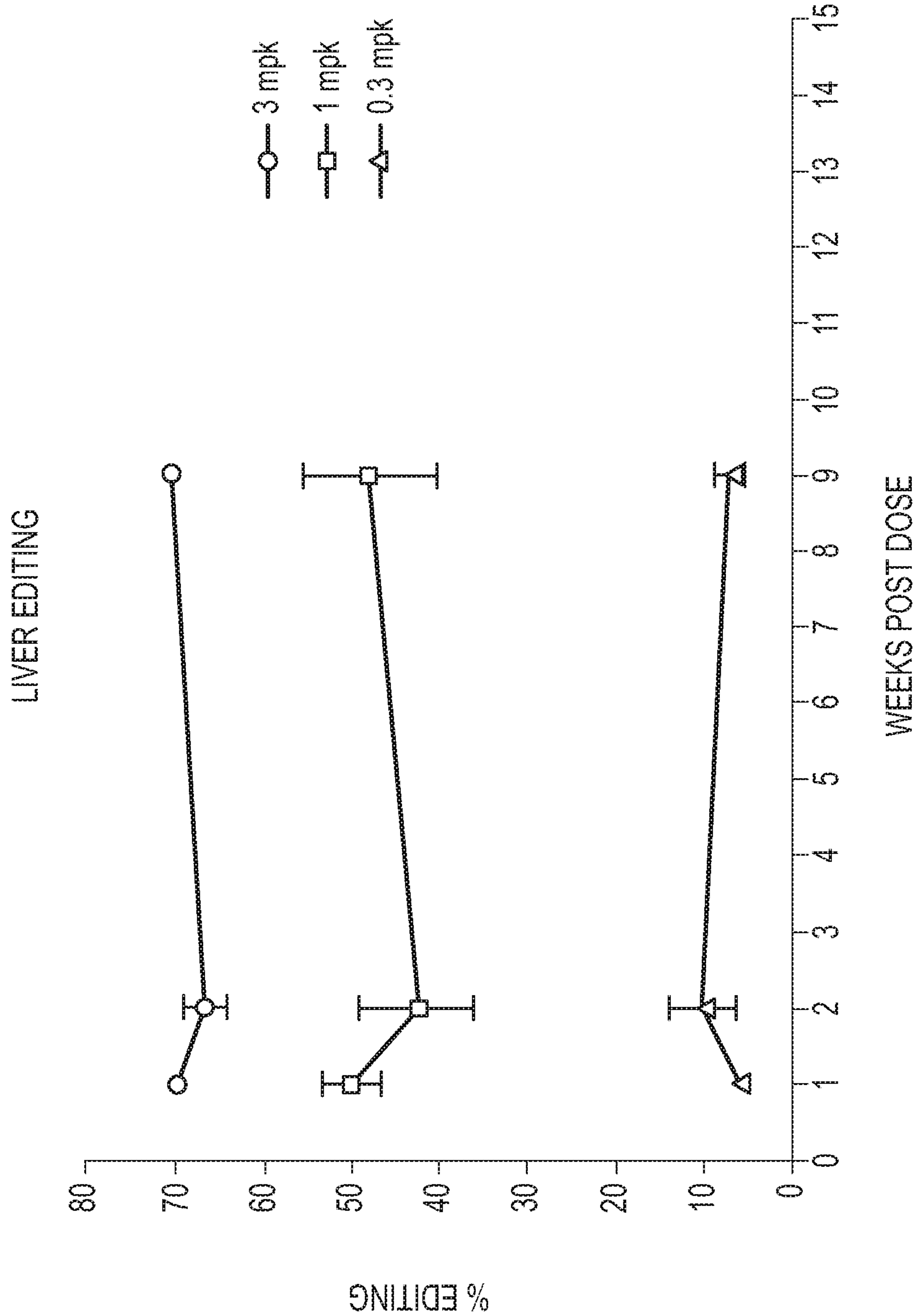
LIVER EDITING
% EDITING
80
70
60
50
40
30
20
10
0
0
1
2
3
4
5
6
7
8
9
10
11
12
13
14
15
WEEKS POST DOSE
3 mpk
1 mpk
0.3 mpk

FIG. 29A

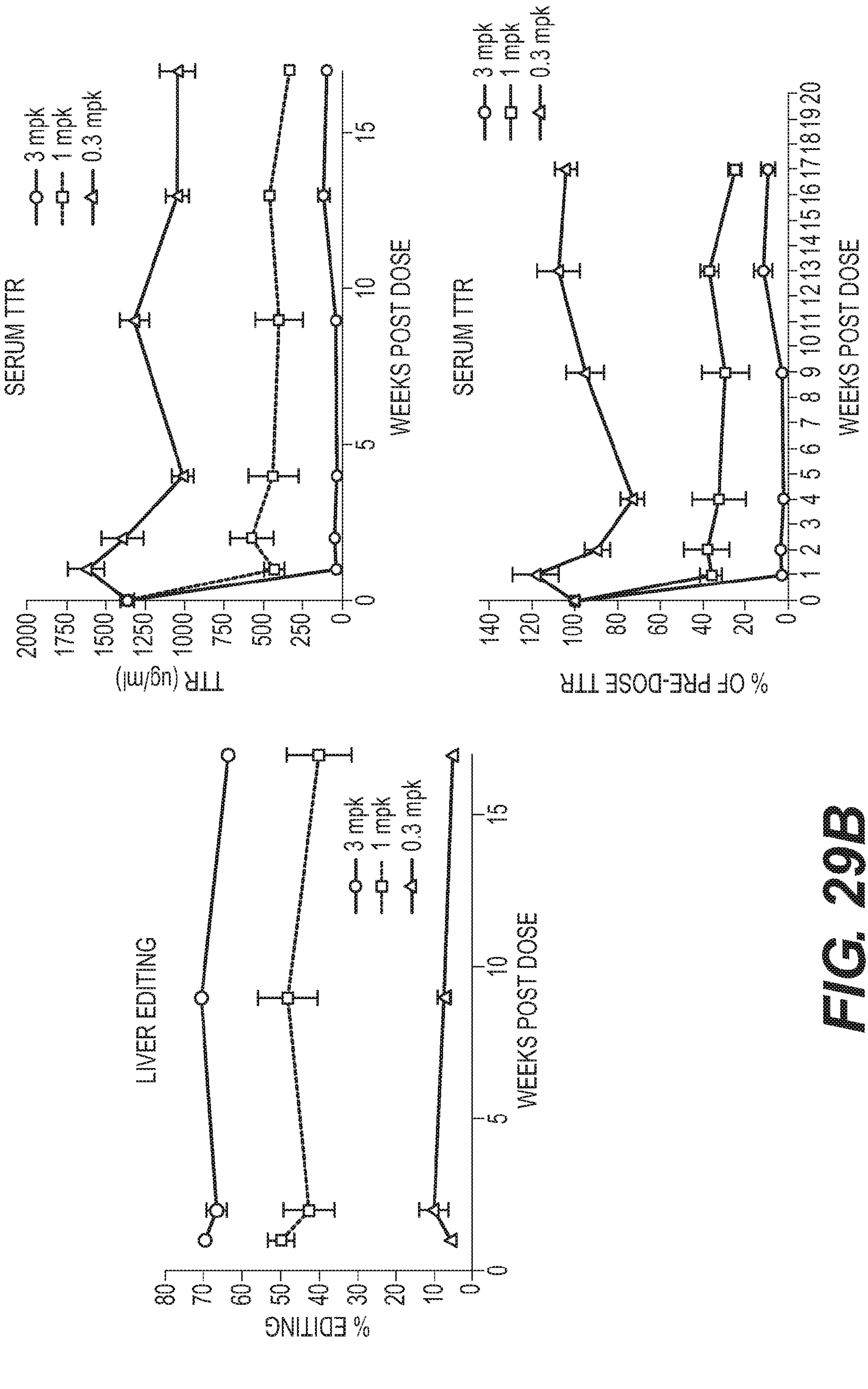
SERUM TTR
TTR (ug/ml)
WEEKS POST DOSE
3 mpk
1 mpk
0.3 mpk
SERUM TTR
% OF PRE-DOSE TTR
WEEKS POST DOSE
3 mpk
1 mpk
0.3 mpk
LIVER EDITING
% EDITING
WEEKS POST DOSE
3 mpk
1 mpk
0.3 mpk

FIG. 29B

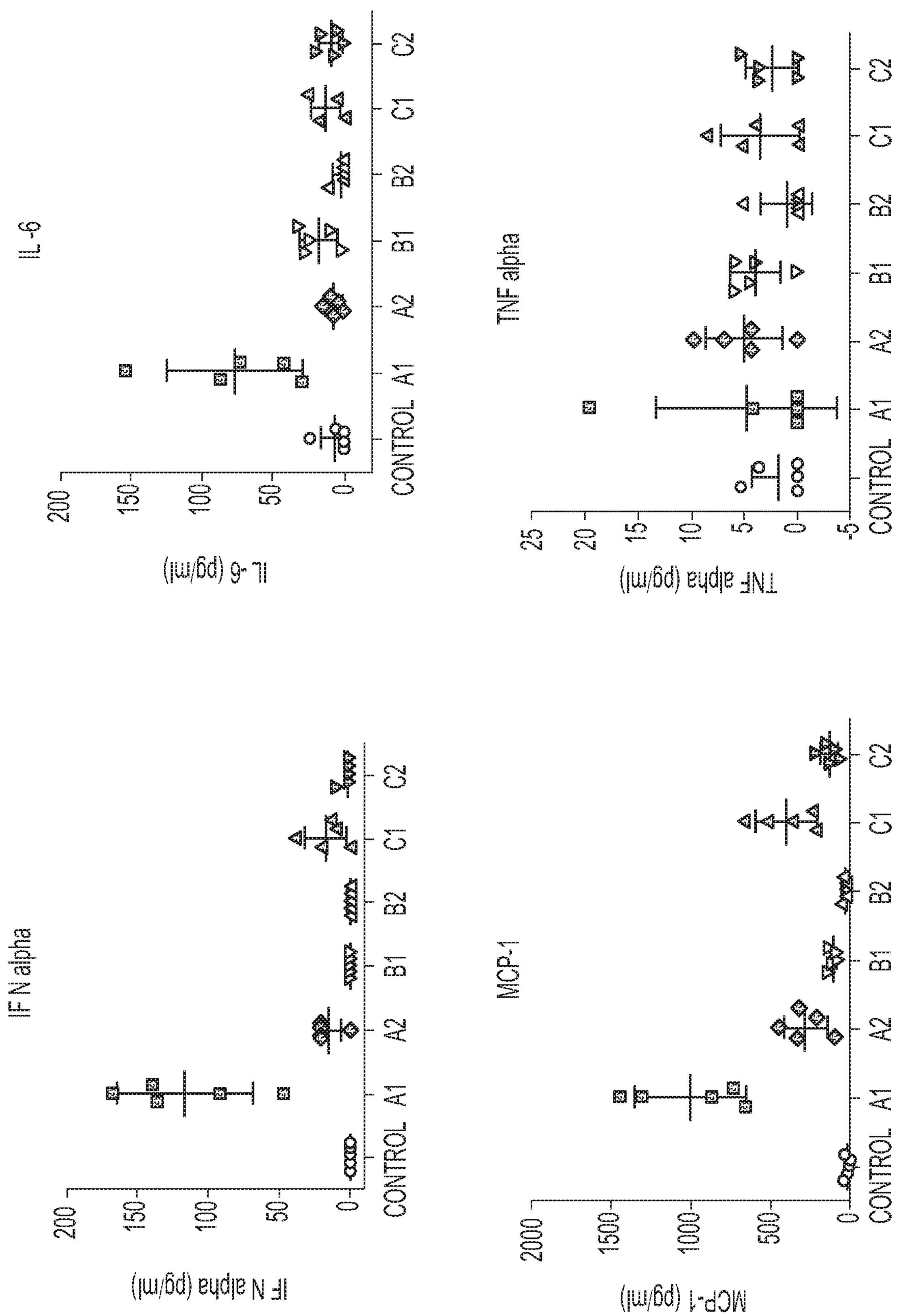
IF N alpha
IF N alpha (pg/ml)
IL-6
IL-6 (pg/ml)
MCP-1
MCP-1 (pg/ml)
TNF alpha
TNF alpha (pg/ml)
CONTROL
A1
A2
B1
B2
C1
C2

FIG. 30

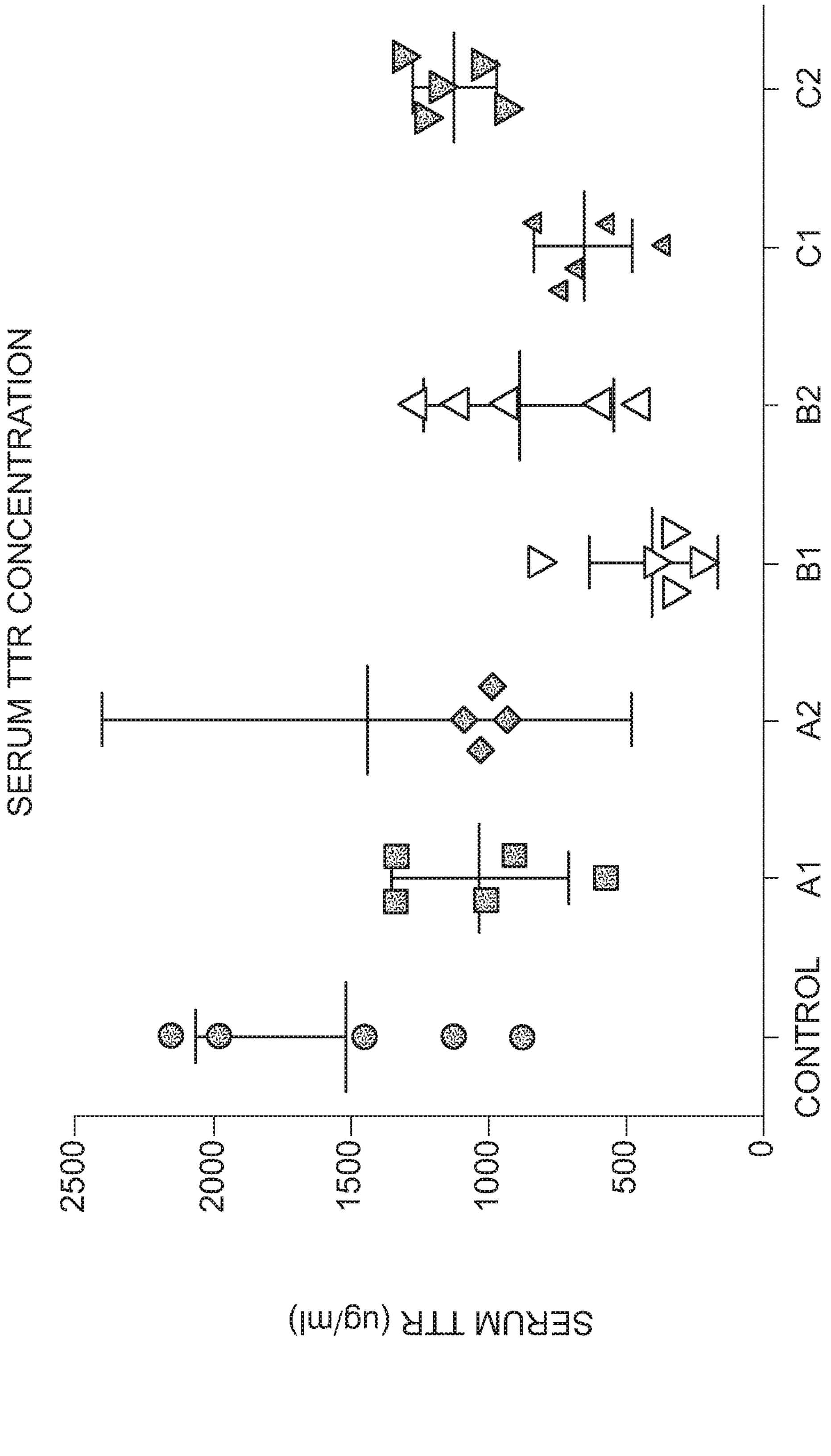
SERUM TTR CONCENTRATION
SERUM TTR (ug/ml)
0
500
1000
1500
2000
2500
CONTROL
A1
A2
B1
B2
C1
C2

FIG. 31

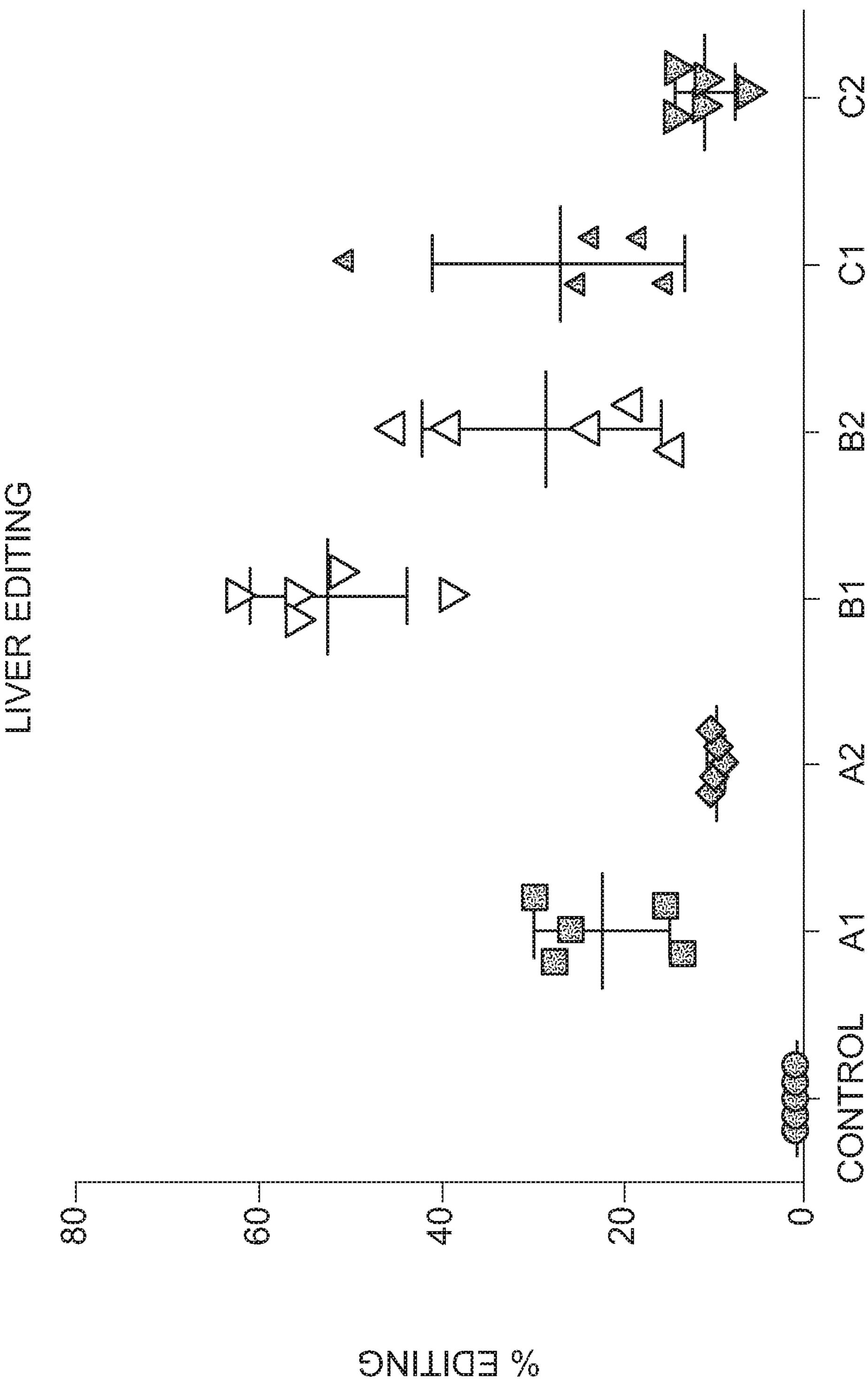


*FIG. 32*

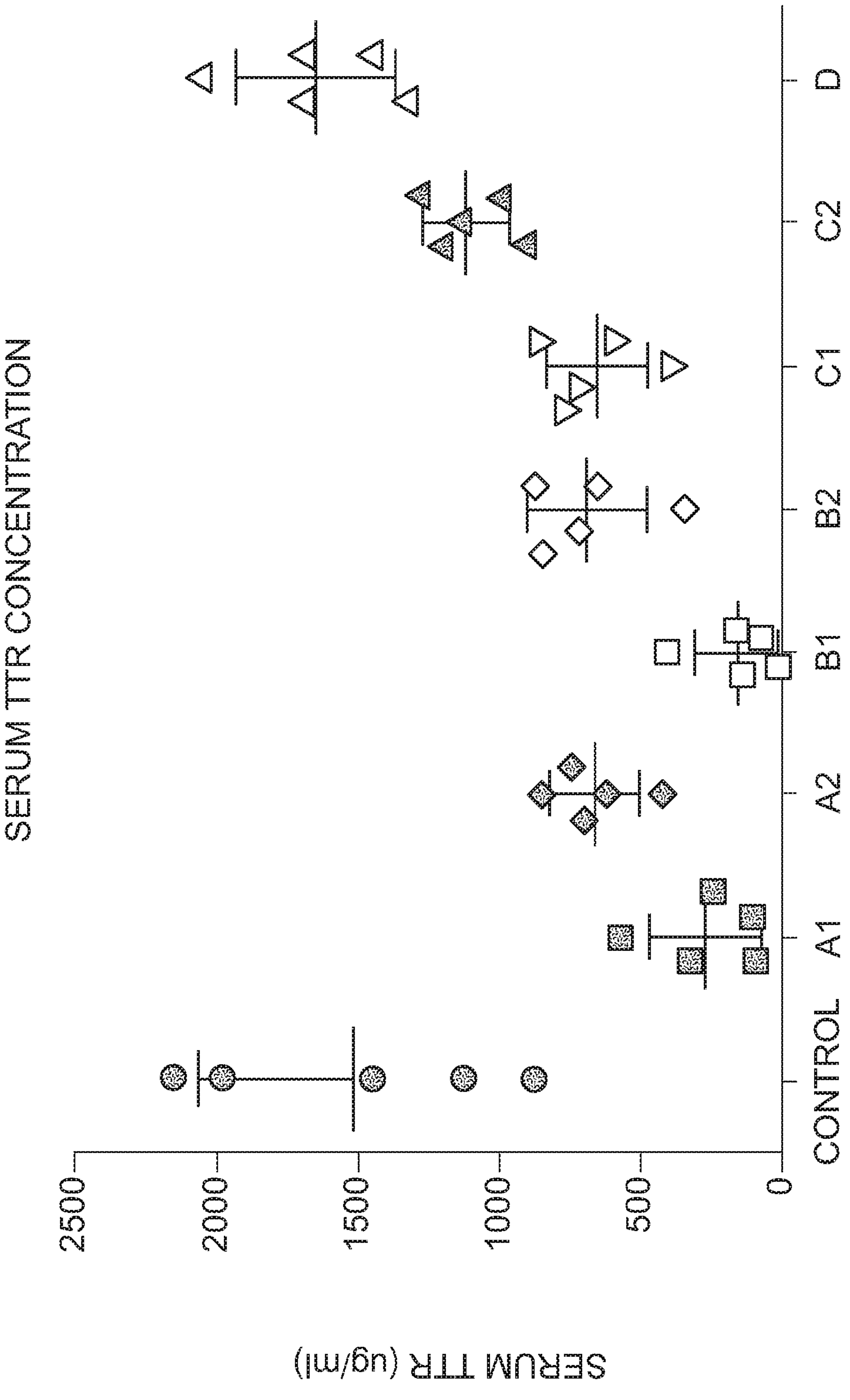
SERUM TTR CONCENTRATION
SERUM TTR (ug/ml)
2500
2000
1500
1000
500
0
CONTROL
A1
A2
B1
B2
C1
C2
D

FIG. 33

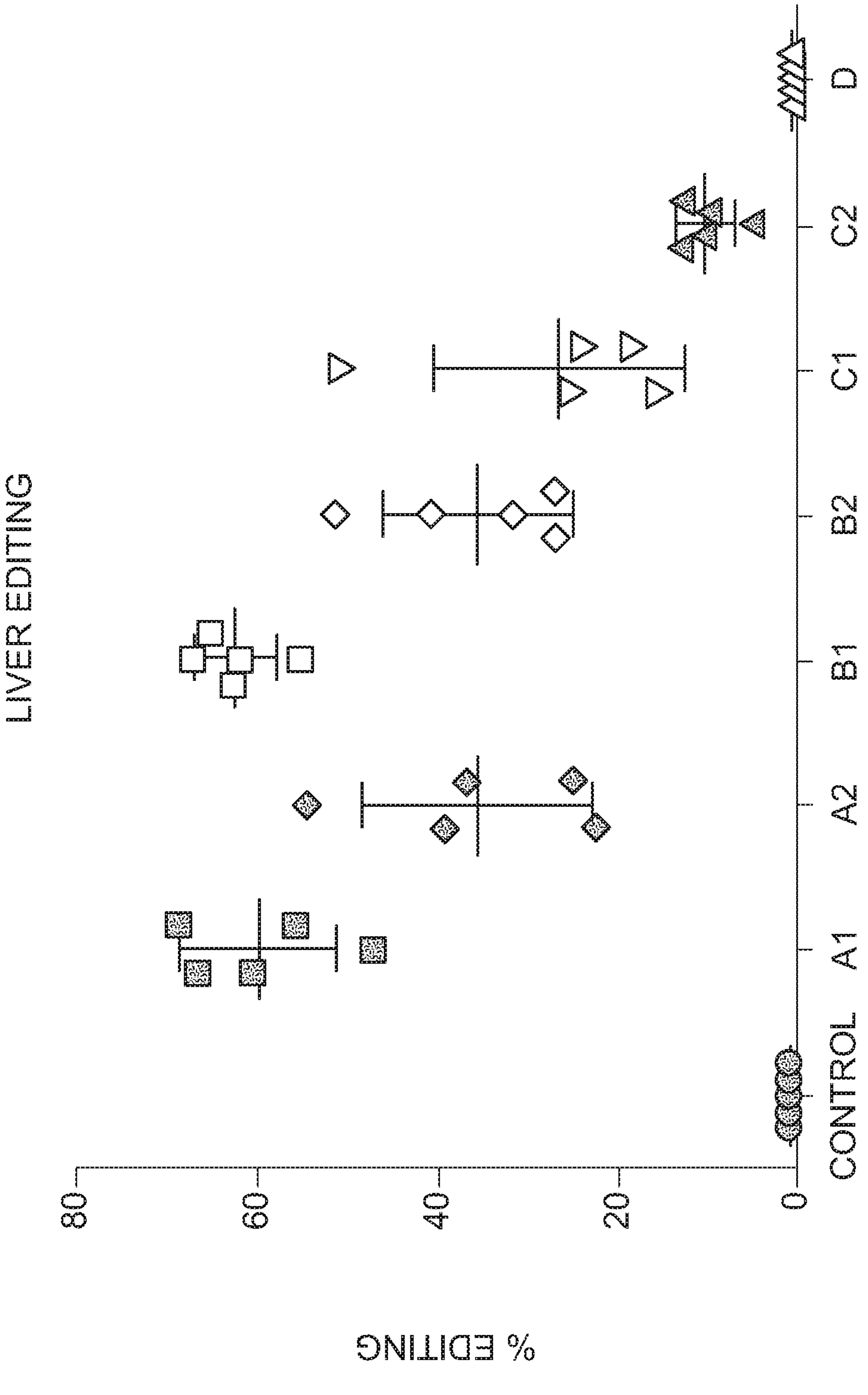


FIG. 34

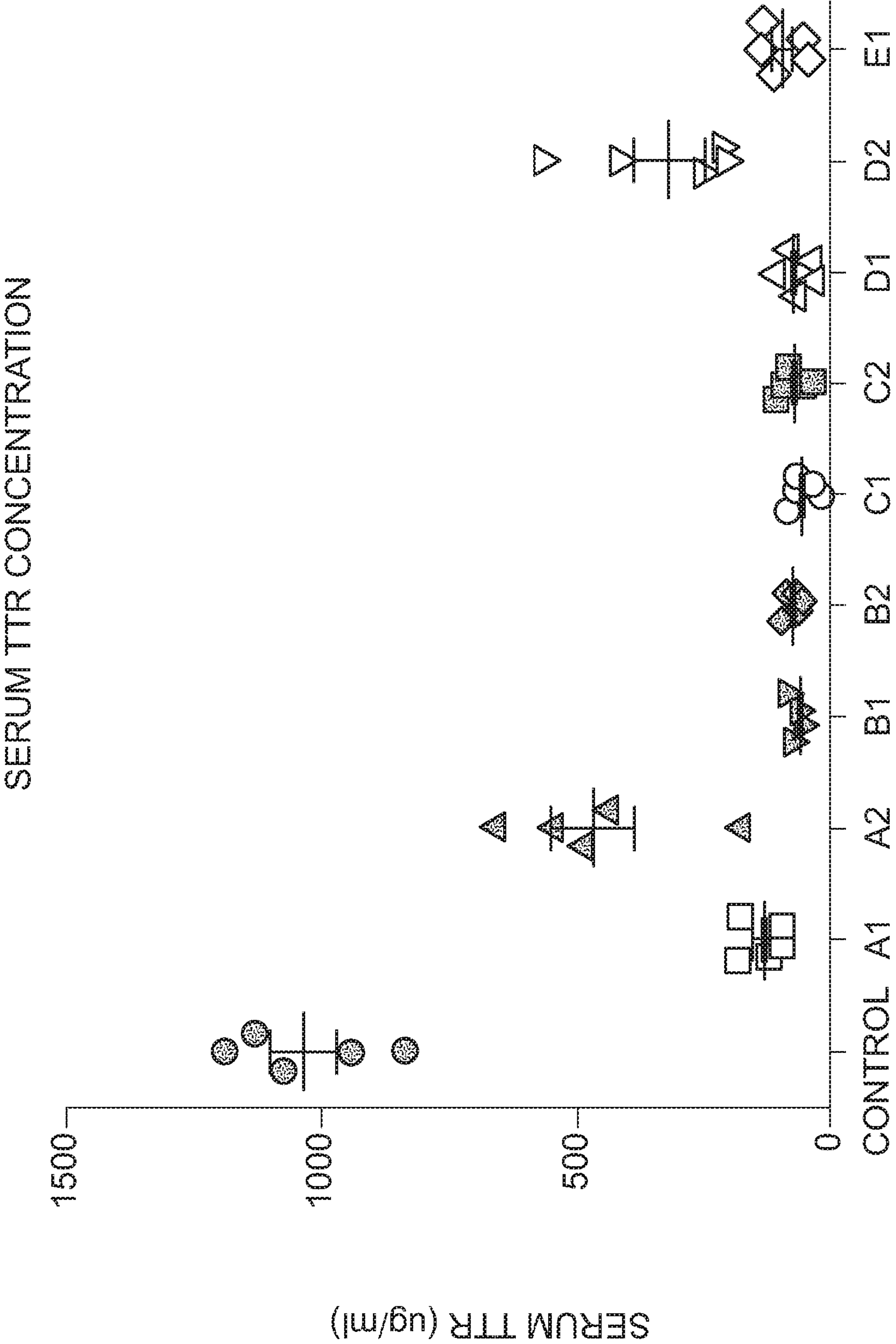
SERUM TTR CONCENTRATION
SERUM TTR (ug/ml)
1500
1000
500
0
CONTROL
A1
A2
B1
B2
C1
C2
D1
D2
E1
E2

*FIG. 35*

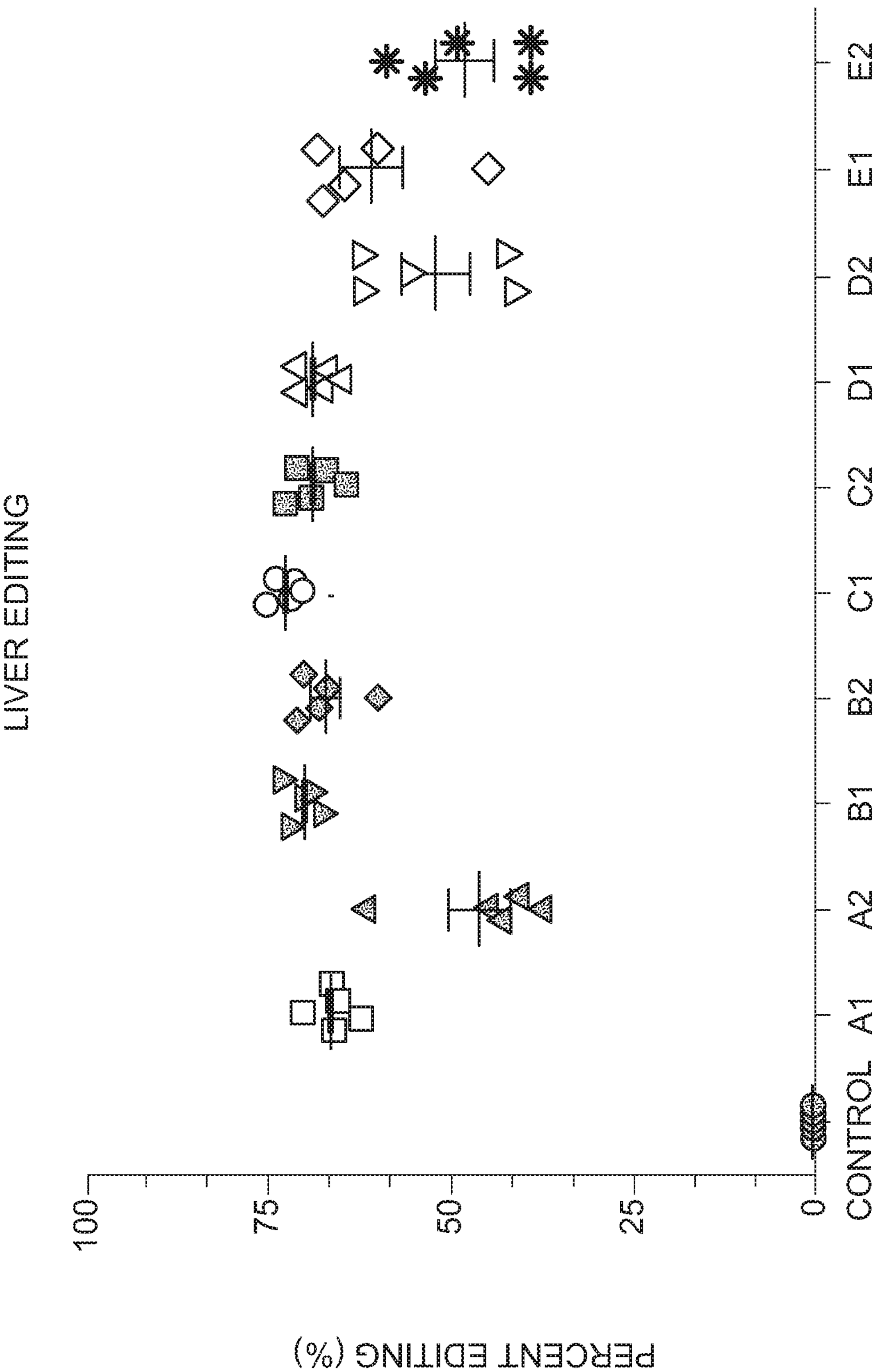
LIVER EDITING
PERCENT EDITING (%)
100
75
50
25
0
CONTROL
A1
A2
B1
B2
C1
C2
D1
D2
E1
E2

FIG. 36

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62315602
- US 62375776
- US 62433228
- US 62468300
- WO 2015089406 A
- US 20160304846 A
- WO 2015095340 A
- WO 2014136086 A
- WO 2006007712 A


### Non-patent literature cited in the description


- **MAKAROVA et al.** *Nat Rev Microbiol*, 2015, vol. 13 (11), 722-36
- **SHMAKOV et al.** *Molecular Cell*, 2015, vol. 60, 385-397
- **ZETSCHE et al.** *Cell*, 2015, vol. 163, 1-13
- **MEFFERD et al.** *RNA*, 2015, vol. 21, 1683-9
- **SCHERER et al.** *Nucleic Acids Res.*, 2007, vol. 35, 2620-2628
- **WANG et al.** *Nature*, 2015
- **FONT-BURGADA et al.** *Cell*, 2015, vol. 162, 766-799
- **RAN et al.** *Nature*, 2015, vol. 520, 186-191
- **SCHERPHOF** ; **KAMPS**. The role of hepatocytes in the clearance of liposomes from the blood circulation. *Prog Lipid Res*, May 2001, vol. 40 (3), 149-66
- **MAIER, M.A. et al.** Biodegradable Lipids Enabling Rapidly Eliminated Lipid Nanoparticles for Systemic Delivery of RNAi Therapeutics.. *Mol. Ther.*, 2013, vol. 21 (8), 1570-78
- **ROMBERG et al.** *Pharmaceutical Research*, 2008, vol. 25 (1), 55-71
- **HOEKSTRA et al.** *Biochimica et Biophysica Acta*, 2004, vol. 1660, 41-52
- **J. MILTON HARRIS**. *Poly(ethylene glycol) chemistry: biotechnical and biomedical applications*, 1992
- **LAPECORELLA, M.** ; **MARIANI, G**. Factor VII deficiency: defining the clinical picture and optimizing therapeutic options.. *Haemophilia*, 2008, vol. 14, 1170-1175
- **PATEL, K** ; **HAWKINS, P**. Cardiac amyloidosis: where are we today?. *J. Intern. Med.*, 2008, vol. 278, 126-144
- **KARIKO et al.** *Nucleic Acids Research*, 2011, vol. 39 (21), e142
- **CHO et al.** Targeted genome engineering in human cells with the Cas9 RNA-guided endonuclease. *Nature Biotechnology*, 2013, vol. 31, 230-232